(19) Europäisches Patentamt
European Patent Office
Office européen des brevets

(11) EP 3 619 196 B1

(12) EUROPÄISCHE PATENTSCHRIFT

(45) Veröffentlichungstag und Bekanntmachung des Hinweises auf die Patenterteilung:
**30.03.2022 Patentblatt 2022/13**

(21) Anmeldenummer: **18719889.0**

(22) Anmeldetag: **26.04.2018**

(51) Internationale Patentklassifikation (IPC):
***C07D 213/64*** (2006.01) ***C07D 401/12*** (2006.01)
***C07D 405/12*** (2006.01) ***C07D 409/12*** (2006.01)
***C07D 401/04*** (2006.01) ***A61K 31/44*** (2006.01)
***A61K 31/443*** (2006.01) ***A61K 31/4436*** (2006.01)
***A61K 31/4439*** (2006.01) ***A61K 31/444*** (2006.01)
***A61K 31/4545*** (2006.01) ***A61K 31/496*** (2006.01)
***A01N 43/40*** (2006.01) ***A01N 43/60*** (2006.01)
***A61P 33/00*** (2006.01) ***A61P 33/14*** (2006.01)

(52) Gemeinsame Patentklassifikation (CPC):
**C07D 213/64; A01N 43/40; A01N 43/60; A61P 33/00; A61P 33/14; C07D 401/04; C07D 401/12; C07D 405/12; C07D 409/12**

(86) Internationale Anmeldenummer:
**PCT/EP2018/060678**

(87) Internationale Veröffentlichungsnummer:
**WO 2018/202524 (08.11.2018 Gazette 2018/45)**

(54) **2-{[2-(PHENYLOXYMETHYL)PYRIDIN-5-YL]OXY}-ETHANAMIN-DERIVATE UND VERWANDTE VERBINDUNGEN ALS SCHÄDLINGSBEKÄMPFUNGSMITTEL Z.B. FÜR DEN PFLANZENSCHUTZ**

2-{[2-(PHENYLOXYMETHYL)PYRIDIN-5-YL]OXY}-ETHANAMIN-DERIVATIVES AND RELATED COMPOUNDS AS PESTICIDES E.G. FOR CROP PROTECTION

DÉRIVÉS DE 2-{[2-(PHÉNYLOXYMÉTHYL)PYRIDIN-5-YL]OXY}-ÉTHANAMINE ET COMPOSÉS SIMILAIRES EN TANT QUE PESTICIDES P.E. PHYTOSANITAIRES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.05.2017 EP 17169375**

(43) Veröffentlichungstag der Anmeldung:
**11.03.2020 Patentblatt 2020/11**

(73) Patentinhaber: **Bayer CropScience Aktiengesellschaft
40789 Monheim am Rhein (DE)**

(72) Erfinder:
- **VELTEN, Robert
40764 Langenfeld (DE)**
- **ARLT, Alexander
50859 Köln (DE)**
- **BÖHNKE, Niels
10713 Berlin (DE)**
- **ILG, Kerstin
50670 Köln (DE)**
- **HORSTMANN, Sebastian
51381 Leverkusen (DE)**
- **VERMEER, Arnoldus
40789 Monheim (DE)**
- **HORN, Karin
40545 Düsseldorf (DE)**
- **GÖRGENS, Ulrich
40882 Ratingen (DE)**
- **PORTZ, Daniela
52391 Vettweiß (DE)**
- **TURBERG, Andreas
42781 Haan (DE)**

(74) Vertreter: **BIP Patents
c/o Bayer Intellectual Property GmbH
Alfred-Nobel-Straße 10
40789 Monheim am Rhein (DE)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(56) Entgegenhaltungen:

| | |
|---|---|
| **WO-A-2008/073461** | **WO-A-2008/073936** |
| **WO-A1-03/064386** | **WO-A1-2008/073929** |
| **WO-A1-2009/145360** | **WO-A2-2005/082089** |

## Beschreibung

Die vorliegende Erfindung betrifft neue heterocyclische Verbindungen, Verfahren und Zwischenprodukte zu ihrer Herstellung und ihre Verwendung zur Bekämpfung von tierischen Schädlingen, insbesondere von Insekten.

Heterocyclen-Derivate mit fungiziden Eigenschaften sind in der Literatur bereits beschrieben worden, z.B. in WO2014/179144.

Heterocyclen-Derivate mit pharmazeutischen Eigenschaften sind in der Literatur bereits beschrieben worden, z.B. WO2005/082089, WO2008073929, WO2009/145360.

WO2008/073936 und WO2008/073461 beschreiben Carbonsäureamidverbindungen mit pharmazeutischen Eigenschaften, die die Aktivität von Ionenkanälen in einem Säugetier modulieren können.

WO2003/064386 offenbart 5-Phenyl-2-(4-piperazino-phenyl)-3,4-dihydro-2H-pyrrol-Derivate als Pestizide und Insektizide.

Moderne Schädlingsbekämpfungsmittel müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle, sowie die Frage des Aufwands, der für die Synthese eines Wirkstoffs betrieben werden muss. Ferner können Resistenzen auftreten. Schon aus all diesen Gründen kann die Suche nach neuen Schädlingsbekämpfungsmittel nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert wird.

Gelöst wird die Aufgabe, sowie weitere nicht explizit genannte Aufgaben, die aus den hierin diskutierten Zusammenhängen ableitbar oder erschließbar sind, durch neue Verbindungen der Formel (I),

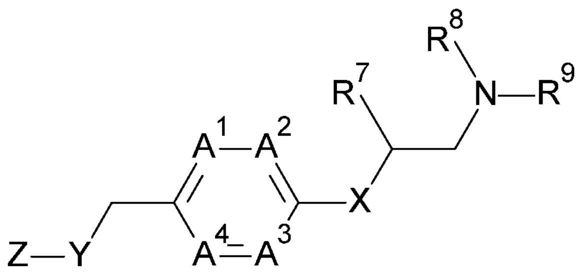

(I)

in welcher (Ausgestaltung 1)

Z für gegebenenfalls ein- oder mehrfach gleich oder verschieden mit Halogen, Cyano, $(C_1\text{-}C_4)$-Alkyl, $(C_1\text{-}C_4)$-Halogenalkyl, $(C_1\text{-}C_4)$-Alkoxy, $(C_1\text{-}C_4)$-Halogenalkoxy, $(C_1\text{-}C_4)$-Alkylsulfanyl, $(C_1\text{-}C_4)$-Halogenalkylsulfanyl, $(C_3\text{-}C_6)$-Cycloalkyl substituiertes Naphthyl, Dibenzo[b,d]furanyl, Dibenzo[b,d]thiophenyl oder für ein Phenyl der Substrukturformel (II) steht,

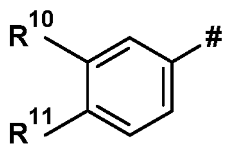

(II)

und das substituierte Phenyl der Substrukturformel (II) gegebenenfalls bis zu zwei weitere Substituenten ausgewählt aus der Gruppe von Halogen, Cyano, $(C_1\text{-}C_4)$-Alkyl, $(C_1\text{-}C_4)$-Halogenalkyl, $(C_1\text{-}C_4)$-Alkoxy, $(C_1\text{-}C_4)$-Halogenalkoxy, $(C_1\text{-}C_4)$-Alkylsulfanyl, $(C_3\text{-}C_6)$-Cycloalkyl oder $(C_3\text{-}C_6)$-Halogencycloalkyl tragen kann,

Y für O steht,

$A^1$ für N oder $-CR^1$ steht,

$A^2$ für N oder $-CR^2$ steht,

$A^3$ für N oder $-CR^3$ steht,

$A^4$ für N oder $-CR^4$ steht,

dabei mindestens eines und maximal zwei der Atome $A_1$, $A_2$, $A_3$ oder $A_4$ im aromatischen Ring für N steht,

X für Sauerstoff oder $-NR^6$ steht,

$R^1$, $R^2$, $R^3$ und $R^4$ jeweils unabhängig voneinander für Wasserstoff, Halogen, Nitro, Cyano, Aminocarbonyl, Aminosulfonyl, jeweils gegebenenfalls substituiertes ($C_1$-$C_4$)-Alkyl, ($C_2$-$C_4$)-Alkenyl, ($C_2$-$C_4$)-Alkinyl, ($C_3$-$C_6$)-Cycloalkyl, ($C_1$-$C_4$)-Alkoxy, ($C_2$-$C_4$)-Alkenyloxy, ($C_2$-$C_4$)-Alkinyloxy, ($C_3$-$C_6$)-Cycloalkoxy, N-Mono-($C_1$-$C_4$)-alkylamino, *N*-Mono-($C_3$-$C_6$)-Cycloalkylamino, *N,N*-Di-($C_1$-$C_4$)-alkylamino, *N,N*-Di-($C_3$-$C_6$)-Cycloalkylamino, *N,N*-($C_3$-$C_6$)-Cycloalkyl-($C_1$-$C_4$)-alkylamino, *N*-($C_1$-$C_4$)-Alkanoylamino, *N*-($C_3$-$C_6$)-Cycloalkanoylamino, *N*-($C_1$-$C_4$)-Alkanoyl-*N*-($C_1$-$C_4$)-Alkylamino, *N*-($C_3$-$C_6$)-Cycloalkanoyl-*N*-($C_1$-$C_4$)-Alkylamino, *N*-($C_3$-$C_6$)-Cycloalkarioyl-*N*-($C_3$-$C_6$)-Cycloalkylamino, *N*-($C_1$-$C_4$)-Alkanoyl-*N*-($C_3$-$C_6$)-Cycloalkylamino, ($C_1$-$C_4$)-Alkoxycarbonyl, ($C_3$-$C_6$)-Cycloalkoxycarbonyl, ($C_1$-$C_4$)-Alkanoyl, ($C_3$-$C_6$)-Cycloalkanoyl, ($C_1$-$C_4$)-Alkylsulfanyl, ($C_1$-$C_4$)-Alkylsulfinyl, ($C_1$-$C_4$)-Alkylsulfonyl, ($C_3$-$C_6$)-Cycloalkylsulfanyl, ($C_3$-$C_6$)-Cycloalkylsulfinyl, ($C_3$-$C_6$)-Cycloalkylsulfonyl, *N*-($C_1$-$C_4$)-Alkylaminocarbonyl, *N*-($C_3$-$C_6$)-Cycloalkylaminocarbonyl, *N,N*-Di-($C_1$-$C_4$)-Alkylaminocarbonyl, *N,N*-Di-($C_3$-$C_6$)-Cycloalkylaminocarbonyl, *N,N*-($C_3$-$C_6$)-Cycloalkyl-($C_1$-$C_4$)-Alkylaminocarbonyl, -CH=N-O-[($C_1$-$C_4$)-Alkyl], -CH=N-O-[($C_3$-$C_6$)-Cycloalkyl], -C[($C_1$-$C_4$)-Alkyl]=N-O-[($C_1$-$C_4$)-Alkyl], -C[($C_3$-$C_6$)-Cycloalkyl]=N-O-[($C_1$-$C_4$)-Alkyl], -C[($C_1$-$C_4$)-Alkyl]=N-O-[($C_3$-$C_6$)-Cycloalkyl], -C[($C_3$-$C_6$)-Cycloalkyl]=N-O-[($C_3$-$C_6$)-Cycloalkyl] ,stehen,

$R^5$ für Wasserstoff oder gegebenenfalls substituiertes ($C_1$-$C_4$)-Alkyl, ($C_3$-$C_6$)-Cycloalkyl, ($C_1$-$C_3$)-Alkanoyl steht,

$R^6$ für Wasserstoff oder gegebenenfalls substituiertes ($C_1$-$C_4$)-Alkyl, ($C_3$-$C_6$)-Cycloalkyl, ($C_1$-$C_4$)-Alkanoyl oder einen Ringschluss mit $R^8$ der aus 1 bis 3 $CH_2$-Gruppen gebildet wird, steht,

$R^7$ für Wasserstoff oder gegebenenfalls substituiertes ($C_1$-$C_4$)-Alkyl, ($C_3$-$C_6$)-Cycloalkyl oder einen Ringschluss mit $R^8$ der aus 1 bis 3 $CH_2$-Gruppen gebildet wird, steht,

$R^8$ für Wasserstoff oder für gegebenenfalls substituiertes ($C_1$-$C_4$)-Alkyl, ($C_2$-$C_4$)-Alkenyl, ($C_2$-$C_4$)-Alkinyl, ($C_3$-$C_6$)-Cycloalkyl steht oder ist zusammen mit $R^6$ oder $R^7$ zu einem Ring geschlossen der aus 1 bis 3 $CH_2$-Gruppen gebildet wird oder ist zusammen mit $R^9$ zu einem 4 bis 6 gliedrigen heterozyklischen Ring geschlossen, welcher im Falle eines 5 bis 6 gliedrigen heterozyklischen Ringes weitere Heteroatome enthalten kann und gegebenenfalls ein- oder mehrfach gleich oder verschieden mit Halogen, Cyano oder jeweils gegebenenfalls ein oder mehrfach gleich oder verschieden mit Halogen substituiertes ($C_1$-$C_4$)-Alkyl, ($C_3$-$C_6$)-Cycloalkyl, ($C_1$-$C_4$)-Alkoxy substituiert ist,

$R^9$ für jeweils gegebenenfalls substituiertes ($C_1$-$C_4$)-Alkyl, ($C_3$-$C_6$)-Cycloalkyl, ($C_2$-$C_4$)-Alkenyl, ($C_2$-$C_4$)-Alkinyl, *N*-Mono-($C_1$-$C_4$)-alkylamino, *N,N*-Di-($C_1$-$C_4$)-alkylamino, ($C_1$-$C_4$)-Alkoxy, ($C_2$-$C_4$)-Alkenyloxy, ($C_2$-$C_4$)-Alkinyloxy, ($C_3$-$C_6$)-Cycloalkoxy, ($C_1$-$C_4$)-Alkylcarbonyl, ($C_1$-$C_4$)-Alkylsulfinyl, ($C_1$-$C_4$)-Alkylsulfonyl, ($C_1$-$C_4$)-Alkoxycarbonyl steht, für den Fall das $R^7$ für Wasserstoff oder gegebenenfalls substituiertes ($C_1$-$C_4$)-Alkyl, ($C_3$-$C_6$)-Cycloalkyl steht oder für den Fall das $R^7$ mit $R^8$ einen 5- oder 6-gliedrigen Ring bildet, oder

$R^9$ für jeweils gegebenenfalls substituiertes ($C_1$-$C_4$)-Alkyl, ($C_3$-$C_6$)-Cycloalkyl, ($C_2$-$C_4$)-Alkenyl, ($C_2$-$C_4$)-Alkinyl, *N*-Mono-($C_1$-$C_4$)-alkylamino, *N,N*-Di-($C_1$-$C_4$)-alkylamino, ($C_1$-$C_4$)-Alkoxy, ($C_2$-$C_4$)-Alkenyloxy, ($C_2$-$C_4$)-Alkinyloxy, ($C_3$-$C_4$)-Cycloalkoxy, ($C_1$-$C_4$)-Alkylsulfinyl, ($C_1$-$C_4$)-Alkylsulfonyl steht, für den Fall das $R^7$ einen 4-gliedrigen Ring zusammen mit $R^8$ bildet, oder

$R^8$ und $R^9$ für einen 4 bis 6 gliedrigen heterozyklischen Ringschluss stehen, welcher im Falle eines 5 bis 6 gliedrigen heterozyklischen Ringes weitere Heteroatome enthalten kann und gegebenenfalls ein oder mehrfach gleich oder verschieden mit Halogen, Cyano oder jeweils gegebenenfalls ein oder mehrfach gleich oder verschieden mit Halogen substituiertes ($C_1$-$C_4$)-Alkyl, ($C_3$-$C_6$)-Cycloalkyl, ($C_1$-$C_4$)-Alkoxy substituiert ist,

$R^{10}$ für Halogen, Nitro, Cyano, $-SF_5$ oder für gegebenenfalls ein- oder mehrfach gleich oder verschieden mit Halogen,

Cyano, Nitro, $-SF_5$, $(C_1-C_4)$-Alkyl, $(C_2-C_4)$-Alkenyl, $(C_2-C_4)$-Alkinyl, $(C_1-C_4)$-Halogenalkyl, $(C_2-C_4)$-Halogenalkenyl, $(C_1-C_4)$-Alkoxy, $(C_1-C_4)$-Halogenalkoxy, $(C_2-C_4)$-Alkenyloxy, $(C_2-C_4)$-Halogenalkenyloxy, $(C_2-C_4)$-Alkinyloxy, $(C_3-C_6)$-Cycloalkoxy, $(C_3-C_6)$-Halogencycloalkoxy, $(C_3-C_6)$-Cycloalkyl, $(C_3-C_6)$-Halogencycloalkyl, $(C_1-C_4)$-Alkylsulfanyl, $(C_1-C_4)$-Alkylsulfinyl, $(C_1-C_4)$-Alkylsulfonyl, $(C_1-C_4)$-Halogenalkylsulfonyl, $(C_1-C_4)$-Halogenalkylsulfanyl, $(C_1-C_4)$-Halogenalkylsulfinyl, $(C_3-C_6)$-Cycloalkylsulfanyl, $(C_3-C_6)$-Cycloalkylsulfinyl, $(C_3-C_6)$-Cycloalkylsulfonyl, $C_6$-,$C_{10}$-,$C_{14}$-Aryl, $C_6$- ,$C_{10}$-,$C_{14}$-Aryloxy, Benzyl, Benzyloxy, Benzylthio, $C_6$-,$C_{10}$-,$C_{14}$-Arylthio, $C_6$-,$C_{10}$-,$C_{14}$-Arylamino, Benzylamino, Heterocyclyl, Trialkylsilyl substituiertes Phenyl, Pyrid-2-yl, Pyrid-3-yl, Pyrid-4-yl, Thiophen-2-yl, Thiophen-3-yl, $(C_1-C_4)$-Alkyl, $(C_1-C_4)$-Alkenyl, $(C_1-C_4)$-Alkinyl, $(C_3-C_6)$-Cycloalkyl, $(C_1-C_4)$-Alkoxy, $(C_2-C_4)$-Alkenyloxy, $(C_2-C_4)$-Alkinyloxy, $(C_3-C_6)$-Cycloalkoxy, $(C_1-C_4)$-Alkylsulfanyl, $(C_1-C_4)$-Alkylsulfinyl, $(C_1-C_4)$-Alkylsulfonyl, $(C_3-C_6)$-Cycloalkylsulfanyl, $(C_3-C_6)$-Cycloalkylsulfinyl, $(C_3-C_6)$-Cycloalkylsulfonyl, -CH=N-O-[$(C_1-C_4)$-Alkyl], -C[$(C_1-C_4)$-Alkyl]=N-O-[$(C_1-C_4)$-Alkyl] steht,

$R^{11}$ für Wasserstoff, Halogen, Cyano oder Nitro oder für jeweils gegebenenfalls substituiertes $(C_1-C_4)$-Alkyl, $(C_3-C_5)$-Cycloalkyl, $(C_1-C_4)$-Alkoxy, $(C_1-C_4)$-Alkylsulfanyl, $(C_1-C_4)$-Alkylsulfinyl, $(C_1-C_4)$-Alkylsulfonyl, -CH=N-O-[$(C_1-C_4)$-Alkyl], -C[$(C_1-C_4)$-Alkyl]=N-O-[$(C_1-C_4)$-Alkyl] steht,

sowie

Salze, Metallkomplexe, N-Oxide und tautomere Formen der Verbindungen der Formel (I).

unter der Maßgabe, dass die Verbindung 1-[4-(6-{2-[3-(Chlormethyl)phenyl]ethyl}pyridin-3-yl)piperazin-1-yl]ethanon ausgenommen ist.

Die ausgenomme Verbindung wurde in der WO2009145360 als Zwischenprodukt zur Herstellung einer pharmazeutisch aktiven Verbindung konkret offenbart.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im Folgenden erläutert:
<u>Ausgestaltung 2:</u>

$R^1$, $R^2$, $R^3$ und $R^4$ stehen bevorzugt jeweils unabhängig voneinander für einen Substituenten ausgewählt aus der Gruppe von Wasserstoff, Halogen, Nitro, Cyano, oder ein jeweils gegebenenfalls ein- oder mehrfach gleich oder verschieden mit Halogen, Cyano, Nitro, $(C_1-C_4)$-Alkyl, $(C_1-C_4)$-Halogenalkyl, $(C_1-C_4)$-Alkoxy, $(C_1-C_4)$-Halogenalkoxy, $(C_3-C_6)$-Cycloalkyl, $(C_3-C_6)$-Halogencycloalkyl, substituiertes $(C_1-C_4)$-Alkyl, $(C_2-C_4)$-Alkenyl, $(C_2-C_4)$-Alkinyl, $(C_3-C_6)$-Cycloalkyl, $(C_1-C_4)$-Alkoxy, $(C_2-C_4)$-Alkenyloxy, $(C_2-C_4)$-Alkinyloxy, $(C_3-C_6)$-Cycloalkoxy, $(C_1-C_4)$-Alkoxycarbonyl, $(C_3-C_6)$-Cycloalkoxycarbonyl, *N*-Mono-$(C_1-C_4)$-alkylamino, *N,N*-Di-$(C_1-C_4)$-alkylamino, $(C_1-C_4)$-Alkylsulfanyl, $(C_1-C_4)$-Alkylsulfinyl, $(C_1-C_4)$-Alkylsulfonyl, $(C_3-C_6)$-Cycloalkylsulfanyl,$(C_3-C_6)$-Cycloalkylsulfinyl,$(C_3-C_6)$-Cycloalkylsulfonyl,

$R^5$ steht bevorzugt für Wasserstoff oder gegebenenfalls ein oder mehrfach gleich oder verschieden mit Halogen substituiertes $(C_1-C_4)$-Alkyl, $(C_3-C_4)$-Cycloalkyl, $(C_1-C_3)$-Alkanoyl,

$R^6$ steht bevorzugt für Wasserstoff oder gegebenenfalls ein oder mehrfach gleich oder verschieden mit Halogen substituiertes $(C_1-C_4)$-Alkyl, $(C_3-C_4)$-Cycloalkyl, $(C_1-C_4)$-Alkanoyl oder einen Ringschluss mit $R^8$ der aus 1 bis 3 $CH_2$-Gruppen gebildet wird,

$R^7$ steht bevorzugt für Wasserstoff oder gegebenenfalls ein oder mehrfach gleich oder verschieden mit Halogen substituiertes $(C_1-C_4)$-Alkyl, $(C_3-C_4)$-Cycloalkyl oder einen Ringschluss mit $R^8$ der aus 1 bis 3 $CH_2$-Gruppen gebildet wird,

$R^8$ steht bevorzugt für Wasserstoff oder für gegebenenfalls ein oder mehrfach gleich oder verschieden mit Halogen, Nitro oder Cyano substituiertes $(C_1-C_4)$-Alkyl, $(C_2-C_4)$-Alkenyl, $(C_2-C_4)$-Alkinyl, $(C_3-C_4)$-Cycloalkyl oder einen Ringschluss zusammen mit $R^6$ oder $R^7$ der aus 1 bis 3 $CH_2$-Gruppen gebildet wird oder einen 4 bis 6 gliedrigen heterozyklischen Ringschluss zusammen mit $R^9$, welcher im Falle eines 5 bis 6 gliedrigen heterozyklischen Ringes weitere Heteroatome ausgewählt aus der Gruppe von N, O enthalten kann und gegebenenfalls ein- oder mehrfach gleich oder verschieden mit Halogen, (Ci-$C_4$)-Alkyl oder $(C_1-C_4)$-Halogenalkyl substituiert ist,

$R^9$ steht bevorzugt für jeweils gegebenenfalls ein- oder mehrfach gleich oder verschieden mit Halogen, Cyano, Nitro oder $(C_3-C_6)$-Cycloalkyl substituiertes $(C_1-C_4)$-Alkyl, $(C_2-C_4)$-Alkenyl, $(C_3-C_4)$-Cycloalkyl, $(C_1-C_4)$-Alkoxy,

$(C_2-C_4)$-Alkenyloxy, $(C_3-C_6)$-Cycloalkoxy, $(C_1-C_4)$-Alkylcarbonyl, $(C_1-C_4)$-Alkylsulfinyl, $(C_1-C_4)$-Alkylsulfonyl, $(C_1-C_4)$-Alkoxycarbonyl, für den Fall das $R^7$ für Wasserstoff steht oder gegebenenfalls ein- oder mehrfach gleich oder verschieden mit Halogen substituiertes $(C_1-C_4)$-Alkyl, $(C_3-C_4)$-Cycloalkyl oder für den Fall das $R^7$ mit $R^8$ einen 5- oder 6-gliedrigen Ring bildet, oder $R^9$ steht bevorzugt für jeweils gegebenenfalls ein- oder mehrfach gleich oder verschieden mit Halogen, Cyano, Nitro oder $(C_3-C_6)$-Cycloalkyl substituiertes $(C_1-C_4)$-Alkyl, $(C_2-C_4)$-Alkenyl, $(C_3-C_4)$-Cycloalkyl, $(C_1-C_4)$-Alkoxy, $(C_2-C_4)$-Alkenyloxy, $(C_3-C_4)$-Cycloalkoxy, $(C_1-C_4)$-Alkylsulfinyl, $(C_1-C_4)$-Alkylsulfonyl, für den Fall das $R^7$ einen 4-gliedrigen Ring zusammen mit $R^8$ bildet, oder $R^8$ und $R^9$ stehen bevorzugt für einen 4 bis 6 gliedrigen heterozyklischen Ringschluss, welcher im Falle eines 5 bis 6 gliedrigen heterozyklischen Ringes weitere Heteroatome ausgewählt aus der Gruppe von N, O enthalten kann und gegebenenfalls ein oder mehrfach gleich oder verschieden mit Halogen, (Ci-$C_4$)-Alkyl oder $(C_1-C_4)$-Halogenalkyl substituiert ist,

$R^{10}$ steht bevorzugt für Halogen oder für gegebenenfalls ein- oder mehrfach gleich oder verschieden mit Halogen, Cyano, Nitro, $(C_1-C_4)$-Alkyl, $(C_1-C_4)$-Halogenalkyl, $(C_1-C_4)$-Alkoxy, $(C_1-C_4)$-Halogenalkoxy, $(C_3-C_6)$-Cycloalkyl, $(C_3-C_6)$-Halogencycloalkyl, substituiertes Phenyl, Pyrid-2-yl, Pyrid-3-yl, Pyrid-4-yl, Thiophen-2-yl, Thiophen-3-yl, $(C_1-C_4)$-Alkyl, $(C_1-C_4)$-Alkenyl, $(C_1-C_4)$-Alkinyl, $(C_3-C_6)$-Cycloalkyl, $(C_1-C_4)$-Alkoxy, $(C_2-C_4)$-Alkenyloxy, $(C_2-C_4)$-Alkinyloxy, $(C_3-C_6)$-Cycloalkoxy, $(C_1-C_4)$-Alkylsulfanyl, $(C_1-C_4)$-Alkylsulfinyl, $(C_1-C_4)$-Alkylsulfonyl, $(C_3-C_6)$-Cycloalkylsulfanyl, $(C_3-C_6)$-Cycloalkylsulfinyl, $(C_3-C_6)$-Cycloalkylsulfonyl,-CH=N-O-[$(C_1-C_4)$-Alkyl],-C[$(C_1-C_4)$-Alkyl]=N-O-[$(C_1-C_4)$-Alkyl,

$R^{11}$ steht bevorzugt für Wasserstoff, Halogen, Cyano oder Nitro oder für gegebenenfalls ein- oder mehrfach gleich oder verschieden mit Halogen, Cyano, Nitro, $(C_1-C_4)$-Alkoxy oder $(C_3-C_6)$-Cycloalkyl substituiertes $(C_1-C_4)$-Alkyl, $(C_1-C_4)$-Alkoxy, $(C_1-C_4)$-Alkylsulfanyl, $(C_1-C_4)$-Alkylsulfinyl, $(C_1-C_4)$-Alkylsulfonyl, $(C_3-C_5)$-Cycloalkyl,-CH=N-O-[$(C_1-C_4)$-Alkyl],-C[$(C_1-C_4)$-Alkyl]=N-O-[$(C_1-C_4)$-Alkyl].

In der Ausgestaltung 2 nicht erwähnte Substituenten sind so definiert wie in Ausgestaltung 1.
Ausgestaltung 3:

zumindest eines der Atome $A_3$ oder $A_4$ im aromatischen Ring steht besonders bevorzugt für Stickstoff,

$R^1$, $R^2$, $R^3$ und $R^4$ stehen besonders bevorzugt jeweils unabhängig voneinander für einen Substituenten ausgewählt aus der Gruppe von Wasserstoff, Halogen, $(C_1-C_4)$-Alkyl, $(C_1-C_4)$-Halogenalkyl, $(C_2-C_4)$-Alkenyl, $(C_2-C_4)$-Halogenalkenyl, $(C_1-C_4)$-Alkoxy, $(C_1-C_4)$-Halogenalkoxy, $(C_3-C_6)$-Cycloalkyl, $(C_3-C_6)$-Halogencycloalkyl, $(C_1-C_4)$-Alkylsulfanyl, $(C_1-C_4)$-Alkylsulfinyl oder $(C_1-C_4)$-Alkylsulfonyl,

$R^6$ steht besonders bevorzugt für Wasserstoff, (Ci-$C_4$)-Alkyl oder einen Ringschluss mit $R^8$ der aus 1 bis 3 $CH_2$-Gruppen gebildet wird,

$R^7$ steht besonders bevorzugt für Wasserstoff, $(C_1-C_4)$-Alkyl, $(C_3-C_4)$-Cycloalkyl oder einen Ringschluss mit $R^8$ der aus 1 bis 3 $CH_2$-Gruppen gebildet wird,

$R^8$ steht besonders bevorzugt für Wasserstoff oder für gegebenenfalls ein- oder mehrfach gleich oder verschieden mit Halogen substituiertes $(C_1-C_4)$-Alkyl, $(C_2-C_4)$-Alkenyl, $(C_2-C_4)$-Alkinyl, $(C_3-C_4)$-Cycloalkyl oder einen Ringschluss zusammen mit $R^6$ oder $R^7$ der aus 1 bis 3 $CH_2$-Gruppen gebildet wird oder einen 4 bis 6 gliedrigen heterozyklischen Ringschluss zusammen mit $R^9$, der aus 3 bis 5 $CH_2$-Gruppen gebildet ist und der gegebenenfalls ein oder mehrfach gleich oder verschieden mit Halogen, (Ci-$C_4$)-Alkyl oder $(C_1-C_4)$-Halogenalkyl substituiert ist,

$R^9$ steht besonders bevorzugt für jeweils gegebenenfalls ein- oder mehrfach gleich oder verschieden mit Halogen, Cyano, Nitro oder $(C_3-C_6)$-Cycloalkyl substituiertes $(C_1-C_4)$-Alkyl, $(C_3-C_6)$-Cycloalkyl, $(C_1-C_6)$-Alkoxy, $(C_1-C_6)$-Alkylcarbonyl, für den Fall das $R^7$ für Wasserstoff steht oder für $(C_1-C_4)$-Alkyl, $(C_3-C_4)$-Cycloalkyl oder für den Fall das $R^7$ mit $R^8$ einen 5- oder 6-gliedrigen Ring bildet, oder

$R^9$ steht besonders bevorzugt für jeweils gegebenenfalls ein- oder mehrfach gleich oder verschieden mit Halogen, Cyano, Nitro oder $(C_3-C_6)$-Cycloalkyl substituiertes $(C_1-C_4)$-Alkyl, $(C_3-C_4)$-Cycloalkyl, $(C_1-C_4)$-Alkoxy, für den Fall das $R^7$ einen 4-gliedrigen Ring zusammen mit $R^8$ bildet, oder

$R^8$ und $R^9$ stehen besonders bevorzugt für einen 4 bis 6 gliedrigen heterozyklischen Ringschluss, der aus 3 bis 5 $CH_2$-Gruppen gebildet ist und der gegebenenfalls ein- oder mehrfach gleich oder verschieden mit Halogen, (Ci-$C_4$)-Alkyl oder $(C_1-C_4)$-Halogenalkyl substituiert ist.

$R^{10}$ steht besonders bevorzugt für Halogen oder für gegebenenfalls ein- oder mehrfach gleich oder verschieden mit Halogen, Cyano, Nitro, $(C_1-C_4)$-Alkyl, $(C_1-C_4)$-Halogenalkyl, $(C_1-C_4)$-Alkoxy, $(C_1-C_4)$-Halogenalkoxy, $(C_3-C_6)$-Cycloalkyl, $(C_3-C_6)$-Halogencycloalkyl substituiertes Phenyl, Pyrid-2-yl, Pyrid-3-yl, Pyrid-4-yl, Thiophen-2-yl, Thiophen-3-yl oder für gegebenenfalls ein- oder mehrfach gleich oder verschieden mit Halogen, Cyano, Nitro, substituiertes $(C_1-C_4)$-Alkyl, $(C_1-C_4)$-Alkenyl, $(C_1-C_4)$-Alkinyl, $(C_3-C_6)$-Cycloalkyl, $(C_1-C_4)$-Alkoxy, $(C_2-C_4)$-Alkenyloxy, $(C_2-C_4)$-Alkinyloxy, $(C_3-C_6)$-Cycloalkoxy, $(C_1-C_4)$-Alkylsulfanyl, $(C_1-C_4)$-Alkylsulfinyl, $(C_1-C_4)$-Alkylsulfonyl, $(C_3-C_6)$-Cycloalkylsulfanyl, $(C_3-C_6)$-Cycloalkylsulfinyl, $(C_3-C_6)$-Cycloalkylsulfonyl, -CH=N-O-[$(C_1-C_4)$-Alkyl], -C[$(C_1-C_4)$-Alkyl]=N-O-[$(C_1-C_4)$-Alkyl,

$R^{11}$ steht besonders bevorzugt für Wasserstoff, Halogen, Cyano oder Nitro oder für gegebenenfalls ein- oder mehrfach gleich oder verschieden mit Halogen, Cyano oder $(C_3-C_6)$-Cycloalkyl substituiertes $(C_1-C_4)$-Alkyl, $(C_1-C_4)$-Alkoxy, $(C_1-C_4)$-Alkylsulfanyl, $(C_1-C_4)$-Alkylsulfinyl, $(C_1-C_4)$-Alkylsulfonyl, $(C_3-C_5)$-Cycloalkyl.

In der Ausgestaltung 3 nicht erwähnte Substituenten sind so definiert wie in Ausgestaltung 1 oder Ausgestaltung 2.

Ausgestaltung 4:

Z steht insbesondere bevorzugt für gegebenenfalls ein- oder mehrfach gleich oder verschieden mit Halogen, Cyano, $(C_1-C_4)$-Alkyl, $(C_1-C_4)$-Halogenalkyl substituiertes Naphthyl, oder für unsubstituiertes Dibenzo[b,d]furanyl oder Dibenzo[b,d]thiophenyl oder für substituiertes Phenyl der Substrukturformel (II),

$R^1$, $R^2$, $R^3$ und $R^4$ stehen insbesondere bevorzugt für einen Substituenten ausgewählt aus der Gruppe von Wasserstoff, Halogen, $(C_1-C_4)$-Alkyl, $(C_1-C_4)$-Halogenalkyl, $(C_1-C_4)$-Alkoxy oder $(C_1-C_4)$-Alkylsulfanyl,

$R^6$ steht insbesondere bevorzugt für Wasserstoff, $(C_1-C_3)$-Alkyl oder einen Ringschluss mit $R^8$ der aus 1 bis 2 $CH_2$-Gruppen gebildet wird,

$R^7$ steht insbesondere bevorzugt für Wasserstoff oder einen Ringschluss mit $R^8$ der aus 1 bis 3 $CH_2$-Gruppen gebildet wird,

$R^8$ steht insbesondere bevorzugt für Wasserstoff oder für gegebenenfalls ein oder mehrfach gleich oder verschieden mit Halogen substituiertes (Ci-$C_4$)-Alkyl oder einen Ringschluss zusammen mit $R^6$ der aus 1 bis 2 $CH_2$-Gruppen gebildet wird oder einen Ringschluss zusammen mit $R^7$ der aus 1 bis 3 $CH_2$-Gruppen gebildet wird oder einen 4 bis 6 gliedrigen heterozyklischen Ringschluss zusammen mit $R^9$ der aus 3 bis 5 $CH_2$-Gruppen gebildet ist und der gegebenenfalls ein- oder mehrfach gleich oder verschieden mit Halogen, $(C_1-C_4)$-Alkyl oder $(C_1-C_4)$-Halogenalkyl substituiert ist,

$R^9$ steht insbesondere bevorzugt für jeweils gegebenenfalls ein- oder mehrfach gleich oder verschieden mit Halogen, Cyano oder $(C_3-C_6)$-Cycloalkyl substituiertes $(C_1-C_6)$-Alkyl, $(C_3-C_6)$-Cycloalkyl oder $(C_1-C_6)$-Alkoxy, oder

$R^8$ und $R^9$ stehen insbesondere bevorzugt für einen 4 bis 6 gliedrigen heterozyklischen Ringschluss der aus 3 bis 5 $CH_2$-Gruppen gebildet ist und der gegebenenfalls ein- oder mehrfach gleich oder verschieden mit Halogen, (Ci-$C_4$)-Alkyl oder $(C_1-C_4)$-Halogenalkyl substituiert ist,

$R^{10}$ steht insbesondere bevorzugt für Halogen, $(C_1-C_4)$-Alkyl, $(C_1-C_4)$-Alkoxy, $(C_1-C_4)$-Halogenalkoxy oder für gegebenenfalls ein- oder mehrfach gleich oder verschieden mit Halogen, Cyano, $(C_1-C_4)$-Alkyl, $(C_1-C_4)$-Halogenalkyl, $(C_1-C_4)$-Alkoxy oder $(C_1-C_4)$-Halogenalkoxy substituiertes Phenyl,

$R^{11}$ steht insbesondere bevorzugt für Wasserstoff, Halogen, Nitro oder Cyano oder für gegebenenfalls ein- oder mehrfach gleich oder verschieden mit Halogen substituiertes $(C_1-C_4)$-Alkyl, $(C_3-C_5)$-Cycloalkyl oder $(C_1-C_4)$-Alkoxy steht.

In der Ausgestaltung 5 nicht erwähnte Substituenten sind so definiert wie in Ausgestaltung 1, Ausgestaltung 2 oder Ausgestaltung 3.

Ausgestaltung 5:

Z steht hervorgehoben für ein Naphthyl der Substrukturformel (III):

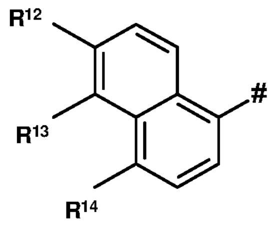

(III)

oder für unsubstituiertes Dibenzo[b,d]furanyl oder Dibenzo[b,d]thiophenyl oder für substituiertes Phenyl der Substrukturformel (II).

$R^1$ steht hervorgehoben für Wasserstoff, Halogen, ($C_1$-$C_4$)-Alkyl oder ($C_1$-$C_4$)-Alkoxy,

$R^2$ steht hervorgehoben für Wasserstoff, Halogen oder ($C_1$-$C_4$)-Halogenalkyl,

$R^3$ steht hervorgehoben für Wasserstoff, Halogen, ($C_1$-$C_4$)-Alkoxy oder ($C_1$-$C_4$)-Alkylsulfanyl,

$R^4$ steht hervorgehoben für Wasserstoff oder Halogen,

$R^6$ steht hervorgehoben für Wasserstoff, oder für einen Piperazin Ringschluss mit $R^8$,

$R^7$ steht hervorgehoben für Wasserstoff oder für einen Pyrrolidin Ringschluss mit $R^8$,

$R^8$ steht hervorgehoben für Wasserstoff, (Ci-$C_4$)-Alkyl oder für einen Piperazin Ringschluss zusammen mit $R^6$ oder für einen Pyrrolidin Ringschluss zusammen mit $R^7$ oder für einen Piperidin Ringschluss zusammen mit $R^9$,

$R^9$ steht hervorgehoben für jeweils gegebenenfalls ein- oder mehrfach gleich oder verschieden mit Halogen oder Cyano substituiertes (Ci-$C_4$)-Alkyl oder ($C_1$-$C_4$)-Alkoxy, oder

$R^8$ und $R^9$ stehen hervorgehoben für einen Piperidin Ringschluss,

$R^{10}$ steht hervorgehoben für Halogen, ($C_1$-$C_4$)-Alkyl, ($C_1$-$C_4$)-Alkoxy, ($C_1$-$C_4$)-Halogenalkoxy oder für ein Phenyl der Substrukturformel (IV):

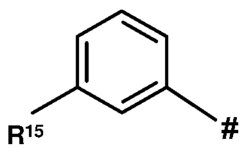

(IV)

$R^{11}$ steht hervorgehoben für Wasserstoff oder Halogen oder für gegebenenfalls ein- oder mehrfach gleich oder verschieden mit Halogen substituiertes (Ci-$C_4$)-Alkyl oder ($C_1$-$C_4$)-Alkoxy,

$R^{12}$, $R^{13}$ und $R^{14}$ stehen hervorgehoben jeweils unabhängig voneinander für Wasserstoff oder Halogen,

$R^{15}$ steht hervorgehoben für Wasserstoff, Halogen, Cyano, ($C_1$-$C_4$)-Alkyl, ($C_1$-$C_4$)-Halogenalkyl, ($C_1$-$C_4$)-Alkoxy oder ($C_1$ $C_4$)-Halogenalkoxy.

In der Ausgestaltung 5 nicht erwähnte Substituenten sind so definiert wie in Ausgestaltung 1, Ausgestaltung 2, Ausgestaltung 3 oder Ausgestaltung 4.

In einer bevorzugten Ausführungsform betrifft die Erfindung die Verbindungen der Formel (I)

wobei Z die in Ausgestaltung (1) beschriebenen Bedeutungen hat und

wobei Y, $A^1$, $A^2$, $A^3$, $A^4$, X, $R^1$, $R^2$, $R^3$, $R^4$ $R^5$. $R^6$, $R^7$, $R^8$, $R^9$, $R^{10}$ und $R^{11}$ die oben beschriebenen Bedeutungen, insbesondere die in Ausgestaltung (2) oder Ausgestaltung (3) oder Ausgestaltung (4) oder Ausgestaltung (5) beschriebenen Bedeutungen haben.

In einer weiteren bevorzugten Ausführungsform betrifft die Erfindung die Verbindungen der Formel (I)

wobei Z die in Ausgestaltung (2) beschriebenen Bedeutungen hat und

wobei Y, $A^1$, $A^2$, $A^3$, $A^4$, X, $R^1$, $R^2$, $R^3$, $R^4$ $R^5$. $R^6$, $R^7$, $R^8$, $R^9$, $R^{10}$ und $R^{11}$ die oben beschriebenen Bedeutungen, insbesondere die in Ausgestaltung (1) oder Ausgestaltung (3) oder Ausgestaltung (4) oder Ausgestaltung (5) beschriebenen Bedeutungen haben.

In einer weiteren bevorzugten Ausführungsform betrifft die Erfindung die Verbindungen der Formel (I)

wobei Z die in Ausgestaltung (3) beschriebenen Bedeutungen hat und

wobei Y, $A^1$, $A^2$, $A^3$, $A^4$, X, $R^1$, $R^2$, $R^3$, $R^4$ $R^5$. $R^6$, $R^7$, $R^8$, $R^9$, $R^{10}$ und $R^{11}$ die oben beschriebenen Bedeutungen, insbesondere die in Ausgestaltung (1) oder Ausgestaltung (2) oder Ausgestaltung (4) oder Ausgestaltung (5) beschriebenen Bedeutungen haben.

In einer weiteren bevorzugten Ausführungsform betrifft die Erfindung die Verbindungen der Formel (I)

wobei Z die in Ausgestaltung (4) beschriebenen Bedeutungen hat und

wobei Y, $A^1$, $A^2$, $A^3$, $A^4$, X, $R^1$, $R^2$, $R^3$, $R^4$ $R^5$. $R^6$, $R^7$, $R^8$, $R^9$, $R^{10}$ und $R^{11}$ die oben beschriebenen Bedeutungen, insbesondere die in Ausgestaltung (1) oder Ausgestaltung (2) oder Ausgestaltung (3) oder Ausgestaltung (5) beschriebenen Bedeutungen haben.

In einer weiteren bevorzugten Ausführungsform betrifft die Erfindung die Verbindungen der Formel (I)

wobei Z die in Ausgestaltung (5) beschriebenen Bedeutungen hat und

wobei Y, $A^1$, $A^2$, $A^3$, $A^4$, X, $R^1$, $R^2$, $R^3$, $R^4$ $R^5$. $R^6$, $R^7$, $R^8$, $R^9$, $R^{10}$ und $R^{11}$ die oben beschriebenen Bedeutungen, insbesondere die in Ausgestaltung (1) oder Ausgestaltung (2) oder Ausgestaltung (3) oder Ausgestaltung (4) beschriebenen Bedeutungen haben.

In einer bevorzugten Ausführungsform betrifft die Erfindung die Verbindungen der Formel (I)

wobei Y die in Ausgestaltung (1) beschriebenen Bedeutungen hat und

wobei Z, $A^1$, $A^2$, $A^3$, $A^4$, X, $R^1$, $R^2$, $R^3$, $R^4$ $R^5$. $R^6$, $R^7$, $R^8$, $R^9$, $R^{10}$ und $R^{11}$ die oben beschriebenen Bedeutungen, insbesondere die in Ausgestaltung (2) oder Ausgestaltung (3) oder Ausgestaltung (4) oder Ausgestaltung (5) beschriebenen Bedeutungen haben.

In einer weiteren bevorzugten Ausführungsform betrifft die Erfindung die Verbindungen der Formel (I)

wobei Y die in Ausgestaltung (2) beschriebenen Bedeutungen hat und

wobei Z, $A^1$, $A^2$, $A^3$, $A^4$, X, $R^1$, $R^2$, $R^3$, $R^4$ $R^5$. $R^6$, $R^7$, $R^8$, $R^9$, $R^{10}$ und $R^{11}$ die oben beschriebenen Bedeutungen, insbesondere die in Ausgestaltung (1) oder Ausgestaltung (3) oder Ausgestaltung (4) oder Ausgestaltung (5) beschriebenen Bedeutungen haben.

In einer bevorzugten Ausführungsform betrifft die Erfindung die Verbindungen der Formel (I)

wobei $A^3$ und $A^4$ die in Ausgestaltung (1) beschriebenen Bedeutungen haben und

wobei Z, Y, $A^1$, $A^2$, X, $R^1$, $R^2$, $R^3$, $R^4$ $R^5$, $R^6$, $R^7$, $R^8$, $R^9$, $R^{10}$ und $R^{11}$ die oben beschriebenen Bedeutungen, insbesondere die in Ausgestaltung (2) oder Ausgestaltung (3) oder Ausgestaltung (4) oder Ausgestaltung (5) beschriebenen Bedeutungen haben.

In einer weiteren bevorzugten Ausführungsform betrifft die Erfindung die Verbindungen der Formel (I)

wobei $A^3$ und $A^4$ die in Ausgestaltung (3) beschriebenen Bedeutungen haben und

wobei Z, Y, $A^1$, $A^2$, X, $R^1$, $R^2$, $R^3$, $R^4$ $R^5$, $R^6$, $R^7$, $R^8$, $R^9$, $R^{10}$ und $R^{11}$ die oben beschriebenen Bedeutungen, insbesondere die in Ausgestaltung (1) oder Ausgestaltung (2) oder Ausgestaltung (4) oder Ausgestaltung (5) beschriebenen Bedeutungen haben.

In einer bevorzugten Ausführungsform betrifft die Erfindung die Verbindungen der Formel (I)

wobei $R^1$, $R^2$, $R^3$und $R^4$ die in Ausgestaltung (1) beschriebenen Bedeutungen haben und

wobei Z, Y, $A^1$, $A^2$, $A^3$, $A^4$, X, $R^5$, $R^6$, $R^7$, $R^8$, $R^9$, $R^{10}$ und $R^{11}$ die oben beschriebenen Bedeutungen, insbesondere die in Ausgestaltung (2) oder Ausgestaltung (3) oder Ausgestaltung (4) oder Ausgestaltung (5) beschriebenen Bedeutungen haben.

In einer weiteren bevorzugten Ausführungsform betrifft die Erfindung die Verbindungen der Formel (I)

wobei $R^1$, $R^2$, $R^3$ und $R^4$ die in Ausgestaltung (2) beschriebenen Bedeutungen haben und

wobei Z, Y, $A^1$, $A^2$, $A^3$, $A^4$, X, $R^5$, $R^6$, $R^7$, $R^8$, $R^9$, $R^{10}$ und $R^{11}$ die oben beschriebenen Bedeutungen, insbesondere die in Ausgestaltung (1) oder Ausgestaltung (3) oder Ausgestaltung (4) oder Ausgestaltung (5) beschriebenen Bedeutungen haben.

In einer weiteren bevorzugten Ausführungsform betrifft die Erfindung die Verbindungen der Formel (I)

wobei $R^1$, $R^2$, $R^3$und $R^4$ die in Ausgestaltung (3) beschriebenen Bedeutungen haben und

wobei Z, Y, $A^1$, $A^2$, $A^3$, $A^4$, X, $R^5$, $R^6$, $R^7$, $R^8$, $R^9$, $R^{10}$ und $R^{11}$ die oben beschriebenen Bedeutungen, insbesondere die in Ausgestaltung (1) oder Ausgestaltung (2) oder Ausgestaltung (4) oder Ausgestaltung (5) beschriebenen Bedeutungen haben.

In einer weiteren bevorzugten Ausführungsform betrifft die Erfindung die Verbindungen der Formel (I)

wobei $R^1$, $R^2$, $R^3$und $R^4$ die in Ausgestaltung (4) beschriebenen Bedeutungen haben und

wobei Z, Y, $A^1$, $A^2$, $A^3$, $A^4$, X, $R^5$, $R^6$, $R^7$, $R^8$, $R^9$, $R^{10}$ und $R^{11}$ die oben beschriebenen Bedeutungen, insbesondere die in Ausgestaltung (1) oder Ausgestaltung (2) oder Ausgestaltung (3) oder Ausgestaltung (5) beschriebenen Bedeutungen haben.

In einer weiteren bevorzugten Ausführungsform betrifft die Erfindung die Verbindungen der Formel (I)

wobei $R^1$, $R^2$, $R^3$und $R^4$ die in Ausgestaltung (5) beschriebenen Bedeutungen haben und

wobei Z, Y, $A^1$, $A^2$, $A^3$, $A^4$, X, $R^5$, $R^6$, $R^7$, $R^8$, $R^9$, $R^{10}$ und $R^{11}$ die oben beschriebenen Bedeutungen, insbesondere die in Ausgestaltung (1) oder Ausgestaltung (2) oder Ausgestaltung (3) oder Ausgestaltung (4) beschriebenen Bedeutungen haben.

In einer bevorzugten Ausführungsform betrifft die Erfindung die Verbindungen der Formel (I)

wobei X die in Ausgestaltung (1) beschriebenen Bedeutungen hat und

wobei Z, Y, $A^1$, $A^2$, $A^3$, $A^4$, $R^1$, $R^2$, $R^3$, $R^4$ $R^5$, $R^6$, $R^7$, $R^8$, $R^9$, $R^{10}$ und $R^{11}$ die oben beschriebenen Bedeutungen, insbesondere die in Ausgestaltung (2) oder Ausgestaltung (3) oder Ausgestaltung (4) oder Ausgestaltung (5) beschriebenen Bedeutungen haben.

In einer weiteren bevorzugten Ausführungsform betrifft die Erfindung die Verbindungen der Formel (I)

wobei X die in Ausgestaltung (3) beschriebenen Bedeutungen hat und

wobei Z, Y, $A^1$, $A^2$, $A^3$, $A^4$, $R^1$, $R^2$, $R^3$, $R^4$ $R^5$, $R^6$, $R^7$, $R^8$, $R^9$, $R^{10}$ und $R^{11}$ die oben beschriebenen Bedeutungen, insbesondere die in Ausgestaltung (1) oder Ausgestaltung (2) oder Ausgestaltung (4) oder Ausgestaltung (5) beschriebenen Bedeutungen haben.

In einer bevorzugten Ausführungsform betrifft die Erfindung die Verbindungen der Formel (I)

wobei $R^6$ die in Ausgestaltung (1) beschriebenen Bedeutungen hat und

wobei Z, Y, $A^1$, $A^2$, $A^3$, $A^4$, X, $R^1$, $R^2$, $R^3$, $R^4$ $R^5$, $R^7$, $R^8$, $R^9$, $R^{10}$ und $R^{11}$ die oben beschriebenen Bedeutungen, insbesondere die in Ausgestaltung (2) oder Ausgestaltung (3) oder Ausgestaltung (4) oder Ausgestaltung (5) beschriebenen Bedeutungen haben.

In einer weiteren bevorzugten Ausführungsform betrifft die Erfindung die Verbindungen der Formel (I)

wobei $R^6$ die in Ausgestaltung (2) beschriebenen Bedeutungen hat und

wobei Z, Y, $A^1$, $A^2$, $A^3$, $A^4$, X, $R^1$, $R^2$, $R^3$, $R^4$ $R^5$, $R^7$, $R^8$, $R^9$, $R^{10}$ und $R^{11}$ die oben beschriebenen Bedeutungen, insbesondere die in Ausgestaltung (1) oder Ausgestaltung (3) oder Ausgestaltung (4) oder Ausgestaltung (5) beschriebenen Bedeutungen haben.

In einer weiteren bevorzugten Ausführungsform betrifft die Erfindung die Verbindungen der Formel (I)

wobei $R^6$ die in Ausgestaltung (3) beschriebenen Bedeutungen hat und

wobei Z, Y, $A^1$, $A^2$, $A^3$, $A^4$, X, $R^1$, $R^2$, $R^3$, $R^4$ $R^5$, $R^7$, $R^8$, $R^9$, $R^{10}$ und $R^{11}$ die oben beschriebenen Bedeutungen, insbesondere die in Ausgestaltung (1) oder Ausgestaltung (2) oder Ausgestaltung (4) oder Ausgestaltung (5) beschriebenen Bedeutungen haben.

In einer weiteren bevorzugten Ausführungsform betrifft die Erfindung die Verbindungen der Formel (I)

wobei $R^6$ die in Ausgestaltung (5) beschriebenen Bedeutungen hat und

wobei Z, Y, $A^1$, $A^2$, $A^3$, $A^4$, X, $R^1$, $R^2$, $R^3$, $R^4$ $R^5$, $R^7$, $R^8$, $R^9$, $R^{10}$ und $R^{11}$ die oben beschriebenen Bedeutungen, insbesondere die in Ausgestaltung (1) oder Ausgestaltung (2) oder Ausgestaltung (3) oder Ausgestaltung (4) beschriebenen Bedeutungen haben.

In einer bevorzugten Ausführungsform betrifft die Erfindung die Verbindungen der Formel (I)

wobei $R^7$ die in Ausgestaltung (1) beschriebenen Bedeutungen hat und

wobei Z, Y, $A^1$, $A^2$, $A^3$, $A^4$, X, $R^1$, $R^2$, $R^3$, $R^4$ $R^5$, $R^6$, $R^8$, $R^9$, $R^{10}$ und $R^{11}$ die oben beschriebenen Bedeutungen, insbesondere die in Ausgestaltung (2) oder Ausgestaltung (3) oder Ausgestaltung (4) oder Ausgestaltung (5) beschriebenen Bedeutungen haben.

In einer weiteren bevorzugten Ausführungsform betrifft die Erfindung die Verbindungen der Formel (I)

wobei $R^7$ die in Ausgestaltung (2) beschriebenen Bedeutungen hat und

wobei Z, Y, $A^1$, $A^2$, $A^3$, $A^4$, X, $R^1$, $R^2$, $R^3$, $R^4$ $R^5$, $R^6$, $R^8$, $R^9$, $R^{10}$ und $R^{11}$ die oben beschriebenen Bedeutungen, insbesondere die in Ausgestaltung (1) oder Ausgestaltung (3) oder Ausgestaltung (4) oder Ausgestaltung (5) beschriebenen Bedeutungen haben.

In einer weiteren bevorzugten Ausführungsform betrifft die Erfindung die Verbindungen der Formel (I)

wobei $R^7$ die in Ausgestaltung (3) beschriebenen Bedeutungen hat und

wobei Z, Y, $A^1$, $A^2$, $A^3$, $A^4$, X, $R^1$, $R^2$, $R^3$, $R^4$ $R^5$, $R^6$, $R^8$, $R^9$, $R^{10}$ und $R^{11}$ die oben beschriebenen Bedeutungen, insbesondere die in Ausgestaltung (1) oder Ausgestaltung (2) oder Ausgestaltung (4) oder Ausgestaltung (5) beschriebenen Bedeutungen haben.

In einer weiteren bevorzugten Ausführungsform betrifft die Erfindung die Verbindungen der Formel (I)

wobei $R^7$ die in Ausgestaltung (4) beschriebenen Bedeutungen hat und

wobei Z, Y, $A^1$, $A^2$, $A^3$, $A^4$, X, $R^1$, $R^2$, $R^3$, $R^4$ $R^5$, $R^6$, $R^8$, $R^9$, $R^{10}$ und $R^{11}$ die oben beschriebenen Bedeutungen, insbesondere die in Ausgestaltung (1) oder Ausgestaltung (2) oder Ausgestaltung (3) oder Ausgestaltung (5) beschriebenen Bedeutungen haben.

In einer weiteren bevorzugten Ausführungsform betrifft die Erfindung die Verbindungen der Formel (I)

wobei $R^7$ die in Ausgestaltung (5) beschriebenen Bedeutungen hat und

wobei Z, Y, $A^1$, $A^2$, $A^3$, $A^4$, X, $R^1$, $R^2$, $R^3$, $R^4$ $R^5$, $R^6$, $R^8$, $R^9$, $R^{10}$ und $R^{11}$ die oben beschriebenen Bedeutungen, insbesondere die in Ausgestaltung (1) oder Ausgestaltung (2) oder Ausgestaltung (3) oder Ausgestaltung (4) beschriebenen Bedeutungen haben.

In einer bevorzugten Ausführungsform betrifft die Erfindung die Verbindungen der Formel (I)

wobei $R^8$ die in Ausgestaltung (1) beschriebenen Bedeutungen hat und

wobei Z, Y, $A^1$, $A^2$, $A^3$, $A^4$, X, $R^1$, $R^2$, $R^3$, $R^4$ $R^5$, $R^6$, $R^7$, $R^9$, $R^{10}$ und $R^{11}$ die oben beschriebenen Bedeutungen, insbesondere die in Ausgestaltung (2) oder Ausgestaltung (3) oder Ausgestaltung (4) oder Ausgestaltung (5) beschriebenen Bedeutungen haben.

In einer weiteren bevorzugten Ausführungsform betrifft die Erfindung die Verbindungen der Formel (I)

wobei $R^8$ die in Ausgestaltung (2) beschriebenen Bedeutungen hat und

wobei Z, Y, $A^1$, $A^2$, $A^3$, $A^4$, X, $R^1$, $R^2$, $R^3$, $R^4$ $R^5$, $R^6$, $R^7$, $R^9$, $R^{10}$ und $R^{11}$ die oben beschriebenen Bedeutungen, insbesondere die in Ausgestaltung (1) oder Ausgestaltung (3) oder Ausgestaltung (4) oder Ausgestaltung (5) beschriebenen Bedeutungen haben.

In einer weiteren bevorzugten Ausführungsform betrifft die Erfindung die Verbindungen der Formel (I)

wobei $R^8$ die in Ausgestaltung (3) beschriebenen Bedeutungen hat und

wobei Z, Y, $A^1$, $A^2$, $A^3$, $A^4$, X, $R^1$, $R^2$, $R^3$, $R^4$ $R^5$, $R^6$, $R^7$, $R^9$, $R^{10}$ und $R^{11}$ die oben beschriebenen Bedeutungen, insbesondere die in Ausgestaltung (1) oder Ausgestaltung (2) oder Ausgestaltung (4) oder Ausgestaltung (5) beschriebenen Bedeutungen haben.

In einer weiteren bevorzugten Ausführungsform betrifft die Erfindung die Verbindungen der Formel (I)

wobei $R^8$ die in Ausgestaltung (4) beschriebenen Bedeutungen hat und

wobei Z, Y, $A^1$, $A^2$, $A^3$, $A^4$, X, $R^1$, $R^2$, $R^3$, $R^4$ $R^5$, $R^6$, $R^7$, $R^9$, $R^{10}$ und $R^{11}$ die oben beschriebenen Bedeutungen, insbesondere die in Ausgestaltung (1) oder Ausgestaltung (2) oder Ausgestaltung (3) oder Ausgestaltung (5) beschriebenen Bedeutungen haben.

In einer weiteren bevorzugten Ausführungsform betrifft die Erfindung die Verbindungen der Formel (I)

wobei $R^8$ die in Ausgestaltung (5) beschriebenen Bedeutungen hat und

wobei Z, Y, $A^1$, $A^2$, $A^3$, $A^4$, X, $R^1$, $R^2$, $R^3$, $R^4$ $R^5$, $R^6$, $R^7$, $R^9$, $R^{10}$ und $R^{11}$ die oben beschriebenen Bedeutungen, insbesondere die in Ausgestaltung (1) oder Ausgestaltung (2) oder Ausgestaltung (3) oder Ausgestaltung (4) beschriebenen Bedeutungen haben.

In einer bevorzugten Ausführungsform betrifft die Erfindung die Verbindungen der Formel (I)

wobei $R^9$ die in Ausgestaltung (1) beschriebenen Bedeutungen hat und

wobei Z, Y, $A^1$, $A^2$, $A^3$, $A^4$, X, $R^1$, $R^2$, $R^3$, $R^4$ $R^5$, $R^6$, $R^7$, $R^8$, $R^{10}$ und $R^{11}$ die oben beschriebenen Bedeutungen, insbesondere die in Ausgestaltung (2) oder Ausgestaltung (3) oder Ausgestaltung (4) oder Ausgestaltung (5) beschriebenen Bedeutungen haben.

In einer weiteren bevorzugten Ausführungsform betrifft die Erfindung die Verbindungen der Formel (I)

wobei $R^9$ die in Ausgestaltung (2) beschriebenen Bedeutungen hat und

wobei Z, Y, $A^1$, $A^2$, $A^3$, $A^4$, X, $R^1$, $R^2$, $R^3$, $R^4$ $R^5$, $R^6$, $R^7$, $R^8$, $R^{10}$ und $R^{11}$ die oben beschriebenen Bedeutungen, insbesondere die in Ausgestaltung (1) oder Ausgestaltung (3) oder Ausgestaltung (4) oder Ausgestaltung (5) beschriebenen Bedeutungen haben.

In einer weiteren bevorzugten Ausführungsform betrifft die Erfindung die Verbindungen der Formel (I)

wobei $R^9$ die in Ausgestaltung (3) beschriebenen Bedeutungen hat und

wobei Z, Y, $A^1$, $A^2$, $A^3$, $A^4$, X, $R^1$, $R^2$, $R^3$, $R^4$ $R^5$, $R^6$, $R^7$, $R^8$, $R^{10}$ und $R^{11}$ die oben beschriebenen Bedeutungen, insbesondere die in Ausgestaltung (1) oder Ausgestaltung (2) oder Ausgestaltung (4) oder Ausgestaltung (5) beschriebenen Bedeutungen haben.

In einer weiteren bevorzugten Ausführungsform betrifft die Erfindung die Verbindungen der Formel (I)

wobei $R^9$ die in Ausgestaltung (4) beschriebenen Bedeutungen hat und

wobei Z, Y, $A^1$, $A^2$, $A^3$, $A^4$, X, $R^1$, $R^2$, $R^3$, $R^4$, $R^5$, $R^6$, $R^7$, $R^8$, $R^{10}$ und $R^{11}$ die oben beschriebenen Bedeutungen, insbesondere die in Ausgestaltung (1) oder Ausgestaltung (2) oder Ausgestaltung (3) oder Ausgestaltung (5) beschriebenen Bedeutungen haben.

In einer weiteren bevorzugten Ausführungsform betrifft die Erfindung die Verbindungen der Formel (I)

wobei $R^9$ die in Ausgestaltung (5) beschriebenen Bedeutungen hat und

wobei Z, Y, $A^1$, $A^2$, $A^3$, $A^4$, X, $R^1$, $R^2$, $R^3$, $R^4$, $R^5$, $R^6$, $R^7$, $R^8$, $R^{10}$ und $R^{11}$ die oben beschriebenen Bedeutungen, insbesondere die in Ausgestaltung (1) oder Ausgestaltung (2) oder Ausgestaltung (3) oder Ausgestaltung (4) beschriebenen Bedeutungen haben.

In einer bevorzugten Ausführungsform betrifft die Erfindung die Verbindungen der Formel (I)

wobei $R^{10}$ und $R^{11}$ die in Ausgestaltung (1) beschriebenen Bedeutungen haben und

wobei Z, Y, $A^1$, $A^2$, $A^3$, $A^4$, X, $R^1$, $R^2$, $R^3$, $R^4$, $R^5$, $R^6$, $R^7$, $R^8$ und $R^9$ die oben beschriebenen Bedeutungen, insbesondere die in Ausgestaltung (2) oder Ausgestaltung (3) oder Ausgestaltung (4) oder Ausgestaltung (5) beschriebenen Bedeutungen haben.

In einer weiteren bevorzugten Ausführungsform betrifft die Erfindung die Verbindungen der Formel (I)

wobei $R^{10}$ und $R^{11}$ die in Ausgestaltung (2) beschriebenen Bedeutungen haben und

wobei Z, Y, $A^1$, $A^2$, $A^3$, $A^4$, X, $R^1$, $R^2$, $R^3$, $R^4$, $R^5$, $R^6$, $R^7$, $R^8$ und $R^9$ die oben beschriebenen Bedeutungen, insbesondere die in Ausgestaltung (1) oder Ausgestaltung (3) oder Ausgestaltung (4) oder Ausgestaltung (5) beschriebenen Bedeutungen haben.

In einer weiteren bevorzugten Ausführungsform betrifft die Erfindung die Verbindungen der Formel (I)

wobei $R^{10}$ und $R^{11}$ die in Ausgestaltung (3) beschriebenen Bedeutungen haben und

wobei Z, Y, $A^1$, $A^2$, $A^3$, $A^4$, X, $R^1$, $R^2$, $R^3$, $R^4$, $R^5$, $R^6$, $R^7$, $R^8$ und $R^9$ die oben beschriebenen Bedeutungen, insbesondere die in Ausgestaltung (1) oder Ausgestaltung (2) oder Ausgestaltung (4) oder Ausgestaltung (5) beschriebenen Bedeutungen haben.

In einer weiteren bevorzugten Ausführungsform betrifft die Erfindung die Verbindungen der Formel (I)

wobei $R^{10}$ und $R^{11}$ die in Ausgestaltung (4) beschriebenen Bedeutungen haben und

wobei Z, Y, $A^1$, $A^2$, $A^3$, $A^4$, X, $R^1$, $R^2$, $R^3$, $R^4$, $R^5$, $R^6$, $R^7$, $R^8$ und $R^9$ die oben beschriebenen Bedeutungen, insbesondere die in Ausgestaltung (1) oder Ausgestaltung (2) oder Ausgestaltung (3) oder Ausgestaltung (5) beschriebenen Bedeutungen haben.

In einer weiteren bevorzugten Ausführungsform betrifft die Erfindung die Verbindungen der Formel (I)

wobei $R^{10}$ und $R^{11}$ die in Ausgestaltung (5) beschriebenen Bedeutungen haben und

wobei Z, Y, $A^1$, $A^2$, $A^3$, $A^4$, X, $R^1$, $R^2$, $R^3$, $R^4$, $R^5$, $R^6$, $R^7$, $R^8$ und $R^9$ die oben beschriebenen Bedeutungen, insbesondere die in Ausgestaltung (1) oder Ausgestaltung (2) oder Ausgestaltung (3) oder Ausgestaltung (4) beschriebenen Bedeutungen haben.

## Definitionen

Der Fachmann ist sich bewusst, dass, wenn nicht ausdrücklich angegeben, die Ausdrücke "ein", "eine" oder "eines" wie in dieser Anmeldung genutzt je nach Situation "ein/eine/eines (1)", "ein/eine/eines (1) oder mehr" oder "mindestens ein/eine/eines (1)" bedeuten kann.

Für alle hierin beschriebenen Strukturen wie cyclischen Systeme und Gruppen gilt, dass benachbarte Atome nicht -O-O- oder -O-S- sein dürfen.

Strukturen mit einer variablen Anzahl an möglichen Kohlenstoffatomen (C-Atomen) können in der vorliegenden Anmeldung als $C_{\text{untere Grenze C-Atome}}$-$C_{\text{obere Grenze C-Atome}}$-Strukturen ($C_{uG}$-$C_{oG}$-Strukturen) bezeichnet werden, um so näher bestimmt zu werden. Beispiel: eine Alkylgruppe kann aus 3 bis 10 C-Atomen bestehen und entspricht dann $C_3$-$C_{10}$-Alkyl. Ringstrukturen aus C-Atomen und Heteroatomen können als "uG bis oGgliedrige" Strukturen bezeichnet werden. Ein Beispiel einer 6-gliedrigen Ringstruktur ist Toluol (eine 6-gliedriges Ringstruktur, die mit einer Methylgruppe substituiert ist).

Steht ein Sammelbegriff für einen Substituenten, z. B. $C_{uG}$-$C_{oG}$-Alkyl, am Ende eines zusammengesetzten Substituenten wie z.B. bei $C_{uG}$-$C_{oG}$-Cycloalkyl-$C_{uG}$-$C_{oG}$-Alkyl, so kann der am Anfang stehende Bestandteil des zusammengesetzten Substituenten, z.B. das $C_{uG}$-$C_{oG}$-Cycloalkyl, ein- bzw. mehrfach, gleich oder verschieden und unabhängig voneinander mit dem letzten Substituenten, $C_{uG}$-$C_{oG}$-Alkyl, substituiert sein. Alle in dieser Anmeldung verwendeten Sammelbegriffe für chemische Gruppen, cyclische Systeme und cyclische Gruppen können durch den Zusatz "$C_{uG}$-$C_{oG}$" oder "uG bis oG-gliedrig(e)" näher bestimmt werden.Die Definition für Sammelbegriffe solange nicht anders definiert gilt auch für diese Sammelbegriffe in zusammengesetzten Substituenten. Beispiel: Die Definition für $C_{uG}$-$C_{oG}$-Alkyl gilt auch für $C_{uG}$-$C_{oG}$-Alkyl als Bestandteil eines zusammengesetzten Substituenten wie z.B. $C_{uG}$-$C_{oG}$-Cycloalkyl-$C_{uG}$-$C_{oG}$-Alkyl.

Bei den in den vorstehenden Formeln angegebenen Definitionen der Symbole wurden Sammelbegriffe verwendet, die allgemein repräsentativ für die folgenden Substituenten stehen:

Halogen bezieht sich auf die Elemente der 7. Hauptgruppe, bevorzugt Fluor, Chlor, Brom und Iod, mehr bevorzugt Fluor, Chlor und Brom und noch bevorzugter Fluor und Chlor.

Beispiele für Heteroatom sind N, O, S, P, B, Si. Bevorzugt bezieht sich der Begriff Heteroatom auf N, S und O.

Sofern nicht an anderer Stelle anders definiert steht "Alkyl" - in Alleinstellung oder als Bestandteil einer chemischen Gruppe - für geradkettige oder verzweigte Kohlenwasserstoffe, vorzugsweise mit 1 bis 6 Kohlenstoffatomen,

wie beispielsweise Methyl, Ethyl, n-Propyl, Isopropyl, n-Butyl, Isobutyl, s-Butyl, t-Butyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,2-Dimethylpropyl, 1,3-Dimethylbutyl, 1,4-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl und 2-Ethylbutyl. Ferner bevorzugt sind Alkyle mit 1 bis 4 Kohlenstoffatomen, wie unter anderem Methyl, Ethyl, Ethyl, n-Propyl, Isopropyl, n-Butyl, Isobutyl, s-Butyl oder t-Butyl. Sofern nicht an anderer Stelle anders definiert steht "Alkenyl" - in Alleinstellung oder als Bestandteil einer chemischen Gruppe - für geradkettige oder verzweigte Kohlenwasserstoffe, vorzugsweise mit 2 bis 6 Kohlenstoffatomen und mindestens einer Doppelbindung, wie beispielsweise Vinyl, 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-dimethyl-3-butenyl, 1,2-Dimethyl-2-butenyl, 1,2-Dimethyl-3-butenyl, 1,3-Dimethyl-2-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl. Ferner bevorzugt sind Alkenyle mit 2 bis 4 Kohlenstoffatomen, wie unter anderem 2-Propenyl, 2-Butenyl oder 1-Methyl-2-propenyl.

Sofern nicht an anderer Stelle anders definiert steht "Alkinyl" - in Alleinstellung oder als Bestandteil einer chemischen Gruppe - für geradkettige oder verzweigte Kohlenwasserstoffe, vorzugsweise mit 2 bis 6 Kohlenstoffatomen und mindestens einer Dreifachbindung wie beispielsweise 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-3-butinyl, 1,2-Dimethyl-3-butinyl, 2,2-Dimethyl-3-butinyl, 1-Ethyl-3-butinyl, 2-Ethyl-3-butinyl, 1-Ethyl-1-methyl-2-propinyl und 2,5-Hexadiynyl. Ferner bevorzugt sind Alkinyle mit 2 bis 4 Kohlenstoffatomen wie unter anderem Ethinyl, 2-Propinyl oder 2-Butinyl-2-propenyl.

Sofern nicht an anderer Stelle anders definiert steht "Cycloalkyl" - in Alleinstellung oder als Bestandteil einer chemischen Gruppe - für mono-, bi- oder tricyclische Kohlenwasserstoffe, vorzugsweise mit 3 bis 10 Kohlenstoffen wie beispielsweise Cyclopropyl, Cyclobutyl, Cyclopentyl, Cyclohexyl, Cycloheptyl, Cyclooctyl, Bicyclo[2.2.1]heptyl, Bicyclo[2.2.2]octyl oder Adamantyl. Ferner bevorzugt sind Cycloalkyle mit 3, 4, 5, 6 oder 7 Kohlenstoffatomen, wie unter anderem Cyclopropyl oder Cyclobutyl. Sofern nicht an anderer Stelle anders definiert steht "Alkylcycloalkyl" für mono-, bi- oder tricyclisches Alkylcycloalkyl, vorzugsweise mit 4 bis 10 oder 4 bis 7 Kohlenstoffatomen, wie beispielsweise Methylcyclopropyl, Ethylcyclopropyl, Isopropylcyclobutyl, 3-Methylcyclopentyl und 4-Methyl-cyclohexyl. Ferner bevorzugt sind Alkylcycloalkyle mit 4, 5 oder 7 Kohlenstoffatomen wie unter anderen Ethylcyclopropyl oder 4-Methyl-cyclohexyl.

Sofern nicht an anderer Stelle anders definiert steht "Hydroxyalkyl" für geradkettigen oder verzweigten Alkohol, vorzugsweise mit 1 bis 6 Kohlenstoffatomen, wie beispielsweise Methanol, Ethanol, n-Propanol, Isopropanol, n-Butanol, Isobutanol, s-Butanol und t-Butanol. Ferner bevorzugt sind Hydroxyalkylgruppen mit 1 bis 4 Kohlenstoffatomen.

Sofern nicht an anderer Stelle anders definiert steht "Alkoxy" für geradkettiges oder verzweigtes O-Alkyl, vorzugsweise mit 1 bis 6 Kohlenstoffatomen, wie beispielsweise Methoxy, Ethoxy, n-Propoxy, Isopropoxy, n-Butoxy, Isobutoxy, s-Butoxy und t-Butoxy. Ferner bevorzugt sind Alkoxygruppen mit 1 bis 4 Kohlenstoffatomen. Sofern nicht an anderer Stelle anders definiert steht "Alkylsulfanyl" für geradkettiges oder verzweigtes S-Alkyl, vorzugsweise mit 1 bis 6 Kohlenstoffatomen, wie beispielsweise Methylthio, Ethylthio, n-Propylthio, Isopropylthio, n-Butylthio, Isobutylthio, s-Butylthio und t-Butylthio. Ferner bevorzugt sind Alkylsulfanylgruppen mit 1 bis 4 Kohlenstoffatomen.

Sofern nicht an anderer Stelle anders definiert steht "Alkylsulfinyl" für geradkettiges oder verzweigtes Alkylsulfinyl, vorzugsweise mit 1 bis 6 Kohlenstoffatomen wie beispielsweise Methylsulfinyl, Ethylsulfinyl, n-Propylsulfinyl, Isopropylsulfinyl, n-Butylsulfinyl, Isobutylsulfinyl, s-Butylsulfinyl und t-Butylsulfinyl. Ferner bevorzugt sind Alkylsulfinylgruppen mit 1 bis 4 Kohlenstoffatomen.

Sofern nicht an anderer Stelle anders definiert steht "Alkylsulfonyl" für geradkettiges oder verzweigtes Alkylsulfonyl, vorzugsweise mit 1 bis 6 Kohlenstoffatomen wie beispielsweise Methylsulfonyl, Ethylsulfonyl, n-Propylsulfonyl, Isopropylsulfonyl, n-Butylsulfonyl, Isobutylsulfonyl, s-Butylsulfonyl und t-Butylsulfonyl. Ferner bevorzugt sind Alkylsulfonylgruppen mit 1 bis 4 Kohlenstoffatomen.

Sofern nicht an anderer Stelle anders definiert steht "Alkylcarbonyl" für geradkettiges oder verzweigtes Alkyl-C(=O), vorzugsweise mit 2 bis 7 Kohlenstoffatomen, wie Methylcarbonyl, Ethylcarbonyl, n-Propylcarbonyl, Isopropylcarbonyl, s-Butylcarbonyl und t-Butylcarbonyl. Ferner bevorzugt sind Alkylcarbonyle mit 1 bis 4 Kohlenstoffatomen.

Sofern nicht an anderer Stelle anders definiert steht "Alkoxycarbonyl" - in Alleinstellung oder als Bestandteil einer chemischen Gruppe - für geradkettiges oder verzweigtes Alkoxycarbonyl, vorzugsweise mit 1 bis 6 Kohlenstoffatomen oder 1 bis 4 Kohlenstoffatomen im Alkoxyteil, wie beispielsweise Methoxycarbonyl, Ethoxycarbonyl, n-Propoxycarbonyl, Isopropoxycarbonyl, s-Butoxycarbonyl und t-Butoxycarbonyl.

Sofern nicht an anderer Stelle anders definiertsteht "Alkylaminocarbonyl" für geradkettiges oder verzweigtes

Alkylaminocarbonyl mit vorzugsweise 1 bis 6 Kohlenstoffatomen oder 1 bis 4 Kohlenstoffatomen im Alkylteil, wie beispielsweise Methylaminocarbonyl, Ethylaminocarbonyl, n-Proylaminocarbonyl, Isopropylaminocarbonyl, s-Butylaminocarbonyl und t-Butylaminocarbonyl. Die erfindungsgemäßen Alkylaminocarbonylgruppen können mit einem oder mehreren, gleichen oder verschiedenen Resten substituiert sein.

Sofern nicht an anderer Stelle anders definiert steht steht "*N*,*N*-Dialkylamino-carbonyl" für geradkettiges oder verzweigtes *N*,*N*-Dialkylamino-carbonyl mit vorzugsweise 1 bis 6 Kohlenstoffatomen oder 1 bis 4 Kohlenstoffatomen im Alkylteil, wie beispielsweise N,N-Dimethylamino-carbonyl, N.N-Diethylaminocarbonyl, N,N-Di(n-propylamino)-carbonyl, N,N-Di-(isopropylamino)-carbonyl und *N*,*N*-Di-(s-butylamino)-carbonyl.

Sofern nicht an anderer Stelle anders definiert steht steht "*N*,*N*-Dialkylamino-" für geradkettiges oder verzweigtes *N*,*N*-Dialkylamino- mit vorzugsweise 1 bis 6 Kohlenstoffatomen oder 1 bis 4 Kohlenstoffatomen im Alkylteil, wie beispielsweise *N*,*N*-Dimethylamino-1, *N*,*N*-Diethylamino-, *N*,*N*-Di(n-propylamino)-, *N,N*-Di-(isopropylamino)- und *N*,*N*-Di-(s-butylamino)-. Sofern nicht an anderer Stelle anders definiert steht "Alkanoyl" oder auch "Alkylcarbonyl" für einen geradkettigen oder verzweigten Alkylrest mit 1 bis 6 Kohlenstoffatomen, der in der 1-Position ein doppelt gebundenes Sauerstoffatom trägt und über die 1 - Position verknüpft ist. Beispielsweise seien genannt: Formyl, Acetyl, Propionyl, n-Butyryl, i-Butyryl, Pivaloyl, n-Hexanoyl.

Sofern nicht an anderer Stelle anders definiert steht "Aryl" für ein mono-, bi- oder polycyclisches aromatisches System mit vorzugsweise 6 bis 14, insbesondere 6 bis 10 Ring-Kohlenstoffatomen, wie beispielsweise Phenyl, Naphthyl, Anthryl, Phenanthrenyl, vorzugsweise Phenyl. Ferner steht Aryl auch für mehrcyclische Systeme, wie Tetrahydronaphtyl, Indenyl, Indanyl, Fluorenyl, Biphenyl, wobei die Bindungsstelle am aromatischen System ist.

Beispiele substitutierter Aryle stellen die Arylalkyle dar, die gleichfalls mit einem oder mehreren, gleichen oder verschiedenen Resten im $C_1$-$C_4$-Alkyl- und/oder $C_6$-$C_{14}$-Arylteil substituiert sein können. Beispiele solcher Arylalkyle sind unter anderem Benzyl und 1-Phenylethyl.

Sofern nicht an anderer Stelle anders definiert steht "Heterocyclus", "heterocyclischer Ring" oder "heterocyclisches Ringsystem" für ein carbocyclisches Ringsystem mit mindestens einem Ring, in dem mindestens ein Kohlenstoffatom durch ein Heteroatom ersetzt ist, vorzugsweise durch ein Heteroatom aus der Gruppe N, O, S, P, B, Si, Se und der gesättigt, ungesättigt oder heteroaromatisch ist und dabei unsubstituiert oder substituiert sein kann, wobei die Bindungsstelle an einem Ringatom lokalisiert ist. Wenn nicht anders definiert, enthält der heterocyclische Ring vorzugsweise 3 bis 9 Ringatome, insbesondere 3 bis 6 Ringatome, und ein oder mehrere, vorzugsweise 1 bis 4, insbesondere 1, 2 oder 3 Heteroatome im heterocyclischen Ring, vorzugsweise aus der Gruppe N, O, und S, wobei jedoch nicht zwei Sauerstoffatome direkt benachbart sein sollen. Die heterocyclischen Ringe enthalten gewöhnlicherweise nicht mehr als 4 Stickstoffatome, und/oder nicht mehr als 2 Sauerstoffatome und/oder nicht mehr als 2 Schwefelatome. Ist der Heterocyclylrest oder der heterocyclische Ring gegebenenfalls substituiert, kann er mit anderen carbocyclischen oder heterocyclischen Ringen annelliert sein. Im Falle von gegebenenfalls substituiertem Heterocyclyl werden erfindungsgemäß auch mehrcyclische Systeme umfaßt, wie beispielsweise 8-Aza-bicyclo[3.2.1]octanyl oder 1-Aza-bicyclo[2.2.1]heptyl. Im Falle von gegebenenfalls substituiertem Heterocyclyl werden erfindungsgemäß auch spirocyclische Systeme umfasst, wie beispielsweise 1-Oxa-5-aza-spiro[2.3]hexyl.

Erfindungsgemäße Heterocyclylgruppen sind beispielsweise Piperidinyl, Piperazinyl, Morpholinyl, Thiomorpholinyl, Dihydropyranyl, Tetrahydropyranyl, Dioxanyl, Pyrrolinyl, Pyrrolidinyl, Imidazolinyl, Imidazolidinyl, Thiazolidinyl, Oxazolidinyl, Dioxolanyl, Dioxolyl, Pyrazolidinyl, Tetrahydrofuranyl, Dihydrofuranyl, Oxetanyl, Oxiranyl, Azetidinyl, Aziridinyl, Oxazetidinyl, Oxaziridinyl, Oxazepanyl, Oxazinanyl, Azepanyl, Oxopyrrolidinyl, Dioxopyrrolidinyl, Oxomorpholinyl, Oxopiperazinyl und Oxepanyl.

Der Begriff "gegebenenfalls substituierte" Gruppen/Substituenten, wie ein substituierter Alkyl-, Alkenyl-, Alkinyl-, Alkoxy-, Alkylsulfanyl-, Alkylsulfinyl-, Alkylsulfonyl-, Cycloalkyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise ein (1) Substituent oder mehrere Substituenten, vorzugsweise 1, 2, 3, 4, 5, 6, oder 7, ausgewählt sind aus einer Gruppe bestehend aus Amino, Hydroxy, Halogen, Nitro, Cyano, Isocyano, Mercapto, Isothiocyanato, $C_1$-$C_4$-Carboxy, Carbonamid, $SF_5$, Aminosulfonyl, $C_1$-$C_4$-Alkyl, $C_3$-$C_4$-Cycloalkyl, $C_2$-$C_4$-Alkenyl, $C_5$-$C_6$-Cycloalkenyl, $C_2$-$C_4$-Alkinyl, *N*-Mono-$C_1$-$C_4$-alkyl-amino, *N*,*N*-Di-$C_1$-$C_4$-alkylamino, *N*-$C_1$-$C_4$-Alkanoylamino, $C_1$-$C_4$-Alkoxy, $C_2$-$C_4$-Alkenyloxy, $C_2$-$C_4$-Alkinyloxy, $C_3$-$C_4$-Cycloalkoxy, $C_5$-$C_6$-Cycloalkenyloxy, $C_1$-$C_4$-Alkoxycarbonyl, $C_2$-$C_4$-Alkenyloxycarbonyl, $C_2$-$C_4$-Alkinyloxycarbonyl, $C_6$-,$C_{10}$-,$C_{14}$-Aryloxycarbonyl, $C_1$-$C_4$-Alkanoyl, $C_2$-$C_4$-Alkenylcarbonyl, $C_2$-$C_4$-Alkinylcarbonyl, $C_6$-,$C_{10}$-,$C_{14}$-Arylcarbonyl, $C_1$-$C_4$-Alkylsulfanyl, $C_3$-$C_4$-Cycloalkylsulfanyl, $C_1$-$C_4$-Alkylthio, $C_2$-$C_4$-Alkenylthio, $C_5$-$C_6$-Cycloalkenylthio, $C_2$-$C_4$-Alkinylthio, $C_1$-$C_4$-Alkylsulfenyl und $C_1$-$C_4$-Alkylsulfinyl, wobei beide Enantiomere der $C_1$-$C_4$-Alkylsulfinylgruppe umfasst ind, $C_1$-$C_4$-Alkylsulfonyl, *N*-Mono-$C_1$-$C_4$-alkylaminosulfonyl, *N*,*N*-Di-$C_1$-$C_4$-alkylaminosulfonyl, $C_1$-$C_4$-Alkylphosphinyl, $C_1$-$C_4$-Alkylphosphonyl, wobei für $C_1$-$C_4$-Alkylphosphinyl bzw. $C_1$-$C_4$-Alkylphosphonyl beide Enantiomere umfasst sind, *N*-$C_1$-$C_4$-Alkylaminocarbonyl, *N*,*N*-Di-$C_1$-$C_4$-alkylamino-carbonyl, N-$C_1$-$C_4$-Alkanoylamino-carbonyl, N-$C_1$-$C_4$-Alkanoyl-N-$C_1$-$C_4$-alkylaminocarbonyl, -CH=N-O-[($C_1$-$C_4$)-Alkyl], -C[($C_1$-$C_4$)-Alkyl]=N-O-[($C_1$-$C_4$)-Alkyl, $C_6$-,$C_{10}$-,$C_{14}$-Aryl, $C_6$-,$C_{10}$-,$C_{14}$-Aryloxy, Benzyl, Benzyloxy, Benzylthio, $C_6$-,$C_{10}$-,$C_{14}$-Arylthio, $C_6$-,$C_{10}$-,$C_{14}$-Arylamino, Benzylamino, Heterocyclyl und Trialkylsilyl, mit einer Doppelbindung verbundene Substituenten

wie $C_1$-$C_4$-Alkyliden (z. B. Methyliden oder Ethyliden), eine Oxogruppe, eine Iminogruppe sowie einer substituierten Iminogruppe. Wenn zwei oder mehrere Reste einen oder mehrere Ringe bilden, so können diese carbocyclisch, heterocyclisch, gesättigt, teilgesättigt, ungesättigt, beispielsweise auch aromatisch und weiter substituiert sein.

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen oder mehreren der Substituenten jeweils unabhängig voneinander ausgewählt aus Halogen, Hydroxy, Amino, Nitro, Cyano, Isocyano, Azido, Acylamino, einer Oxogruppe und einer Iminogruppe. Vorzugsweise werden vom Begriff "gegebenenfalls substituierter" Gruppe nur ein oder zwei Substitutentenebenen umfasst.

Sofern nicht an anderer Stelle anders definiert sind die mit Halogen substituierten chemischen Gruppen (wie z. B. Alkyl, Cycloalkyl oder Alkoxy) einfach oder mehrfach bis zur maximal möglichen Substituentenzahl mit Halogen substituiert. Solche Gruppen werden auch als Halogruppen bezeichnet (wie, z. B. Haloalkyl). Bei mehrfacher Substitution mit Halogen, können die Halogenatome gleich oder verschieden sein und können alle an eines oder an mehrere Kohlenstoffatome gebunden sein. Dabei steht Halogen insbesondere für Fluor, Chlor, Brom oder Iod, vorzugsweise für Fluor, Chlor oder Brom und besonders bevorzugt für Fluor. Insbesondere sind mit Halogen substituierte Gruppen Monohalocycloalkyl wie 1-Fluor-cyclopropyl, 2-Fluor-cyclopropyl oder 1-Fluor-cyclobutyl, Monohaloalkyl wie 2-Chlor-ethyl, 2-Fluor-ethyl, 1-Chlor-ethyl, 1-Fluor-ethyl, Chlormethyl, oder Fluormethyl; Perhaloalkyl wie Trichlormethyl oder Trifluormethyl oder $CF_2CF_3$, Polyhaloalkyl wie Difluormethyl, 2-Fluor-2-Chlor-ethyl, Dichlormethyl, 1,1,2,2-Tetraflourethyl, oder 2,2,2-Trifluorethyl. Weitere Beispiele für Halogenalkyle sind Trichlormethyl, Chlordifluormethyl, Dichlorfluormethyl, Chlormethyl, Brommethyl, 1-Fluorethyl, 2-Fluorethyl, 2,2-Difluorethyl, 2,2,2-Trifluorethyl, 2,2,2-Trichlorethyl, 2-Chlor-2,2-difluorethyl, Pentafluorethyl, 3,3,3-Trifluorpropyl und Pentafluor-t-butyl. Bevorzugt sind Halogenalkyle mit 1 bis 4 Kohlenstoffatomen und 1 bis 9, vorzugsweise 1 bis 5 gleichen oder verschiedenen Halogenatomen, die ausgewählt sind unter Fluor, Chlor oder Brom. Besonders bevorzugt sind Halogenalkyle mit 1 oder 2 Kohlenstoffatomen und mit 1 bis 5 gleichen oder verschiedenen Halogenatomen, die ausgewählt sind unter Fluor oder Chlor, wie unter anderen Difluormethyl, Trifluormethyl oder 2,2-Difluorethyl. Weitere Beispiele für mit Halogen substituierten Verbindungen sind Haloalkoxy wie $OCF_3$, $OCHF_2$, $OCH_2F$, $OCF_2CF_3$, $OCH_2CF_3$, $OCH_2CHF_2$ und $OCH_2CH_2C1$, Halogenalkylsulfanyle wie Difluormethylthio, Trifluormethylthio, Trichlormethylthio, Chlordifluormethylthio, 1-Fluorethylthio, 2-Fluorethylthio, 2,2-Difluorethylthio, 1,1,2,2-Tetrafluorethylthio, 2,2,2-Trifluorethylthio oder 2-Chlor-1,1,2-trifluorethylthio, Halogenalkylsulfinyle wie Difluormethylsulfinyl, Trifluormethylsulfinyl, Trichlormethylsulfinyl, Chlordifluormethylsulfinyl, 1-Fluorethylsulfinyl, 2-Fluorethylsulfinyl, 2,2-Difluorethylsulfinyl, 1,1,2,2-Tetrafluorethylsulfinyl, 2,2,2-Trifluorethylsulfinyl und 2-Chlor-1,1,2-trifluorethylsulfinyl, Halogenalkylsulfinyle wie Difluormethylsulfinyl, Trifluormethylsulfinyl, Trichlormethylsulfinyl, Chlordifluormethylsulfinyl, 1-Fluorethylsulfinyl, 2-Fluorethylsulfinyl, 2,2-Difluorethylsulfinyl, 1,1,2,2-Tetrafluorethylsulfinyl, 2,2,2-Trifluorethylsulfinyl und 2-Chlor-1,1,2-trifluorethylsulfinyl, Halogenalkylsulfonylgrupen wie Difluormethylsulfonyl, Trifluormethylsulfonyl, Trichlormethylsulfonyl, Chlordifluormethylsulfonyl, 1-Fluorethylsulfonyl, 2-Fluorethylsulfonyl, 2,2-Difluorethylsulfonyl, 1,1,2,2-Tetrafluorethylsulfonyl, 2,2,2-Trifluorethylsulfonyl und 2-Chlor-1,1,2-trifluorethylsulfonyl.

Sofern nicht an anderer Stelle anders definiert sind bei Resten mit C-Atomen solche mit 1 bis 4 C-Atomen, insbesondere 1 oder 2 C-Atomen bevorzugt. Bevorzugt sind in der Regel Substituenten aus der Gruppe Halogen, z.B. Fluor und Chlor, ($C_1$-$C_4$)Alkyl, vorzugsweise Methyl oder Ethyl, ($C_1$-$C_4$)Haloalkyl, vorzugsweise Trifluormethyl, ($C_1$-$C_4$)Alkoxy, vorzugsweise Methoxy oder Ethoxy, ($C_1$-$C_4$)Haloalkoxy, Nitro und Cyano. Besonders bevorzugt sind dabei die Substituenten Methyl, Methoxy, Fluor und Chlor.

Sofern nicht an anderer Stelle anders definiert bedeuten substituiertes Amino wie mono- oder disubstituiertes Amino einen Rest aus der Gruppe der substituierten Aminoreste, welche beispielsweise durch einen bzw. zwei gleiche oder verschiedene Reste aus der Gruppe Alkyl, Hydroxy, Amino, Alkoxy, Acyl und Aryl N-substituiert sind; vorzugsweise *N*-Mono- und *N*,*N*-Dialkylamino, (z.B. Methylamino, Ethylamino, *N,N*-Dimethylamino, *N*,*N*-Diethylamino, *N*,*N*-Di-n-propylamino, *N*,*N*-Diisopropylamino oder *N,N*-Dibutylamino), *N*-Mono- oder *N*,*N*-Dialkoxyalkylaminogruppen (z.B. *N*-Methoxymethylamino, N-Methoxyethylamino, *N*,*N*-Di-(methoxymethyl)-amino oder *N*,*N*-Di-(methoxyethyl)-amino), *N*-Mono- und *N*,*N*-Diarylamino, wie gegebenenfalls substituierte Aniline, Acylamino, *N*,*N*-diacylamino, *N*-Alkyl-*N*-arylamino, *N*-Alkyl-*N*-acylamino sowie gesättigte *N*-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise ($C_1$-$C_4$)Alkanoyl. Entsprechenes gilt für substituiertes Hydroxylamino oder Hydrazino.

Substituiertes Amino schließt auch quartäre Ammoniumverbindungen (Salze) mit vier organischen Substituenten am Stickstoffatom ein.

Sofern nicht an anderer Stelle anders definiert ist gegebenenfalls substituiertes Phenyl vorzugsweise Phenyl, das unsubstituiert oder ein- oder mehrfach, vorzugsweise bis zu dreifach durch gleiche oder verschiedene Reste aus der Gruppe Halogen, $C_1$-$C_4$-Alkyl, $C_1$-$C_4$-Alkoxy, $C_1$-$C_4$-Alkoxy-$C_1$-$C_4$-alkoxy , $C_1$-$C_4$-Alkoxy-$C_1$-$C_4$-alkyl, $C_1$-$C_4$-Halogenalkyl, $C_1$-$C_4$-Halogenalkoxy, $C_1$-$C_4$-Alkylsulfanyl, $C_1$-$C_4$-Halogenalkylsulfanyl, Cyano, Isocyano und Nitro substituiert ist, z.B. o-, m- und p-Tolyl, Dimethylphenyle, 2-, 3- und 4-Chlorphenyl, 2-, 3- und 4-Fluorphenyl, 2-, 3- und 4-Trifluormethyl- und -Trichlormethylphenyl, 2,4-, 3,5-, 2,5- und 2,3-Dichlorphenyl, o-, m- und p-Methoxyphenyl, 4-Heptafluorphenyl.

Sofern nicht an anderer Stelle anders definiert ist substituiertes Cycloalkyl vorzugsweise Cycloalkyl, das unsubstituiert oder ein- oder mehrfach, vorzugsweise bis zu dreifach durch gleiche oder verschiedene Reste aus der Gruppe Halogen, Cyano, $C_1$-$C_4$-Alkyl, $C_1$-$C_4$-Alkoxy, $C_1$-$C_4$-Alkoxy-$C_1$-$C_4$-alkoxy , $C_1$-$C_4$-Alkoxy-$C_1$-$C_4$-alkyl, $C_1$-$C_4$-Halogenalkyl und $C_1$-$C_4$-Halogenalkoxy substituiert ist, insbesondere durch einen oder zwei $C_1$-$C_4$-Alkylreste substituiert ist.

Erfindungsgemäße Verbindungen können in bevorzugten Ausführungsformen vorkommen. Einzelne hierin beschriebene Ausführungsformen können dabei miteinander kombiniert werden. Nicht umfasst sind solche Kombinationen, die den Naturgesetzen widersprechen und die der Fachmann daher aufgrund seines Fachwissens ausgeschlossen hätte. Beispielsweise sind Ringstrukturen mit drei oder mehreren benachbarten O-Atomen ausgeschlossen.

**Isomere**

Die Verbindungen der Formel (I) können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit sowohl reine Stereoisomere als auch beliebige Gemische dieser Isomere.

**Verfahren und Verwendungen**

Die Erfindung betrifft auch Verfahren zur Bekämpfung von tierischen Schädlingen, bei dem man Verbindungen der Formel (I) auf tierische Schädlinge und/oder ihren Lebensraum einwirken lässt. Bevorzugt wird die Bekämpfung der tierischen Schädlinge in der Land- und Forstwirtschaft und im Materialschutz durchgeführt. Hierunter vorzugsweise ausgeschlossen sind Verfahren zur chirurgischen oder therapeutischen Behandlung des menschlichen oder tierischen Körpers und Diagnostizierverfahren, die am menschlichen oder tierischen Körper vorgenommen werden.

Die Erfindung betrifft ferner die Verwendung der Verbindungen der Formel (I) als Schädlingsbekämpfungsmittel, insbesondere Pflanzenschutzmittel.

Im Rahmen der vorliegenden Anmeldung umfasst der Begriff Schädlingsbekämpfungsmittel jeweils immer auch den Begriff Pflanzenschutzmittel.

Die Verbindungen der Formel (I) eignen sich bei guter Pflanzenverträglichkeit, günstiger Warmblütertoxizität und guter Umweltverträglichkeit zum Schutz von Pflanzen und Pflanzenorganen vor biotischen und abiotischen Stressfaktoren, zur Steigerung der Ernteerträge, Verbesserung der Qualität des Erntegutes und zur Bekämpfung von tierischen Schädlingen, insbesondere Insekten, Spinnentieren, Helminthen, insbesondere Nematoden, und Mollusken, die in der Landwirtschaft, im Gartenbau, bei der Tierzucht, in Aquakulturen, in Forsten, in Gärten und Freizeiteinrichtungen, im Vorrats- und Materialschutz sowie auf dem Hygienesektor vorkommen.

Im Rahmen der vorliegenden Patentanmeldung ist der Begriff "Hygiene" so zu verstehen, dass damit jegliche und alle Maßnahmen, Vorschriften und Verfahrensweisen gemeint sind, deren Ziel es ist, Krankheiten, insbesondere Infektionskrankheiten, zu verhindern, und die dazu dienen, die Gesundheit von Menschen und Tieren zu schützen und/oder die Umwelt zu schützen, und/oder die Sauberkeit aufrechterhalten. Erfindungsgemäß schließt dies insbesondere Maßnahmen zur Reinigung, Desinfektion und Sterilisation beispielsweise von Textilien oder harten Oberflächen, insbesondere Oberflächen aus Glas, Holz, Zement, Porzellan, Keramik, Kunststoff oder auch Metall(en) ein, um sicherzustellen, dass diese frei von Hygieneschädlingen und/oder ihren Ausscheidungen sind. Vorzugsweise ausgeschlossen vom Schutzbereich der Erfindung sind in dieser Hinsicht chirurgische oder therapeutische, auf den menschlichen Körper oder die Körper von Tieren anzuwendende Behandlungsvorschriften und diagnostische Vorschriften, die am menschlichen Körper oder den Körpern von Tieren durchgeführt werden.

Der Begriff "Hygienesektor" deckt alle Gebiete, technischen Felder und industriellen Anwendungen ab, bei denen diese Hygienemaßnahmen, -vorschriften und -verfahrensweisen wichtig sind, zum Beispiel im Hinblick auf Hygiene in Küchen, Bäckereien, Flughäfen, Badezimmern, Schwimmbecken, Kaufhäusern, Hotels, Krankenhäusern, Ställen, Tierhaltungen usw.

Der Begriff "Hygieneschädling" ist daher so zu verstehen, dass damit ein oder mehrere Tierschädlinge gemeint sind, deren Gegenwart im Hygienesektor problematisch ist, insbesondere aus Gesundheitsgründen. Es ist daher ein Hauptziel, das Vorhandensein von Hygieneschädlingen und/oder das Ausgesetztsein ihnen gegenüber im Hygienesektor zu vermeiden oder auf ein Mindestmaß zu begrenzen. Dies lässt sich insbesondere durch die Anwendung eines Pestizids erreichen, das sich sowohl zum Verhindern eines Befalls als auch zum Verhindern eines bereits vorhandenen Befalls einsetzen lässt. Man kann auch Zubereitungen verwenden, die eine Exposition gegenüber Schädlingen verhindern oder reduzieren. Hygieneschädlinge schließen zum Beispiel die unten erwähnten Organismen ein.

Der Begriff "Hygieneschutz" deckt somit alle Handlungen ab, mit denen diese Hygienemaßnahmen, -vorschriften und -verfahrensweisen aufrechterhalten und/oder verbessert werden.

Die Verbindungen der Formel (I) können vorzugsweise als Schädlingsbekämpfungsmittel eingesetzt werden. Sie sind gegen normal sensible und resistente Arten sowie gegen alle oder einzelne Entwicklungsstadien wirksam. Zu den oben erwähnten Schädlingen gehören:

Schädlinge aus dem Stamm der Arthropoda, insbesondere aus der Klasse der Arachnida z. B. Acarus spp., z. B. Acarus siro, Aceria kuko, Aceria sheldoni, Aculops spp., Aculus spp., z. B. Aculus fockeui, Aculus schlechtendali, Amblyomma spp., Amphitetranychus viennensis, Argas spp., Boophilus spp., Brevipalpus spp., z. B. Brevipalpus phoenicis, Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., z. B. Eotetranychus hicoriae, Epitrimerus pyri, Eutetranychus spp., z. B. Eutetranychus banksi, Eriophyes spp., z. B. Eriophyes pyri, Glycyphagus domesticus, Halotydeus destructor, Hemitarsonemus spp., z. B. Hemitarsonemus latus (=Polyphagotarsonemus latus), Hyalomma spp., Ixodes spp., Latrodectus spp., Loxosceles spp., Neutrombicula autumnalis, Nuphersa spp., Oligonychus spp., z. B. Oligonychus coffeae, Oligonychus coniferarum, Oligonychus ilicis, Oligonychus indicus, Oligonychus mangiferus, Oligonychus pratensis, Oligonychus punicae, Oligonychus yothersi, Ornithodorus spp., Ornithonyssus spp., Panonychus spp., z. B. Panonychus citri (=Metatetranychus citri), Panonychus ulmi (=Metatetranychus ulmi), Phyllocoptruta oleivora, Platytetranychus multidigituli, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., z. B. Tarsonemus confusus, Tarsonemus pallidus, Tetranychus spp., z. B. Tetranychus canadensis, Tetranychus cinnabarinus, Tetranychus turkestani, Tetranychus urticae, Trombicula alfreddugesi, Vaejovis spp., Vasates lycopersici;

aus der Klasse der Chilopoda z. B. Geophilus spp., Scutigera spp.;

aus der Ordnung oder der Klasse der Collembola z. B. Onychiurus armatus; Sminthurus viridis;

aus der Klasse der Diplopoda z. B. Blaniulus guttulatus;

aus der Klasse der Insecta, z. B. aus der Ordnung der Blattodea z. B. Blatta orientalis, Blattella asahinai, Blattella germanica, Leucophaea maderae, Loboptera decipiens, Neostylopyga rhombifolia, Panchlora spp., Parcoblatta spp., Periplaneta spp., z. B. Periplaneta americana, Periplaneta australasiae, Pycnoscelus surinamensis, Supella longipalpa;

aus der Ordnung der Coleoptera z. B. Acalymma vittatum, Acanthoscelides obtectus, Adoretus spp., Aethina tumida, Agelastica alni, Agrilus spp., z. B. Agrilus planipennis, Agrilus coxalis, Agrilus bilineatus, Agrilus anxius, Agriotes spp., z. B. Agriotes linneatus, Agriotes mancus, Alphitobius diaperinus, Amphimallon solstitialis, Anobium punctatum, Anoplophora spp., z. B. Anoplophora glabripennis, Anthonomus spp., z. B. Anthonomus grandis, Anthrenus spp., Apion spp., Apogonia spp., Atomaria spp., z. B. Atomaria linearis, Attagenus spp., Baris caerulescens, Bruchidius obtectus, Bruchus spp., z. B. Bruchus pisorum, Bruchus rufimanus, Cassida spp., Cerotoma trifurcata, Ceutorrhynchus spp., z. B. Ceutorrhynchus assimilis, Ceutorrhynchus quadridens, Ceutorrhynchus rapae, Chaetocnema spp., z. B. Chaetocnema confinis, Chaetocnema denticulata, Chaetocnema ectypa, Cleonus mendicus, Conoderus spp., Cosmopolites spp., z. B. Cosmopolites sordidus, Costelytra zealandica, Ctenicera spp., Curculio spp., z. B. Curculio caryae, Curculio caryatrypes, Curculio obtusus, Curculio sayi, Cryptolestes ferrugineus, Cryptolestes pusillus, Cryptorhynchus lapathi, Cryptorhynchus mangiferae, Cylindrocopturus spp., Cylindrocopturus adspersus, Cylindrocopturus furnissi, Dendroctonus spp., z. B. Dendroctonus ponderosae, Dermestes spp., Diabrotica spp., z. B. Diabrotica balteata, Diabrotica barberi, Diabrotica undecimpunctata howardi, Diabrotica undecimpunctata undecimpunctata, Diabrotica virgifera virgifera, Diabrotica virgifera zeae, Dichocrocis spp., Dicladispa armigera, Diloboderus spp., Epicaerus spp., Epilachna spp., z. B. Epilachna borealis, Epilachna varivestis, Epitrix spp., z. B. Epitrix cucumeris, Epitrix fuscula, Epitrix hirtipennis, Epitrix subcrinita, Epitrix tuberis, Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., z. B. Hypothenemus hampei, Hypothenemus obscurus, Hypothenemus pubescens, Lachnosterna consanguinea, Lasioderma serricorne, Latheticus oryzae, Lathridius spp., Lema spp., Leptinotarsa decemlineata, Leucoptera spp., z. B. Leucoptera coffeella, Limonius ectypus, Lissorhoptrus oryzophilus, Listronotus (=Hyperodes) spp., Lixus spp., Luperodes spp., Luperomorpha xanthodera, Lyctus spp., Megacyllene spp., z. B. Megacyllene robiniae, Megascelis spp., Melanotus spp., z. B. Melanotus longulus oregonensis, Meligethes aeneus, Melolontha spp., z. B. Melolontha melolontha, Migdolus spp., Monochamus spp., Naupactus xanthographus, Necrobia spp., Neogalerucella spp., Niptus hololeucus, Oryctes rhinoceros, Oryzaephilus surinamensis, Oryzaphagus oryzae, Otiorhynchus spp., z. B. Otiorhynchus cribricollis, Otiorhynchus ligustici, Otiorhynchus ovatus, Otiorhynchus rugosostriarus, Otiorhynchus sulcatus, Oulema spp., z. B. Oulema melanopus, Oulema oryzae, Oxycetonia jucunda, Phaedon cochleariae, Phyllophaga spp., Phyllophaga helleri, Phyllotreta spp., z. B. Phyllotreta armoraciae, Phyllotreta pusilla, Phyllotreta ramosa, Phyllotreta striolata, Popillia japonica, Premnotrypes spp., Prostephanus truncatus, Psylliodes spp., z. B. Psylliodes affinis, Psylliodes chrysocephala, Psylliodes punctulata, Ptinus spp., Rhizobius ventralis, Rhizopertha dominica, Rhynchophorus spp., Rhynchophorus ferrugineus, Rhynchophorus palmarum, Scolytus spp., z. B. Scolytus multistriatus, Sinoxylon perforans, Sitophilus spp.,

z. B. Sitophilus granarius, Sitophilus linearis, Sitophilus oryzae, Sitophilus zeamais, Sphenophorus spp., Stegobium paniceum, Sternechus spp., z. B. Sternechus paludatus, Symphyletes spp., Tanymecus spp., z. B. Tanymecus dilaticollis, Tanymecus indicus, Tanymecus palliatus, Tenebrio molitor, Tenebrioides mauretanicus, Tribolium spp., z. B. Tribolium audax, Tribolium castaneum, Tribolium confusum, Trogoderma spp., Tychius spp., Xylotrechus spp., Zabrus spp., z. B. Zabrus tenebrioides;

aus der Ordnung der Dermaptera z. B. Anisolabis maritime, Forficula auricularia, Labidura riparia;

aus der Ordnung der Diptera z. B. Aedes spp., z. B. Aedes aegypti, Aedes albopictus, Aedes sticticus, Aedes vexans, Agromyza spp., z. B. Agromyza frontella, Agromyza parvicornis, Anastrepha spp., Anopheles spp., z. B. Anopheles quadrimaculatus, Anopheles gambiae, Asphondylia spp., Bactrocera spp., z. B. Bactrocera cucurbitae, Bactrocera dorsalis, Bactrocera oleae, Bibio hortulanus, Calliphora erythrocephala, Calliphora vicina, Ceratitis capitata, Chironomus spp., Chrysomya spp., Chrysops spp., Chrysozona pluvialis, Cochliomya spp., Contarinia spp., z. B. Contarinia johnsoni, Contarinia nasturtii, Contarinia pyrivora, Contarinia schulzi, Contarinia sorghicola, Contarinia tritici, Cordylobia anthropophaga, Cricotopus sylvestris, Culex spp., z. B. Culex pipiens, Culex quinquefasciatus, Culicoides spp., Culiseta spp., Cuterebra spp., Dacus oleae, Dasineura spp., z. B. Dasineura brassicae, Delia spp., z. B. Delia antiqua, Delia coarctata, Delia florilega, Delia platura, Delia radicum, Dermatobia hominis, Drosophila spp., z. B. Drosphila melanogaster, Drosophila suzukii, Echinocnemus spp., Euleia heraclei, Fannia spp., Gasterophilus spp., Glossina spp., Haematopota spp., Hydrellia spp., Hydrellia griseola, Hylemya spp., Hippobosca spp., Hypoderma spp., Liriomyza spp., z. B. Liriomyza brassicae, Liriomyza huidobrensis, Liriomyza sativae, Lucilia spp., z. B. Lucilia cuprina, Lutzomyia spp., Mansonia spp., Musca spp., z. B. Musca domestica, Musca domestica vicina, Oestrus spp., Oscinella frit, Paratanytarsus spp., Paralauterborniella subcincta, Pegomya oder Pegomyia spp., z. B. Pegomya betae, Pegomya hyoscyami, Pegomya rubivora, Phlebotomus spp., Phorbia spp., Phormia spp., Piophila casei, Platyparea poeciloptera, Prodiplosis spp., Psila rosae, Rhagoletis spp., z. B. Rhagoletis cingulata, Rhagoletis completa, Rhagoletis fausta, Rhagoletis indifferens, Rhagoletis mendax, Rhagoletis pomonella, Sarcophaga spp., Simulium spp., z. B. Simulium meridionale, Stomoxys spp., Tabanus spp., Tetanops spp., Tipula spp., z. B. Tipula paludosa, Tipula simplex, Toxotrypana curvicauda;

aus der Ordnung der Hemiptera z. B. Acizzia acaciaebaileyanae, Acizzia dodonaeae, Acizzia uncatoides, Acrida turrita, Acyrthosipon spp., z. B. Acyrthosiphon pisum, Acrogonia spp., Aeneolamia spp., Agonoscena spp., Aleurocanthus spp., Aleyrodes proletella, Aleurolobus barodensis, Aleurothrixus floccosus, Allocaridara malayensis, Amrasca spp., z. B. Amrasca bigutulla, Amrasca devastans, Anuraphis cardui, Aonidiella spp., z. B. Aonidiella aurantii, Aonidiella citrina, Aonidiella inornata, Aphanostigma piri, Aphis spp., z. B. Aphis citricola, Aphis craccivora, Aphis fabae, Aphis forbesi, Aphis glycines, Aphis gossypii, Aphis hederae, Aphis illinoisensis, Aphis middletoni, Aphis nasturtii, Aphis nerii, Aphis pomi, Aphis spiraecola, Aphis viburniphila, Arboridia apicalis, Arytainilla spp., Aspidiella spp., Aspidiotus spp., z. B. Aspidiotus nerii, Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., z. B. Cacopsylla pyricola, Calligypona marginata, Capulinia spp., Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus aonidum, Chrysomphalus ficus, Cicadulina mbila, Coccomytilus halli, Coccus spp., z. B. Coccus hesperidum, Coccus longulus, Coccus pseudomagnoliarum, Coccus viridis, Cryptomyzus ribis, Cryptoneossa spp., Ctenarytaina spp., Dalbulus spp., Dialeurodes chittendeni, Dialeurodes citri, Diaphorina citri, Diaspis spp., Diuraphis spp., Doralis spp., Drosicha spp., Dysaphis spp., z. B. Dysaphis apiifolia, Dysaphis plantaginea, Dysaphis tulipae, Dysmicoccus spp., Empoasca spp., z. B. Empoasca abrupta, Empoasca fabae, Empoasca maligna, Empoasca solana, Empoasca stevensi, Eriosoma spp., z. B. Eriosoma americanum, Eriosoma lanigerum, Eriosoma pyricola, Erythroneura spp., Eucalyptolyma spp., Euphyllura spp., Euscelis bilobatus, Ferrisia spp., Fiorinia spp., Furcaspis oceanica, Geococcus coffeae, Glycaspis spp., Heteropsylla cubana, Heteropsylla spinulosa, Homalodisca coagulata, Hyalopterus arundinis, Hyalopterus pruni, Icerya spp., z. B. Icerya purchasi, Idiocerus spp., Idioscopus spp., Laodelphax striatellus, Lecanium spp., z. B. Lecanium corni (=Parthenolecanium corni), Lepidosaphes spp., z. B. Lepidosaphes ulmi, Lipaphis erysimi, Lopholeucaspis japonica, Lycorma delicatula, Macrosiphum spp., z. B. Macrosiphum euphorbiae, Macrosiphum lilii, Macrosiphum rosae, Macrosteles facifrons, Mahanarva spp., Melanaphis sacchari, Metcalfiella spp., Metcalfa pruinosa, Metopolophium dirhodum, Monellia costalis, Monelliopsis pecanis, Myzus spp., z. B. Myzus ascalonicus, Myzus cerasi, Myzus ligustri, Myzus ornatus, Myzus persicae, Myzus nicotianae, Nasonovia ribisnigri, Neomaskellia spp., Nephotettix spp., z. B. Nephotettix cincticeps, Nephotettix nigropictus, Nettigoniclla spectra, Nilaparvata lugens, Oncometopia spp., Orthezia praelonga, Oxya chinensis, Pachypsylla spp., Parabemisia myricae, Paratrioza spp., z. B. Paratrioza cockerelli, Parlatoria spp., Pemphigus spp., z. B. Pemphigus bursarius, Pemphigus populivenae, Peregrinus maidis, Perkinsiella spp., Phenacoccus spp., z. B. Phenacoccus madeirensis, Phloeomyzus passerinii, Phorodon humuli, Phylloxera spp., z. B. Phylloxera devastatrix, Phylloxera notabilis, Pinnaspis aspidistrae, Planococcus spp., z. B. Planococcus citri, Prosopidopsylla flava, Protopulvinaria pyriformis, Pseudaulacaspis pentagona, Pseudococcus spp., z. B. Pseudococcus calceolariae, Pseudococcus comstocki, Pseudococcus longispinus, Pseudococcus maritimus, Pseudococcus viburni, Psyllopsis spp., Psylla spp., z. B. Psylla buxi, Psylla mali, Psylla pyri, Pteromalus spp., Pulvinaria spp., Pyrilla spp., Quadraspidiotus spp., z. B. Quadraspidiotus juglansregiae, Quadraspidiotus ostreaeformis, Quadraspidiotus perniciosus, Quesada gigas, Rastrococcus spp., Rhopalosiphum spp., z. B. Rhopalosiphum maidis, Rhopalosiphum oxyacanthae, Rhopalosiphum padi, Rhopalosiphum rufiabdominale, Saissetia spp., z. B. Saissetia coffeae, Saissetia miranda, Saissetia neglecta, Saissetia oleae, Scaphoideus titanus, Schizaphis graminum, Selenaspidus articulatus, Sipha flava, Sitobion avenae, Sogata spp., Sogatella furcifera, Sogatodes spp., Stictocephala festina, Siphoninus phillyreae, Tenalaphara malayensis, Tetragonocephela spp., Tinocallis caryaefoliae, Tomaspis spp., Toxoptera spp., z. B. Toxoptera aurantii, Toxoptera citricidus, Trialeurodes vaporariorum, Trioza spp., z. B. Trioza diospyri, Typhlocyba spp., Unaspis spp., Viteus vitifolii, Zygina spp.;

aus der Unterordnung der Heteroptera z. B. Aelia spp., Anasa tristis, Antestiopsis spp., Boisea spp., Blissus spp., Calocoris spp., Campylomma livida, Cavelerius spp., Cimex spp., z. B. Cimex adjunctus, Cimex hemipterus, Cimex lectularius, Cimex pilosellus, Collaria spp., Creontiades dilutus, Dasynus piperis, Dichelops furcatus, Diconocoris hewetti, Dysdercus spp., Euschistus spp., z. B. Euschistus heros, Euschistus servus, Euschistus tristigmus, Euschistus variolarius, Eurydema spp., Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygocoris spp., z. B. Lygocoris pabulinus, Lygus spp., z. B. Lygus elisus, Lygus hesperus, Lygus lineolaris, Macropes excavatus, Megacopta cribraria, Miridae, Monalonion atratum, Nezara spp., z. B. Nezara viridula, Nysius spp., Oebalus spp., Pentomidae, Piesma quadrata, Piezodorus spp., z. B. Piezodorus guildinii, Psallus spp., Pseudacysta persea, Rhodnius spp., Sahlbergella singularis, Scaptocoris castanea, Scotinophora spp., Stephanitis nashi, Tibraca spp., Triatoma spp.;

aus der Ordnung der Hymenoptera z. B. Acromyrmex spp., Athalia spp., z. B. Athalia rosae, Atta spp., Camponotus spp., Dolichovespula spp., Diprion spp., z. B. Diprion similis, Hoplocampa spp., z. B. Hoplocampa cookei, Hoplocampa testudinea, Lasius spp., Linepithema (Iridiomyrmex) humile, Monomorium pharaonis, Paratrechina spp., Paravespula spp., Plagiolepis spp., Sirex spp., z. B. Sirex noctilio, Solenopsis invicta, Tapinoma spp., Technomyrmex albipes, Urocerus spp., Vespa spp., z. B. Vespa crabro, Wasmannia auropunctata, Xeris spp.;

aus der Ordnung der Isopoda z. B. Armadillidium vulgare, Oniscus asellus, Porcellio scaber;

aus der Ordnung der Isoptera z. B. Coptotermes spp., z. B. Coptotermes formosanus, Cornitermes cumulans, Cryptotermes spp., Incisitermes spp., Kalotermes spp., Microtermes obesi, Nasutitermis spp., Odontotermes spp., Porotermes spp., Reticulitermes spp., z. B. Reticulitermes flavipes, Reticulitermes hesperus;

aus der Ordnung der Lepidoptera z. B. Achroia grisella, Acronicta major, Adoxophyes spp., z. B. Adoxophyes orana, Aedia leucomelas, Agrotis spp., z. B. Agrotis segetum, Agrotis ipsilon, Alabama spp., z. B. Alabama argillacea, Amyelois transitella, Anarsia spp., Anticarsia spp., z. B. Anticarsia gemmatalis, Argyroploce spp., Autographa spp., Barathra brassicae, Blastodacna atra, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., z. B. Chilo plejadellus, Chilo suppressalis, Choreutis pariana, Choristoneura spp., Chrysodeixis chalcites, Clysia ambiguella, Cnaphalocerus spp., Cnaphalocrocis medinalis, Cnephasia spp., Conopomorpha spp., Conotrachelus spp., Copitarsia spp., Cydia spp., z. B. Cydia nigricana, Cydia pomonella, Dalaca noctuides, Diaphania spp., Diparopsis spp., Diatraea saccharalis, Dioryctria spp., z. B. Dioryctria zimmermani, Earias spp., Ecdytolopha aurantium, Elasmopalpus lignosellus, Eldana saccharina, Ephestia spp., z. B. Ephestia elutella, Ephestia kuehniella, Epinotia spp., Epiphyas postvittana, Erannis spp., Erschoviella musculana, Etiella spp., Eudocima spp., Eulia spp., Eupoecilia ambiguella, Euproctis spp., z. B. Euproctis chrysorrhoea, Euxoa spp., Feltia spp., Galleria mellonella, Gracillaria spp., Grapholitha spp., z. B. Grapholita molesta, Grapholita prunivora, Hedylepta spp., Helicoverpa spp., z. B. Helicoverpa armigera, Helicoverpa zea, Heliothis spp., z. B. Heliothis virescens , Hofmannophila pseudospretella, Homoeosoma spp., Homona spp., Hyponomeuta padella, Kakivoria flavofasciata, Lampides spp., Laphygma spp., Laspeyresia molesta, Leucinodes orbonalis, Leucoptera spp., z. B. Leucoptera coffeella, Lithocolletis spp., z. B. Lithocolletis blancardella, Lithophane antennata, Lobesia spp., z. B. Lobesia botrana, Loxagrotis albicosta, Lymantria spp., z. B. Lymantria dispar, Lyonetia spp., z. B. Lyonetia clerkella, Malacosoma neustria, Maruca testulalis, Mamestra brassicae, Melanitis leda, Mocis spp., Monopis obviella, Mythimna separata, Nemapogon cloacellus, Nymphula spp., Oiketicus spp., Omphisa spp., Operophtera spp., Oria spp., Orthaga spp., Ostrinia spp., z. B. Ostrinia nubilalis, Panolis flammea, Parnara spp., Pectinophora spp., z. B. Pectinophora gossypiella, Perileucoptera spp., Phthorimaea spp., z. B. Phthorimaea operculella, Phyllocnistis citrella, Phyllonorycter spp., z.

B. Phyllonorycter blancardella, Phyllonorycter crataegella, Pieris spp., z. B. Pieris rapae, Platynota stultana, Plodia interpunctella, Plusia spp., Plutella xylostella (=Plutella maculipennis), Podesia spp., z. B. Podesia syringae, Prays spp., Prodenia spp., Protoparce spp., Pseudaletia spp., z. B. Pseudaletia unipuncta, Pseudoplusia includens, Pyrausta nubilalis, Rachiplusia nu, Schoenobius spp., z. B. Schoenobius bipunctifer, Scirpophaga spp., z. B. Scirpophaga innotata, Scotia segetum, Sesamia spp., z. B. Sesamia inferens, Sparganothis spp., Spodoptera spp., z. B. Spodoptera eradiana, Spodoptera exigua, Spodoptera frugiperda, Spodoptera praefica, Stathmopoda spp., Stenoma spp., Stomopteryx subsecivella, Synanthedon spp., Tecia solanivora, Thaumetopoea spp., Thermesia gemmatalis, Tinea cloacella, Tinea pellionella, Tineola bisselliella, Tortrix spp., Trichophaga tapetzella, Trichoplusia spp., z. B. Trichoplusia ni, Tryporyza incertulas, Tuta absoluta, Virachola spp.;

aus der Ordnung der Orthoptera oder Saltatoria z. B. Acheta domesticus, Dichroplus spp., Gryllotalpa spp., z. B. Gryllotalpa gryllotalpa, Hieroglyphus spp., Locusta spp., z. B. Locusta migratoria, Melanoplus spp., z. B. Melanoplus devastator, Paratlanticus ussuriensis, Schistocerca gregaria;

aus der Ordnung der Phthiraptera z. B. Damalinia spp., Haematopinus spp., Linognathus spp., Pediculus spp., Phylloxera vastatrix, Phthirus pubis, Trichodectes spp.;

aus der Ordnung der Psocoptera z. B. Lepinotus spp., Liposcelis spp.;

aus der Ordnung der Siphonaptera z. B. Ceratophyllus spp., Ctenocephalides spp., z. B. Ctenocephalides canis, Ctenocephalides felis, Pulex irritans, Tunga penetrans, Xenopsylla cheopis;

aus der Ordnung der Thysanoptera z. B. Anaphothrips obscurus, Baliothrips biformis, Chaetanaphothrips leeuweni, Drepanothrips reuteri, Enneothrips flavens, Frankliniella spp., z. B. Frankliniella fusca, Frankliniella occidentalis, Frankliniella schultzei, Frankliniella tritici, Frankliniella vaccinii, Frankliniella williamsi, Haplothrips spp., Heliothrips spp., Hercinothrips femoralis, Kakothrips spp., Rhipiphorothrips cruentatus, Scirtothrips spp., Taeniothrips cardamomi, Thrips spp., z. B. Thrips palmi, Thrips tabaci;

aus der Ordnung der Zygentoma (= Thysanura), z. B. Ctenolepisma spp., Lepisma saccharina, Lepismodes inquilinus, Thermobia domestica;

aus der Klasse der Symphyla z. B. Scutigerella spp., z. B. Scutigerella immaculata;

Schädlinge aus dem Stamm der Mollusca, z. B. aus der Klasse der Bivalvia, z. B. Dreissena spp.;

sowie aus der Klasse der Gastropoda z. B. Arion spp., z. B. Arion ater rufus, Biomphalaria spp., Bulinus spp., Deroceras spp., z. B. Deroceras laeve, Galba spp., Lymnaea spp., Oncomelania spp., Pomacea spp., Succinea spp.;

Pflanzenschädlinge aus dem Stamm der Nematoda, d. h. pflanzenparasitäre Nematoden, insbesondere Aglenchus spp., z. B. Aglenchus agricola, Anguina spp., z. B. Anguina tritici, Aphelenchoides spp., z. B. Aphelenchoides arachidis, Aphelenchoides fragariae, Belonolaimus spp., z. B. Belonolaimus gracilis, Belonolaimus longicaudatus, Belonolaimus nortoni, Bursaphelenchus spp., z. B. Bursaphelenchus cocophilus, Bursaphelenchus eremus, Bursaphelenchus xylophilus, Cacopaurus spp., z. B. Cacopaurus pestis, Criconemella spp., z. B. Criconemella curvata, Criconemella onoensis, Criconemella ornata, Criconemella rusium, Criconemella xenoplax (= Mesocriconema xenoplax), Criconemoides spp., z. B. Criconemoides ferniae, Criconemoides onoense, Criconemoides ornatum, Ditylenchus spp., z. B. Ditylenchus dipsaci, Dolichodorus spp., Globodera spp., z. B. Globodera pallida, Globodera rostochiensis, Helicotylenchus spp., z. B. Helicotylenchus dihystera, Hemicriconemoides spp., Hemicycliophora spp., Heterodera spp., z. B. Heterodera avenae, Heterodera glycines, Heterodera schachtii, Hirschmaniella spp., Hoplolaimus spp., Longidorus spp., z. B. Longidorus africanus, Meloidogyne spp., z. B. Meloidogyne chitwoodi, Meloidogyne fallax, Meloidogyne hapla, Meloidogyne incognita, Meloinema spp., Nacobbus spp., Neotylenchus spp., Paralongidorus spp., Paraphelenchus spp., Paratrichodorus spp., z. B. Paratrichodorus minor, Paratylenchus spp., Pratylenchus spp., z. B. Pratylenchus penetrans, Pseudohalenchus spp., Psilenchus spp., Punctodera spp., Quinisulcius spp., Radopholus spp., z. B. Radopholus citrophilus, Radopholus similis, Rotylenchulus spp., Rotylenchus spp., Scutellonema spp., Subanguina spp., Trichodorus spp., z. B. Trichodorus obtusus, Trichodorus primitivus, Tylenchorhynchus spp., z. B. Tylenchorhynchus annulatus, Tylenchulus spp., z. B. Tylenchulus semipenetrans, Xiphinema spp., z. B. Xiphinema index.

Die Verbindungen der Formel (I) können gegebenenfalls in bestimmten Konzentrationen bzw. Aufwandmengen

auch als Herbizide, Safener, Wachstumsregulatoren oder Mittel zur Verbesserung der Pflanzeneigenschaften, als Mikrobizide oder Gametozide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

**Formulierungen**

Die vorliegende Erfindung betrifft weiterhin Formulierungen und daraus bereitete Anwendungsformen als Schädlingsbekämpfungsmittel wie z. B. Drench-, Drip- und Spritzbrühen, umfassend mindestens eine Verbindung der Formel (I). Gegebenenfalls enthalten die Anwendungsformen weitere Schädlingsbekämpfungsmittel und/oder die Wirkung verbessernde Adjuvantien wie Penetrationsförderer, z. B. pflanzliche Öle wie beispielsweise Rapsöl, Sonnenblumenöl, Mineralöle wie beispielsweise Paraffinöle, Alkylester pflanzlicher Fettsäuren wie beispielsweise Rapsöl- oder Sojaölmethylester oder Alkanolalkoxylate und/oder Spreitmittel wie beispielsweise Alkylsiloxane und/oder Salze, z. B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar-Polymere und/oder Humectants wie z. B. Glycerin und/oder Dünger wie beispielsweise Ammonium, Kalium oder Phosphor enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einer oder mehreren Verbindungen der Formel (I) weitere agrochemische Wirkstoffe.

Vorzugsweise handelt es sich um Formulierungen oder Anwendungsformen, welche zumindest einen Hilfsstoff wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z. B. durch Vermischen der Verbindungen der Formel (I) mit Hilfsstoffen wie beispielsweise Streckmitteln, Lösemitteln und/oder festen Trägerstoffen und/oder weiteren Hilfsstoffen wie beispielsweise oberflächenaktiven Stoffen. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung der Verbindungen der Formel (I) oder den aus diesen Formulierungen bereiteten Anwendungsformen (wie z. B. gebrauchsfähigen Schädlingsbekämpfungsmitteln wie Spritzbrühen oder Saatgutbeizen) besondere Eigenschaften, wie bestimmte physikalische, technische und/oder biologische Eigenschaften zu verleihen.

Als Streckmittel eignen sich z. B. Wasser, polare und unpolare organische chemische Flüssigkeiten z. B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggfs. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z. B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe, wie Cyclohexan oder Paraffine, z. B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z. B. Xylol, Toluol oder Alkylnaphthaline, chlorierte aromatische oder chlorierte aliphatische Kohlenwasserstoffe wie z. B. Chlorbenzol, Chlorethylen, oder Methylenchlorid, aliphatische Kohlenwasserstoffe wie z. B. Cyclohexan, Paraffine, Erdölfraktionen, mineralische und pflanzliche Öle, Alkohole wie z. B. Methanol, Ethanol, iso-Propanol, Butanol oder Glykol sowie deren Ether und Ester, Ketone wie z. B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z. B. Ammoniumsalze und natürliche Gesteinsmehle wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und natürliche oder synthetische Silikate, Harze, Wachse und/oder feste Düngemittel. Mischungen solcher Trägerstoffe können ebenfalls verwendet werden. Als Trägerstoffe für Granulate kommen infrage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z. B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulfonsäure, Salze von Phenolsulfonsäure oder Naphthalinsulfonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulfobernsteinsäureestern, Taurinderivate (vorzugsweise Alkyltaurate), Phosphorsäureester von polyethoxylierten Alkoholen oder Phenolen, Fettsäureester von Polyolen und Derivate der Verbindungen enthaltend Sulfate, Sulfonate und Phosphate, z. B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn eine der Verbindungen der Formel (I) und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z. B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und/oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und pflanzliche Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Feuchthaltemittel, Spreitmittel. Im Allgemeinen können die Verbindungen der Formel (I) mit jedem festen oder flüssigen Zusatzstoff, welcher für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar-Polymere.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Beweglichkeit der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettaminalkoxylate wie beispielsweise Tallowamine-ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% der Verbindung der Formel (I), besonders bevorzugt zwischen 0,01 und 95 Gew.-% der Verbindung der Formel (I), ganz besonders bevorzugt zwischen 0,5 und 90 Gew.-% der Verbindung der Formel (I), bezogen auf das Gewicht der Formulierung.

Der Gehalt an der Verbindung der Formel (I) in den aus den Formulierungen bereiteten Anwendungsformen (insbesondere Schädlingsbekämpfungsmittel) kann in weiten Bereichen variieren. Die Konzentration der Verbindung der Formel (I) in den Anwendungsformen kann üblicherweise zwischen 0,00000001 und 95 Gew.-% der Verbindung der Formel (I), vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

**Mischungen**

Die Verbindungen der Formel (I) können auch in Mischung mit einem oder mehreren geeigneten Fungiziden, Bakteriziden, Akariziden, Molluskiziden, Nematiziden, Insektiziden, Mikrobiologika, Nützlingen, Herbiziden, Düngemitteln, Vogelrepellentien, Phytotonics, Sterilantien, Safenern, Semiochemicals und/oder Pflanzenwachstumsregulatoren verwendet werden, um so z. B. das Wirkungsspektrum zu verbreitern, die Wirkdauer zu verlängern, die Wirkgeschwindigkeit zu steigern, Repellenz zu verhindern oder Resistenzentwicklungen vorzubeugen. Des Weiteren können solche Wirkstoffkombinationen das Pflanzenwachstum und/oder die Toleranz gegenüber abiotischen Faktoren wie z. B. hohen oder niedrigen Temperaturen, gegen Trockenheit oder gegen erhöhten Wasser- bzw. Bodensalzgehalt verbessern. Auch lässt sich das Blüh- und Fruchtverhalten verbessern, die Keimfähigkeit und Bewurzelung optimieren, die Ernte erleichtern und Ernteertrag steigern, die Reife beeinflussen, die Qualität und/oder der Ernährungswert der Ernteprodukte steigern, die Lagerfähigkeit verlängern und/oder die Bearbeitbarkeit der Ernteprodukte verbessern.

Weiterhin können die Verbindungen der Formel (I) in Mischung mit weiteren Wirkstoffen oder Semiochemicals, wie Lockstoffen und/oder Vogelrepellentien und/oder Pflanzenaktivatoren und/oder Wachstumsregulatoren und/oder Düngemitteln vorliegen. Gleichfalls können die Verbindungen der Formel (I) zur Verbesserung der Pflanzeneigenschaften wie zum Beispiel Wuchs, Ertrag und Qualität des Erntegutes eingesetzt werden.

In einer besonderen erfindungsgemäßen Ausführungsform liegen die Verbindungen der Formel (I) in Formulierungen bzw. in den aus diesen Formulierungen bereiteten Anwendungsformen in Mischung mit weiteren Verbindungen vor, vorzugsweise solchen wie nachstehend beschrieben.

Wenn eine der im Folgenden genannten Verbindungen in verschiedenen tautomeren Formen vorkommen kann, sind auch diese Formen mit umfasst, auch wenn sie sie nicht in jedem Fall explizit genannt wurden. Alle genannten Mischungspartner können außerdem, wenn sie auf Grund ihrer funktionellen Gruppen dazu imstande sind, gegebenenfalls mit geeigneten Basen oder Säuren Salze bilden.

**Insektizide/Akarizide/Nematizide**

Die hier mit ihrem "Common Name" genannten Wirkstoffe sind bekannt und beispielsweise im Pestizidhandbuch ("The Pesticide Manual" 16th Ed., British Crop Protection Council 2012) beschrieben oder im Internet recherchierbar (z. B. http://www.alanwood.net/pesticides). Die Klassifizierung basiert auf dem zum Zeitpunkt der Einreichung dieser Patentanmeldung gültigen IRAC Mode of Action Classification Scheme.

(1) Acetylcholinesterase(AChE)-Inhibitoren, wie beispielsweise Carbamate, z. B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb oder Organophosphate, z. B. Acephat, Azamethiphos, Azinphos-ethyl, Azinphos-methyl, Cadusafos, Chlorethoxyfos, Chlorfenvinphos, Chlormephos, Chlorpyrifos-methyl, Coumaphos, Cyanophos, Demeton-S-methyl, Diazinon, Dichlorvos/DDVP, Dicrotophos, Dimethoat, Dimethylvinphos, Disulfoton, EPN, Ethion, Ethoprophos, Famphur, Fenamiphos, Fenitrothion, Fenthion, Fosthiazat, Heptenophos, Imicyafos, Isofenphos, Isopropyl-O-(methoxyaminothio-phosphoryl)salicylat, Isoxathion, Malathion, Mecarbam, Methamidophos, Methidathion, Mevinphos, Monocrotophos, Naled, Omethoate, Oxydemeton-methyl, Parathion-methyl, Phenthoat, Phorat, Phosalon, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.

(2) GABA-gesteuerte Chlorid-Kanal-Blocker, wie beispielsweise Cyclodien-organochlorine, z. B. Chlordan und Endosulfan oder Phenylpyrazole (Fiprole), z. B. Ethiprol und Fipronil.

(3) Natrium-Kanal-Modulatoren, wie beispielsweise Pyrethroide, z. B. Acrinathrin, Allethrin, d-cis-trans-Allethrin, d-trans-Allethrin, Bifenthrin, Bioallethrin, Bioallethrin-S-cyclopentenyl-Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomer], Deltamethrin, Empenthrin [(EZ)-(1R)-Isomer], Esfenvalerat, Etofenprox, Fenpropathrin, Fenvalerat, Flucythrinat, Flumethrin, tau-Fluvalinat, Halfenprox, Imiprothrin, Kadethrin, Momfluorothrin, Permethrin, Phenothrin [(1R)-trans-Isomer], Prallethrin, Pyrethrine (pyrethrum), Resmethrin, Silafluofen, Tefluthrin, Tetramethrin, Tetramethrin [(1R)-Isomer], Tralomethrin und Transfluthrin oder DDT oder Methoxychlor.

(4) Kompetitive Modulatoren des nicotinischen Acetylcholin-Rezeptors (nAChR), wie beispielsweise Neonicotinoide, z. B. Acetamiprid, Clothianidin, Dinotefuran, Imidacloprid, Nitenpyram, Thiacloprid und Thiamethoxam oder Nicotin

oder Sulfoxaflor oder Flupyradifurone.

(5) Allosterische Modulatoren des nicotinischen Acetylcholin-Rezeptors (nAChR), wie beispielsweise Spinosyne, z. B. Spinetoram und Spinosad.

(6) Allosterische Modulatoren des Glutamat-abhängigen Chloridkanals(GluC1), wie beispielsweise Avermectine/Milbemycine, z. B. Abamectin, Emamectin-benzoat, Lepimectin und Milbemectin.

(7) Juvenilhormon-Mimetika, wie beispielsweise Juvenilhormon-Analoge, z. B. Hydropren, Kinopren und Methopren oder Fenoxycarb oder Pyriproxyfen.

(8) Verschiedene nicht spezifische (multi-site) Inhibitoren, wie beispielsweise Alkylhalogenide, z. B. Methylbromid und andere Alkylhalogenide; oder Chloropicrin oder Sulfurylfluorid oder Borax oder Brechweinstein oder Methylisocyanaterzeuger, z. B. Diazomet und Metam.

(9) Modulatoren chordotonaler Organe, z. B. Pymetrozin oder Flonicamid.

(10) Milbenwachstumsinhibitoren, wie z. B. Clofentezin, Hexythiazox und Diflovidazin oder Etoxazol.

(11) Mikrobielle Disruptoren der Insektendarmmembran, wie z. B. *Bacillus thuringiensis* Subspezies *israelensis, Bacillus sphaericus, Bacillus thuringiensis* Subspezies *aizawai*, *Bacillus thuringiensis* Subspezies *kurstaki, Bacillus thuringiensis* Subspezies *tenebrionis* und B.t.-Pflanzenproteine: Cry1Ab, CrylAc, Cry1Fa, CrylA.105, Cry2Ab, VIP3A, mCry3A, Cry3Ab, Cry3Bb, Cry34Ab1/35Ab1.

(12) Inhibitoren der mitochondrialen ATP-Synthase, wie ATP-Disruptoren, wie beispielsweise Diafenthiuron oder Organozinnverbindungen, z. B. Azocyclotin, Cyhexatin und Fenbutatin-oxid oder Propargit oder Tetradifon.

(13) Entkoppler der oxidativen Phoshorylierung durch Störung des Protonengradienten, wie beispielsweise Chlorfenapyr, DNOC und Sulfluramid.

(14) Blocker des nicotinischen Acetylcholinrezeptorkanals, wie beispielsweise Bensultap, Cartaphydrochlorid, Thiocyclam und Thiosultap-Natrium.

(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.

(16) Inhibitoren der Chitinbiosynthese, Typ 1, wie beispielsweise Buprofezin.

(17) Häutungsdisruptor (insbesondere bei Dipteren, d. h. Zweiflüglern), wie beispielsweise Cyromazin.

(18) Ecdyson-Rezeptor-Agonisten, wie beispielsweise Chromafenozid, Halofenozid, Methoxyfenozid und Tebufenozid.

(19) Oktopamin-Rezeptor-Agonisten, wie beispielsweise Amitraz.

(20) Mitochondriale Komplex-III-Elektronentransportinhibitoren, wie beispielsweise Hydramethylnon oder Acequinocyl oder Fluacrypyrim.

(21) Mitochondriale Komplex-I-Elektronentransportinhibitoren, wie beispielsweise METI-Akarizide, z. B. Fenazaquin, Fenpyroximat, Pyrimidifen, Pyridaben, Tebufenpyrad und Tolfenpyrad oder Rotenon (Derris).

(22) Blocker des spannungsabhängigen Natriumkanals, wie z. B. Indoxacarb oder Metaflumizone.

(23) Inhibitoren der Acetyl-CoA-Carboxylase, wie beispielsweise Tetron- und Tetramsäurederivate, z. B. Spirodiclofen, Spiromesifen und Spirotetramat.

(24) Inhibitoren des mitochondrialen Komplex-IV-Elektronentransports, wie beispielsweise Phosphine, z. B. Aluminiumphosphid, Calciumphosphid, Phosphin und Zinkphosphid oder Cyanide, Calciumcyanid, Kaliumcyanid und

Natriumcyanid.

(25) Inhibitoren des mitochondrialen Komplex-II-Elektronentransports, wie beispielsweise beta-Ketonitrilderivate, z. B. Cyenopyrafen und Cyflumetofen und Carboxanilide, wie beispielsweise Pyflubumid.

(28) Ryanodinrezeptor-Modulatoren, wie beispielsweise Diamide, z. B. Chlorantraniliprol, Cyantraniliprol und Flubendiamid,

weitere Wirkstoffe wie beispielsweise Afidopyropen, Afoxolaner, Azadirachtin, Benclothiaz, Benzoximat, Bifenazat, Broflanilid, Bromopropylat, Chinomethionat, Chloroprallethrin, Cryolit, Cyclaniliprol, Cycloxaprid, Cyhalodiamid, Dicloromezotiaz, Dicofol, epsilon-Metofluthrin, epsilon-Momfluthrin, Flometoquin, Fluazaindolizin, Fluensulfon, Flufenerim, Flufenoxystrobin, Flufiprol, Fluhexafon, Fluopyram, Fluralaner, Fluxametamid, Fufenozid, Guadipyr, Heptafluthrin, Imidaclothiz, Iprodione, kappa-Bifenthrin, kappa-Tefluthrin, Lotilaner, Meperfluthrin, Paichongding, Pyridalyl, Pyrifluquinazon, Pyriminostrobin, Spirobudiclofen, Tetramethylfluthrin, Tetraniliprol, Tetrachlorantraniliprol, Tioxazafen, Thiofluoximat, Triflumezopyrim und Iodmethan; des Weiteren Präparate auf Basis von *Bacillus firmus* (I-1582, BioNeem, Votivo), sowie folgende Verbindungen: 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635) (CAS 885026-50-6), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457) (CAS 637360-23-7), 2-Chlor-N-[2-{1-[(2E)-3-(4-chlorphenyl)prop-2-en-1-yl]piperidin-4-yl}-4-(trifluormethyl)phenyl]isonicotinamid (bekannt aus WO2006/003494) (CAS 872999-66-1), 3-(4-Chlor-2,6-dimethylphenyl)-4-hydroxy-8-methoxy-1,8-diazaspiro[4.5]dec-3-en-2-on (bekannt aus WO 2010052161) (CAS 1225292-17-0), 3-(4-Chlor-2, 6-dimethylphenyl)-8-methoxy-2-oxo-1,8-diazaspiro[4.5]dec-3-en-4-yl-ethylcarbonat (bekannt aus EP 2647626) (CAS-1440516-42-6), 4-(But-2-in-1-yloxy)-6-(3,5-dimethylpiperidin-1-yl)-5-fluorpyrimidin (bekannt aus WO2004/099160) (CAS 792914-58-0), PF1364 (bekannt aus JP2010/018586) (CAS-Reg.No. 1204776-60-2), N-[(2E)-1-[(6-Chlorpyridin-3-yl)methyl]pyridin-2(1H)-yliden]-2,2,2-trifluoracetamid (bekannt aus WO2012/029672) (CAS 1363400-41-2), (3E)-3-[1-[(6-Chlor-3-pyridyl)methyl]-2-pyridyliden]-1,1,1-trifluorpropan-2-on (bekannt aus WO2013/144213) (CAS 1461743-15-6), N-[3-(Benzylcarbamoyl)-4-chlorphenyl]-1-methyl-3-(pentafluorethyl)-4-(trifluormethyl)-1H-pyrazol-5-carboxamid (bekannt aus WO2010/051926) (CAS 1226889-14-0), 5-Brom-4-chlor-N-[4-chlor-2-methyl-6-(methylcarbamoyl)phenyl]-2-(3-chlor-2-pyridyl)pyrazol-3-carboxamid (bekannt aus CN103232431) (CAS 1449220-44-3), 4-[5-(3,5-Dichlorphenyl)-4,5-dihydro-5-(trifluormethyl)-3-isoxazolyl]-2-methyl-N-(cis-1-oxido-3-thietanyl)benzamid, 4-[5-(3,5-Dichlorphenyl)-4,5-dihydro-5-(trifluormethyl)-3-isoxazolyl]-2-methyl-N-(trans-1-oxido-3-thietanyl)benzamid und 4-[(5S)-5-(3,5-Dichlorphenyl)-4,5-dihydro-5-(trifluormethyl)-3-isoxazolyl]-2-methyl-N-(cis-1-oxido-3-thietanyl)benzamid (bekannt aus WO 2013/050317 A1) (CAS 1332628-83-7), N-[3-Chlor-1-(3-pyridinyl)-1H-pyrazol-4-yl]-N-ethyl-3-[(3,3,3-trifluorpropyl)sulfinyl]propanamid, (+)-N-[3-Chlor-1-(3-pyridinyl)-1H-pyrazol-4-yl]-N-ethyl-3-[(3,3,3-trifluorpropyl)sulfinyl]propanamid und (-)-N-[3-Chlor-1-(3-pyridinyl)-1H-pyrazol-4-yl]-N-ethyl-3-[(3,3,3-trifluorpropyl)sulfinyl]propanamid (bekannt aus WO 2013/162715 A2, WO 2013/162716 A2, US 2014/0213448 A1) (CAS 1477923-37-7), 5-[[(2E)-3-Chlor-2-propen-1-yl]amino]-1-[2,6-dichlor-4-(trifluormethyl)phenyl]-4-[(trifluormethyl)sulfinyl]-1H-pyrazol-3-carbonitrile (bekannt aus CN 101337937 A) (CAS 1105672-77-2), 3-Brom-N-[4-chlor-2-methyl-6-[(methylamino)thioxomethyl]phenyl]-1-(3-chlor-2-pyridinyl)-1H-pyrazol-5-carboxamid, (Liudaibenjiaxuanan, bekannt aus CN 103109816 A) (CAS 1232543-85-9); N-[4-Chlor-2-[[(1,1-dimethylethyl)amino]carbonyl]-6-methylphenyl]-1-(3-chlor-2-pyridinyl)-3-(fluormethoxy)-1H-pyrazol-5-carboxamid (bekannt aus WO 2012/034403 A1) (CAS 1268277-22-0), N-[2-(5-Amino-1,3,4-thiadiazol-2-yl)-4-chlor-6-methylphenyl]-3-brom-1-(3-chlor-2-pyridinyl)-1H-pyrazol-5-carboxamid (bekannt aus WO 2011/085575 A1) (CAS 1233882-22-8), 4-[3-[2,6-Dichlor-4-[(3,3-dichlor-2-propen-1-yl)oxy]phenoxy]propoxy]-2-methoxy-6-(trifluormethyl)pyrimidin (bekannt aus CN 101337940 A) (CAS 1108184-52-6); (2E)- und 2(Z)-2-[2-(4-Cyanophenyl)-1-[3-(trifluormethyl)phenyl]ethyliden]-N-[4-(difluormethoxy)phenyl]hydrazincarboxamid (bekannt aus CN 101715774 A) (CAS 1232543-85-9); Cyclopropancarbonsäure-3-(2,2-dichlorethenyl)-2,2-dimethyl-4-(1H-benzimidazol-2-yl)phenylester (bekannt aus CN 103524422 A) (CAS 1542271-46-4); (4aS)-7-Chlor-2,5-dihydro-2-[[(methoxycarbonyl)[4-[(trifluormethyl)thio]phenyl]amino]carbonyl]indeno[1,2-e][1,3,4]oxadiazin-4a(3H)-carbonsäuremethylester (bekannt aus CN 102391261 A) (CAS 1370358-69-2); 6-Desoxy-3-O-ethyl-2,4-di-O-methyl-1-[N-[4-[1-[4-(1,1,2,2,2-pentafluorethoxy)phenyl]-1H-1,2,4-triazol-3-yl]phenyl]carbamat]-α-L-mannopyranose (bekannt aus US 2014/0275503 A1) (CAS 1181213-14-8); 8-(2-Cyclopropylmethoxy-4-trifluormethylphenoxy)-3-(6-trifluormethylpyridazin-3-yl)-3-azabicyclo[3.2.1]octan (CAS 1253850-56-4), (8-anti)-8-(2-Cyclopropylmethoxy-4-trifluormethylphenoxy)-3-(6-trifluormethylpyridazin-3-yl)-3-azabicyclo[3.2.1]octan (CAS 933798-27-7), (8-syn)-8-(2-Cyclopropylmethoxy-4-trifluormethylphenoxy)-3-(6-trifluormethylpyridazin-3-yl)-3-azabicyclo[3.2.1]octan (bekannt aus WO 2007040280 A1, WO 2007040282 A1) (CAS 934001-66-8), N-[3-Chlor-1-(3-pyridinyl)-1H-pyrazol-4-yl]-N-ethyl-3-[(3,3,3-trifluorpropyl)thio]-propanamid (bekannt aus WO 2015/058021 A1, WO 2015/058028 A1) (CAS 1477919-27-9) und N-[4-(Aminothioxomethyl)-2-methyl-6-[(methylamino)carbonyl]phenyl]-3-bromo-1-(3-chloro-2-pyridinyl)-1H-pyrazol-5-carboxamid (bekannt aus CN 103265527 A) (CAS 1452877-50-7).

**Fungizide**

Die hier mit ihrem "Common Name" spezifizierten Wirkstoffe sind bekannt und beispielsweise im "Pesticide Manual" (16. Aufl. British Crop Protection Council) oder im Internet recherchierbar (beispielsweise: http://www.alanwood.net/pesticides) beschrieben.

Alle genannten Mischungspartner der Klassen (1) bis (15) können, wenn sie auf Grund ihrer funktionellen Gruppen dazu imstande sind, gegebenenfalls mit geeigneten Basen oder Säuren Salze bilden. Alle genannten fungiziden Mischungspartner der Klassen (1) bis (15) können gegebenenfalls tautomere Formen einschließen.

1) Inhibitoren der Ergosterol-Biosynthese, beispielsweise (1.001) Cyproconazol, (1.002) Difenoconazol, (1.003) Epoxiconazol, (1.004) Fenhexamid, (1.005) Fenpropidin, (1.006) Fenpropimorph, (1.007) Fenpyrazamin, (1.008) Fluquinconazol, (1.009) Flutriafol, (1.010) Imazalil, (1.011) Imazalil Sulfat, (1.012) Ipconazol, (1.013) Metconazol, (1.014) Myclobutanil, (1.015) Paclobutrazol, (1.016) Prochloraz, (1.017) Propiconazol, (1.018) Prothioconazol, (1.019) Pyrisoxazol, (1.020) Spiroxamin, (1.021) Tebuconazol, (1.022) Tetraconazol, (1.023) Triadimenol, (1.024) Tridemorph, (1.025) Triticonazol, (1.026) (1R,2S,5S)-5-(4-Chlorbenzyl)-2-(chlormethyl)-2-methyl-1-(1H-1,2,4-triazol-1-ylmethyl)cyclopentanol, (1.027) (1S,2R,5R)-5-(4-Chlorbenzyl)-2-(chlormethyl)-2-methyl-1-(1H-1,2,4-triazol-1-ylmethyl)cyclopentanol, (1.028) (2R)-2-(1-Chlorcyclopropyl)-4-[(1R)-2,2-dichlorcyclopropyl]-1-(1H-1,2,4-triazol-1-yl)butan-2-ol (1.029) (2R)-2-(1-Chlorcyclopropyl)-4-[(1S)-2,2-dichlorcyclopropyl]-1-(1H-1,2,4-triazol-1-yl)butan-2-ol, (1.030) (2R)-2-[4-(4-Chlorphenoxy)-2-(trifluormethyl)phenyl]-1-(1H-1,2,4-triazol-1-yl)propan-2-ol, (1.031) (2S)-2-(1-Chlorcyclopropyl)-4-[(1R)-2,2-dichlorcyclopropyl]-1-(1H-1,2,4-triazol-1-yl)butan-2-ol, (1.032) (2S)-2-(1-Chlorcyclopropyl)-4-[(1S)-2,2-dichlorcyclopropyl]-1-(1H-1,2,4-triazol-1-yl)butan-2-ol, (1.033) (2S)-2-[4-(4-Chlorphenoxy)-2-(trifluormethyl)phenyl]-1-(1H-1,2,4-triazol-1-yl)propan-2-ol, (1.034) (R)-[3-(4-Chlor-2-fluorphenyl)-5-(2,4-difluorphenyl)-1,2-oxazol-4-yl](pyridin-3-yl)methanol, (1.035) (S)-[3-(4-Chlor-2-fluorphenyl)-5-(2,4-difluorphenyl)-1,2-oxazol-4-yl](pyridin-3-yl)methanol, (1.036) [3-(4-Chlor-2-fluorphenyl)-5-(2,4-difluorphenyl)-1,2-oxazol-4-yl](pyridin-3-yl)methanol, (1.037) 1-({(2R,4S)-2-[2-Chlor-4-(4-chlorphenoxy)phenyl]-4-methyl-1,3-dioxolan-2-yl}methyl)-1H-1,2,4-triazol, (1.038) 1-({(2S,4S)-2-[2-Chlor-4-(4-chlorphenoxy)phenyl]-4-methyl-1,3-dioxolan-2-yl}methyl)-1H-1,2,4-triazol, (1.039) 1-{[3-(2-Chlorphenyl)-2-(2,4-difluorphenyl)oxiran-2-yl]methyl}-1H-1,2,4-triazol-5-yl-thiocyanat, (1.040) 1-{[rel(2R,3R)-3-(2-Chlorphenyl)-2-(2,4-difluorphenyl)oxiran-2-yl]methyl}-1H-1,2,4-triazol-5-yl-thiocyanat, (1.041) 1-{[rel(2R,3S)-3-(2-Chlorphenyl)-2-(2,4-difluorphenyl)oxiran-2-yl]methyl}-1H-1,2,4-triazol-5-yl-thiocyanat, (1.042) 2-[(2R,4R,5R)-1-(2,4-Dichlorphenyl)-5-hydroxy-2,6,6-trimethylheptan-4-yl]-2,4-dihydro-3H-1,2,4-triazol-3-thion, (1.043) 2-[(2R,4R,5S)-1-(2,4-Dichlorphenyl)-5-hydroxy-2,6,6-trimethylheptan-4-yl]-2,4-dihydro-3H-1,2,4-triazol-3-thion, (1.044) 2-[(2R,4S,5R)-1-(2,4-Dichlorophenyl)-5-hydroxy-2,6,6-trimethylheptan-4-yl]-2,4-dihydro-3H-1,2,4-triazol-3-thion, (1.045) 2-[(2R,4S,5S)-1-(2,4-Dichlorphenyl)-5-hydroxy-2,6,6-trimethylheptan-4-yl]-2,4-dihydro-3H-1,2,4-triazol-3-thion, (1.046) 2-[(2S,4R,5R)-1-(2,4-Dichlorphenyl)-5-hydroxy-2,6,6-trimethylheptan-4-yl]-2,4-dihydro-3H-1,2,4-triazol-3-thion, (1.047) 2-[(2S,4R,SS)-1-(2,4-Dichlorphenyl)-5-hydroxy-2,6,6-trimethylheptan-4-yl]-2,4-dihydro-3H-1,2,4-triazol-3-thion, (1.048) 2-[(2S,4S,SR)-1-(2,4-Dichlorphenyl)-5-hydroxy-2,6,6-trimethylheptan-4-yl]-2,4-dihydro-3H-1,2,4-triazol-3-thion, (1.049) 2-[(2S,4S,5S)-1-(2,4-Dichlorphenyl)-5-hydroxy-2,6,6-trimethylheptan-4-yl]-2,4-dihydro-3H-1,2,4-triazol-3-thion, (1.050) 2-[1-(2,4-Dichlorphenyl)-5-hydroxy-2,6,6-trimethylheptan-4-yl]-2,4-dihydro-3H-1,2,4-triazol-3-thion, (1.051) 2-[2-Chlor-4-(2,4-dichlorophenoxy)phenyl]-1-(1H-1,2,4-triazol-1-yl)propan-2-ol, (1.052) 2-[2-Chlor-4-(4-chlorphenoxy)phenyl]-1-(1H-1,2,4-triazol-1-yl)butan-2-ol, (1.053) 2-[4-(4-Chlorphenoxy)-2-(trifluormethyl)phenyl]-1-(1H-1,2,4-triazol-1-yl)butan-2-ol, (1.054) 2- [4-(4-Chlorphenoxy)-2-(trifluormethyl)phenyl]-1-(1H-1,2,4-triazol-1-yl)pentan-2-ol, (1.055) 2-[4-(4-Chlorphenoxy)-2-(trifluormethyl)phenyl]-1-(1H-1,2,4-triazol-1-yl)propan-2-ol, (1.056) 2-{[3-(2-Chlorphenyl)-2-(2,4-difluorphenyl)oxiran-2-yl]methyl}-2,4-dihydro-3H-1,2,4-triazol-3-thion, (1.057) 2-{[rel(2R,3R)-3-(2-Chlorphenyl)-2-(2,4-difluorphenyl)oxiran-2-yl]methyl]-2,4-dihydro-3H-1,2,4-triazol-3-thion, (1.058) 2-{[rel(2R,3S)-3-(2-Chlorphenyl)-2-(2,4-dinuorphenyl)oxiran-2-yl]methyl}-2,4-dihydro-3H-1,2,4-triazol-3-thion, (1.059) 5-(4-Chlorbenzyl)-2-(chlormethyl)-2-methyl-1-(1H-1,2,4-triazol-1-ylmethyl)cyclopentanol, (1.060) 5-(Allylsulfanyl)-1-{[3-(2-chlorphenyl)-2-(2,4-difluorphenyl)oxiran-2-yl]methyl}-1H-1,2,4-triazol, (1.061) 5-(Allylsulfanyl)-1-{[rel(2R,3R)-3-(2-chlorphenyl)-2-(2,4-difluorphenyl)oxiran-2-yl]methyl} -1H-1,2,4-triazol, (1.062) 5-(Allylsulfanyl)-1-{[rel(2R,3S)-3-(2-chlorphenyl)-2-(2,4-difluorphenyl)oxiran-2-yl]methyl}-1H-1,2,4-triazol, (1.063) N'-(2,5-Dimethyl-4-{[3-(1,1,2,2-tetrafluorethoxy)phenyl]sulfanyl}phenyl)-N-ethyl-N-methylimidoformamid, (1.064) N'-(2,5-Dimethyl-4-{[3-(2,2,2-trifluorethoxy)phenyl]sulfanyl}phenyl)-N-ethyl-N-methylimidoformamid, (1.065) N'-(2,5-Dimethyl-4-{[3-(2,2,3,3-tetrafluorpropoxy)phenyl]sulfanyl}phenyl)-N-ethyl-N-methylimidoformamid, (1.066) N'-(2,5-Dimethyl-4-{[3-(pentafluorethoxy)phenyl]sulfanyl}phenyl)-N-ethyl-N-methylimidoformamid, (1.067) N'-(2,5-Dimethyl-4-{3-[(1,1,2,2-tetrafluorethyl)sulfanyl]phenoxy}phenyl)-N-ethyl-N-methylimidoformamid, (1.068) N'-(2,5-Dimethyl-4-{3-[(2,2,2-trifluorethyl)sulfanyl]phenoxy}phenyl)-N-ethyl-N-methylimidoformamid, (1.069) N'-(2,5-Dimethyl-4-{3-[(2,2,3,3-tetrafluorpropyl)sulfanyl]phenoxy}phenyl)-N-ethyl-N-methylimidoformamid, (1.070) N'-(2,5-Dimethyl-4-{3-[(pentafluorethyl)sulfanyl]phenoxy}phenyl)-N-ethyl-N-methylimidoformamid, (1.071) N'-(2,5-Dimethyl-4-phenoxyphenyl)-N-ethyl-N-methylimidoformamid, (1.072)

N'-(4-{ [3-(Difluormethoxy)phenyl]sulfanyl}-2,5-dimethylphenyl)-N-ethyl-N-methylimidoformamid, (1.073) N'-(4-{3-[(Difluormethyl)sulfanyl]phenoxy}-2,5-dimethylphenyl)-N-ethyl-N-methylimidoformamid, (1.074) N'-[5-Brom-6-(2,3-dihydro-1H-inden-2-yloxy)-2-methylpyridin-3-yl]-N-ethyl-N-methylimidoformamid, (1.075) N'- {4-[(4,5-Dichlor-1,3 -thiazol-2-yl)oxy] -2,5 -dimethylphenyl}-N-ethyl-N-methylimidoformamid, (1.076) N'-{5-Brom-6-[(1R)-1-(3,5-difluorophenyl)ethoxy]-2-methylpyridin-3-yl}-N-ethyl-N-methylimidoformamid, (1.077) N'-{5-Brom-6-[(1S)-1-(3,5-difluorphenyl)ethoxy]-2-methylpyridin-3-yl}-N-ethyl-N-methylimidoformamid, (1.078) N'-{5-Brom-6-[(cis-4-isopropylcyclohexyl)oxy]-2-methylpyridin-3-yl}-N-ethyl-N-methylimidoformamid, (1.079) N'- {5 -Brom-6- [(trans-4-isopropylcyclohexyl)oxy] -2-methylpyridin-3-yl} -N-ethyl-N-methylimidoformamid, (1.080) N'-{5-Bromo-6-[1-(3,5-difluorphenyl)ethoxy]-2-methylpyridin-3-yl} -N-ethyl-N-methylimidoformamid, (1.081) Mefentrifluconazole, (1.082) Ipfentrifluconazole.

2) Inhibitoren der Atmungskette am Komplex I oder II beispielsweise (2.001) Benzovindiflupyr, (2.002) Bixafen, (2.003) Boscalid, (2.004) Carboxin, (2.005) Fluopyram, (2.006) Flutolanil, (2.007) Fluxapyroxad, (2.008) Furametpyr, (2.009) Isofetamid, (2.010) Isopyrazam (anti-epimeres Enantiomer 1R,4S,9S), (2.011) Isopyrazam (anti-epimeres Enantiomer 1S,4R,9R), (2.012) Isopyrazam (anti-epimeres Racemat 1RS,4SR,9SR), (2.013) Isopyrazam (Mischung des syn-epimeren Razemates 1RS,4SR,9RS und des antiepimeren Razemates 1RS,4SR,9SR), (2.014) Isopyrazam (syn-epimeres Enantiomer 1R,4S,9R), (2.015) Isopyrazam (syn-epimeres Enantiomer 1S,4R,9S), (2.016) Isopyrazam (syn-epimeres Racemat 1RS,4SR,9RS), (2.017) Penflufen, (2.018) Penthiopyrad, (2.019) Pydiflumetofen, (2.020) Pyraziflumid, (2.021) Sedaxane, (2.022) 1,3-Dimethyl-N-(1,1,3-trimethyl-2,3-dihydro-1H-inden-4-yl)-1H-pyrazol-4-carboxamid, (2.023) 1,3-Dimethyl-N-[(3R)-1,1,3-trimethyl-2,3-dihydro-1H-inden-4-yl]-1H-pyrazol-4-carboxamid, (2.024) 1,3-Dimethyl-N-[(3S)-1,1,3-trimethyl-2,3-dihydro-1H-inden-4-yl]-1H-pyrazol-4-carboxamid, (2.025) 1-Methyl-3-(trifluormethyl)-N-[2'-(trifluormethyl)biphenyl-2-yl]-1H-pyrazol-4-carboxamid, (2.026) 2-Fluor-6-(trifluoromethyl)-N-(1,1,3-trimethyl-2,3-dihydro-1H-inden-4-yl)benzamid, (2.027) 3-(Difluormethyl)-1-methyl-N-(1,1,3-trimethyl-2,3-dihydro-1H-inden-4-yl)-1H-pyrazol-4-carboxamid, (2.028) 3-(Difluormethyl)-1-methyl-N-[(3R)-1,1,3-trimethyl-2,3-dihydro-1H-inden-4-yl]-1H-pyrazol-4-carboxamid, (2.029) 3-(Difluormethyl)-1-methyl-N-[(3S)-1,1,3-trimethyl-2,3-dihydro-1H-inden-4-yl]-1H-pyrazol-4-carboxamid, (2.030) 3-(Difluormethyl)-N-(7-fluor-1,1,3-trimethyl-2,3-dihydro-1H-inden-4-yl)-1-methyl-1H-pyrazol-4-carboxamid, (2.031) 3-(Difluormethyl)-N-[(3R)-7-fluor-1,1,3-trimethyl-2,3-dihydro-1H-inden-4-yl]-1-methyl-1H-pyrazol-4-carboxamid, (2.032) 3-(Difluoromethyl)-N-[(3S)-7-fluor-1,1,3-trimethyl-2,3-dihydro-1H-inden-4-yl]-1-methyl-1H-pyrazol-4-carboxamid, (2.033) 5,8-Difluor-N-[2-(2-fluor-4-{[4-(trifluormethyl)pyridin-2-yl]oxy}phenyl)ethyl]quinazolin-4-amin, (2.034) N-(2-Cyclopentyl-5-fluorbenzyl)-N-cyclopropyl-3-(difluormethyl)-5-fluor-1-methyl-1H-pyrazol-4-carboxamid, (2.035) N-(2-tert-Butyl-5-methylbenzyl)-N-cyclopropyl-3-(difluormethyl)-5-fluor-1-methyl-1H-pyrazol-4-carboxamid, (2.036) N-(2-tert-Butylbenzyl)-N-cyclopropyl-3-(difluormethyl)-5-fluor-1-methyl-1H-pyrazol-4-carboxamid, (2.037) N-(5-Chlor-2-ethylbenzyl)-N-cyclopropyl-3-(difluormethyl)-5-fluor-1-methyl-1H-pyrazol-4-carboxamid, (2.038) N-(5-Chlor-2-isopropylbenzyl)-N-cyclopropyl-3-(difluormethyl)-5-fluor-1-methy|-1H-pyrazo|-4-carboxamid, (2.039) N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid, (2.040) N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid, (2.041) N-[1-(2,4-Dichlorphenyl)-1-methoxypropan-2-yl]-3-(difluormethyl)-1-methyl-1H-pyrazol-4-carboxamid, (2.042) N-[2-Chlor-6-(trifluormethyl)benzyl]-N-cyclopropyl-3-(difluormethyl)-5-fluor-1-methyl-1H-pyrazol-4-carboxamid, (2.043) N-[3-Chlor-2-fluor-6-(trifluormethyl)benzyl]-N-cyclopropyl-3-(difluormethyl)-5-fluor-1-methyl-1H-pyrazol-4-carboxamid, (2.044) N-[5-Chlor-2-(trifluormethyl)benzyl]-N-cyclopropyl-3-(difluormethyl)-5-fluor-1-methyl-1H-pyrazol-4-carboxamid, (2.045) N-Cyclopropyl-3-(difluormethyl)-5-fluor-1-methyl-N-[5-methyl-2-(trifluormethyl)benzyl]-1H-pyrazol-4-carboxamid, (2.046) N-Cyclopropyl-3-(difluormethyl)-5-fluor-N-(2-fluor-6-isopropylbenzyl)-1-methyl-1H-pyrazol-4-carboxamid, (2.047) N-Cyclopropyl-3-(difluormethyl)-5-fluor-N-(2-isopropyl-5-methylbenzyl)-1-methyl-1H-pyrazol-4-carboxamid, (2.048) N-Cyclopropyl-3-(difluormethyl)-5-fluor-N-(2-isopropylbenzyl)-1-methyl-1H-pyrazol-4-carbothioamid, (2.049) N-Cyclopropyl-3-(difluoromethyl)-5-fluor-N-(2-isopropylbenzyl)-1-methyl-1H-pyrazol-4-carboxamid, (2.050) N-Cyclopropyl-3-(difluormethyl)-5-fluor-N-(5-fluor-2-isopropylbenzyl)-1-methyl-1H-pyrazol-4-carboxamid, (2.051) N-Cyclopropyl-3-(difluormethyl)-N-(2-ethyl-4,5-dimethylbenzyl)-5-fluor-1-methyl-1H-pyrazol-4-carboxamid, (2.052) N-Cyclopropyl-3-(difluormethyl)-N-(2-ethyl-5-fluorbenzyl)-5-fluor-1-methyl-1H-pyrazol-4-carboxamid, (2.053) N-Cyclopropyl-3-(difluormethyl)-N-(2-ethyl-5-methylbenzyl)-5-fluor-1-methyl-1H-pyrazole-4-carboxamid, (2.054) N-Cyclopropyl-N-(2-cyclopropyl-5-fluorbenzyl)-3-(difluormethyl)-5-fluor-1-methyl-1H-pyrazole-4-carboxamid, (2.055) N-Cyclopropyl-N-(2-cyclopropyl-5-methylbenzyl)-3-(difluormethyl)-5-fluor-1-methyl-1H-pyrazole-4-carboxamid, (2.056) N-Cyclopropyl-N-(2-cyclopropylbenzyl)-3-(difluormethyl)-5-fluor-1-methyl-1H-pyrazole-4-carboxamid.

3) Inhibitoren der Atmungskette am Komplex III, beispielsweise (3.001) Ametoctradin, (3.002) Amisulbrom, (3.003) Azoxystrobin, (3.004) Coumethoxystrobin, (3.005) Coumoxystrobin, (3.006) Cyazofamid, (3.007) Dimoxystrobin, (3.008) Enoxastrobin, (3.009) Famoxadon, (3.010) Fenamidon, (3.011) Flufenoxystrobin, (3.012) Fluoxastrobin,

(3.013) Kresoxim-Methyl, (3.014) Metominostrobin, (3.015) Orysastrobin, (3.016) Picoxystrobin, (3.017) Pyraclostrobin, (3.018) Pyrametostrobin, (3.019) Pyraoxystrobin, (3.020) Trifloxystrobin (3.021) (2E)-2-{2-[({[(1E)-1-(3-{[(E)-1-Fluor-2-phenylvinyl]oxy}phenyl)ethyliden]amino}oxy)methyl]phenyl}-2-(methoxyimino)-N-methylacetamid, (3.022) (2E,3Z)-5-{[1-(4-Chlorphenyl)-1H-pyrazol-3-yl]oxy}-2-(methoxyimino)-N,3-dimethylpent-3-enamid, (3.023) (2R)-2-{2-[(2,5-Dimethylphenoxy)methyl]phenyl}-2-methoxy-N-methylacetamid, (3.024) (2S)-2-{2-[(2,5-Dimethylphenoxy)methyl]phenyl}-2-methoxy-N-methylacetamid, (3.025) (3S,6S,7R,8R)-8-Benzyl-3-[(13-[(isobutyryloxy)methoxy]-4-methoxypyridin-2-yllcarbonyl)amino]-6-methyl-4,9-dioxo-1,5-dioxonan-7-yl-2-methylpropanoat, (3.026) 2-{2-[(2,5-Dimethylphenoxy)methyl]phenyl}-2-methoxy-N-methylacetamid, (3.027) N-(3-Ethyl-3,5,5-trimethylcyclohexyl)-3-formamido-2-hydroxybenzamid, (3.028) (2E,3Z)-5-{ [1-(4-Chlor-2-fluorphenyl)-1H-pyrazol-3-yl]oxy}-2-(methoxyimino)-N,3-dimethylpent-3-enamid, (3.029) Methyl {5-[3-(2,4-dimethylphenyl)-1H-pyrazol-1-yl]-2-methylbenzyl}carbamate.

4) Inhibitoren der Mitose und Zellteilung, beispielsweise (4.001) Carbendazim, (4.002) Diethofencarb, (4.003) Ethaboxam, (4.004) Fluopicolid, (4.005) Pencycuron, (4.006) Thiabendazol, (4.007) Thiophanat-Methyl, (4.008) Zoxamid, , (4.009) 3-Chlor-4-(2,6-difluorphenyl)-6-methyl-5-phenylpyridazin, (4.010) 3-Chlor-5-(4-chlorphenyl)-4-(2,6-difluorphenyl)-6-methylpyridazin, (4.011) 3-Chlor-5-(6-chlorpyridin-3-yl)-6-methyl-4-(2,4,6-trifluorphenyl)pyridazin, (4.012) 4-(2-Brom-4-fluorphenyl)-N-(2,6-difluorphenyl)-1,3-dimethyl-1H-pyrazol-5-amin, (4.013) 4-(2-Brom-4-fluorphenyl)-N-(2-brom-6-fluorphenyl)-1,3-dimethyl-1H-pyrazol-5-amin, (4.014) 4-(2-Brom-4-fluorphenyl)-N-(2-bromphenyl)-1,3-dimethyl-1H-pyrazol-5-amin, (4.015) 4-(2-Brom-4-fluorphenyl)-N-(2-chlor-6-fluorphenyl)-1,3-dimethyl-1H-pyrazol-5-amin, (4.016) 4-(2-Brom-4-fluorphenyl)-N-(2-chlorphenyl)-1,3-dimethyl-1H-pyrazol-5-amin, (4.017) 4-(2-Brom-4-fluorphenyl)-N-(2-fluorphenyl)-1,3-dimethyl-1H-pyrazol-5-amin, (4.018) 4-(2-Chlor-4-fluorphenyl)-N-(2,6-difluorphenyl)-1,3-dimethyl-1H-pyrazol-5-amin, (4.019) 4-(2-Chlor-4-fluorphenyl)-N-(2-chlor-6-fluorphenyl)-1,3-dimethyl-1H-pyrazol-5-amin, (4.020) 4-(2-Chlor-4-fluorphenyl)-N-(2-chlorphenyl)-1,3-dimethyl-1H-pyrazol-5-amin, (4.021) 4-(2-Chlor-4-fluorphenyl)-N-(2-fluorphenyl)-1,3-dimethyl-1H-pyrazol-5-amin, (4.022) 4-(4-Chlorphenyl)-5-(2,6-difluorphenyl)-3,6-dimethylpyridazin, (4.023) N-(2-Brom-6-fluorphenyl)-4-(2-chlor-4-fluorphenyl)-1,3-dimethyl-1H-pyrazol-5-amin, (4.024) N-(2-Bromphenyl)-4-(2-chlor-4-fluorphenyl)-1,3-dimethyl-1H-pyrazol-5-amin, (4.025) N-(4-Chlor-2,6-difluorphenyl)-4-(2-chlor-4-fluorphenyl)-1,3-dimethyl-1H-pyrazol-5-amin.

5) Verbindungen mit Befähigung zu Multisite-Aktivität, beispielsweise (5.001) Bordeauxmischung, (5.002) Captafol, (5.003) Captan, (5.004) Chlorthalonil, (5.005) Kupferhydroxid, (5.006) Kupfernaphthenat, (5.007) Kupferoxid, (5.008) Kupferoxychlorid, (5.009) Kupfer(2+)-sulfat, (5.010) Dithianon, (5.011) Dodin, (5.012) Folpet, (5.013) Mancozeb, (5.014) Maneb, (5.015) Metiram, (5.016) Zinkmetiram, (5.017) Kupfer-Oxin, (5.018) Propineb, (5.019) Schwefel und Schwefelzubereitungen einschließlich Calciumpolysulfid, (5.020) Thiram, (5.021) Zineb, (5.022) Ziram, (5.023) 6-Ethyl-5,7-dioxo-6,7-dihydro-5H-pyrrolo[3',4':5,6][1,4]dithiino[2,3-c][1,2]thiazole-3-carbonitrile.

6) Verbindungen, die zum Auslösen einer Wirtsabwehr befähigt sind, beispielsweise (6.001) Acibenzolar-S-Methyl, (6.002) Isotianil, (6.003) Probenazol, (6.004) Tiadinil.

7) Inhibitoren der Aminosäure- und/oder Protein-Biosynthese, beispielsweise (7.001) Cyprodinil, (7.002) Kasugamycin, (7.003) Kasugamycinhydrochlorid-hydrat, (7.004) Oxytetracyclin (7.005) Pyrimethanil, (7.006) 3-(5-Fluor-3,3,4,4-tetramethyl-3,4-dihydroisochinolin-1-yl)chinolin.

8) Inhibitoren der ATP-Produktion, beispielsweise (8.001) Silthiofam.

9) Inhibitoren der Zellwandsynthese, beispielsweise (9.001) Benthiavalicarb, (9.002) Dimethomorph, (9.003) Flumorph, (9.004) Iprovalicarb, (9.005) Mandipropamid, (9.006) Pyrimorph, (9.007) Valifenalat, (9.008) (2E)-3-(4-tert.-Butylphenyl)-3-(2-chlorpyridin-4-yl)-1-(morpholin-4-yl)prop-2-en-1-on, (9.009) (2Z)-3-(4-tert.-Butylphenyl)-3-(2-chlorpyridin-4-yl)-1-(morpholin-4-yl)prop-2-en-1-on.

10) Inhibitoren der Lipid- und Membran-Synthese, beispielsweise (10.001) Propamocarb, (10.002) Propamocarbhydrochlorid, (10.003) Tolclofos-Methyl.

11) Inhibitoren der Melanin-Biosynthese, beispielsweise (11.001) Tricyclazol, (11.002) 2,2,2-Trifluorethyl-{3-methyl-1-[(4-methylbenzoyl)amino]butan-2-yl}carbamat.

12) Inhibitoren der Nukleinsäuresynthese, beispielsweise (12.001) Benalaxyl, (12.002) Benalaxyl-M (Kiralaxyl), (12.003) Metalaxyl, (12.004) Metalaxyl-M (Mefenoxam).

13) Inhibitoren der Signaltransduktion, beispielsweise (13.001) Fludioxonil, (13.002) Iprodion, (13.003) Procymidon, (13.004) Proquinazid, (13.005) Quinoxyfen, (13.006) Vinclozolin.

14) Verbindungen, die als Entkoppler wirken können, beispielsweise (14.001) Fluazinam, (14.002) Meptyldinocap.

15) Weitere Verbindungen, beispielsweise (15.001) Abscisinsäure, (15.002) Benthiazol, (15.003) Bethoxazin, (15.004) Capsimycin, (15.005) Carvon, (15.006) Chinomethionat, (15.007) Cufraneb, (15.008) Cyflufenamid, (15.009) Cymoxanil, (15.010) Cyprosulfamid, (15.011) Flutianil, (15.012) Fosetyl-Aluminium, (15.013) Fosetyl-Calcium, (15.014) Fosetyl-Natrium, (15.015) Methylisothiocyanat, (15.016) Metrafenon, (15.017) Mildiomycin, (15.018) Natamycin, (15.019) Nickel-Dimethyldithiocarbamat, (15.020) Nitrothal-Isopropyl, (15.021) Oxamocarb, (15.022) Oxathiapiprolin, (15.023) Oxyfenthiin, (15.024) Pentachlorphenol und Salze, (15.025) Phosphonsäure und deren Salze, (15.026) Propamocarb-fosetylat, (15.027) Pyriofenone (Chlazafenone) (15.028) Tebufloquin, (15.029) Tecloftalam, (15.030) Tolnifanide, (15.031) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.032) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.033) 2-(6-Benzylpyridin-2-yl)quinazolin, (15.034) 2,6-Dimethyl-1H,5H-[1,4]dithiino[2,3-c:5,6-c']dipyrrol-1,3,5,7(2H,6H)-tetron, (15.035) 2-[3,5-Bis(difluormethyl)-1H-pyrazol-1-yl]-1-[4-(4-{5-[2-(prop-2-in-1-yloxy)phenyl]-4,5-dihydro-1,2-oxazol-3-yl}-1,3-thiazol-2-yl)piperidin-1-yl]ethanon, (15.036) 2-[3,5-Bis(difluormethyl)-1H-pyrazol-1-yl]-1-[4-(4-{5-[2-chlor-6-(prop-2-in-1-yloxy)phenyl]-4,5-dihydro-1,2-oxazol-3-yl}-1,3-thiazol-2-yl)piperidin-1-yl]ethanon, (15.037) 2-[3,5-Bis(difluormethyl)-1H-pyrazol-1-yl]-1-[4-(4-{5-[2-fluor-6-(prop-2-in-1-yloxy)phenyl]-4,5-dihydro-1,2-oxazol-3-yl}-1,3-thiazol-2-yl)piperidin-1-yl]ethanon, (15.038) 2-[6-(3-Fluor-4-methoxyphenyl)-5-methylpyridin-2-yl]quinazolin, (15.039) 2-{(5R)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenyl methanesulfonat, (15.040) 2-{(5S)-3-[2-(1-1[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenyl methanesulfonat, (15.041) 2-{2-[(7,8-Difluor-2-methylquinolin-3-yl)oxy]-6-fluorphenyl}propan-2-ol, (15.042) 2-{2-Fluor-6-[(8-fluor-2-methylquinolin-3-yl)oxy]phenyl}propan-2-ol, (15.043) 2-13-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenyl-methansulfonat, (15.044) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenyl methanesulfonat, (15.045) 2-Phenylphenol und deren Salze, (15.046) 3-(4,4,5-Trifluor-3,3-dimethyl-3,4-dihydroisoquinolin-1-yl)quinolin, (15.047) 3-(4,4-Difluor-3,3-dimethyl-3,4-dihydroisoquinolin-1-yl)quinolin, (15.048) 4-Amino-5-fluorpyrimidin-2-ol (Tautomere Form: 4-Amino-5-fluorpyrimidin-2(1H)-on), (15.049) 4-Oxo-4-[(2-phenylethyl)amino]buttersäure, (15.050) 5-Amino-1,3,4-thiadiazol-2-thiol, (15.051) 5-Chlor-N'-phenyl-N'-(prop-2-yn-1-yl)thiophen-2-sulfonohydrazid, (15.052) 5 -Fluor-2- [(4-fluorbenzyl)oxy]pyrimidin-4-amin, (15.053) 5-Fluor-2-[(4-methylbenzyl)oxy]pyrimidin-4-amin, (15.054) 9-Fluor-2,2-dimethyl-5-(quinolin-3-yl)-2,3-dihydro-1,4-benzoxazepin, (15.055) But-3-yn-1-yl {6-[({[(Z)-(1-methyl-1H-tetrazol-5-yl)(phenyl)methylen]amino}oxy)methyl]pyridin-2-yl}carbamat, (15.056) Ethyl (2Z)-3-amino-2-cyano-3-phenylacrylat, (15.057) Phenazin-1-carbonsäure, (15.058) Propyl 3,4,5-trihydroxybenzoat, (15.059) Quinolin-8-ol, (15.060) Quinolin-8-ol sulfat (2:1), (15.061) tert-Butyl {6-[({[(1-methyl-1H-tetrazol-5-yl)(phenyl)methylene]amino}oxy)methyl]pyridin-2-yl}carbamat, (15.062) 5-Fluor-4-imino-3-methyl-1-[(4-methylphenyl)sulfonyl]-3,4-dihydropyrimidin-2(1H)-one.

**Biologische Schädlingsbekämpfungsmittel als Mischungskomponenten**

Die Verbindungen der Formel (I) können mit biologischen Schädlingsbekämpfungsmitteln kombiniert werden.

Biologische Schädlingsbekämpfungsmittel umfassen insbesondere Bakterien, Pilze, Hefen, Pflanzenextrakte und solche Produkte, die von Mikroorganismen gebildet wurden inklusive Proteine und sekundäre Stoffwechselprodukte.

Biologische Schädlingsbekämpfungsmittel umfassen Bakterien wie sporenbildende Bakterien, wurzelbesiedelnde Bakterien und Bakterien, die als biologische Insektizide, Fungizide oder Nematizide wirken.

Beispiele für solche Bakterien, die als biologische Schädlingsbekämpfungsmittel eingesetzt werden bzw. verwendet werden können, sind:
*Bacillus amyloliquefaciens,* Stamm FZB42 (DSM 231179), oder *Bacillus cereus,* insbesondere *B. cereus* Stamm CNCM I-1562 oder *Bacillus firmus,* Stamm I-1582 (Accession number CNCM I-1582) oder *Bacillus pumilus,* insbesondere Stamm GB34 (Accession No. ATCC 700814) und Stamm QST2808 (Accession No. NRRL B-30087), oder *Bacillus subtilis,* insbesondere Stamm GB03 (Accession No. ATCC SD-1397), oder *Bacillus subtilis* Stamm QST713 (Accession No. NRRL B-21661) oder *Bacillus subtilis* Stamm OST 30002 (Accession No. NRRL B-50421), *Bacillus thuringiensis,* insbesondere *B. thuringiensis* Subspezies *israelensis* (Serotyp H-14), Stamm AM65-52 (Accession No. ATCC 1276), oder *B. thuringiensis subsp. aizawai,* insbesondere Stamm ABTS-1857 (SD-1372), oder *B. thuringiensis subsp. kurstaki* Stamm HD-1, oder *B. thuringiensis subsp. tenebrionis* Stamm NB 176 (SD-5428), *Pasteuria penetrans, Pasteuria spp.* (Rotylenchulus reniformis nematode)-PR3 (Accession Number ATCC SD-5834), *Streptomyces microflavus* Stamm

AQ6121 (= QRD 31.013, NRRL B-50550), *Streptomyces galbus* Stamm AQ 6047 (Acession Number NRRL 30232).

Beispiele für Pilze und Hefen, die als biologische Schädlingsbekämpfungsmittel eingesetzt werden bzw. verwendet werden können, sind:

*Beauveria bassiana,* insbesondere Stamm ATCC 74040, *Coniothyrium minitans,* insbesondere Stamm CON/M/91-8 (Accession No. DSM-9660), *Lecanicillium spp.,* insbesondere Stamm HRO LEC 12, *Lecanicillium lecanii* (ehemals bekannt als *Verticillium lecanii),* insbesondere Stamm KV01, *Metarhizium anisopliae,* insbesondere Stamm F52 (DSM3884/ ATCC 90448), *Metschnikowia fructicola,* insbesondere Stamm NRRL Y-30752, *Paecilomyces fumosoroseus* (neu: *Isaria fumosorosea),* insbesondere Stamm IFPC 200613, oder Stamm Apopka 97 (Accesion No. ATCC 20874), *Paecilomyces lilacinus,* insbesondere P. *lilacinus* Stamm 251 (AGAL 89/030550), *Talaromyces flavus,* insbesondere Stamm V117b, *Trichoderma atroviride,* insbesondere Stamm SC1 (Accession Number CBS 122089), *Trichoderma harzianum,* insbesondere *T. harzianum rifai T39.* (Accession Number CNCM I-952).

Beispiele für Viren, die als biologische Schädlingsbekämpfungsmittel eingesetzt werden bzw. verwendet werden können, sind:

*Adoxophyes orana* (Apfelschalenwickler) Granulosevirus (GV), *Cydia pomonella* (Apfelwickler) Granulosevirus (GV), *Helicoverpa armigera* (Baumwollkapselwurm) Nuklear Polyhedrosis Virus (NPV), *Spodoptera exigua* (Zuckerrübeneule) mNPV, *Spodoptera frugiperda* (Heerwurm) mNPV, *Spodoptera littoralis* (Afrikanischer Baumwollwurm) NPV.

Es sind auch Bakterien und Pilze umfasst, die als 'Inokulant' Pflanzen oder Pflanzenteilen oder Pflanzenorganen beigegeben werden und durch ihre besonderen Eigenschaften das Pflanzenwachstum und die Pflanzengesundheit fördern. Als Beispiele sind genannt:

*Agrobacterium spp., Azorhizobium caulinodans, Azospirillum spp., Azotobacter spp., Bradyrhizobium spp., Burkholderia spp.,* insbesondere *Burkholderia cepacia* (ehemals bekannt als *Pseudomonas cepacia), Gigaspora spp.,* oder *Gigaspora monosporum, Glomus spp., Laccaria spp., Lactobacillus buchneri, Paraglomus spp., Pisolithus tinctorus, Pseudomonas spp., Rhizobium spp.,* insbesondere *Rhizobium trifolii, Rhizopogon spp., Scleroderma spp., Suillus spp., Streptomyces spp..*

Beispiele für Pflanzenextrakte und solche Produkte, die von Mikroorganismen gebildet wurden inklusive Proteine und sekundäre Stoffwechselprodukte, die als biologische Schädlingsbekämpfungsmittel eingesetzt werden bzw. verwendet werden können, sind:

Allium sativum, Artemisia absinthium, Azadirachtin, Biokeeper WP, Cassia nigricans, Celastrus angulatus, Chenopodium anthelminticum, Chitin, Armour-Zen, Dryopteris filix-mas, Equisetum arvense, Fortune Aza, Fungastop, Heads Up (Chenopodium quinoa-Saponinextrakt), Pyrethrum/Pyrethrine, Quassia amara, Quercus, Quillaja, Regalia, "Requiem ™ Insecticide", Rotenon, Ryania/Ryanodine, Symphytum officinale, Tanacetum vulgare, Thymol, Triact 70, TriCon, Tropaeulum majus, Urtica dioica, Veratrin, Viscum album, Brassicacaeen-Extrakt, insbesondere Raps- oder Senfpulver.

### Safener als Mischungskomponenten

Die Verbindungen der Formel (I) können mit Safenern kombiniert werden, wie zum Beispiel Benoxacor, Cloquintocet (-mexyl), Cyometrinil, Cyprosulfamide, Dichlormid, Fenchlorazole (-ethyl), Fenclorim, Flurazole, Fluxofenim, Furilazole, Isoxadifen (-ethyl), Mefenpyr (-diethyl), Naphthalic anhydride, Oxabetrinil, 2-Methoxy-N-({4-[(methylcarbamoyl)amino]phenyl}sulfonyl)benzamid (CAS 129531-12-0), 4-(Dichloracetyl)-1-oxa-4-azaspiro[4.5]decan (CAS 71526-07-3), 2,2,5-Trimethyl-3-(dichloracetyl)-1,3-oxazolidin (CAS 52836-31-4).

### Pflanzen und Pflanzenteile

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen), beispielsweise Getreide (Weizen, Reis, Triticale, Gerste, Roggen, Hafer), Mais, Soja, Kartoffel, Zuckerrüben, Zuckerrohr, Tomaten, Paprika, Gurke, Melone, Möhre, Wassermelone, Zwiebel, Salat, Spinat, Porree, Bohnen, *Brassica oleracea* (z. B. Kohl) und andere Gemüsesorten, Baumwolle, Tabak, Raps, sowie Obstpflanzen (mit den Früchten Äpfel, Birnen, Zitrusfrüchte und Weintrauben). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzen sollen alle Entwicklungsstadien wie Saatgut, Stecklinge, junge (unausgereifte) Pflanzen bis hin zu ausgereiften Pflanzen verstanden werden. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehören auch geerntete Pflanzen oder geerntete Pflanzenteile sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Verbindungen der Formel (I) erfolgt direkt oder durch Einwirkung der Verbindungen auf die Umgebung, den Lebensraum oder den Lagerraum nach den üblichen Behandlungsmethoden, z. B. durch Eintauchen, Spritzen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die durch konventionelle Züchtung, durch Mutagenese oder durch rekombinante DNA-Techniken erhalten worden sind. Dies können Sorten, Rassen, Bio- und Genotypen sein.

**Transgene Pflanze, Saatgutbehandlung und Integrationsereignisse**

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehrfähigkeit der Pflanzen gegen tierische und mikrobielle Schädlinge, wie Insekten, Spinnentiere, Nematoden, Milben, Schnecken, bewirkt z. B. durch in den Pflanzen entstehende Toxine, insbesondere solche, die durch das genetische Material aus Bacillus Thuringiensis (z. B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden, ferner eine erhöhte Abwehrfähigkeit der Pflanzen gegen pflanzenpathogene Pilze, Bakterien und/oder Viren, bewirkt z. B. durch Systemisch Akquirierte Resistenz (SAR), Systemin, Phytoalexine, Elicitoren sowie Resistenzgene und entsprechend exprimierte Proteine und Toxine, sowie eine erhöhte Toleranz der Pflanzen gegen bestimmte herbizide Wirkstoffe, beispielsweise Imidazolinone, Sulfonylharnstoffe, Glyphosat oder Phosphinotricin (z. B. "PAT"-Gen). Die jeweils die gewünschten Eigenschaften ("Traits") verleihenden Gene können auch in Kombinationen miteinander in den transgenen Pflanzen vorkommen. Als Beispiele transgener Pflanzen werden die wichtigen Kulturpflanzen, wie Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchte und Weintrauben) erwähnt, wobei Mais, Soja, Weizen, Reis, Kartoffel, Baumwolle, Zuckerrohr, Tabak und Raps besonders hervorgehoben werden. Als Eigenschaften ("Traits") werden besonders hervorgehoben die erhöhte Abwehrfähigkeit der Pflanzen gegen Insekten, Spinnentiere, Nematoden und Schnecken.

**Pflanzenschutz - Behandlungsarten**

Die Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z. B. durch Tauchen, Spritzen, Sprühen, Berieseln, Verdampfen, Zerstäuben, Vernebeln, Verstreuen, Verschäumen, Bestreichen, Verstreichen, Injizieren, Gießen (drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen, usw. Es ist ferner möglich, die Verbindungen der Formel (I) nach dem Ultra-Low-Volume-Verfahren auszubringen oder die Anwendungsform oder die Verbindung der Formel (I) selbst in den Boden zu injizieren.

Eine bevorzugte direkte Behandlung der Pflanzen ist die Blattapplikation, d. h. die Verbindungen der Formel (I) werden auf das Blattwerk aufgebracht, wobei die Behandlungsfrequenz und die Aufwandmenge auf den Befallsdruck des jeweiligen Schädlings abgestimmt sein sollte.

Bei systemisch wirksamen Wirkstoffen gelangen die Verbindungen der Formel (I) auch über das Wurzelwerk in die Pflanzen. Die Behandlung der Pflanzen erfolgt dann durch Einwirkung der Verbindungen der Formel (I) auf den Lebensraum der Pflanze. Das kann beispielsweise durch Drenchen, Einmischen in den Boden oder die Nährlösung sein, d. h. der Standort der Pflanze (z. B. Boden oder hydroponische Systeme) wird mit einer flüssigen Form der Verbindungen der Formel (I) getränkt, oder durch die Bodenapplikation, d. h. die erfindungsgemäßen Verbindungen der Formel (I) werden in fester Form (z. B. in Form eines Granulats) in den Standort der Pflanzen eingebracht. Bei Wasserreiskulturen kann das auch durch Zudosieren der Verbindung der Formel (I) in einer festen Anwendungsform (z. B. als Granulat) in ein überflutetes Reisfeld sein.

**Saatgutbehandlung**

Die Bekämpfung von tierischen Schädlingen durch die Behandlung des Saatguts von Pflanzen ist seit langem bekannt und ist Gegenstand ständiger Verbesserungen. Dennoch ergeben sich bei der Be-handlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Schädlingsbekämpfungsmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. - toleranter transgener Pflanzen einbeziehen, um einen optimalen Schutz des Saatguts und auch der keimenden Pflanze bei einem minimalen Aufwand an Schädlingsbekämpfungsmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einer der Verbindungen der Formel (I) behandelt wird. Das erfindungsgemäße Verfahren zum Schutz von Saatgut und keimenden Pflanzen vor dem Befall von Schädlingen umfasst ferner ein Verfahren, in dem das Saatgut gleichzeitig in einem Vorgang oder sequentiell mit einer Verbindung der Formel (I) und einer Mischungskomponente behandelt wird. Es umfasst ferner auch ein Verfahren, in dem das Saatgut zu unterschiedlichen Zeiten mit einer Verbindung der Formel (I) und einer Mischungskomponente behandelt wird.

Die Erfindung bezieht sich ebenfalls auf die Verwendung der Verbindungen der Formel (I) zur Behandlung von Saatgut zum Schutz des Saatguts und der daraus entstehenden Pflanze vor tierischen Schädlingen.

Weiterhin bezieht sich die Erfindung auf Saatgut, welches zum Schutz vor tierischen Schädlingen mit einer erfindungsgemäßen Verbindung der Formel (I) behandelt wurde. Die Erfindung bezieht sich auch auf Saatgut, welches zur gleichen Zeit mit einer Verbindung der Formel (I) und einer Mischungskomponente behandelt wurde. Die Erfindung bezieht sich weiterhin auf Saatgut, welches zu unterschiedlichen Zeiten mit einer Verbindung der Formel (I) und einer Mischungskomponente behandelt wurde. Bei Saatgut, welches zu unterschiedlichen Zeiten mit einer Verbindung der Formel (I) und einer Mischungskomponente behandelt wurde, können die einzelnen Substanzen in unterschiedlichen Schichten auf dem Saatgut vorhanden sein. Dabei können die Schichten, die eine Verbindung der Formel (I) und Mischungskomponenten enthalten, gegebenenfalls durch eine Zwischenschicht getrennt sein. Die Erfindung bezieht sich auch auf Saatgut, bei dem eine Verbindung der Formel (I) und eine Mischungskomponente als Bestandteil einer Umhüllung oder als weitere Schicht oder weitere Schichten zusätzlich zu einer Umhüllung aufgebracht sind.

Des Weiteren bezieht sich die Erfindung auf Saatgut, welches nach der Behandlung mit einer Verbindung der Formel (**I**) einem Filmcoating-Verfahren unterzogen wird, um Staubabrieb am Saatgut zu vermeiden.

Einer der auftretenden Vorteile, wenn eine Verbindung der Formel (I) systemisch wirkt, ist es, dass die Behandlung des Saatguts nicht nur das Saatgut selbst, sondern auch die daraus hervorgehenden Pflanzen nach dem Auflaufen vor tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit einer Verbindung der Formel (I) Keimung und Auflauf des behandelten Saatguts gefördert werden können.

Ebenso ist es als vorteilhaft anzusehen, dass Verbindungen der Formel (I) insbesondere auch bei transgenem Saatgut eingesetzt werden können.

Verbindungen der Formel (I) können ferner in Kombination mit Mitteln der Signaltechnologie eingesetzt werden, wodurch eine bessere Besiedlung mit Symbionten, wie zum Beispiel Rhizobien, Mycorrhiza und/oder endophytischen Bakterien oder Pilzen, stattfindet und/oder es zu einer optimierten Stickstofffixierung kommt.

Die Verbindungen der Formel (I) eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baumwolle, Soja, Reis, Kartoffeln, Sonnenblume, Kaffee, Tabak, Canola, Raps, Rübe (z. B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps, Gemüse und Reis zu.

Wie vorstehend bereits erwähnt, kommt auch der Behandlung von transgenem Saatgut mit einer Verbindung der Formel (I) eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw.

nematiziden Eigenschaften steuert. Die heterologen Gene in transgenem Saatgut können dabei aus Mikroorganismen wie Bacillus, Rhizobium, Pseudomonas, Serratia, Trichoderma, Clavibacter, Glomus oder Gliocladium stammen. Die vorliegende Erfindung eignet sich besonders für die Behandlung von transgenem Saatgut, das zumindest ein heterologes Gen enthält, das aus Bacillus sp. stammt. Besonders bevorzugt handelt es sich dabei um ein heterologes Gen, das aus Bacillus thuringiensis stammt.

Im Rahmen der vorliegenden Erfindung wird die Verbindung der Formel (I) auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hüllen, Wolle oder Fruchtfleisch befreit wurde. So kann zum Beispiel Saatgut verwendet werden, das geerntet, gereinigt und bis zu einem lagerfähigen Feuchtigkeitsgehalt getrocknet wurde. Alternativ kann auch Saatgut verwendet werden, das nach dem Trocknen z. B. mit Wasser behandelt und dann erneut getrocknet wurde, zum Beispiel Priming. Im Fall von Reis-Saatgut ist es auch möglich, Saatgut zu verwenden, das getränkt wurde, zum Beispiel in Wasser bis zu einem bestimmten Stadium des Reisembryos ("Pigeon Breast Stage"), wodurch die Keimung und ein einheitlicheres Auflaufen stimuliert wird.

Im Allgemeinen muss bei der Behandlung des Saatguts darauf geachtet werden, dass die Menge der auf das Saatgut aufgebrachten Verbindung der Formel (I) und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die Verbindungen der Formel (I) werden in der Regel in Form einer geeigneten Formulierung auf das Saatgut aufgebracht. Geeignete Formulierungen und Verfahren für die Saatgutbehandlung sind dem Fachmann bekannt.

Die Verbindungen der Formel (I) können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Verbindungen der Formel (I) mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalinsulfonate, wie Diisopropyl- oder Diisobutylnaphthalinsulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vor-zugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid-Blockpolymere, Alkylphenolpolyglykolether sowie Tri-stryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formu-lierungen enthalten sein können, kommen alle für derartige Zwecke in agrochemischen Mitteln ein-setzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, be-sonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zu-bereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen, Soja und Rüben oder auch von Gemüsesaatgut der verschiedensten Natur. Die erfindungsgemäß verwendbaren Beizmittel-Formulierungen oder deren verdünnte Anwendungsformen können auch zum Beizen von Saatgut transgener Pflanzen eingesetzt werden.

Zur Behandlung von Saatgut mit den erfindungsgemäß verwendbaren Beizmittel-Formulierungen oder dem daraus durch Zugabe von Wasser hergestellten Anwendungsformen kommen alle üblicherweise für die Beizung einsetzbaren Mischgeräte in Betracht. Im Einzelnen geht man bei der Beizung so vor, dass man das Saatgut in einen Mischer im diskontinuierlichen oder kontinuierlichen Betrieb gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann inner-halb eines größeren Bereiches variiert werden. Sie richtet sich nach dem jeweiligen Gehalt der Verbindungen der Formel (I) in den Formulierungen und nach dem Saatgut. Die Aufwandmengen bei der Verbindung der Formel (I) liegen im Allgemeinen zwischen 0,001 und 50 g pro Kilogramm Saatgut, vorzugsweise zwischen 0,01 und 15 g pro Kilogramm Saatgut.

**Tiergesundheit**

Auf dem Gebiet der Tiergesundheit, d. h. dem Gebiet der Tiermedizin, sind die Verbindungen der Formel (I) gegen Tierparasiten, insbesondere Ektoparasiten oder Endoparasiten, wirksam. Der Begriff Endoparasit umfasst insbesondere Helminthen und Protozoen wie Kokzidien. Ektoparasiten sind typischerweise und bevorzugt Arthropoden, insbesondere Insekten oder Akariden.

Auf dem Gebiet der Tiermedizin eignen sich die Verbindungen der Formel (I), die eine günstige Toxizität gegenüber Warmblütern aufweisen, für die Bekämpfung von Parasiten, die in der Tierzucht und Tierhaltung bei Nutztieren, Zuchttieren, Zootieren, Laboratoriumstieren, Versuchstieren und Haustieren auftreten. Sie sind gegen alle oder einzelne Entwicklungsstadien der Parasiten wirksam.

Zu den landwirtschaftlichen Nutztieren zählen zum Beispiel Säugetiere wie Schafe, Ziegen, Pferde, Esel, Kamele, Büffel, Kaninchen, Rentiere, Damhirsche und insbesondere Rinder und Schweine; oder Geflügel wie Truthähne, Enten, Gänse und insbesondere Hühner; oder Fische oder Krustentiere, z. B. in der Aquakultur, oder gegebenenfalls Insekten wie Bienen.

Zu den Haustieren zählen zum Beispiel Säugetiere wie Hamster, Meerschweinchen, Ratten, Mäuse, Chinchillas, Frettchen und insbesondere Hunde, Katzen, Stubenvögel; Reptilien, Amphibien oder Aquariumfische.

Gemäß einer bestimmten Ausführungsform werden die Verbindungen der Formel (I) an Säugetiere verabreicht.

Gemäß einer weiteren bestimmten Ausführungsform werden die Verbindungen der Formel (I) an Vögel, nämlich Stubenvögel oder insbesondere Geflügel, verabreicht.

Durch Verwendung der Verbindungen der Formel (I) für die Bekämpfung von Tierparasiten sollen Krankheit, Todesfälle und Leistungsminderungen (bei Fleisch, Milch, Wolle, Häuten, Eiern, Honig und dergleichen) verringert bzw. vorgebeugt werden, so dass eine wirtschaftlichere und einfachere Tierhaltung ermöglicht wird und ein besseres Wohlbefinden der Tiere erzielbar ist.

In Bezug auf das Gebiet der Tiergesundheit bedeutet der Begriff "Bekämpfung" oder "bekämpfen" im vorliegenden Zusammenhang, dass durch die Verbindungen der Formel (I) wirksam das Auftreten des jeweiligen Parasiten in einem Tier, das mit solchen Parasiten in einem harmlosen Ausmaß infiziert ist, reduziert wird. Genauer gesagt bedeutet "bekämpfen" im vorliegenden Zusammenhang, dass die Verbindungen der Formel (I) den jeweiligen Parasiten abtöten, sein Wachstum verhindern oder seine Vermehrung verhindern.

Zu den Arthropoden zählen beispielsweise, ohne hierauf beschränkt zu sein,

aus der Ordnung Anoplurida zum Beispiel Haematopinus spp., Linognathus spp., Pediculus spp., Phtirus spp., Solenopotes spp.;

aus der Ordnung Mallophagida und den Unterordnungen Amblycerina und Ischnocerina, zum Beispiel Bovicola spp., Damalina spp., Felicola spp.; Lepikentron spp., Menopon spp., Trichodectes spp., Trimenopon spp., Trinoton spp., Werneckiella spp;

aus der Ordnung Diptera und den Unterordnungen Nematocerina und Brachycerina, zum Beispiel Aedes spp., Anopheles spp., Atylotus spp., Braula spp., Calliphora spp., Chrysomyia spp., Chrysops spp., Culex spp., Culicoides

spp., Eusimulium spp., Fannia spp., Gasterophilus spp., Glossina spp., Haematobia spp., Haematopota spp., Hippobosca spp., Hybomitra spp., Hydrotaea spp., Hypoderma spp., Lipoptena spp., Lucilia spp., Lutzomyia spp., Melophagus spp., Morellia spp., Musca spp., Odagmia spp., Oestrus spp., Philipomyia spp., Phlebotomus spp., Rhinoestrus spp., Sarcophaga spp., Simulium spp., Stomoxys spp., Tabanus spp., Tipula spp., Wilhelmia spp., Wohlfahrtia spp.;

aus der Ordnung Siphonapterida, zum Beispiel Ceratophyllus spp., Ctenocephalides spp., Pulex spp., Tunga spp., Xenopsylla spp.;

aus der Ordnung Heteropterida, zum Beispiel Cimex spp., Panstrongylus spp., Rhodnius spp., Triatoma spp.; sowie Lästlinge und Hygieneschädlinge aus der Ordnung Blattarida.

Weiterhin sind bei den Arthropoden beispielhaft, ohne hierauf beschränkt zu sein, die folgenden Akari zu nennen: Aus der Unterklasse Akari (Acarina) und der Ordnung Metastigmata, zum Beispiel aus der Familie Argasidae, wie Argas spp., Ornithodorus spp., Otobius spp., aus der Familie Ixodidae, wie Amblyomma spp., Dermacentor spp., Haemaphysalis spp., Hyalomma spp., Ixodes spp., Rhipicephalus (Boophilus) spp., Rhipicephalus spp. (die ursprüngliche Gattung der mehrwirtigen Zecken); aus der Ordnung Mesostigmata, wie Dermanyssus spp., Ornithonyssus spp., Pneumonyssus spp., Raillietia spp., Sternostoma spp., Tropilaelaps spp., Varroa spp.; aus der Ordnung Actinedida (Prostigmata), zum Beispiel Acarapis spp., Cheyletiella spp., Demodex spp., Listrophorus spp., Myobia spp., Neotrombicula spp., Ornithocheyletia spp., Psorergates spp., Trombicula spp.; und aus der Ordung der Acaridida (Astigmata), zum Beispiel Acarus spp., Caloglyphus spp., Chorioptes spp., Cytodites spp., Hypodectes spp., Knemidocoptes spp., Laminosioptes spp., Notoedres spp., Otodectes spp., Psoroptes spp., Pterolichus spp., Sarcoptes spp., Trixacarus spp., Tyrophagus spp.

Zu Beispielen für parasitäre Protozoen zählen, ohne hierauf beschränkt zu sein:
Mastigophora (Flagellata), wie:

Metamonada: aus der Ordnung Diplomonadida zum Beispiel Giardia spp., Spironucleus spp.

Parabasala: aus der Ordnung Trichomonadida zum Beispiel Histomonas spp., Pentatrichomonas spp., Tetratrichomonas spp., Trichomonas spp., Tritrichomonas spp.

Euglenozoa: aus der Ordnung Trypanosomatida zum Beispiel Leishmania spp., Trypanosoma spp.

Sarcomastigophora (Rhizopoda), wie Entamoebidae, zum Beispiel Entamoeba spp., Centramoebidae, zum Beispiel Acanthamoeba sp., Euamoebidae, z. B. Hartmanella sp.

Alveolata wie Apicomplexa (Sporozoa): z. B. Cryptosporidium spp.; aus der Ordnung Eimeriida zum Beispiel Besnoitia spp., Cystoisospora spp., Eimeria spp., Hammondia spp., Isospora spp., Neospora spp., Sarcocystis spp., Toxoplasma spp.; aus der Ordnung Adeleida z. B. Hepatozoon spp., Klossiella spp.; aus der Ordnung Haemosporida z. B. Leucocytozoon spp., Plasmodium spp.; aus der Ordnung Piroplasmida z. B. Babesia spp., Ciliophora spp., Echinozoon spp., Theileria spp.; aus der Ordnung Vesibuliferida z. B. Balantidium spp., Buxtonella spp.

Microspora wie Encephalitozoon spp., Enterocytozoon spp., Globidium spp., Nosema spp., und außerdem z. B. Myxozoa spp.

Zu den für Menschen oder Tiere pathogenen Helminthen zählen zum Beispiel Acanthocephala, Nematoden, Pentastoma und Platyhelminthen (z.B. Monogenea, Cestodes und Trematodes).

Zu beispielhaften Helminthen zählen, ohne hierauf beschränkt zu sein:

Monogenea: z. B.: Dactylogyrus spp., Gyrodactylus spp., Microbothrium spp., Polystoma spp., Troglecephalus spp.;

Cestodes: aus der Ordnung Pseudophyllidea zum Beispiel: Bothridium spp., Diphyllobothrium spp., Diplogonoporus spp. Ichthyobothrium spp., Ligula spp., Schistocephalus spp., Spirometra spp.

Aus der Ordnung Cyclophyllida zum Beispiel: Andyra spp., Anoplocephala spp., Avitellina spp., Bertiella spp., Cittotaenia spp., Davainea spp., Diorchis spp., Diplopylidium spp., Dipylidium spp., Echinococcus spp., Echinocotyle spp., Echinolepis spp., Hydatigera spp., Hymenolepis spp., Joyeuxiella spp., Mesocestoides spp., Moniezia spp., Paranoplocephala spp., Raillietina spp., Stilesia spp., Taenia spp., Thysaniezia spp., Thysanosoma spp.

Trematodes: aus der Klasse Digenea zum Beispiel: Austrobilharzia spp., Brachylaima spp., Calicophoron spp., Catatropis spp., Clonorchis spp. Collyriclum spp., Cotylophoron spp., Cyclocoelum spp., Dicrocoelium spp., Diplostomum spp., Echinochasmus spp., Echinoparyphium spp., Echinostoma spp., Eurytrema spp., Fasciola spp., Fasciolides spp., Fasciolopsis spp., Fischoederius spp., Gastrothylacus spp., Gigantobilharzia spp., Gigantocotyle spp., Heterophyes spp., Hypoderaeum spp., Leucochloridium spp., Metagonimus spp., Metorchis spp., Nanophyetus spp., Notocotylus spp., Opisthorchis spp., Ornithobilharzia spp., Paragonimus spp., Paramphistomum spp., Plagiorchis spp., Posthodiplostomum spp., Prosthogonimus spp., Schistosoma spp., Trichobilharzia spp., Troglotrema spp., Typhlocoelum spp.

Nematoden: aus der Ordnung Trichinellida zum Beispiel: Capillaria spp., Trichinella spp., Trichomosoides spp., Trichuris spp.

Aus der Ordnung Tylenchida zum Beispiel: Micronema spp., Parastrangyloides spp., Strongyloides spp.

Aus der Ordnung Rhabditina zum Beispiel: Aelurostrongylus spp., Amidostomum spp., Ancylostoma spp., Angiostrongylus spp., Bronchonema spp., Bunostomum spp., Chabertia spp., Cooperia spp., Cooperioides spp., Crenosoma spp., Cyathostomum spp., Cyclococercus spp., Cyclodontostomum spp., Cylicocyclus spp., Cylicostephanus spp., Cylindropharynx spp., Cystocaulus spp., Dictyocaulus spp., Elaphostrongylus spp., Filaroides spp., Globocephalus spp., Graphidium spp., Gyalocephalus spp., Haemonchus spp., Heligmosomoides spp., Hyostrongylus spp., Marshallagia spp., Metastrongylus spp., Muellerius spp., Necator spp., Nematodirus spp., Neostrongylus spp., Nippostrongylus spp., Obeliscoides spp., Oesophagodontus spp., Oesophagostomum spp., Ollulanus spp.; Ornithostrongylus spp., Oslerus spp., Ostertagia spp., Paracooperia spp., Paracrenosoma spp., Parafilaroides spp., Parelaphostrongylus spp., Pneumocaulus spp., Pneumostrongylus spp., Poteriostomum spp., Protostrongylus spp., Spicocaulus spp., Stephanurus spp., Strongylus spp., Syngamus spp., Teladorsagia spp., Trichonema spp., Trichostrongylus spp., Triodontophorus spp., Troglostrongylus spp., Uncinaria spp.

Aus der Ordnung Spirurida zum Beispiel: Acanthocheilonema spp., Anisakis spp., Ascaridia spp.; Ascaris spp., Ascarops spp., Aspiculuris spp., Baylisascaris spp., Brugia spp., Cercopithifilaria spp., Crassicauda spp., Dipetalonema spp., Dirofilaria spp., Dracunculus spp.; Draschia spp., Enterobius spp., Filaria spp., Gnathostoma spp., Gongylonema spp., Habronema spp., Heterakis spp.; Litomosoides spp., Loa spp., Onchocerca spp., Oxyuris spp., Parabronema spp., Parafilaria spp., Parascaris spp., Passalurus spp., Physaloptera spp., Probstmayria spp., Pseudofilaria spp., Setaria spp., Skjrabinema spp., Spirocerca spp., Stephanofilaria spp., Strongyluris spp., Syphacia spp., Thelazia spp., Toxascaris spp., Toxocara spp., Wuchereria spp.

Acanthocephala: aus der Ordnung Oligacanthorhynchida z.B: Macracanthorhynchus spp., Prosthenorchis spp.; aus der Ordnung Moniliformida zum Beispiel: Moniliformis spp.,

Aus der Ordnung Polymorphida zum Beispiel: Filicollis spp.; aus der Ordnung Echinorhynchida zum Beispiel Acanthocephalus spp., Echinorhynchus spp., Leptorhynchoides spp.

Pentastoma: aus der Ordnung Porocephalida zum Beispiel Linguatula spp.

Auf dem Gebiet der Tiermedizin und der Tierhaltung erfolgt die Verabreichung der Verbindungen der Formel (I) nach allgemein fachbekannten Verfahren, wie enteral, parenteral, dermal oder nasal in Form von geeigneten Präparaten. Die Verabreichung kann prophylaktisch; metaphylaktisch oder therapeutisch erfolgen. So bezieht sich eine Ausführungsform der vorliegenden Erfindung auf die Verbindungen der Formel (I) zur Verwendung als Arzneimittel.

Ein weiterer Aspekt bezieht sich auf die Verbindungen der Formel (I) zur Verwendung als Antiendoparasitikum.

Ein weiterer spezieller Aspekt der Erfindung betrifft die Verbindungen der Formel (I) zur Verwendung als Antihelminthikum, insbesondere zur Verwendung als Nematizid, Platymelminthizid, Acanthocephalizid oder Pentastomizid.

Ein weiterer spezieller Aspekt der Erfindung betrifft die Verbindungen der Formel (I) zur Verwendung als Antiprotozoikum.

Ein weiterer Aspekt betrifft die Verbindungen der Formel (I) zur Verwendung als Antiektoparasitikum, insbesondere ein Arthropodizid, ganz besonders ein Insektizid oder ein Akarizid.

Weitere Aspekte der Erfindung sind veterinärmedizinische Formulierungen, die eine wirksame Menge mindestens einer Verbindung der Formel (I) und mindestens einen der folgenden umfassen: einen pharmazeutisch unbedenklichen Exzipienten (z.B. feste oder flüssige Verdünnungsmittel), ein pharmazeutisch unbedenkliches Hilfsmittel (z.B. Tenside), insbesondere einen herkömmlicherweise in veterinärmedizinischen Formulierungen verwendeten pharmazeutisch unbedenklichen Exzipienten und/oder ein herkömmlicherweise in veterinärmedizinischen Formulierungen verwendetes pharmazeutisch unbedenkliches Hilfsmittel.

Ein verwandter Aspekt der Erfindung ist ein Verfahren zur Herstellung einer wie hier beschriebenen veterinärmedizinischen Formulierung, welches den Schritt des Mischens mindestens einer Verbindung der Formel (I) mit pharmazeutisch unbedenklichen Exzipienten und/oder Hilfsmitteln, insbesondere mit herkömmlicherweise in veterinärmedizinischen Formulierungen verwendeten pharmazeutisch unbedenklichen Exzipienten und/oder herkömmlicherweise in veterinärmedizinischen Formulierungen verwendeten Hilfsmitteln umfasst.

Ein anderer spezieller Aspekt der Erfindung sind veterinärmedizinische Formulierungen ausgewählt aus der Gruppe ektoparasitizider und endoparasitizider Formulierungen, insbesondere ausgewählt aus der Gruppe anthelmintischer, antiprotozolischer und arthropodizider Formulierungen, ganz besonders ausgewählt aus der Gruppe nematizider, platyhelminthizider, acanthocephalizider, pentastomizider, insektizider und akkarizider Formulierungen, gemäß den erwähnten Aspekten, sowie Verfahren zu ihrer Herstellung.

Ein anderer Aspekt bezieht sich auf ein Verfahren zur Behandlung einer parasitischen Infektion, insbesondere einer Infektion durch einen Parasiten ausgewählt aus der Gruppe der hier erwähnten Ektoparasiten und Endoparasiten, durch Anwendung einer wirksamen Menge einer Verbindung der Formel (I) bei einem Tier, insbesondere einem nichthumanen Tier, das dessen bedarf.

Ein anderer Aspekt bezieht sich auf ein Verfahren zur Behandlung einer parasitischen Infektion, insbesondere einer Infektion durch einen Parasiten ausgewählt aus der Gruppe der hier erwähnten Ektoparasiten und Endoparasiten, durch Anwendung einer wie hier definierten veterinärmedizinischen Formulierung bei einem Tier, insbesondere einem nichthumanen Tier, das dessen bedarf.

Ein anderer Aspekt bezieht sich auf die Verwendung der Verbindungen der Formel (I) bei der Behandlung einer Parasiteninfektion, insbesondere einer Infektion durch einen Parasiten ausgewählt aus der Gruppe der hier erwähnten Ektoparasiten und Endoparasiten, bei einem Tier, insbesondere einem nichthumanen Tier.

Im vorliegenden tiergesundheitlichen oder veterinärmedizinischen Zusammenhang schließt der Begriff "Behandlung" die prophylaktische, die metaphylaktische und die therapeutische Behandlung ein.

Bei einer bestimmten Ausführungsform werden hiermit Mischungen mindestens einer Verbindung der Formel (I) mit anderen Wirkstoffen, insbesondere mit Endo- und Ektoparasitiziden, für das veterinärmedizinische Gebiet bereitgestellt.

Auf dem Gebiet der Tiergesundheit bedeutet "Mischung" nicht nur, dass zwei (oder mehr) verschiedene Wirkstoffe in einer gemeinsamen Formulierung formuliert werden und entsprechend zusammen angewendet werden, sondern bezieht sich auch auf Produkte, die für jeden Wirkstoff getrennte Formulierungen umfassen. Dementsprechend können, wenn mehr als zwei Wirkstoffe angewendet werden sollen, alle Wirkstoffe in einer gemeinsamen Formulierung formuliert werden oder alle Wirkstoffe in getrennten Formulierungen formuliert werden; ebenfalls denkbar sind gemischte Formen, bei denen einige der Wirkstoffe gemeinsam formuliert und einige der Wirkstoffe getrennt formuliert sind. Getrennte Formulierungen erlauben die getrennte oder aufeinanderfolgende Anwendung der in Rede stehenden Wirkstoffe.

Die hier mit ihrem "Common Name" spezifizierten Wirkstoffe sind bekannt und beispielsweise im "Pesticide Manual" (siehe oben) beschrieben oder im Internet recherchierbar (z.B. http://www.alanwood.net/pesticides).

Beispielhafte Wirkstoffe aus der Gruppe der Ektoparasitizide als Mischungspartner schließen, ohne dass dies eine Einschränkung darstellen soll, die oben ausführlich aufgelisteten Insektizide und Akkarizide ein. Weitere verwendbare Wirkstoffe sind unten gemäß der oben erwähnten Klassifikation, die auf dem aktuellen IRAC Mode of Action Classification Scheme beruht, aufgeführt: (1) Acetylcholinesterase (AChE)-Inhibitoren; (2) GABA-gesteuerte Chlorid-Kanal-Blocker; (3) Natrium-Kanal-Modulatoren; (4) kompetitive Modulatoren des nicotinischen Acetylcholin-Rezeptors (nAChR); (5) allosterische Modulatoren des nicotinischen Acetylcholin-Rezeptors (nAChR); (6) allosterische Modulatoren des Glutamat-abhängigen Chloridkanals (GluCl); (7) Juvenilhormon-Mimetika; (8) verschiedene nichtspezifische (Multi-Site) Inhibitoren; (9) Modulatoren Chordotonaler Organe; (10) Milbenwachstumsinhibitoren; (12) Inhibitoren der mitochondrialen ATP-Synthase, wie ATP-Disruptoren; (13) Entkoppler der oxidativen Phosphorylierung durch Störung des Protonengradienten; (14) Blocker des nicotinischen Acetylcholinrezeptorkanals; (15) Inhibitoren der Chitinbiosynthese, Typ 0; (16) Inhibitoren der Chitinbiosynthese, Typ 1; (17) Häutungsdisruptor (insbesondere bei Dipteren, d.h. Zweiflüglern); (18) Ecdyson-Rezeptor-Agonisten; (19) Octopamin-Rezeptor-Agonisten; (21) mitochondriale Komplex-I-Elektronentransportinhibitoren; (25) mitochondriale Komplex-II-Elektronentransportinhibitoren; (20) mitochondriale Komplex-III-Elektronentransportinhibitoren; (22) Blocker des spannungsabhängigen Natriumkanals; (23) Inhibitoren der Acetyl-CoA-Carboxylase; (28) Ryanodinrezeptor-Modulatoren;

Wirkstoffe mit unbekannten oder nicht spezifischen Wirkmechanismen, z. B. Fentrifanil, Fenoxacrim, Cyclopren, Chlorobenzilat, Chlordimeform, Flubenzimin, Dicyclanil, Amidoflumet, Quinomethionat, Triarathen, Clothiazoben, Tetrasul, Kaliumoleat, Petroleum, Metoxadiazon, Gossyplur, Flutenzin, Brompropylat, Cryolit;

Verbindungen aus anderen Klassen, z.B. Butacarb, Dimetilan, Cloethocarb, Phosphocarb, Pirimiphos(-ethyl), Parathion(-ethyl), Methacrifos, Isopropyl-o-salicylat, Trichlorfon, Sulprofos, Propaphos, Sebufos, Pyridathion, Prothoat, Dichlofenthion, Demeton-S-methylsulfon, Isazofos, Cyanofenphos, Dialifos, Carbophenothion, Autathiofos, Aromfenvinfos(-methyl), Azinphos(-ethyl), Chlorpyrifos(-ethyl), Fosmethilan, Iodofenphos, Dioxabenzofos, Formothion, Fonofos, Flupyrazofos, Fensulfothion, Etrimfos;

Organochlorverbindungen, z. B. Camphechlor, Lindan, Heptachlor; oder Phenylpyrazole, z. B. Acetoprol, Pyrafluprol, Pyriprol, Vaniliprol, Sisapronil; oder Isoxazoline, z. B. Sarolaner, Afoxolaner, Lotilaner, Fluralaner;

Pyrethroide, z. B. (cis-, trans-)Metofluthrin, Profluthrin, Flufenprox, Flubrocythrinat, Fubfenprox, Fenfluthrin, Protrifenbut, Pyresmethrin, RU15525, Terallethrin, cis-Resmethrin, Heptafluthrin, Bioethanomethrin, Biopermethrin, Fenpyrithrin, cis-Cypermethrin, cis-Permethrin, Clocythrin, Cyhalothrin (lambda-), Chlovaporthrin, oder halogenierte Kohlenwasserstoffverbindungen (HCHs),

Neonicotinoide, z. B. Nithiazin

Dicloromezotiaz, Triflumezopyrim
makrocyclische Lactone, z. B. Nemadectin, Ivermectin, Latidectin, Moxidectin, Selamectin, Eprinomectin, Doramectin, Emamectinbenzoat; Milbemycinoxim

Tripren, Epofenonan, Diofenolan;

Biologicals, Hormone oder Pheromone, zum Beispiel natürliche Produkte, z.B. Thuringiensin, Codlemon oder Neem-Komponenten

Dinitrophenole, z. B. Dinocap, Dinobuton, Binapacryl;

Benzoylharnstoffe, z. B. Fluazuron, Penfluron,

Amidinderivate, z. B. Chlormebuform, Cymiazol, Demiditraz

Bienenstockvarroa-Akarizide, zum Beispiel organische Säuren, z.B. Ameisensäure, Oxalsäure.

Zu beispielhaften Wirkstoffen aus der Gruppe der Endoparasitizide, als Mischungspartner, zählen, ohne hierauf beschränkt zu sein, anthelmintische Wirkstoffe und antiprotozoische Wirkstoffe.
Zu den anthelmintischen Wirkstoffen zählen, ohne hierauf beschränkt zu sein, die folgenden nematiziden, trematiziden und/oder cestoziden Wirkstoffe:

aus der Klasse der makrocyclischen Lactone zum Beispiel: Eprinomectin, Abamectin, Nemadectin, Moxidectin, Doramectin, Selamectin, Lepimectin, Latidectin, Milbemectin, Ivermectin, Emamectin, Milbemycin;

aus der Klasse der Benzimidazole und Probenzimidazole zum Beispiel: Oxibendazol, Mebendazol, Triclabendazol, Thiophanat, Parbendazol, Oxfendazol, Netobimin, Fenbendazol, Febantel, Thiabendazol, Cyclobendazol, Cambendazol, Albendazol-sulfoxid, Albendazol, Flubendazol;

aus der Klasse der Depsipeptide, vorzugsweise cyclischen Depsipetide, insbesondere 24-gliedrigen cyclischen Depsipeptide, zum Beispiel: Emodepsid, PF1022A;

aus der Klasse der Tetrahydropyrimidine zum Beispiel: Morantel, Pyrantel, Oxantel;

aus der Klasse der Imidazothiazole zum Beispiel: Butamisol, Levamisol, Tetramisol;

aus der Klasse der Aminophenylamidine zum Beispiel: Amidantel, deacyliertes Amidantel (dAMD), Tribendimidin;

aus der Klasse der Aminoacetonitrile zum Beispiel: Monepantel;

aus der Klasse der Paraherquamide zum Beispiel: Paraherquamid, Derquantel;

aus der Klasse der Salicylanilide zum Beispiel: Tribromsalan, Bromoxanid, Brotianid, Clioxanid, Closantel, Niclosamid, Oxyclozanid, Rafoxanid;

aus der Klasse der substituierten Phenole zum Beispiel: Nitroxynil, Bithionol, Disophenol, Hexachlorophen, Niclofolan, Meniclopholan;

aus der Klasse der Organophosphate zum Beispiel: Trichlorfon, Naphthalofos, Dichlorvos/DDVP, Crufomat, Coumaphos, Haloxon;

aus der Klasse der Piperazinone/Chinoline zum Beispiel: Praziquantel, Epsiprantel;

aus der Klasse der Piperazine zum Beispiel: Piperazin, Hydroxyzin;

aus der Klasse der Tetracycline zum Beispiel: Tetracyclin, Chlorotetracyclin, Doxycyclin, Oxytetracyclin, Rolitetracyclin;

aus diversen anderen Klassen zum Beispiel: Bunamidin, Niridazol, Resorantel, Omphalotin, Oltipraz, Nitroscanat, Nitroxynil, Oxamniquin, Mirasan, Miracil, Lucanthon, Hycanthon, Hetolin, Emetin, Diethylcarbamazin, Dichlorophen, Diamfenetid, Clonazepam, Bephenium, Amoscanat, Clorsulon.

Antiprotozoische Wirkstoffe, darunter, ohne hierauf beschränkt zu sein, die folgenden Wirkstoffe:

aus der Klasse der Triazine zum Beispiel: Diclazuril, Ponazuril, Letrazuril, Toltrazuril;

aus der Klasse Polyletherionophor zum Beispiel: Monensin, Salinomycin, Maduramicin, Narasin;

aus der Klasse der makrocyclischen Lactone zum Beispiel: Milbemycin, Erythromycin;

aus der Klasse der Chinolone zum Beispiel: Enrofloxacin, Pradofloxacin;

aus der Klasse der Chinine zum Beispiel: Chloroquin;

aus der Klasse der Pyrimidine zum Beispiel: Pyrimethamin;

aus der Klasse der Sulfonamide zum Beispiel: Sulfachinoxalin, Trimethoprim, Sulfaclozin;

aus der Klasse der Thiamine zum Beispiel: Amprolium;

aus der Klasse der Lincosamide zum Beispiel: Clindamycin;

aus der Klasse der Carbanilide zum Beispiel: Imidocarb;

aus der Klasse der Nitrofurane zum Beispiel: Nifurtimox;

aus der Klasse der Chinazolinonalkaloide zum Beispiel: Halofuginon;

aus diversen anderen Klassen zum Beispiel: Oxamniquin, Paromomycin;

aus der Klasse der Vakzine oder Antigene aus Mikroorganismen zum Beispiel: Babesia canis rossi, Eimeria tenella, Eimeria praecox, Eimeria necatrix, Eimeria mitis, Eimeria maxima, Eimeria brunetti, Eimeria acervulina, Babesia canis vogeli, Leishmania infantum, Babesia canis canis, Dictyocaulus viviparus.

Alle genannten Mischungspartner können außerdem, wenn sie auf Grund ihrer funktionellen Gruppen dazu imstande sind, gegebenenfalls mit geeigneten Basen oder Säuren Salze bilden.

**Vektorbekämpfung**

Die Verbindungen der Formel (I) können auch in der Vektorbekämpfung eingesetzt werden. Ein Vektor im Sinne der vorliegenden Erfindung ist ein Arthropode, insbesondere ein Insekt oder Arachnide, der in der Lage ist, Krankheitserreger wie z. B. Viren, Würmer, Einzeller und Bakterien aus einem Reservoir (Pflanze, Tier, Mensch, etc.) auf einen Wirt zu übertragen. Die Krankheitserreger können entweder mechanisch (z. B. Trachoma durch nicht-stechende Fliegen) auf einem Wirt, oder nach Injektion (z. B. Malaria-Parasiten durch Mücken) in einen Wirt übertragen werden.
Beispiele für Vektoren und die von ihnen übertragenen Krankheiten bzw. Krankheitserreger sind:

1) Mücken

- Anopheles: Malaria, Filariose;

- Culex: Japanische Encephalitis, Filariasis, weitere virale Erkrankungen, Übertragung von anderen Würmern;
- Aedes: Gelbfieber, Dengue-Fieber, weitere virale Erkrankungen, Filariasis;
- Simulien: Übertragung von Würmern, insbesondere Onchocerca volvulus;
- Psychodidae: Übertragung von Leishmaniose

2) Läuse: Hautinfektionen, epidemisches Fleckfieber;

3) Flöhe: Pest, endemisches Fleckfieber, Bandwürmer;

4) Fliegen: Schlafkrankheit (Trypanosomiasis); Cholera, weitere bakterielle Erkrankungen;

5) Milben: Acariose, epidemisches Fleckfieber, Rickettsipocken, Tularämie, Saint-Louis-Enzephalitis, Frühsommer-Meningoenzephalitis (FSME), Krim-Kongo-Fieber, Borreliose;

6) Zecken: Borelliosen wie Borrelia bungdorferi sensu lato., Borrelia duttoni, Frühsommer-Meningoenzephalitis, Q-Fieber (Coxiella burnetii), Babesien (Babesia canis canis), Ehrlichiose.

Beispiele für Vektoren im Sinne der vorliegenden Erfindung sind Insekten, zum Beispiel Aphiden, Fliegen, Zikaden oder Thripse, die Pflanzenviren auf Pflanzen übertragen können. Weitere Vektoren, die Pflanzenviren übertragen können, sind Spinnmilben, Läuse, Käfer und Nematoden.

Weitere Beispiele für Vektoren im Sinne der vorliegenden Erfindung sind Insekten und Arachniden wie Mücken, insbesondere der Gattungen Aedes, Anopheles, z. B. A. gambiae, A. arabiensis, A. funestus, A. dirus (Malaria) und Culex, Psychodide wie Phlebotomus, Lutzomyia, Läuse, Flöhe, Fliegen, Milben und Zecken, die Krankheitserreger auf Tiere und/oder Menschen übertragen können.

Eine Vektorbekämpfung ist auch möglich, wenn die Verbindungen der Formel (I) Resistenz-brechend sind.

Verbindungen der Formel (I) sind zur Verwendung in der Prävention von Krankheiten und/oder Krankheitserregern, die durch Vektoren übertragen werden, geeignet. Somit ist ein weiterer Aspekt der vorliegenden Erfindung die Verwendung von Verbindungen der Formel (I) zur Vektorbekämpfung, z. B. in der Landwirtschaft, im Gartenbau, in Forsten, in Gärten und Freizeiteinrichtungen sowie im Vorrats- und Materialschutz.

**Schutz von technischen Materialen**

Die Verbindungen der Formel (I) eignen sich zum Schutz von technischen Materialien gegen Befall oder Zerstörung durch Insekten, z. B. aus den Ordnungen Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer weiteren Ausführungsform werden die Verbindungen der Formel (I) zusammen mit mindestens einem weiteren Insektizid und/oder mindestens einem Fungizid eingesetzt.

In einer weiteren Ausführungsform liegen die Verbindungen der Formel (I) als ein anwendungsfertiges (ready-to-use) Schädlingsbekämpfungsmittel vor, d. h., sie können ohne weitere Änderungen auf das entsprechende Material aufgebracht werden. Als weitere Insektizide oder Fungizide kommen insbesondere die oben genannten in Frage.

Überraschenderweise wurde auch gefunden, dass die Verbindungen der Formel (I) zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, verwendet werden können. Gleichfalls können die Verbindungen der Formel (I) allein oder in Kombinationen mit anderen Wirkstoffen als Antifouling-Mittel eingesetzt werden.

**Bekämpfung von tierischen Schädlingen auf dem Hygienesektor**

Die Verbindungen der Formel (I) eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren, Zecken und Milben, die in geschlossenen Räumen, wie beispielsweise Wohnungen, Fabrikhallen, Büros, Fahrzeugkabinen, Tierzuchtanlagen vorkommen. Zur Bekämpfung der tierischen Schädlinge werden die Verbindungen der Formel (I) allein oder in Kombination mit anderen Wirk- und/oder Hilfsstoffen verwendet. Bevorzugt werden sie in Haushaltsinsektizid-Produkten verwendet. Die Verbindungen der Formel (I) sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera, Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z. B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

## Herstellungsverfahren

Die erfindungsgemäßen Verbindungen können nach üblichen, dem Fachmann bekannten Methoden hergestellt werden.

**Reaktionsschema I – Methode A**

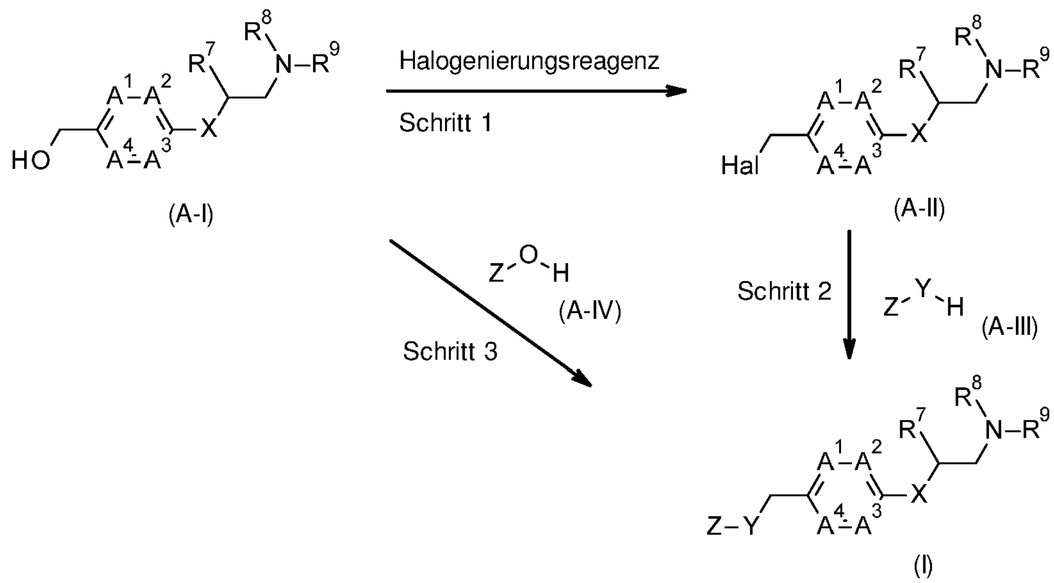

(A-I) (A-II) (A-III) (A-IV) (I)

Verbindungen der Formel (I) können beispielsweise gemäß Reaktionsschema I in ein oder zwei Schritten hergestellt werden. Die Reste $A^1$, $A^2$, $A^3$, $A^4$, Z, X, $R^5$, $R^7$, $R^8$ und $R^9$ haben die oben genannten und gegebenenfalls weitere, für die Herstellverfahren relevante Bedeutungen, die aus dem Text bzw. dem Zusammenhang hervorgehen. Y steht für O, S oder $NR^5$. Hal steht für Chlor oder Brom.

Beispielsweise können Alkohole der Formel (A-I) unter Verwendung von Halogenierungsreagenzien in Benzylhalogenide der Formel (A-II) überführt werden. Diese reagieren dann in Gegenwart von basischen Reaktionshilfsmitteln in einem zweiten Reaktionsschritt mit Verbindungen der Formel (A-III) in einer nukleophilen Substitutionsreaktion zu Verbindungen der Formel (I).

Für Verbindungen der Formel (I) in denen Y für Sauerstoff steht kann diese Reaktion alternativ auch in einem einstufigen Prozess durchgeführt werden. Dabei wird der Benzylalkohol der Formel (A-I) in situ in Gegenwart eines Trialkyl- oder Triarylphosphans und eines Azodicarboxylats aktiviert und reagiert mit einem Alkohol der Formel (A-IV) in einer nukleophilen Substitutionsreaktion zu Verbindungen der Formel (I).

**Methode A** - **Schritt 1**: Die Verbindungen der Formel (A-I) sind bekannt (z.B. [6-(4-Ethylpiperazin-1-yl)pyridin-3-yl]methanol aus WO2010/132999, tert-Butyl-4-[3-chlor-5-(hydroxymethyl)pyridin-2-yl]piperazin-1-carboxylat aus EP2050734 und tert-Butyl-3-{ [3-fluor-5-(hydroxymethyl)pyridin-2-yl]amino}pyrrolidin-1-carboxylat aus US2006/0052599) oder können nach bekannten Herstellungsverfahren erhalten werden. Weitere Verfahren zu ihrer Herstellung sind in dieser Anmeldung beschrieben worden, u.a. von (6-{2-[Methoxy(methyl)amino]ethoxy}pyridin-3-yl)methanol und (3-Fluor-5-{2-[methoxy(methyl)amino]ethoxy}pyridin-2-yl)methanol (siehe außerdem Schemata II und

VI).

Für die Halogenierungsreaktion sind zahlreiche Reaktionsbedingungen beschrieben worden. Sie sind dem Fachmann bekannt. Eine Zusammenfassung findet sich in Houben-Weyl, Methoden der Organischen Chemie, Band V/3 (Georg Thieme Verlag Stuttgart), S. 760 für Hal = Cl und für Hal = Br in Houben-Weyl, Methoden der Organischen Chemie, Band V/4 (Georg Thieme Verlag Stuttgart), S. 361. Bevorzugt werden die Benzylchloride der Formel (A-II), in denen Hal für Chlor steht, hergestellt. Sie lassen sich z.B. in Gegenwart von Thionylchlorid aus den Benzylalkoholen der Formel (A-I) herstellen. Die Reaktion kann in einem geeigneten inerten Lösungs- oder Verdünnungsmittel, z.B. Toloul oder Dichlormethan und ggfs. in Gegenwart einer katalytischen Mengen N,N-Dimethylformamid durchgeführt werden (vgl. z. B. 5-(Chlormethyl)-2-(2,2,3,3-tetrafluorpropoxy)pyridin aus WO2013/161312).

**Methode A** - **Schritt 2:** Die Verbindungen der Formel (A-III) sind bekannt, kommerziell erhältlich oder können nach bekannten Herstellungsverfahren erhalten werden (siehe z.B. WO2002017712 für 5,6-Dichlor-1-naphthol). Weitere Verfahren zu ihrer Herstellung sind in dieser Anmeldung beschrieben worden (siehe z.B. die Herstellung von 6-Fluor-3'-(trifluormethoxy)biphenyl-3-ol und 4-Chlor-3-methoxyphenol).

Die Halogenide der Formel (A-II) reagieren unter Erhitzen in Gegenwart eines basischen Reaktionshilfsmittels, wie z.B. Kalium- oder Cäsiumcarbonat, in einer nukleophilen Substitutionsreaktion mit Verbindungen der Formel (A-III) zu Verbindungen der Formel (I) in denen Y für O, S und $NR^5$ steht. Die Reaktion wird gegebenenfalls in Gegenwart eines inerten Lösungs- oder Verdünnungsmittels durchgeführt. Beispielhaft genannt seien N,N-Dimethylformamid, N-Methyl-2-pyrrolidon und Acetonitril. Die Reaktion wird für Y = O oder $NR^5$ bevorzugt bei Temperaturen von 50 - 120 °C durchgeführt, für Y = S bei Raumtemperatur (siehe WO2015/089139 für Y = O, WO2013/019621 und WO2005/049572 für Y = $NR^5$, WO2012/082566 für Y = S).

**Methode A** - **Schritt 3:** Die Verbindungen der Formel (A-IV) sind bekannt, kommerziell erhältlich oder können nach bekannten Herstellungsverfahren erhalten werden. Weitere Verfahren zu ihrer Herstellung sind in dieser Anmeldung beschrieben (siehe z.B. die Herstellung von 6-Fluor-3'-(trifluormethoxy)biphenyl-3-ol oder 4-Chlor-3-methoxyphenol).

Die Benzylalkohole der Formel (A-I) reagieren in Gegenwart eines Trialkyl- oder Triarylphosphans und eines Azodicarboxylats mit einem Alkohol der Formel (A-IV) in einer nukleophilen Substitutionsreaktion zu

Verbindungen der Formel (I) in den Y für Sauerstoff steht. Diese Reaktion ist dem Fachmann als "Mitsunobu-Reaktion" bekannt. Als Phosphan wird bevorzugt Triphenylphosphan und als Azodicarboxylat bevorzug Diethylazodicarboxylat verwendet. Die Reaktion findet in Gegenwart eines inerten Lösungs- oder Verdünnungsmittels, wie z.B. Tetrahydrofuran statt. (siehe z.B. WO2016/044789).

**Reaktionsschema II – Methode B**

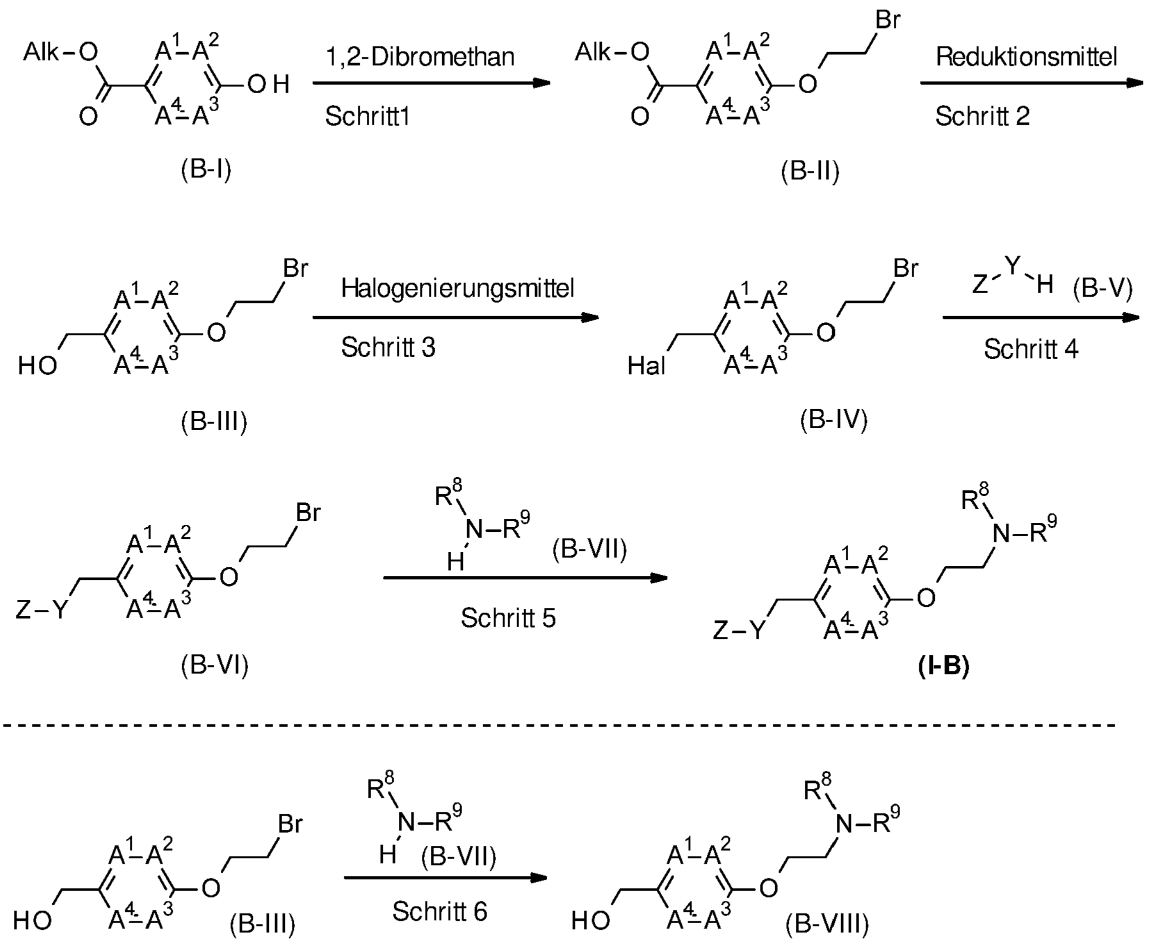


Verbindungen der Formel (I-B) können beispielsweise gemäß Reaktionsschema II hergestellt werden. Die Reste $A^1$, $A^2$, $A^3$, $A^4$, Z, $R^5$, $R^8$ und $R^9$ haben die oben genannten (wobei durch dieses Vefahren keine Ringbildung zwischen $R^8$ und $R^6$ bzw. $R^7$ möglich ist) und gegebenenfalls weitere, für die Herstellverfahren relevante Bedeutungen, die aus dem Text bzw. dem Zusammenhang hervorgehen. Y steht für O, S oder $NR^5$. Hal steht für Chlor oder Brom. Alk steht für ein ($C_1$-$C_3$)-Alkyl.

Beispielsweise können Alkohole der Formel (B-I) in Gegenwart von basischen Reaktionshilfsmitteln in einer nukleophilen Substitutionsreaktion mit 1,2-Dibromethan zu Verbindungen der Formel (B-II) reagieren. In einem nächsten Schritt kann die Alkylesterfunktion in Verbindungen der Formel (B-II) unter Einsatz eines Reduktionsmittels zu Benzylalkoholen der Formel (B-III) reduziert werden. Anschließend können Benzylalkohole der Formel (B-III) unter Verwendung von Halogenierungsreagenzien in Benzylhalogenide der Formel (B-IV) überführt werden. Diese können dann in Gegenwart von basischen Reaktionshilfsmitteln in einem weiteren Reaktionsschritt mit Verbindungen der Formel (B-V) in einer nukleophilen Substitutionsreaktion zu Verbindungen der Formel (B-VI) reagieren. In einer weiteren nukleophilen Substitutionsreaktion können Amine der Formel (B-VII) mit den Bromiden der Formel (B-VI) zu Verbindungen der Formel (I-B) umgesetzt werden.

**Methode B** - **Schritt 1**:

Die Verbindungen der Formel (B-I) sind bekannt, kommerziell erhältlich oder können nach bekannten Herstellungsverfahren erhalten werden (siehe z.B. WO2011/044181 für Methyl-3-fluor-5-hydroxypyridin-2-carboxylat).

Die Alkohole der Formel (B-I) reagieren in Gegenwart eines basischen Hilfstoffs, wie z.B. Kaliumcarbonat oder Cäsiumcarbonat, und ggfs. in Gegenwart einer katalytischen Menge Kaliumiodid in einer nukleophilen Substitutionsreaktion mit 1,2-Dibromethan. Die Reaktion wird gegebenenfalls in Gegenwart eines inerten Lösungs- oder Verdünnungsmittels durchgeführt. Beispielhaft genannt seien N,N-Dimethylformamid, N-Methyl-2-pyrrolidon und Acetonitril. Die Reaktion wird bei erhöhter Temperatur, bevorzugt bei 50 °C bis 120 °C durchgeführt (für Beispiele in denen $A^1$ bis $A^4$ für -CH steht siehe z.B. WO1998/48800).

**Methode B** - **Schritt 2:**

Die Ester der Formel (B-II) reagieren in Gegenwart eines geeigneten Reduktionsmittels, wie z.B. Natriumborhydrid oder Diisobutylaluminiumhydrid, zu Benzylalkoholen der Formel (B-III). Wird die Reaktion wird in Gegenwart von Natriumborhydrid durchgeführt wird ein geeignetes Lösungs- oder Verdünnungsmittels wie z.B. Ethanol oder Methanol verwendet. Die Reaktion wird dabei bevorzugt bei Temperaturen von 0 °C - 50 °C durchgeführt. Bei der Verwendung von Diisobutylaluminiumhydrid wird die Reaktion in einem geeigneten inerten Lösungsmittel, z.B. Toloul oder Dichlormethan durchgeführt. Die Reaktion wird dabei bevorzugt bei Temperaturen von -78 °C bis +30 °C durchgeführt (siehe z.B. die in dieser Anmeldung beschriebene Synthese von Methyl-5-(2-bromethoxy)-3-fluorpyridin-2-carboxylat). Bei der Verwendung von Diisobutylaluminiumhydrid kann eine Mischung der Benzyalkohole der Formel (B-III) mit den analogen Benzaldehyden erhalten werden. In diesem Fall wird in einer zweiten Stufe die Aldehydfunktion zu den Benzylalkoholen der Formel (B-III) reduziert. Diese Reaktion kann in Gegenwart von Natriumhydrid unter den oben genannten Bedingungen durchgeführt werden.

**Methode B** - **Schritt 3:**

Für die Halogenierungsreaktion sind zahlreiche Reaktionsbedingungen beschrieben worden. Sie sind dem Fachmann bekannt und unter Schritt 1 der Methode A bereits beschrieben worden.

**Methode B** - **Schritt 4:** Die Verbindungen der Formel (B-V) sind bekannt, kommerziell erhältlich oder können nach bekannten Herstellungsverfahren erhalten werden. Weitere Verfahren zu ihrer Herstellung sind in dieser Anmeldung beschrieben worden (siehe z.B. die Herstellung von 6-Fluor-3'-(trifluormethoxy)biphenyl-3-ol oder 4-Chlor-3-methoxyphenol).

Die Halogenide der Formel (B-IV) reagieren unter Erhitzen in Gegenwart eines basischen Reaktionshilfsmittels, wie z.B. Kalium- oder Cäsiumcarbonat, in einer nukleophilen Substitutionsreaktion mit Verbindungen der Formel (B-V) zu Verbindungen der Formel (B-VI). Die Reaktion wird gegebenenfalls in Gegenwart eines inerten Lösungs- oder Verdünnungsmittels durchgeführt. Beispielhaft genannt seien N,N-Dimethylformamid, N-Methyl-2-pyrrolidon und Acetonitril. Die Reaktion wird bevorzugt bei Temperaturen von 50 °C bis 120 °C durchgeführt.

**Methode B - Schritt 5:** Die Verbindungen der Formel (B-VII) sind bekannt, kommerziell erhältlich oder können nach bekannten Herstellungsverfahren erhalten werden (siehe z.B. EP0678504 für 1-(Aminooxy)propan). Teilweise sind sie auch als ihre Hydrochloride oder Hydrobromide bekannt und kommerziell erhältlich.

Die Bromide der Formel (B-VI) reagieren unter Erhitzen in einer nukleophilen Substitutionsreaktion mit Verbindungen der Formel (B-VII) zu Verbindungen der Formel (I-B). Die Reaktion wird gegebenenfalls in Gegenwart eines inerten Lösungs- oder Verdünnungsmittels durchgeführt. Beispielhaft genannt seien N,N-Dimethylformamid, N-Methyl-2-pyrrolidon, Acetonitril, Tetrahydrofuran. Die Reaktion wird gegebenenfalls in Gegenwart eines basischen Reaktionshilfsmittels, wie z.B. Kaliumcarbonat, Cäsiumcarbonat oder N,N-Diisopropylethylamin durchgeführt. Die Reaktion wird bevorzugt bei Temperaturen von 10 °C bis 120 °C durchgeführt (ohne basisches Reaktionshilfsmittel: WO2006/078619, in Gegenwart von $K_2CO_3$: WO2010/071885, in Gegenwart von N,N-Diisopropylethylamin: WO2006/105304)

**Methode B** - **Schritt 6:**

Die Bromide der Formel (B-III) reagieren unter Erhitzen in einer nukleophilen Substitutionsreaktion mit Verbindungen der Formel (B-VII) zu Verbindungen der Formel (B-VIII). Die Reaktionsbedingungen entsprechen den in Schritt 5 beschriebenen. Diese können unter Anwendung der Methode A zu Verbindungen der Formel (I-B) umgesetzt werden.

**Reaktionsschema III– Methode C**

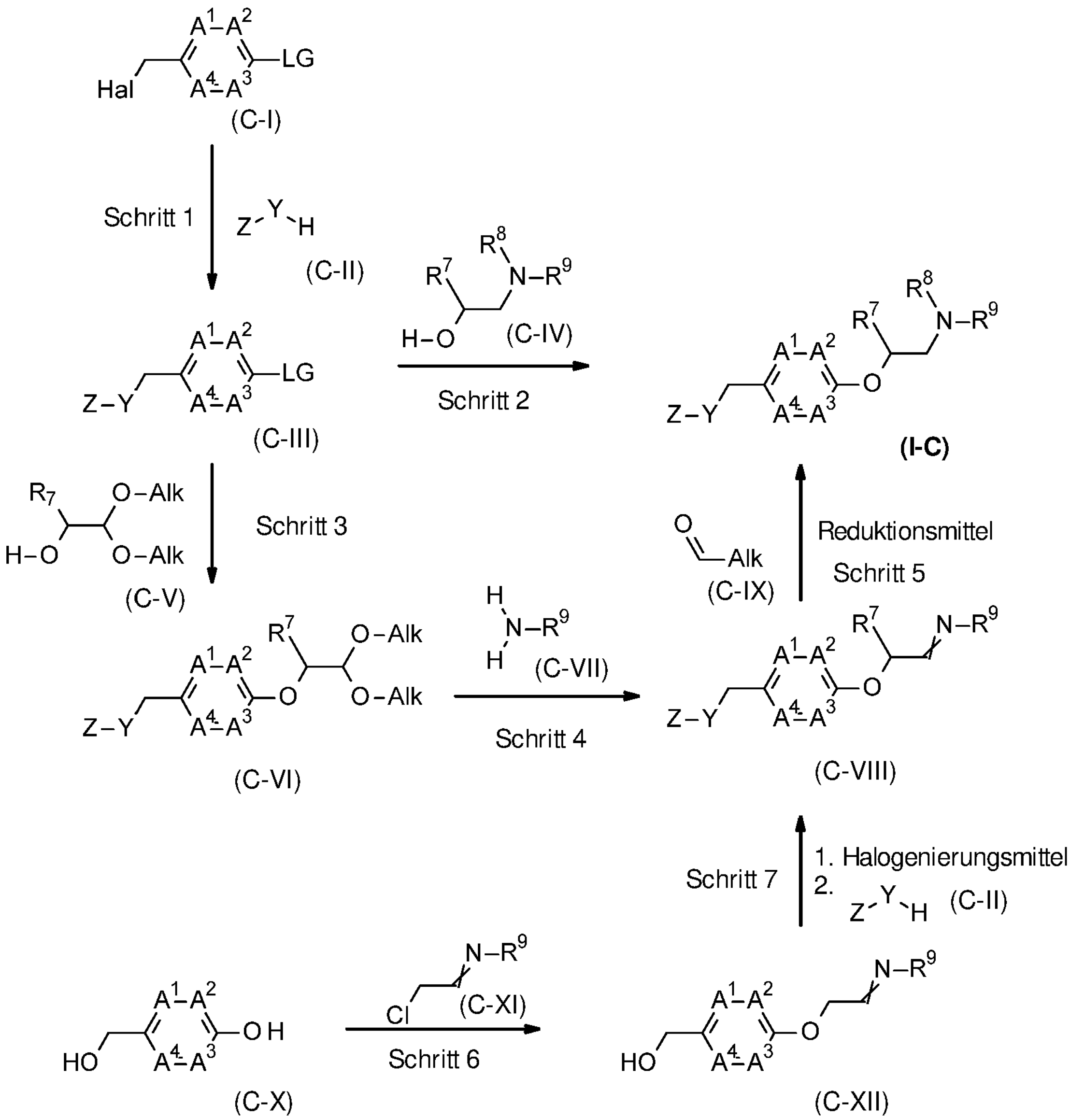


Verbindungen der Formel (I-C) können beispielsweise gemäß Reaktionsschema III hergestellt werden. Die Reste $A^1$, $A^2$, $A^3$, $A^4$, Z, $R^5$, $R^7$, $R^8$ und $R^9$ haben die oben genannten und gegebenenfalls weitere, für die Herstellverfahren relevante Bedeutungen, die aus dem Text bzw. dem Zusammenhang hervorgehen. Y steht für O, S oder $NR^5$. Hal steht für Chlor oder Brom. LG steht für Fluor oder Chlor. Alk steht für ein ($C_1$-$C_5$)-Alkyl.

Beispielsweise können Verbindungen der Formel (C-I) in Gegenwart von basischen Reaktionshilfsmitteln in einer nukleophilen Substitutionsreaktion mit Verbindungen der Formel (C-II) zu Verbindungen der Formel (C-III) reagieren. In einem nächsten Schritt können die Verbindungen der Formel (C-III) in einer nukleophilen aromatischen Substitutionsreaktion mit Verbindungen der Formel (C-IV) direkt zu Verbindungen der Formel (I-C) umgesetzt werden. Alternativ können die Verbindungen der Formel (C-III) in einer nukleophilen aromatischen Substitutionsreaktion mit Verbindungen der Formel (C-V) zu Verbindungen der Formel (C-VI) reagieren. Diese können mit Aminen der Formel (C-VII) oder ihren Hydrochloriden oder Hydrobromiden zu Verbindungen der Formel (C-VIII) kondensiert werden. Die Verbindungen der Formel (C-VIII) können anschließend in einem ersten Schritt reduziert und in einem zweiten Schritt in einer reduktiven Aminierungsreaktion mit Aldehyden der Formel (C-IX) zu Verbindungen der Formel (I-C) umgesetzt werden.

Entsprechend eines weiteren Weges zur Herstellung von Verbindungen der Formel (C-VIII) können Benzylalkhole der Formel (C-X) mit Chloriden der Formel (C-XI) zur Reaktion gebracht und so zunächst Verbindungen der Formel (C-XII) erhalten werden. Nach Überführung des Alkohols in ein Halogen, mittels eines Halogenierungsmittels, kann dieses halogenierte Intermediat in einer nukleophilen Substitutionsreaktion mit Verbindungen der Formel (C-II) zu Verbindungen der Formel (C-VIII) reagieren.

**Methode C** - **Schritt 1**: Die Benzylhalogenide der Formel (C-I) sind bekannt, kommerziell erhältlich oder können nach bekannten Herstellungsverfahren erhalten werden (siehe z.B. EP0366085 für 2-Chlor-5-(chlormethyl)pyridin oder WO2014/028669 für 5-(Brommethyl)-2-chlorpyrimidin).

Die Benzylhalogenide der Formel (C-I) reagieren unter Erhitzen in Gegenwart eines basischen Reaktionshilfsmittels, wie z.B. Kalium- oder Cäsiumcarbonat, in einer nukleophilen Substitutionsreaktion mit Verbindungen der Formel (C-II) zu Verbindungen der Formel (C-III). Die Reaktion wird gegebenenfalls in Gegenwart eines inerten Lösungs- oder Verdünnungsmittels durchgeführt. Beispielhaft genannt seien N,N-Dimethylformamid, N-Methyl-2-pyrrolidon und Acetonitril. Die Reaktion wird bevorzugt bei Temperaturen von 10 °C bis 120 °C durchgeführt (siehe z.B. US2003/0092739).

**Methode C - Schritt 2:** Die Alkohole der Formel (C-IV) sind bekannt, kommerziell erhältlich oder können nach bekannten Herstellungsverfahren erhalten werden (siehe z.B. Journal of Organic Chemistry, 1957, 22, 579 für 2-[Methoxy(methyl)amino]ethanol).

Die Verbindungen der Formel (C-III) reagieren in Gegenwart eines basischen Reaktionshilfsmittels, wie z.B. Natriumhydrid, in einer nukleophilen aromatischen Substitutionsreaktion mit Alkoholen der Formel (C-IV) zu Verbindungen der Formel (I-C). Die Reaktion wird in Gegenwart eines inerten Lösungs- oder Verdünnungsmittels durchgeführt. Beispielhaft genannt seien N,N-Dimethylformamid, N-Methyl-2-pyrrolidon und Tetrahydrofuran. Die Reaktion wird bevorzugt bei Temperaturen von 0 °C bis 120 °C durchgeführt (siehe z.B. EP2236507 für LG = Cl oder EP0135894 für LG = F).

**Methode C - Schritt 3:** Die Verbindungen der Formel (C-III) reagieren in einer nukleophilen aromatischen Substitutionsreaktion mit Verbindungen der Formel (C-V) zu Acetalen der Formel (C-VI). Diese Reaktion verläuft unter den in Schritt 2 der Methode C beschriebenen Bedingungen.

**Methode C** - **Schritt 4:** Die Verbindungen der Formel (C-VII) sind bekannt, kommerziell erhältlich oder können nach bekannten Herstellungsverfahren erhalten werden (siehe z.B. EP0678504 für 1-(Aminooxy)propan). Teilweise sind sie auch als ihre Hydrochloride oder Hydrobromide bekannt und kommerziell erhältlich.

Die Acetale der Formel (C-VI) reagieren ggfs. in Gegenwart einer Säure, wie z.B. para-Toluolsulfonsäure (siehe z.B. WO2012/038851), oder eines Dehydratisierungsreagenzes, wie z.B. Magnesiumsulfat (siehe z.B. WO2010/054024), mit Verbindungen der Formel (C-VII) oder ihren Hydrochloriden oder Hydrobromiden in einer Kondensationsreaktion zu Verbindungen der Formel (C-VIII). Die Reaktion wird in Gegenwart eines inerten Lösungs- oder Verdünnungsmittels durchgeführt. Beispielhaft genannt seien Ethanol, Methanol, Acetonitril, Tetrahydrofuran und Wasser sowie Mischungen dieser Lösungsmittel. Die Reaktion wird bevorzugt bei Temperaturen von 10 °C bis 80 °C durchgeführt.

**Methode C - Schritt 5**: Die Aldehyde der Formel (C-IX) sind bekannt, kommerziell erhältlich oder können nach bekannten Herstellungsverfahren erhalten werden.

Die Verbindungen der Formel (C-VIII) können in einer zweistufigen Eintopf-Reaktion zu Verbindungen der Formel (I-C) umgesetzt werden. In der ersten Stufe reagieren die Verbindungen der Formel (C-VIII) zunächst in Gegenwart eines Reduktionsmittels, wie z.B. Natriumcyanoborhydrid, zu einer Zwischenstufe der allgemeinen Formel (I-C) in der $R^8$ für Wasserstoff steht. Die Reaktion wird in Gegenwart eines Lösungs-oder Verdünnungsmittels, wie z.B. Essigsäure, bei Temperaturen von 10 °C bis 80 °C durchgeführt.

In der zweiten Stufe reagiert diese Zwischenstufe in Gegenwart eines Reduktionsmittels mit Aldehyden der Formel (C-IX) in einer reduktiven Alkylierung zu Verbindungen der Formel (I-C) in denen $R^8$ für gegebenenfalls an $C_2$ bis $C_6$ substituiertes ($C_1$-$C_6$)-Alkyl steht. Die Reaktion wird in Gegenwart eines Lösungs- oder Verdünnungsmittels, wie z.B. Essigsäure, bei Temperaturen von 10 °C bis 80 °C durchgeführt.

**Methode C - Schritt 6**: Die Verbindungen der Formel (C-X) und der Formel (C-XI) sind bekannt, kommerziell erhältlich oder können nach bekannten Herstellungsverfahren erhalten werden. Weitere Verfahren zu ihrer Herstellung sind in dieser Anmeldung beschrieben z.B. von 2-Fluor-6-(hydroxymethyl)pyridin-3-ol.

Die Verbindungen der Formel (C-X) reagieren mit Chloriden der Formel (C-XI) in Gegenwart eines basischen Reaktionshilfsmittels, wie z.B. Kalium- oder Cäsiumcarbonat, in einer nukleophilen Substitutionsreaktion mit Verbindungen der Formel (C-XI) zu Verbindungen der Formel (C-XII). Die Reaktion wird gegebenenfalls in Gegenwart eines inerten Lösungs- oder Verdünnungsmittels durchgeführt. Beispielhaft genannt seien N,N-Dimethylformamid, N-Methyl-2-pyrrolidon und Acetonitril. Die Reaktion wird bevorzugt bei Temperaturen von 20 °C bis 120 °C durchgeführt.

**Methode C** - **Schritt 7:** Die Benzylalkohole der Formel (C-XII) können nach Überführung der Alkoholfunktion in ein Halogen mit Verbindungen der Formel (C-II) in einer nukleophilen Substitutionsreaktion zu Verbindungen der Formel (C-VIII) reagieren. Diese Reaktion verläuft unter den in Schritt 1 und Schritt 2 der Methode A beschriebenen Bedingungen.

**Reaktionsschema IV– Methode D**

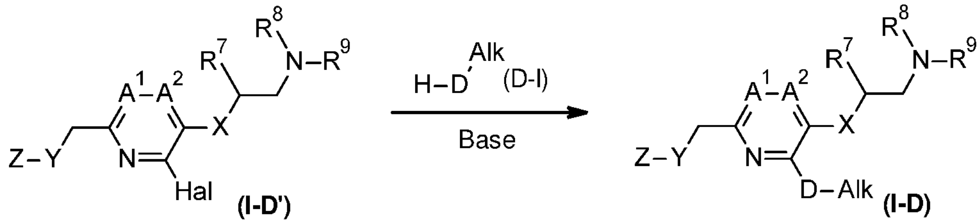


Verbindungen der Formel (I-D) können beispielsweise gemäß Reaktionsschema IV hergestellt werden. Die Reste $A^1$, $A^2$, X, Y, Z, $R^7$, $R^8$ und $R^9$ haben die oben genannten und gegebenenfalls weitere, für die Herstellverfahren relevante Bedeutungen, die aus dem Text bzw. dem Zusammenhang hervorgehen. Hal steht für Chlor oder Fluor. D steht für O oder S. Alk steht für ein gegebenenfalls substituiertes ($C_1$-$C_4$) Alkyl.

Die Verbindungen der Formel (I-D') können nach bekannten oder analog der, in dieser Anmeldung beschriebenen, Herstellungsverfahren erhalten werden. Die Thiole und Alkohole (D-I) und ihre Natriumsalze sind bekannt, kommerziell erhältlich oder können nach bekannten Herstellungsverfahren erhalten werden. Die Verbindungen der Formel (I-D') reagieren in Gegenwart eines basischen Reaktionshilfsmittels, wie z.B. Natriumhydrid, in einer nukleophilen aromatischen Substitutionsreaktion mit Alkoholen und Thiolen der Formel (D-I) zu Verbindungen der Formel (I-D). Es können auch direkt die Natriumsalze der Alkohole und Thiole der Formel (D-I) eingesetzt werden. In diesem Fall erübrigt sich die Zugabe einer Base. Die Reaktion wird in Gegenwart eines inerten Lösungs- oder Verdünnungsmittels durchgeführt. Beispielhaft genannt seien N,N-Dimethylformamid, N,N-Dimethylacetamid, N-Methyl-2-pyrrolidon und Tetrahydrofuran. Die Reaktion wird bevorzugt bei Temperaturen von 0 °C bis 80 °C durchgeführt.

**Reaktionsschema V– Methode E**

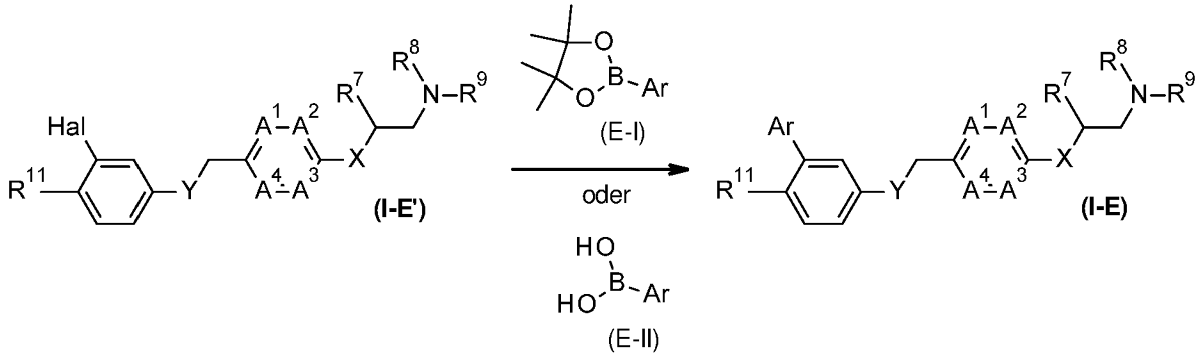


Verbindungen der Formel (I-E) können beispielsweise gemäß Reaktionsschema V hergestellt werden. Die Reste $A^1$, $A^2$, $A^3$, $A^4$, X, Y, $R^7$, $R^8$, $R^9$ und $R^{11}$ haben die oben genannten und gegebenenfalls weitere, für die Herstellverfahren relevante Bedeutungen, die aus dem Text bzw. dem Zusammenhang hervorgehen. Hal steht für Brom oder Iod. Ar steht für jeweils gegebenenfalls substituiertes Phenyl, Pyrid-2-yl, Pyrid-3-yl, Pyrid-4-yl, Thiophen-2-yl oder Thiophen-3-yl.

Die Verbindungen der Formel (I-E') können nach im Prinzip bekannten oder analog der, in dieser Anmeldung beschriebenen, Herstellungsverfahren erhalten werden. Die Boronsäureester (E-I) und die Boronsäuren (E-II) sind bekannt, kommerziell erhältlich oder können nach bekannten Herstellungsverfahren erhalten werden.

Die Verbindungen der Formel (I-E') reagieren in Gegenwart von geeigneten Kupplungskatalysatoren, wie z. B. Tetrakis(triphenylphosphin)-palladium(0) oder [1,1'-Bis(diphenylphosphino)ferrocen]-dichlorpalladium(II) in Anwesenheit einer Base, wie z. B. Kaliumcarbonat oder Natriumcarbonat mit gegebenenfalls substituierten Arylboronsäuren (E-II) bzw. Arylboronsäurepinakolestern (E-I) zu Verbindungen der Formel (I-E) (siehe z.B. WO2014/201206 oder WO2016/059097) Die Reaktion findet in einem inerten Lösungs- bzw. Verdünnungsmittel statt. Beispielhaft genannt seien 1,4-Dioxan, Toluol oder Tetrahydrofuran jeweils ggfs. in Kombination mit Wasser. Die Reaktion wird bevorzugt bei Temperaturen von 40 °C bis 120 °C durchgeführt.

**Reaktionsschema VI– Methode F**

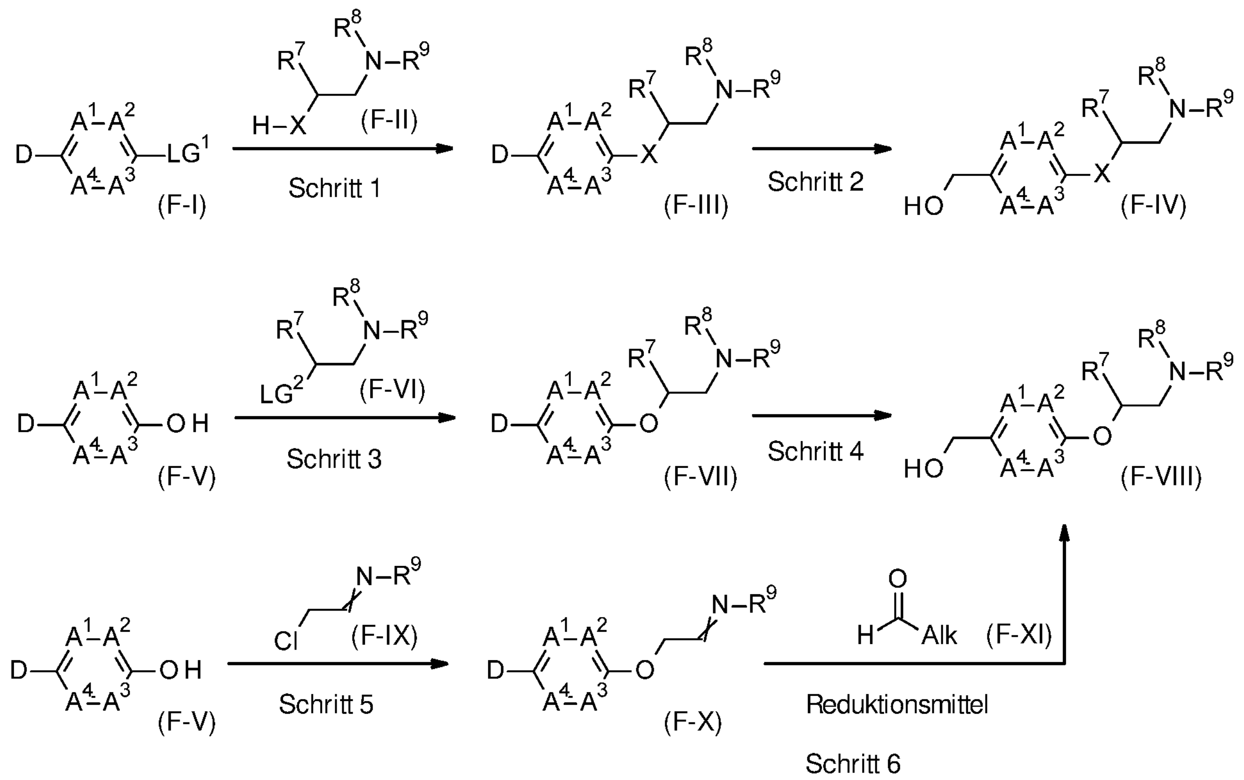


Verbindungen der Formel (F-VIII) und (F-IV) können beispielsweise gemäß Reaktionsschema VI hergestellt werden. Die Reste $A^1$, $A^2$, $A^3$, $A^4$, $R^6$, $R^7$, $R^8$ und $R^9$ haben die oben genannten und gegebenenfalls weitere, für die Herstellverfahren relevante, Bedeutungen, die aus dem Text bzw. dem Zusammenhang hervorgehen. X steht für O, $NR^6$ oder S. $LG^1$ steht für Chlor oder Fluor. $LG^2$ steht für Chlor oder Brom. Alk steht für ein gegebenenfalls substituiertes ($C_1$-$C_5$)-Alkyl. D steht für Cyano, ($C_1$-$C_3$)-Alkoxycarbonyl, Formyl oder $CH_2OPG$. PG steht für eine Schutzgruppe wie z.B. tert-Butyldimethylsilyl. Schutzgruppen für Alkohole, ihre Einführung und Spaltung sind dem Fachmann bekannt. Eine Zusammenfassung findet sich z.B. in T. W. Greene, P. G. M. Wuts, Protective Groups in Organic Synthesis, 3.Auflage, 1999 (Wiley-Interscience).

**Methode F** - **Schritt 1**: Die Verbindungen der Formel (F-I) und der Formel (F-II) sind bekannt, kommerziell erhältlich oder können nach bekannten Herstellungsverfahren erhalten werden (siehe z.B. Journal of Organic Chemistry, 1957, 22, 579 für 2-[Methoxy(methyl)amino]ethanol).

Die Verbindungen der Formel (F-I) reagieren in Gegenwart eines basischen Reaktionshilfsmittels, wie z.B. Natriumhydrid oder Kaliumcarbonat, in einer nukleophilen aromatischen Substitutionsreaktion mit Verbindungen der Formel (F-II) zu Verbindungen der Formel (F-III). Die Reaktion wird in Gegenwart eines inerten Lösungs- oder Verdünnungsmittels durchgeführt. Beispielhaft genannt seien N,N-Dimethylformamid, N,N-Dimethylacetamid, N-Methyl-2-pyrrolidon und Acetonitril. Die Reaktion wird bevorzugt bei Temperaturen von 0 °C bis 100 °C durchgeführt. (siehe z.B. die, in dieser Anmeldung, beschriebenen Synthesen von 5-{2-[Methoxy(methyl)amino]ethoxy}-3-methylpyridin-2-carbonitril, 6-{2-[Methoxy(methyl)amino]ethoxy} nicotinaldehyd, Methyl-3-methoxy-5- {2-[methoxy(methyl)amino]ethoxy}pyridin-2-carboxylat) und Ethyl-5-(4-ethylpiperazin-1-yl)-3-fluorpyridin-2-carboxylat).

**Methode F** - **Schritt 2:** Die Verbindungen der Formel (F-III) können in Abhängigkeit vom Substituenten D in einem einstufigen oder ggfs. mehrstufigen Verfahren in Verbindungen der Struktur (F-IV) überführt werden.

Für den Fall, dass D für Cyano steht kann die Cyanofunktion in Gegenwart einer Base, wie z.B. Natriumhydroxid zur Säurefunktion hydrolysiert werden. Die Reaktion wird in Gegenwart eines inerten Lösungs- oder Verdünnungsmittels durchgeführt. Beispielhaft genannt seien Ethanol, Methanol, Tetrahydrofuran und Wasser, oder ggfs. Mischungen dieser Stoffe. Die Reaktion wird bevorzugt bei Temperaturen von 20 °C bis 120 °C durchgeführt (siehe z.B. WO2010/043377 für die Synthese von (6-Methoxypyridin-3-yl)methanol oder die Synthese von Methyl-5-{2-[methoxy(methyl)amino]ethoxy}-3-methylpyridin-2-carboxylat in dieser Anmeldung). Anschließend kann diese Säurefunktion in Gegenwart einer Mineralsäure, wie z.B. Schwefelsäure und eines Alkanols der Kettenlänge ($C_1$-$C_3$) in Verbindungen der Formel (F-III) überführt werden in denen D für ($C_1$-$C_3$)-Alkoxycarbonyl steht (siehe z.B. WO2003/084917 für die Synthese von Methyl-3-brompyridin-2-carboxylat oder die Synthese von Methyl-5-{2-[methoxy(methyl)amino]ethoxy}-3-methylpyridin-2-carboxylat in dieser Anmeldung). Die Reaktion wird bevorzugt in Gegenwart eines Überschusses des ($C_1$-$C_3$)-Alkanols als Lösungs- oder Verdünnungsmittel durchgeführt, ggfs. in Mischung mit einem anderen Lösungs- oder Verdünnungsmittels. Die Reaktion wird bevorzugt bei Temperaturen von 40 °C bis 100 °C durchgeführt.

Für den Fall, dass D für ($C_1$-$C_3$)-Alkoxycarbonyl steht kann die Esterfunktion in Gegenwart eines geeigneten Reduktionsmittels, wie z.B. Natriumborhydrid oder Diisobutylaluminiumhydrid, zu Benzylalkoholen der Formel (F-IV) reduziert werden. Wird die Reaktion in Gegenwart von Natriumborhydrid durchgeführt wird ein geeignetes Lösungs- oder Verdünnungsmittels wie z.B. Ethanol oder Methanol verwendet. Die Reaktion wird dabei bevorzugt bei Temperaturen von 0 °C bis 50 °C durchgeführt. Bei der Verwendung von Diisobutylaluminiumhydrid wird die Reaktion in einem geeigneten inerten Lösungsmittel, z.B. Toluol oder Dichlormethan durchgeführt. Die Reaktion wird dabei bevorzugt bei Temperaturen von -78 °C bis +30 °C durchgeführt (siehe z.B. die in dieser Anmeldung beschriebenen Synthesen von (5-{2-[Methoxy(methyl)amino]ethoxy}-3-methylpyridin-2-yl)methanol) und (3-Methoxy-5-{2-[methoxy(methyl)amino]ethoxy}pyridin-2-yl)methanol).

Für den Fall, dass D für Formyl steht kann die Formylgruppe in Gegenwart eines geeigneten Reduktionsmittels, wie z.B. Natriumborhydrid, zu Benzylalkoholen der Formel (F-IV) reduziert werden. Wird die Reaktion in Gegenwart von Natriumborhydrid durchgeführt wird ein geeignetes Lösungs- oder Verdünnungsmittels wie z.B. Ethanol oder Methanol ggfs. in Mischung mit Tetrahydrofuran verwendet. Die Reaktion wird dabei bevorzugt bei Temperaturen von 0 °C bis 50 °C durchgeführt.

Für den Fall, dass D für $-CH_2OPG$ steht, kann die Schutzgruppe in Abhängigkeit von ihrer Art unter verschiedenen Bedingungen abgespalten werden. Die Bedingungen für die Spaltung von Alkohol-Schutzgruppen sind dem Fachmann bekannt. Eine Zusammenfassung findet sich z.B. in T. W. Greene, P. G. M. Wuts, Protective Groups in Organic Synthesis, 3.Auflage, 1999 (Wiley-Interscience).

**Methode F - Schritt 3:** Die Halogenide der Formel (F-VI) sind bekannt, kommerziell erhältlich oder können nach den, in dieser Anmeldung beschriebenen, Herstellungsverfahren erhalten werden (siehe z.B. Herstellung von 2-Brom-N-methoxy-N-methylethanaminhydrobromid (1:1)). Die Verbindungen der Formel (F-V) sind bekannt, kommerziell erhältlich oder können nach bekannten Herstellverfahren erhalten werden (siehe z.B. die, in dieser Anmeldung beschriebene, Herstellung von 2-({ [tert-Butyl(dimethyl)silyl]oxy}methyl)pyrimidin-5-ol).

Die Verbindungen der Formel (F-VI) reagieren in Gegenwart eines basischen Reaktionshilfsmittels, wie z.B. Kaliumcarbonat, und ggfs. in Gegenwart von Natriumiodid in einer nukleophilen Substitutionsreaktion mit Alkoholen der Formel (F-V) zu Verbindungen der Formel (F-VII). Die Reaktion wird in Gegenwart eines inerten Lösungs- oder Verdünnungsmittels durchgeführt. Beispielhaft genannt seien N,N-Dimethylformamid, N-Methyl-2-pyrrolidon und Tetrahydrofuran. Die Reaktion wird bevorzugt bei Temperaturen von 0 °C bis 100 °C durchgeführt (siehe z.B. WO2016/040185 für die Reaktion von 2-Brom-N,N-dimethylethanamin mit 5-Brompyridin-3-ol).

**Methode F - Schritt 4:** Die Verbindungen der Formel (F-VII) können in Abhängigkeit vom Substituenten D in einem einstufigen oder ggfs. mehrstufigen Verfahren in Verbindungen der Struktur (F-VIII) überführt werden. Die Reaktionsbedingungen entsprechen denen für die Reaktion von Verbindungen der Formel (F-III) zu Verbindungen der Formel (F-IV) in Schritt 2 beschriebenen.

**Methode F** - **Schritt 5:** Die Verbindungen der Formel (F-IX) sind bekannt, kommerziell erhältlich oder können nach bekannten Herstellungsverfahren erhalten werden.

Die Verbindungen der Formel (F-V) reagieren mit Chloriden der Formel (F-IX) in Gegenwart eines basischen Reaktionshilfsmittels, wie z.B. Kalium- oder Cäsiumcarbonat, in einer nukleophilen Substitutionsreaktion zu Verbindungen der Formel (F-X). Die Reaktion wird gegebenenfalls in Gegenwart eines inerten Lösungs- oder Verdünnungsmittels durchgeführt. Beispielhaft genannt seien N,N-Dimethylformamid, N-Methyl-2-pyrrolidon und Acetonitril. Die Reaktion wird bevorzugt bei Temperaturen von 20°C bis 120 °C durchgeführt (siehe z.B. die in dieser Anmeldung beschriebenen Synthese von Methyl-3-fluor-5-[2-(methoxyimino)ethoxy]pyridin-2-carboxylat).

**Methode F** - **Schritt 6:** Die Aldehyde der Formel (F-XI) sind bekannt, kommerziell erhältlich oder können nach bekannten Herstellungsverfahren erhalten werden.

Die Verbindungen der Formel (F-X) können in einer zweistufigen Eintopf-Reaktion zu Verbindungen der Formel (F-VIII) umgesetzt werden. In der ersten Stufe reagieren die Verbindungen der Formel (F-X) zunächst in Gegenwart eines Reduktionsmittels, wie z.B. Natriumcyanoborhydrid, zu einer Zwischenstufe der Formel (F-VIII) in der $R^8$ für Wasserstoff steht. Die Reaktion wird in Gegenwart eines Lösungs- oder Verdünnungsmittels, wie z.B. Essigsäure, bei Temperaturen von 10°C bis 80 °C durchgeführt.

In der zweiten Stufe reagiert diese Zwischenstufe in Gegenwart eines Reduktionsmittels mit Aldehyden der Formel (F-XI) in einer reduktiven Alkylierung zu Verbindungen der Formel (F-VIII) in denen $R^8$ für gegebenenfalls an $C_2$ bis $C_6$ substituiertes ($C_1$-$C_6$)-Alkyl steht. Die Reaktion wird in Gegenwart eines Lösungs- oder Verdünnungsmittels, wie z.B. Essigsäure, bei Temperaturen von 10 °C bis 80 °C durchgeführt.

**Reaktionsschema VII- Methode G**

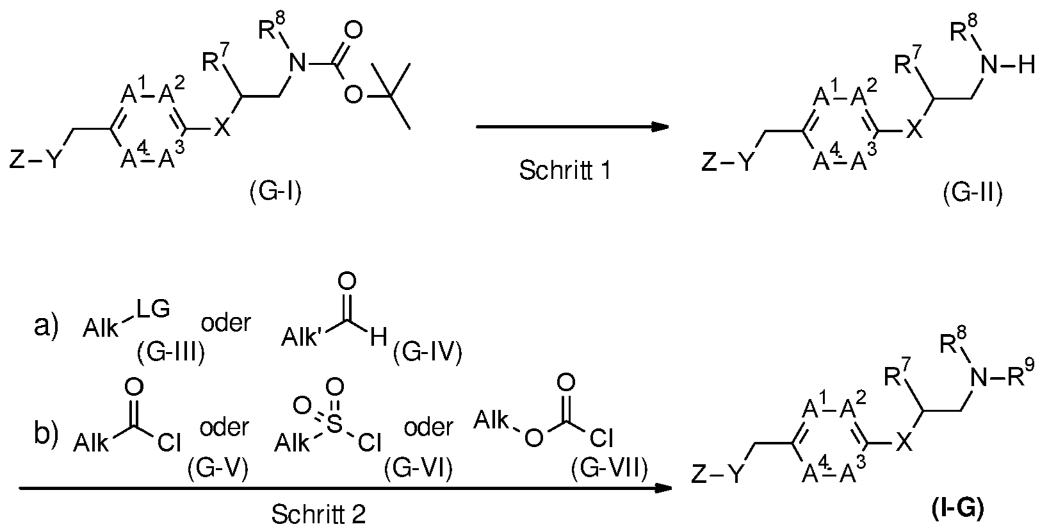


Verbindungen der Formel (I-G) können beispielsweise gemäß Reaktionsschema VII hergestellt werden. Die Reste $A^1$, $A^2$, $A^3$, $A^4$, $R^7$, $R^8$, X, Y, und Z haben die oben genannten und gegebenenfalls weitere, für die Herstellverfahren relevante Bedeutungen, die aus dem Text bzw. dem Zusammenhang hervorgehen. $R^6$ steht für gegebenenfalls substituiertes ($C_1$-$C_4$)-Alkyl, ($C_3$-$C_6$)-Cycloalkyl, ($C_1$-$C_4$)-Alkanoyl oder einen Ringschluss mit $R^8$ der aus 1 bis 3 $CH_2$-Gruppen gebildet wird. $R^5$ steht für gegebenenfalls substituiertes ($C_1$-$C_4$)-Alkyl. $R^9$ steht für gegebenenfalls substituiertes ($C_1$-$C_6$)-Alkyl, ($C_1$-$C_6$)-Alkylcarbonyl, ($C_1$-$C_6$)-Alkylsulfonyl oder ($C_1$-$C_6$)-Alkoxycarbonyl. Alk steht für eine gegebenenfalls substituierte ($C_1$-$C_6$)-Alkylgruppe. Alk' steht für eine gegebenenfalls substituierte ($C_1$-$C_5$)-Alkylgruppe. LG steht für eine Abgangsgruppe wie Halogen oder den Ester einer Sulfonsäure.

**Methode G** - **Schritt 1**: Die Verbindungen der Formel (G-I) können entsprechend der in Reaktionsschemata I und III dargestellten Methoden erhalten werden.

Die tert-Butoxycarbonyl-Gruppe von Verbindungen der Formel (G-I) lässt sich unter sauren Bedingungen abspalten. Als Säuren werden bevorzugt Chlorwasserstoff oder Trifluoressigsäure verwendet. Die Reaktion wird gegebenenfalls in Gegenwart eines inerten Lösungs- oder Verdünnungsmittels durchgeführt oder in reiner Trifluoressigsäure. Als Lösungsmittels seien beispielhaft genannt 1,4-Dioxan, Dichlormethan und Wasser. Die Reaktion wird bevorzugt bei Temperaturen von 0°C bis 60 °C durchgeführt. (siehe z.B. die, in dieser Anmeldung, beschriebenen Synthesen von 5-{[(4-Chlor-1-naphthyl)oxy]methyl}-2-(pyrrolidin-3-yloxy)pyridin oder US2015/238641).

**Methode G** - **Schritt 2:** Die Verbindungen der Formel (G-II) können in Abhängigkeit vom Substituenten $R^9$ in einem einstufigen Verfahren in Verbindungen der Struktur (**1-G**) überführt werden.

a) Die Verbindungen der Struktur (I-G) in denen $R^9$ für gegebenenfalls substituiertes ($C_1$-$C_6$)-Alkyl steht werden erhalten indem Verbindungen der Formel (G-II) mit Alkylierungsmitteln der allgemeinen Struktur (G-III) in Gegenwart eines basischen Reaktionshilfsmittels wie z.B. Kaliumcarbonat oder Triethylamin zur Reaktion gebracht werden. Als Abgangsgruppe LG kommen bevorzugt die Halogene Chlor, Brom oder Iod zum Einsatz oder die Ester einer Sulfonsäure wie z.B. der Methansulfonsäure oder der p-Toluolsufonsäure. Die Reaktion wird gegebenenfalls in Gegenwart eines inerten Lösungs- oder Verdünnungsmittels durchgeführt, wie z.B. Acetonitril, Dichlormethan oder N,N-Dimethylformamid. Die Reaktion wird bevorzugt bei Temperaturen von 0 °C bis 100 °C durchgeführt (siehe z.B. WO2014/207240 oder WO2014/116684).
Alternativ reagieren Verbindungen der Formel (G-II) in Gegenwart eines Reduktionsmittels wie z.B. Natriumcyanoborhydrid mit Aldehyden der Formel (G-IV) in einer reduktiven Alkylierung zu Verbindungen der Formel (I-G) in denen $R^9$ für gegebenenfalls an $C_2$ bis $C_6$ substituiertes ($C_1$-$C_6$)-Alkyl steht. Die Reaktion wird in Gegenwart eines Lösungs- oder Verdünnungsmittels, wie z.B. Essigsäure, bei Temperaturen von 10 °C bis 80 °C durchgeführt

b) Die Verbindungen der Struktur (I-G) in denen $R^9$ für gegebenenfalls substituiertes ($C_1$-$C_6$)-Alkylcarbonyl, ($C_1$-$C_6$)-Alkylsulfonyl oder ($C_1$-$C_6$)-Alkoxycarbonyl steht werden erhalten indem Verbindungen der Formel (G-II) mit Acylchloriden der allgemeinen Struktur (G-V) oder mit oder mit Sulfonsäurechloriden der allgemeinen Struktur (G-VI) oder mit Chlorameisesäureestern der allgemeinen Struktur (G-VII) zur Reaktion gebracht werden. Die Reaktionen werden bevorzugt in Gegenwart eines basischen Reaktionshilfsmittels wie z.B. Triethylamin oder Diisopropylethylamin und eines inerten Lösungs-oder Verdünnungsmittels durchgeführt, wie z.B. Dichlormethan, Tetrahydrofuran

oder Acetonitril. Die Reaktionen werden bevorzugt bei Temperaturen von 0 °C bis 100 °C durchgeführt (siehe z.B. US2015/259317 für eine Reaktion mit einem Acylchloriden, WO2004/041264 für eine Reaktion mit einem Sulfonsäurechlorid oder WO2004/089925 für eine Reaktion mit einem Chlorameisensäureester).

Eine weitere Ausführungsform der Erfindung bezieht sich auf Zwischenprodukte und umfasst folgende Verbindungen der Formel (Va) oder (Vb):

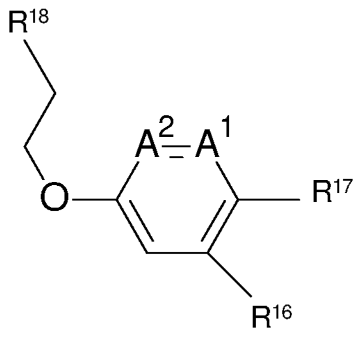

(Va)

,

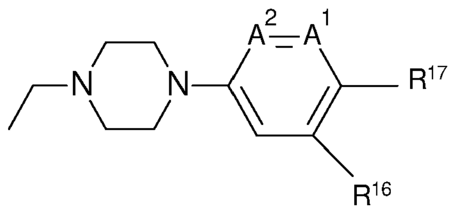

(Vb)

in welcher

$A^1$ für N oder CH steht,
$A^2$ für N oder CH steht, wobei zumindest $A^1$ oder $A^2$ für N stehen und vorzugsweise $A^1$ für N und $A^2$ für CH steht,
$R^{16}$ für Wasserstoff, Methyl, Fluor oder Methoxy steht,
$R^{17}$ für Cyano, Hydroxymethyl, Methylcarboxy, Ethylcarboxy oder 3-Bromphenoxymethyl steht,
$R^{18}$ bei den Verbindungen der Formel (Va) für einen Rest:

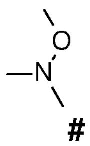 , 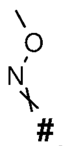 , 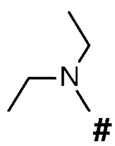

oder Brom steht, mit der Maßgabe, dass folgende Verbindungen nicht umfasst sind:

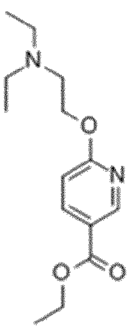 , 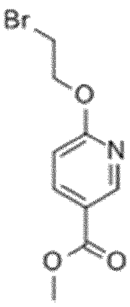 , 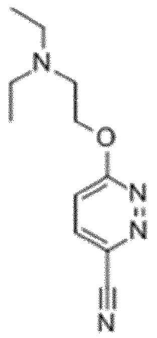 , 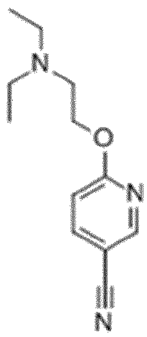 , 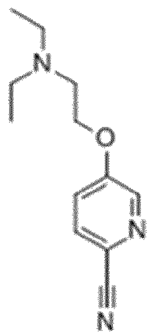 , 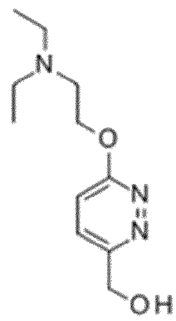 ,

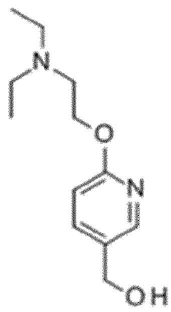 , 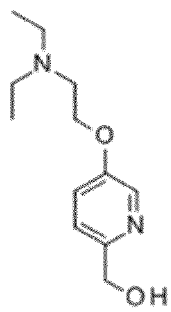 .

In einer bevorzugten Ausführungsform der Erfindung steht $R^{18}$ für einen Rest:

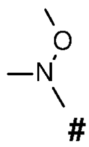 oder 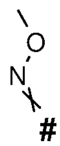 .

In einer weiteren bevorzugten Ausführungsform der Erfindung stehen die Verbindungen der Formel (Vb) für:

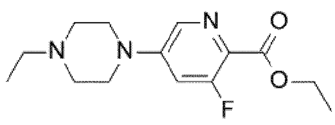

(Vb-1)

Weiterhin bezieht sich eine besonders bevorzugte Ausführungsform der Erfindung auf Verbindungen gemäß Formel (Va-1 bis Va-14) und gemäß Formel (Vb-1):

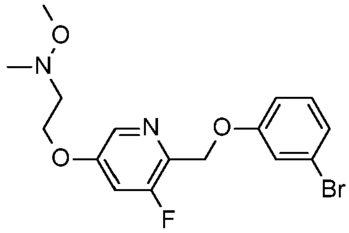

(Va-I)

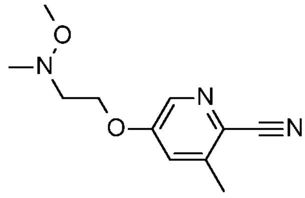

(Va-2)

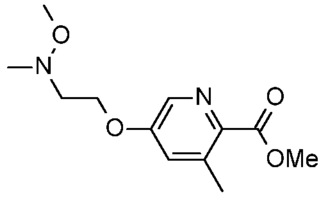

(Va-3)

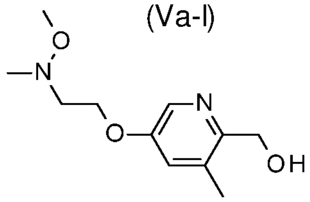

(Va-4)

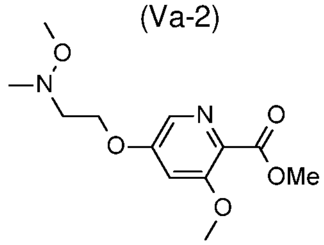

(Va-5)

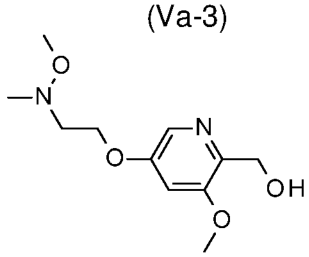

(Va-6)

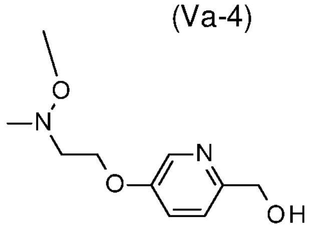

(Va-7)

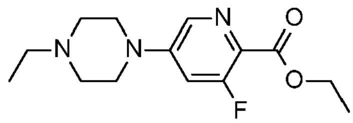

(Vb-1)

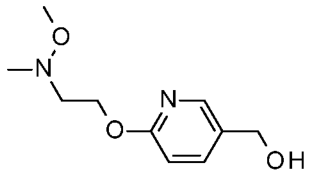

(Va-8)

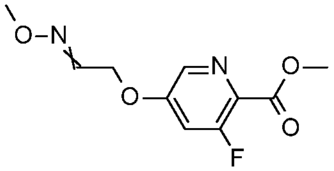

(Va-9)

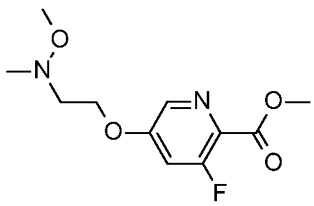

(Va-10)

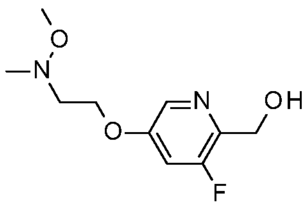

(Va-11)

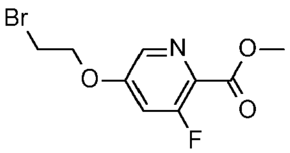

(Va-12)

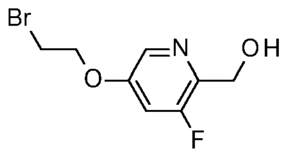

(Va-13)

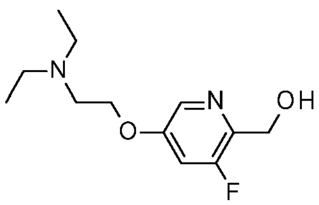

(Va-14)

Gemäß oben beschriebenen Herstellverfahren wurden u.a. die in Tabelle 1 beschriebenen Verbindungen der Formel (I) erhalten.

**Tabelle 1**

Verbindungen der Formel (I-a)

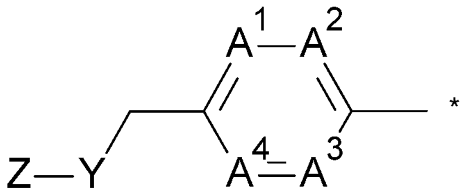

Wobei die Substituenten Z, Y, $A^1$, $A^2$, $A^3$, $A^4$ und * folgende Bedeutung haben:

| **Bsp.-Nr.** | **Z** | **Y** | **$A^1$** | **$A^2$** | **$A^3$** | **$A^4$** | ** |
|---|---|---|---|---|---|---|---|
| 1 | 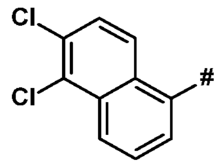 | O | CH | CH | N | CH | 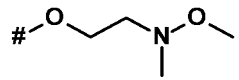 |
| 2 | 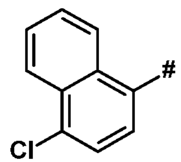 | O | CH | CH | N | CH | 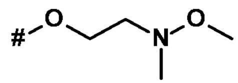 |
| 3 | 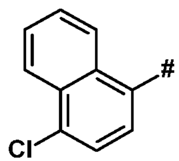 | O | CH | CH | N | CF | 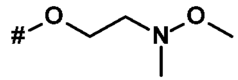 |
| 4 | 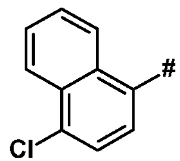 | O | CH | CH | N | CH | 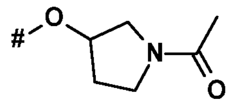 |

(fortgesetzt)

| Bsp.-Nr. | Z | Y | $A^1$ | $A^2$ | $A^3$ | $A^4$ | * |
|---|---|---|---|---|---|---|---|
| 5 | 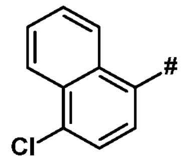 | O | CH | CH | N | CH | 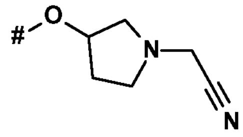 |
| 6 | 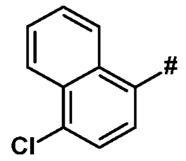 | O | CH | $CCHF_2$ | N | CH | 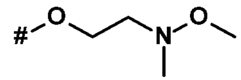 |
| 7 | 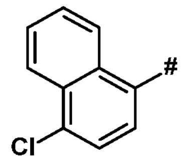 | O | CH | CCl | N | CH | 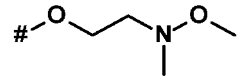 |
| 8 | 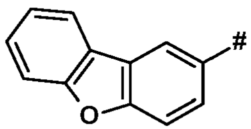 | O | CH | CH | N | CH | 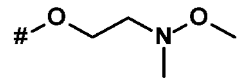 |
| 9 | 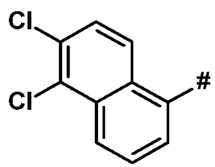 | O | CH | CH | N | CH | 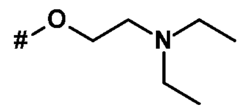 |
| 10 | 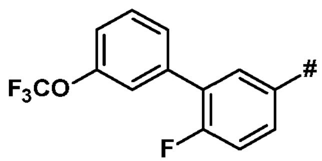 | O | CH | CH | N | CH | 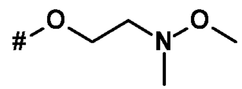 |

(fortgesetzt)

| Bsp.-Nr. | Z | Y | $A^1$ | $A^2$ | $A^3$ | $A^4$ | * |
|---|---|---|---|---|---|---|---|
| 11 | 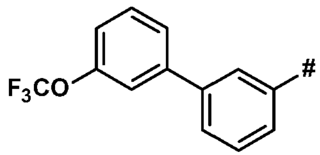 | O | CH | CH | N | CH | 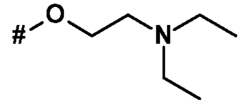 |
| 12 | 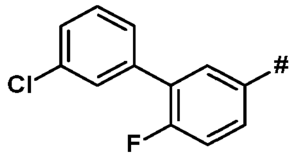 | O | CH | CH | N | CH | 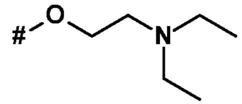 |
| 13 | 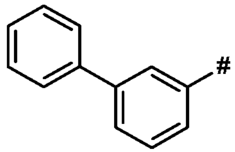 | O | CH | CH | N | CH | 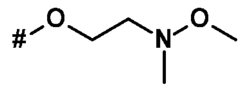 |
| 14 | 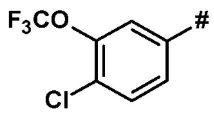 | O | CH | CH | N | CH | 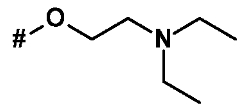 |
| 15 | 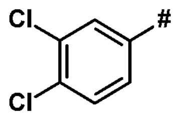 | O | CH | CH | N | CH | 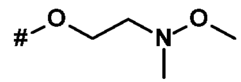 |
| 16 | 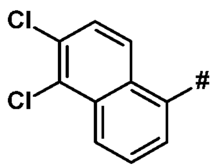 | O | CH | CH | CH | N | 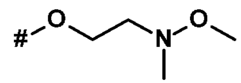 |

(fortgesetzt)

| Bsp.-Nr. | Z | Y | $A^1$ | $A^2$ | $A^3$ | $A^4$ | * |
|---|---|---|---|---|---|---|---|
| 17 | 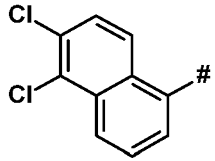 | O | CH | CH | CF | N | 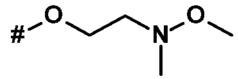 |
| 18 | 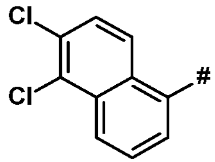 | O | CH | CH | CCl | N | 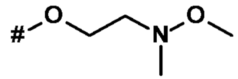 |
| 19 | 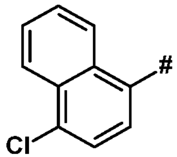 | O | CF | CH | CH | N | 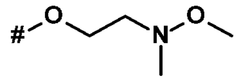 |
| 20 | 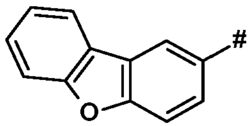 | O | CF | CH | CH | N | 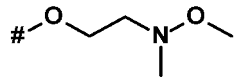 |
| 21 | 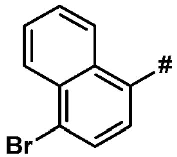 | O | CF | CH | CH | N | 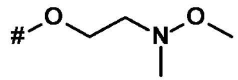 |
| 22 | 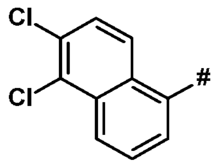 | O | CF | CH | CH | N | 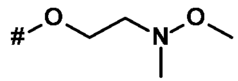 |

(fortgesetzt)

| Bsp.-Nr. | Z | Y | A$^1$ | A$^2$ | A$^3$ | A$^4$ | * |
|---|---|---|---|---|---|---|---|
| 23 | 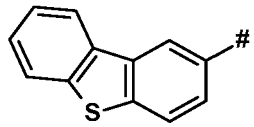 | O | CF | CH | CH | N | 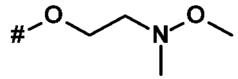 |
| 24 | 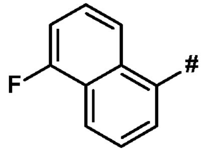 | O | CF | CH | CH | N | 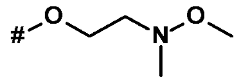 |
| 25 | 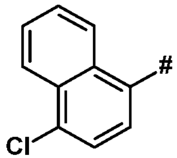 | O | CCl | CH | CH | N | 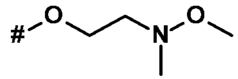 |
| 26 | 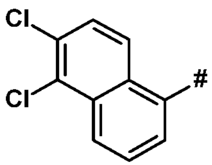 | O | CCl | CH | CH | N | 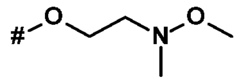 |
| 27 | 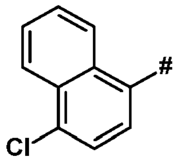 | O | CF | CH | CH | N | 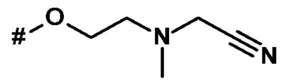 |
| 28 | 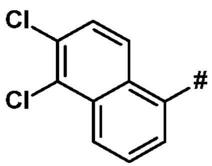 | O | CF | CH | CH | N | 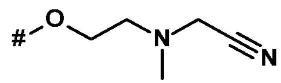 |

(fortgesetzt)

| Bsp.-Nr. | Z | Y | $A^1$ | $A^2$ | $A^3$ | $A^4$ | * |
|---|---|---|---|---|---|---|---|
| 29 | 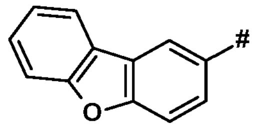 | O | CF | CH | CH | N | 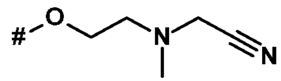 |
| 30 | 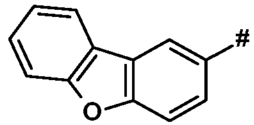 | O | $CCH_3$ | CH | CH | N | 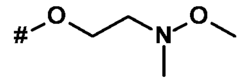 |
| 31 | 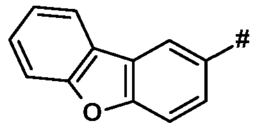 | O | CH | CH | CH | N | 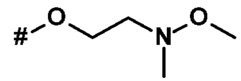 |
| 32 | 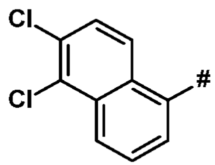 | O | CF | CH | CH | N | 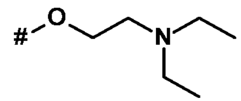 |
| 33 | 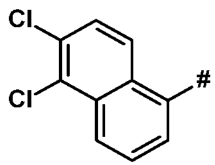 | O | CH | CH | CH | N | 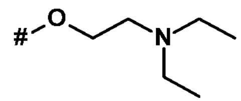 |
| 34 | 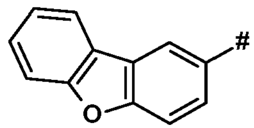 | O | CF | CH | CH | N | 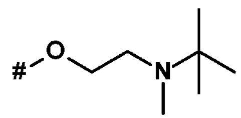 |
| 35 | 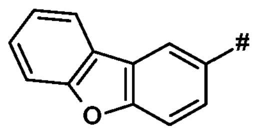 | O | CF | CH | CH | N | 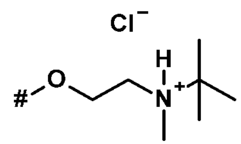 |

(fortgesetzt)

| Bsp.-Nr. | Z | Y | $A^1$ | $A^2$ | $A^3$ | $A^4$ | * |
|---|---|---|---|---|---|---|---|
| 36 | 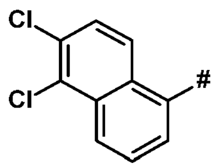 | O | CF | CH | CH | N | 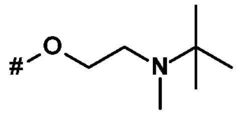 |
| 37 | 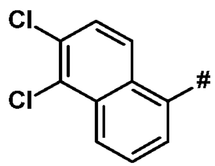 | O | CF | CH | CH | N | 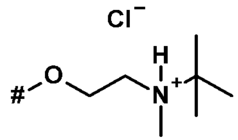 |
| 38 | 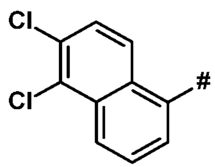 | O | CF | CH | CH | N | 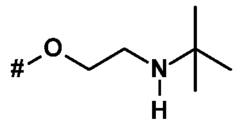 |
| 39 | 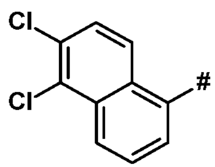 | O | CF | CH | CH | N | 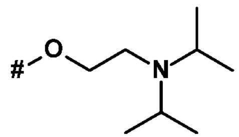 |
| 40 | 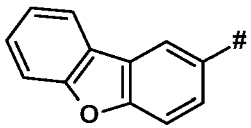 | O | CF | CH | CH | N | 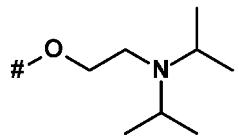 |
| 41 | 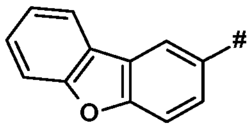 | O | CF | CH | CH | N | 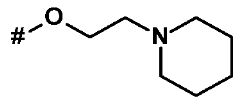 |

(fortgesetzt)

| Bsp.-Nr. | Z | Y | A¹ | A² | A³ | A⁴ | * |
|---|---|---|---|---|---|---|---|
| 42 | 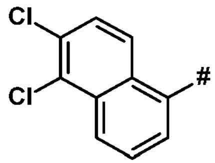 | O | CF | CH | CH | N | 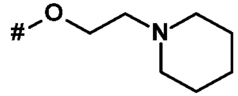 |
| 43 | 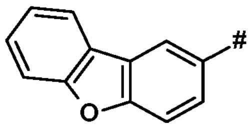 | O | CF | CH | CH | N | 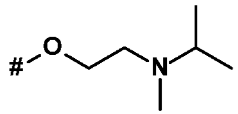 |
| 44 | 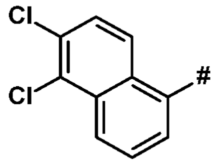 | O | CF | CH | CH | N | 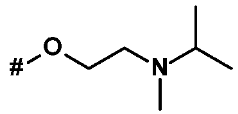 |
| 45 | 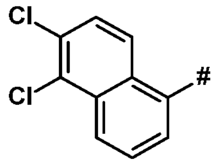 | O | CF | CH | CH | N | 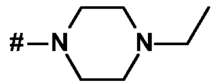 |
| 46 | 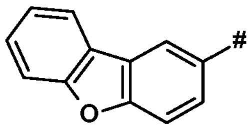 | O | CF | CH | CH | N | 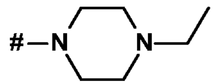 |
| 47 | 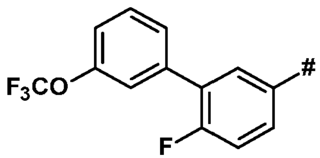 | O | CF | CH | CH | N | 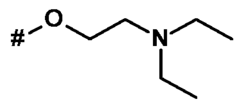 |

(fortgesetzt)

| Bsp.-Nr. | Z | Y | $A^1$ | $A^2$ | $A^3$ | $A^4$ | * |
|---|---|---|---|---|---|---|---|
| 48 | 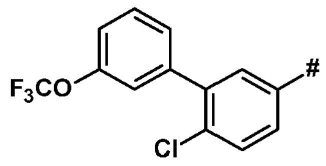 | O | CF | CH | CH | N | 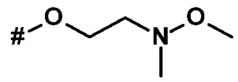 |
| 49 | 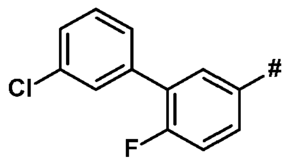 | O | CF | CH | CH | N | 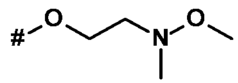 |
| 50 | 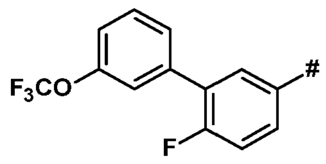 | O | CF | CH | CH | N | 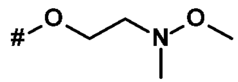 |
| 51 | 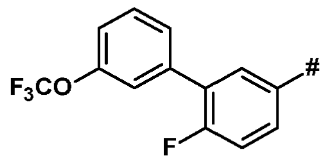 | O | CF | CH | CH | N | 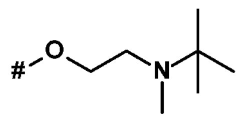 |
| 52 | 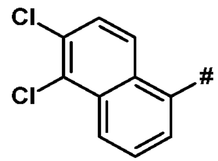 | O | CH | CH | $CSCH_3$ | N | 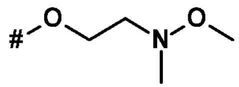 |
| 53 | 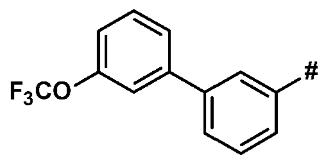 | O | CF | CH | CH | N | 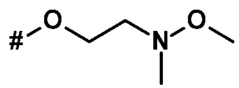 |

(fortgesetzt)

| Bsp.-Nr. | Z | Y | $A^1$ | $A^2$ | $A^3$ | $A^4$ | * |
|---|---|---|---|---|---|---|---|
| 54 | 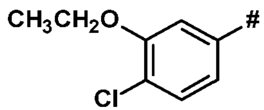 | O | CF | CH | CH | N | 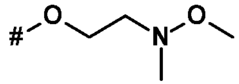 |
| 55 | 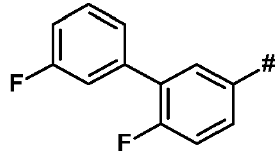 | O | CF | CH | CH | N | 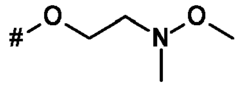 |
| 56 | 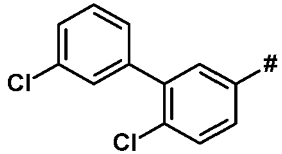 | O | CF | CH | CH | N | 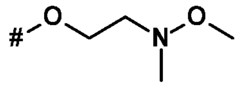 |
| 57 | 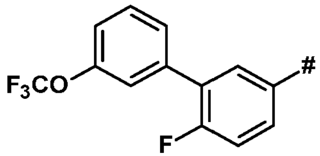 | O | CF | CH | CH | N | 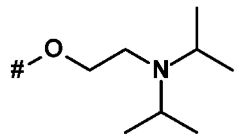 |
| 58 | 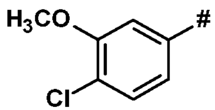 | O | CF | CH | CH | N | 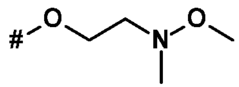 |
| 59 | 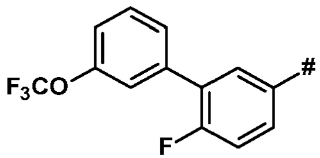 | O | $CCH_3$ | CH | CH | N | 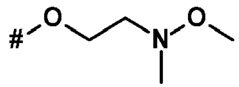 |

(fortgesetzt)

| Bsp.-Nr. | Z | Y | $A^1$ | $A^2$ | $A^3$ | $A^4$ | * |
|---|---|---|---|---|---|---|---|
| 60 | 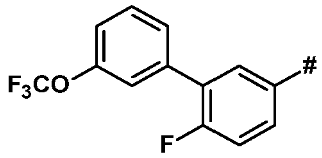 | O | CF | CH | CH | N | 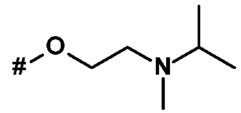 |
| 61 | 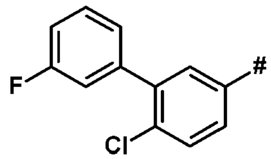 | O | CF | CH | CH | N | 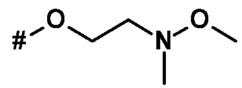 |
| 62 | 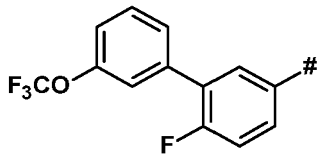 | O | CF | CH | CH | N | 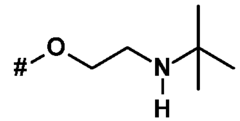 |
| 63 | 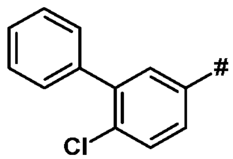 | O | CF | CH | CH | N | 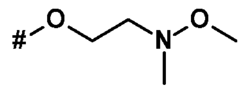 |
| 64 | 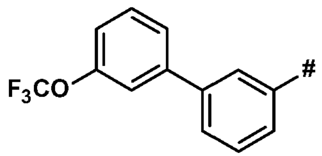 | O | CH | CH | CH | N | 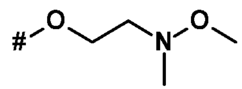 |
| 65 | 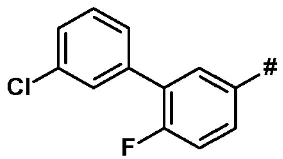 | O | CH | CH | CH | N | 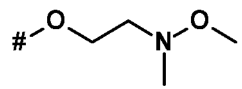 |

(fortgesetzt)

| Bsp.-Nr. | Z | Y | $A^1$ | $A^2$ | $A^3$ | $A^4$ | * |
|---|---|---|---|---|---|---|---|
| 66 | 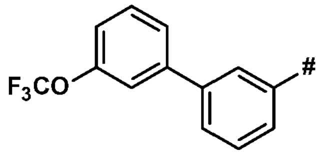 | O | CF | CH | CH | N | 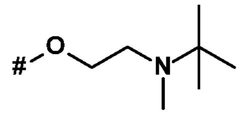 |
| 67 | 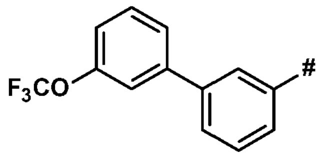 | O | CF | CH | CH | N | 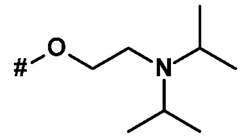 |
| 68 | 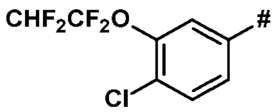 | O | CF | CH | CH | N | 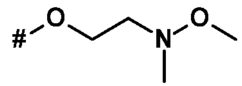 |
| 69 | 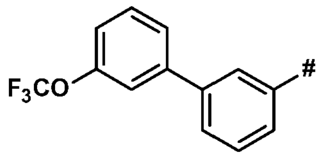 | O | CF | CH | CH | N | 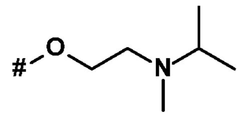 |
| 70 | 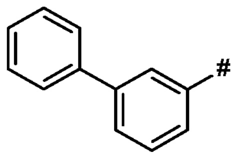 | O | CF | CH | CH | N | 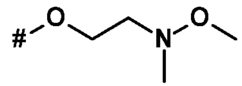 |
| 71 | 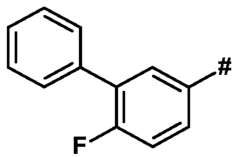 | O | CF | CH | CH | N | 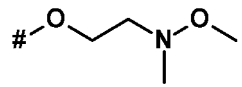 |

(fortgesetzt)

| Bsp.-Nr. | Z | Y | A¹ | A² | A³ | A⁴ | * |
|---|---|---|---|---|---|---|---|
| 72 | 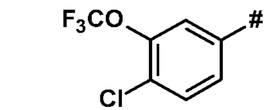 | O | CF | CH | CH | N | 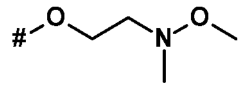 |
| 73 | 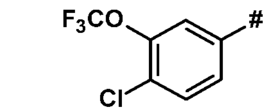 | O | CH | CH | CH | N | 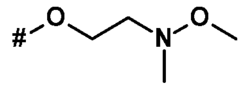 |
| 74 | 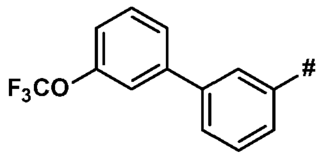 | O | CF | CH | CH | N | 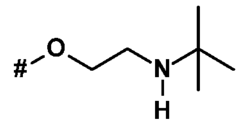 |
| 75 | 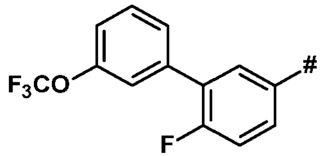 | O | CF | CH | CH | N | 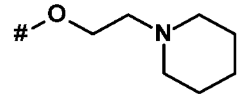 |
| 76 | 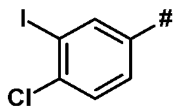 | O | CF | CH | CH | N | 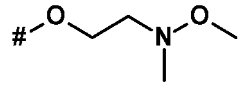 |
| 77 | 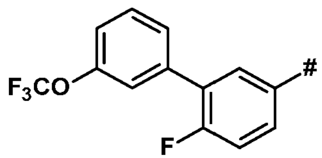 | O | CF | CH | CH | N | 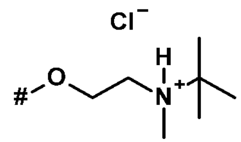 |
| 78 | 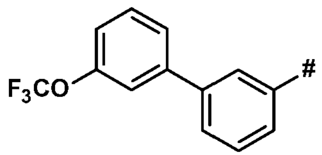 | O | CF | CH | CH | N | 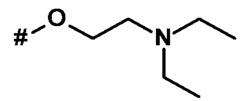 |

(fortgesetzt)

| Bsp.-Nr. | Z | Y | $A^1$ | $A^2$ | $A^3$ | $A^4$ | * |
|---|---|---|---|---|---|---|---|
| 79 | 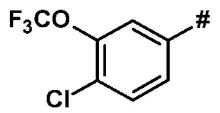 | O | CF | CH | CH | N | 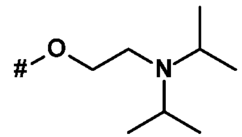 |
| 80 | 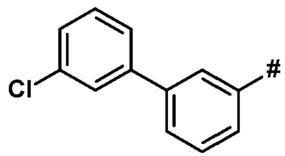 | O | CF | CH | CH | N | 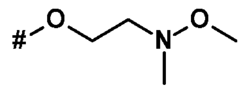 |
| 81 | 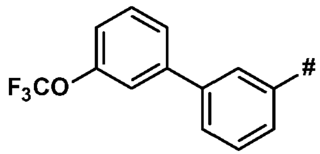 | O | CH | CH | CH | N | 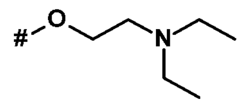 |
| 82 | 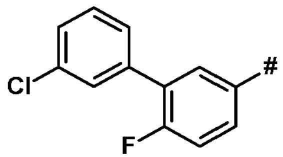 | O | CH | CH | CH | N | 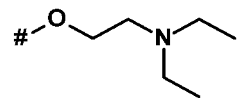 |
| 83 | 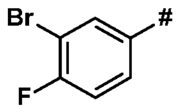 | O | CF | CH | CH | N | 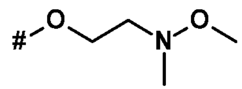 |
| 84 | 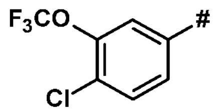 | O | CF | CH | CH | N | 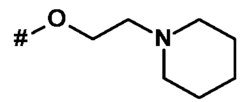 |

(fortgesetzt)

| Bsp.-Nr. | Z | Y | $A^1$ | $A^2$ | $A^3$ | $A^4$ | * |
|---|---|---|---|---|---|---|---|
| 85 | 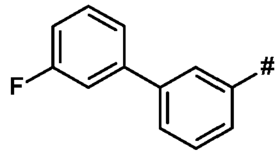 | O | CF | CH | CH | N | 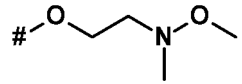 |
| 86 | 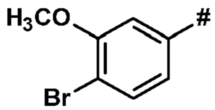 | O | CF | CH | CH | N | 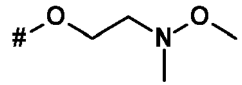 |
| 87 | 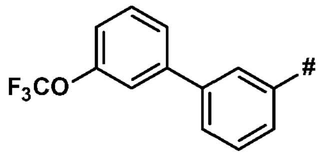 | O | CF | CH | CH | N | 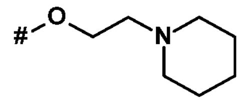 |
| 88 | 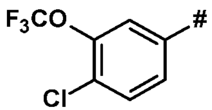 | O | CH | CH | CH | N | 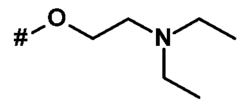 |
| 89 | 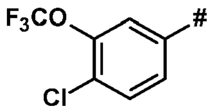 | O | CF | CH | CH | N | 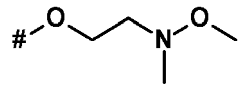 |
| 90 | 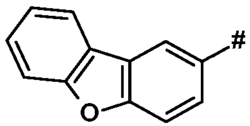 | O | $COCH_3$ | CH | CH | N | 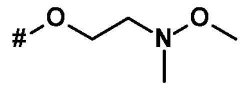 |
| 91 | 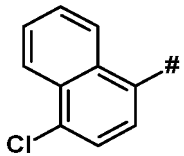 | O | $COCH_3$ | CH | CH | N | 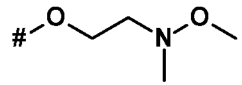 |

(fortgesetzt)

| Bsp.-Nr. | Z | Y | $A^1$ | $A^2$ | $A^3$ | $A^4$ | * |
|---|---|---|---|---|---|---|---|
| 92 | 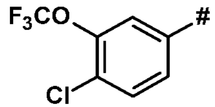 | O | CF | CH | CH | N | 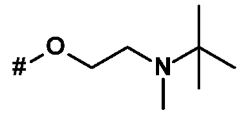 |
| 93 | 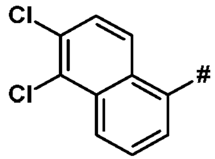 | O | $COCH_3$ | CH | CH | N | 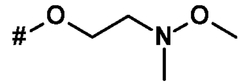 |
| 94 | 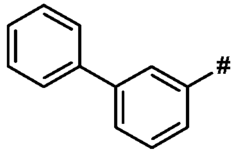 | O | $COCH_3$ | CH | CH | N | 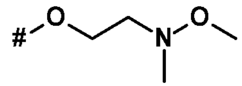 |
| 95 | 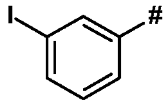 | O | CF | CH | CH | N | 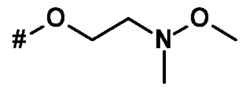 |
| 96 | 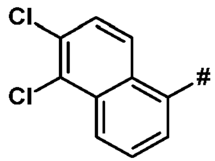 | O | CH | CH | $COCH_3$ | N | 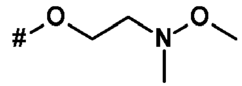 |
| 97 | 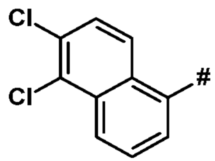 | O | CH | CH | $COCH_2CH_3$ | N | 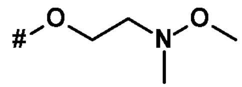 |

(fortgesetzt)

| Bsp.-Nr. | Z | Y | $A^1$ | $A^2$ | $A^3$ | $A^4$ | * |
|---|---|---|---|---|---|---|---|
| 98 | 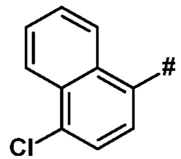 | O | CH | N | N | CH | 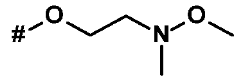 |
| 99 | 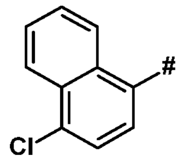 | O | N | CH | CH | N | 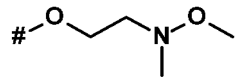 |

Herstellung von 2-[(5-Fluor-6-{[(6-fluorbiphenyl-3-yl)oxy]methyl}pyridin-3-yl)oxy}-N-methoxy-N-methylethanamin

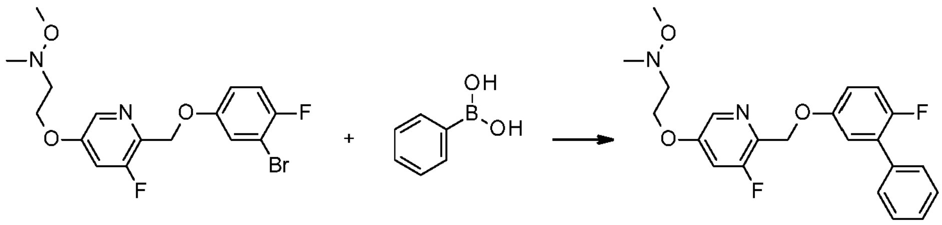

Eine Lösung von 85 mg (0,21 mmol) 2-({6-[(3-Brom-4-fluorphenoxy)methyl]-5-fluorpyridin-3-yl}oxy)-N-methoxy-N-methylethanamin in 5 mL Tetrahydrofuran wurde unter Argon mit 58 mg (0,42 mmol) Kaliumcarbonat, 49 mg (42 μmol) Tetrakis-(triphenylphosphin)-palladium, 1 mL Wasser und 51 mg (0,42 mmol) Phenylboronsäure versetzt. Das Reaktionsgemisch wurde durch Einleiten eines Argonstroms von gelöstem Sauerstoff befreit, über Nacht bei 80 °C gerührt und anschließend mit Kieselgel versetzt bevor das Lösungsmittel wurde unter reduziertem Druck entfernt wurde. Der Rückstand wurde zunächst mittels MPLC an Kieselgel (Gradient: Essigsäureethylester/Cyclohexan) und anschließend erneut per HPLC (Gradient: $H_2O$/Acetonitril) chromatographisch aufgetrennt. Es wurden 80 mg 2-[(5-Fluor-6-{[(6-fluorbiphenyl-3-yl)oxy]methyl}pyridin-3-yl)oxy]-N-methoxy-N-methylethanamin erhalten.

$^1$H-NMR(400.0 MHz, $d_6$-DMSO): δ = 8.2304(2.2);8.2263(2.2);7.5763(1.4);7.5681(2.6);7.5640(1.8); 7.5471(4.3);7.4984(1.9);7.4804(3.7);7.4611(2.0);7.4291(0.8);7.4259(1.4);7.4227(0.9);7.4135(0.5);7.4076 (1.7);7.3895(0.6);7.2630(1.2);7.2404(1.7);7.2374(1.6);7.2147(1.5);7.1731(1.2);7.1653(1.4);7.1570(1.3); 7.1492(1.4);7.0680(0.8);7.0587(1.3);7.0503(0.8);7.0456(0.8);7.0367(1.0);7.0280(0.6);5.1873(4.3);5.1829 (4.6);4.2340(1.9);4.2203(4.0);4.2066(2.1);3.4025(19.6);3.3189(19.4);2.9769(1.8);2.9633(3.6);2.9495(1.8); 2.6708(0.4);2.5643(16.0);2.5409(36.2);2.5239(0.9);2.5104(22.3);2.5061(46.1);2.5016(61.5);2.4971(45.7); 2.4929(23.3);2.3283(0.4);0.0079(1.5);-0.0002(41.0);-0.0084(1.9).

Herstellung von 2-[(6-{[(5,6-Dichlor-1-naphthyl)oxy]methyl}-2-ethoxypyridin-3-yl)oxy]-N-methoxy-N-methylethanamin

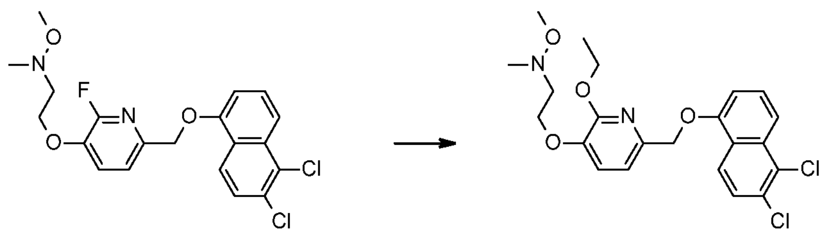

Eine Lösung von 20 mg (47 μmol) 2-[(6-{[(5,6-Dichlor-1-naphthyl)oxy]methyl}-2-fluorpyridin-3-yl)oxy]-N-methoxy-N-methylethanamin in 1 mL Dimethylacetamid wurde bei Raumtemperatur mit 9,6 mg (0,14 mmol) Natriumethylat versetzt. Das Reaktionsgemisch wurde über Nacht gerührt und anschließend per HPLC (Gradient: $H_2O$/Acetonitril) chromatographisch aufgetrennt. Es wurden 7 mg 2-[(6-{[(5,6-Dichlor-1-naphthyl)oxy]methyl}-2-ethoxypyridin-3-yl)oxy]-N-methoxy-N-methylethanamin erhalten.

$^1$H-NMR(400.0 MHz, $d_6$-DMSO): δ = 9.9594(0.4);8.2457(2.4);8.2230(2.6);7.7922(1.9);7.7708(2.7); 7.6965(3.2);7.6791(1.9);7.6739(3.3);7.6593(2.4);7.6384(1.3);7.3698(2.4);7.3498(2.8);7.2878(2.3);7.2684 (2.1);7.1449(2.7);7.1251(2.3);5.2364(7.8);4.5171(0.3);4.3665(1.3);4.3490(4.1);4.3315(4.2);4.3139(1.4); 4.1371(2.1);4.1226(4.5);4.1080(2.3);3.4096(18.6);3.4001(2.0);3.3204(31.9);2.9799(2.3);2.9655(4.6);2.95 11(2.3);2.6709(0.6);2.5723(16.0);2.5558(1.6);2.5058(75.0);2.5019(95.4);2.4980(72.3);2.3286(0.5);1.3184 (4.2);1.3009(8.7);1.2834(4.2);1.2308(0.6);0.1457(0.5);-0.0004(99.6);-0.1499(0.5).

Herstellung von 2-({6-[(Dibenzo[b,d]furan-2-yloxy)methyl]-5-methylpyridin-3-yl}oxy)-N-methoxy-N-methylethanamin

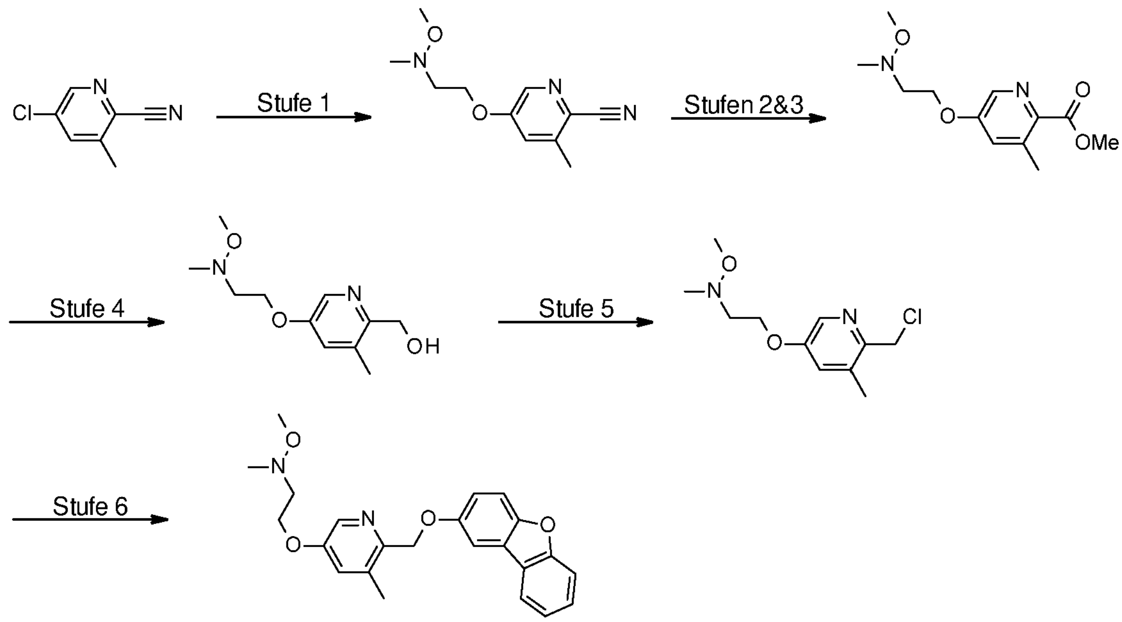


Stufe 1: Herstellung von 5-{2-[Methoxy(methyl)amino]ethoxy}-3-methylpyndin-2-carbonitril

Zu einer gekühlten Suspension von 686 mg (15,7 mmol) Natriumhydrid (55%ige Dispersion in Mineralöl) in 32 mL N-Methyl-2-pyrrolidon wurde langsam eine Lösung von 1,38 g (13,1 mmol) 2-[Methoxy(methyl)amino]ethanol in 4 mL N-Methyl-2-pyrrolidon getropft. Das Reaktionsgemisch wurde für 30 min bei Raumtemperatur gerührt und anschließend eine Lösung von 2,00 g (13,1 mmol) 5-Chlor-3-methylpyridin-2-carbonitril in 4 mL N-Methyl-2-pyrrolidon hinzugetropft. Es wurde über Nacht bei Raumtemperatur weitergerührt und dann mit 20 mL Essigsäureethylester versetzt. Nach 5 min wurde Wasser hinzugefügt und das Gemisch mehrfach mit Essigsäureethylester extrahiert. Die vereinigten organischen Phasen wurden mit Natriumsulfat getrocknet, filtriert und das Lösungsmittel unter reduziertem Druck entfernt. Der Rückstand wurde an RP-Kieselgel adsorbiert und mittels MPLC an RP-Kieselgel chromatographisch aufgetrennt (Gradient: $H_2O$/Acetonitril). Es wurden 1,20 g 5-{2-[Methoxy(methyl)amino]ethoxy}-3-methylpyridin-2-carbonitril erhalten.

$^1$H-NMR(400.0 MHz, CDCl3): $\delta$ = 8.7253(0.4);8.7197(0.4);8.2526(1.8);8.2459(1.9);7.7789(0.3);7.7733 (0.3);7.2654(4.4);7.1286(1.7);7.1221(1.7);4.2396(1.4);4.2259(2.9);4.2122(1.6);3.9097(0.7);3.5168(16.0); 3.0561(2.1);3.0424(4.0);3.0286(2.0);2.7222(0.6);2.6748(14.6);2.6247(1.3);2.5715(0.4);2.5393(11.4);2.00 97(3.7);1.6280(0.5);-0.0002(3.7).

Stufe 2&3: Herstellung von Methyl-5-{2-[methoxy(methyl)amino]ethoxy}-3-methylpyridin-2-carboxylat

Eine Lösung von 1,20 g (5,43 mmol) 5-{2-[Methoxy(methyl)amino]ethoxy}-3-methylpyridin-2-carbonitril in 5,4 mL Tetrahydrofuran wurde mit 2,17 g (54,3 mmol) Natriumhydroxid, 2,7 ml Wasser und 2,7 mL Ethanol versetzt und für 12 h bei 70 °C gerührt. Das Reaktionsgemisch wurde anschließend mit Wasser verdünnt, mit 1 M Salzsäure angesäuert und mehrfach mit Essigsäureethylester extrahiert. Die vereinigten organischen Phasen wurden mit Natriumsulfat getrocknet, filtriert und das Lösungsmittel unter reduziertem Druck entfernt. Es wurden 245 mg eines Rückstands erhalten. Die wässrige Phase wurde ebenfalls unter reduziertem Druck eingeengt und der Rückstand azeotrop mit $CH_2Cl_2$ getrocknet. Es wurden 4,28 g eines Rückstands erhalten, welcher in 45 mL Methanol gelöst und mit einer katalytischen Menge Schwefelsäure versetzt wurde. Die Reaktionslösung wurde für 5 d unter Rückfluss erhitzt und anschließend das Lösungsmittel unter reduziertem Druck entfernt. Der Rückstand wurde mit gesättigter $NaHCO_3$-Lösung versetzt und die erhaltene Lösung mehrfach mit Essigsäureethylester extrahiert. Die vereinigten organischen Phasen wurden mit Natriumsulfat getrocknet, filtriert und das Lösungsmittel unter reduziertem Druck entfernt. Es wurden 787 mg Methyl-5-{2-[methoxy(methyl)amino]ethoxy}-3-methylpyridin-2-carboxylat erhalten.

$^1$H-NMR(400.0 MHz, CDCl3): $\delta$ = 8.2899(1.8);8.2832(1.8);7.2673(4.3);7.1065(1.6);7.1003(1.6); 5.3010(0.6);4.2446(1.2);4.2311(2.5);4.2174(1.4);4.0111(1.3);3.9793(1.3);3.9549(15.8);3.9047(0.5);3.524 4(16.0);3.0596(1.9);3.0459(3.7);3.0322(1.9);2.6789(14.2);2.6359(11.1);1.6127(0.4);-0.0002(2.9)

Stufe 4: Herstellung von (5-{2-[Methoxy(methyl)amino]ethoxy}-3-methylpyridin-2-yl)methanol

Eine Lösung von 750 mg (2,95 mmol) Methyl-5-{2-[methoxy(methyl)amino]ethoxy}-3-methylpyridin-2-carboxylat in 32 mL Toluol wurde bei -78 °C mit 14,8 mL (14,8 mmol) einer 1 M Lösung von Diisobutylaluminiumhydrid tropfenweise versetzt. Die Reaktionslösung wurde für 60 min bei -10 °C gerührt, mit 50 mL einer gesättigten Kaliumnatriumtartrat-Lösung versetzt und anschließend mehrfach mit Essigsäureethylester extrahiert. Die vereinigten organischen Phasen wurden mit Natriumsulfat getrocknet, filtriert und das Lösungsmittel unter reduziertem Druck entfernt. Der Rückstand wurde in 10 mL Methanol gelöst und unter Eiskühlung mit 64 mg (1,7 mmol) Natriumborhydrid versetzt. Die Lösung wurde für 2 h bei Raumtemperatur gerührt. Das Lösungsmittel wurde unter reduziertem Druck entfernt. Der Rückstand wurde mit Wasser versetzt und mehrfach mit Essigsäureethylester extrahiert. Die vereinigten organischen Phasen wurden mit Natriumsulfat getrocknet, filtriert und das Lösungsmittel unter reduziertem Druck entfernt. Es wurden 498 mg (5-{2-[Methoxy(methyl)amino]ethoxy}-3-methylpyridin-2-yl)methanol erhalten.

$^{1}$H-NMR(400.0 MHz, CDCl3): δ = 8.1296(1.7);8.1232(1.8);7.2654(3.6);7.1012(1.6);7.0955(1.6); 4.6307(3.8);4.5215(0.5);4.1966(1.3);4.1829(2.6);4.1692(1.4);3.8572(0.5);3.5324(16.0);3.0446(2.0);3.0308(3.9);3.0171(2.0);2.6788(14.8);2.2275(0.4);2.2064(10.4);1.6595(0.9);-0.0002(2.0).

Stufe 5: Herstellung von 2-{[6-(Chlormethyl)-5-methylpyridin-3-yl]oxy}-N-methoxy-N-methylethanamin

Eine Lösung von 100 mg (442 μmol) (5-{2-[Methoxy(methyl)amino]ethoxy}-3-methylpyridin-2-yl)methanol in 3 mL $CH_2Cl_2$ wurde bei 0 °C mit 0,23 mL (1,3 mmol) Diisopropylethylamin und 34 μL (0,44 mmol) Methansulfonsäurechlorid versetzt. Die Reaktionslösung wurde über Nacht bei Raumtemperatur gerührt, anschließend mit $CH_2Cl_2$ verdünnt und mit gesättigter $NaHCO_3$-Lösung sowie Wasser gewaschen. Die organische Phase wurde mit Natriumsulfat getrocknet, filtriert und das Lösungsmittel unter reduziertem Druck entfernt. Es wurden 102 mg 2-{[6-(Chlormethyl)-5-methylpyridin-3-yl]oxy}-N-methoxy-N-methylethanamin erhalten.

$^{1}$H-NMR(400.0 MHz, CDCl3): δ = 8.1510(1.8);8.1442(1.8);7.2641(5.4);7.0808(1.7);7.0743(1.7);4.7037(0.5);4.6904(9.1);4.1959(1.4);4.1823(2.7);4.1686(1.5);3.8559(0.5);3.5265(16.0);3.0400(2.0);3.0263(3.8); 3.0125(2.0);2.6738(14.6);2.4657(0.4);2.4289(11.1);1.6500(0.9);-0.0002(3.5)

Stufe 6: Herstellung von 2-({6-[(Dibenzo[b,d]furan-2-yloxy)methyl]-5-methylpyridin-3-yl}oxy)-N-methoxy-N-methylethanamin

Zu einer Lösung von 100 mg (409 μmol) 2-{ [6-(Chlormethyl)-5-methylpyridin-3-yl]oxy}-N-methoxy-N-methylethanamin in 3 mL N,N-Dimethylformamid wurden 61 mg (0,33 mmol) Dibenzo[b,d]furan-2-ol und 227 mg (1,64 mmol) $K_2CO_3$ gegeben. Das Reaktionsgemisch wurde für 4 h bei 70 °C und über Nacht bei Raumtemperatur gerührt. Anschließend wurde es mit Wasser versetzt und mehrmals mit Essigsäureethylester extrahiert. Die vereinigten organischen Phasen wurden mit Natriumsulfat getrocknet, filtriert und das Lösungsmittel unter reduziertem Druck entfernt. Der Rückstand wurde mittels HPLC (Gradient: $H_2O$/Acetonitril) chromatographisch aufgetrennt. So wurden 93 mg 2-({6-[(Dibenzo[b,d]furan-2-yloxy)methyl]-5-methylpyridin-3-yl}oxy)-N-methoxy-N-methylethanamin erhalten.

$^{1}$H-NMR(400.0 MHz, CDCl3): δ = 8.2065(2.0);8.1997(2.0);7.9262(1.5);7.9069(1.6);7.5833(2.4); 7.5768(2.5);7.5423(1.4);7.5217(2.2);7.4626(2.3);7.4559(1.0);7.4536(0.9);7.4401(3.2);7.4172(0.8);7.3375(1.2);7.3184(1.8);7.3000(0.8);7.2610(7.7);7.1522(1.4);7.1457(1.4);7.1299(1.3);7.1233(1.4);7.1162(1.9); 7.1096(1.8);5.2523(8.4);4.2062(1.4);4.1927(2.7);4.1790(1.5);3.8629(0.5);3.5287(16.0);3.0459(1.9);3.0323(3.8);3.0186(1.9);2.6759(14.0);2.4599(11.8);2.0045(1.6);1.6104(5.7);-0.0002(5.0).

Herstellung von 2-({6-[(Dibenzo[b,d]furan-2-yloxy)methyl]-5-methoxypyridin-3-yl}oxy)-N-methoxy-N-methylethanamin

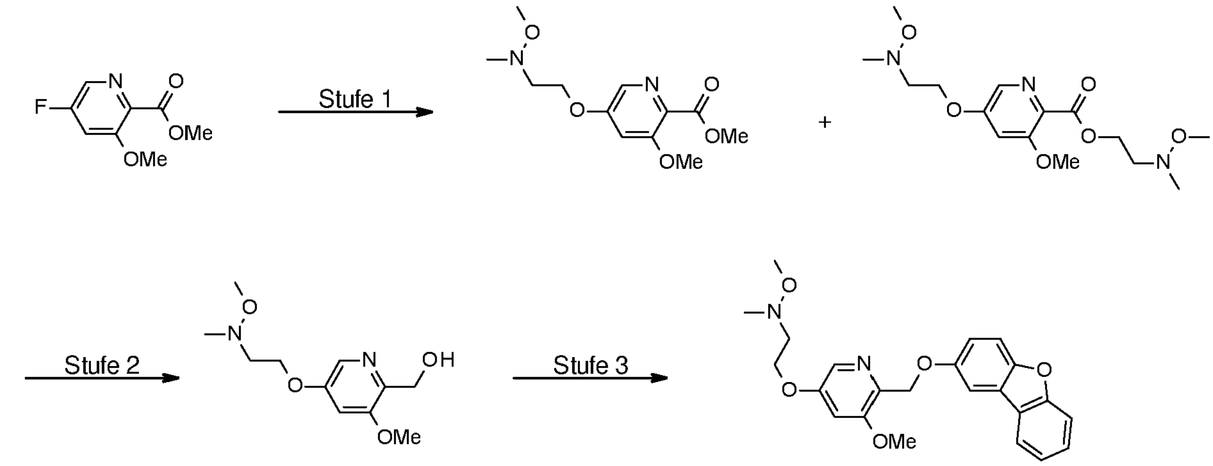


Stufe 1: Herstellung von Methyl-3-methoxy-5-{2-[methoxy(methyl)amino]ethoxy}pyridin-2-carboxylat

Eine Suspension von 673 mg (15,4 mmol) Natriumhydrid (55%ige Dispersion in Mineralöl) in 80 mL N,N-Dimethylformamid wurde tropfenweise mit einer Lösung von 1,35 g (12,9 mmol) 2-[Methoxy(methyl)amino]ethanol in 10 mL N,N-Dimethylformamid versetzt. Die Reaktionslösung wurde für 30 min bei Raumtemperatur gerührt und dann eine Lösung von 2,38 g (12,9 mmol) Methyl-5-fluor-3-methoxypyridin-2-carboxylat in 10 mL N,N-Dimethylformamid hinzugetropft. Das Reaktionsgemisch wurde über Nacht bei Raumtemperatur gerührt, anschließend mit Wasser versetzt und mit Essigsäureethylester extrahiert. Die vereinigten organischen Phasen wurden mit Natriumsulfat getrocknet, filtriert und das Lösungsmittel unter reduziertem Druck entfernt. Der Rückstand wurde mittels HPLC (Gradient: $H_2O$/Acetonitril) chromatographisch aufgetrennt. Es wurden 2,76 g Rohprodukt erhalten welches erneut mittels HPLC (Gradient: $H_2O$/Acetonitril) chromatographisch gereinigt wurde. So konnten 1,43 g Methyl-3-methoxy-5-{2-[methoxy(methyl)amino]ethoxy}pyridin-2-carboxylat (Reinheit ca. 69%) als Mischung mit 2-[Methoxy(methyl)amino]ethyl-3-methoxy-5-{2-[methoxy(methyl)amino]ethoxy}pyridin-2-carboxylat erhalten werden.

$^1$H-NMR(400.0 MHz, CDCl3): $\delta$ = 8.0372(1.9);8.0315(1.9);8.0009(0.9);7.9951(0.9);7.2691(3.6);6.9242(1.7);6.9186(1.7);6.8404(0.8);6.8346(0.8);4.2815(1.0);4.2681(1.9);4.2547(1.1);3.9713(0.6);3.9495(16.0); 3.9380(6.1);3.9351(6.2);3.9249(10.7);3.9142(0.6);3.5489(0.5);3.5312(11.7);3.0632(1.4);3.0497(2.7); 3.0362(1.4);2.6847(10.4);2.6366(0.4);1.6400(2.2);-0.0002(3.8).

Stufe 2: Herstellung von (3-Methoxy-5-{2-fmethoxy(methyl)amino]ethoxy}pyridin-2-yl)methanol

Eine Lösung von 1,43 g (5,29 mmol) Methyl-3-methoxy-5-{2-[methoxy(methyl)amino]ethoxy}pyridin-2-carboxylat in 56 mL Toluol wurde bei -78 °C tropfenweise mit 3,3 mL (3,3 mmol) einer 1 M Lösung von Diisobutylaluminiumhydrid versetzt. Die Reaktionslösung wurde über Nacht bei 0 °C gerührt. Anschließend wurden bei 0 °C weitere 10,6 mL (10,6 mmol) einer 1 M Lösung von Diisobutylaluminiumhydrid zugesetzt. Nach 1 h Rühren bei 0 °C wurden tropfenweise 3 mL Methanol hinzugefügt und 5 min später 56 mL einer gesättigten Kaliumnatriumtartrat-Lösung. Das Gemisch wurde mehrfach mit Essigsäureethylester extrahiert. Die vereinigten organischen Phasen wurden mit Natriumsulfat getrocknet, filtriert und das Lösungsmittel unter reduziertem Druck entfernt. Der Rückstand wurde in 5 mL Methanol gelöst und unter Eiskühlung mit 114 mg (3,02 mmol) Natriumborhydrid versetzt. Die Lösung wurde für 2 h bei Raumtemperatur gerührt. Das Reaktionsgemisch wurde unter reduziertem Druck eingeengt, mit Wasser versetzt und mehrfach mit Essigsäureethylester extrahiert. Die vereinigten organischen Phasen wurden mit Natriumsulfat getrocknet, filtriert und das Lösungsmittel unter reduziertem Druck entfernt. Der Rückstand wurde mittels HPLC (Gradient: $H_2O$/Acetonitril) chromatographisch aufgetrennt. Es wurden 949 mg (3-Methoxy-5-{2-[methoxy(methyl)amino]ethoxy}pyridin-2-yl)methanol erhalten.

$^1$H-NMR(400.0 MHz, CDCl3): $\delta$= 7.8658(0.8);7.2645(3.4);6.8295(2.2);6.8242(2.2);4.6788(2.4); 4.6682(2.5);4.2230(1.2);4.2096(2.3);4.1962(1.3);4.0111(0.5);3.9995(0.9);3.9882(0.4);3.8247(14.4); 3.5389(16.0);3.0482(1.8);3.0347(3.5);3.0212(1.9);2.6855(14.5);1.6298(0.8);-0.0002(3.3.)

Stufe 3: Herstellung von 2-({6-[(Dibenzo[b,d]furan-2-yloxy)methyl]-5-methoxypyridin-3-yl}oxy)-N-methoxy-N-methylethanamin

100 mg (413 $\mu$mol) (3-Methoxy-5-{2-[methoxy(methyl)amino]ethoxy}pyridin-2-yl)methanol wurden mit 246 mg (2,06 mmol) $SOCl_2$ und einer katalytischen Menge N,N-Dimethylformamid versetzt. Das Reaktionsgemisch wurde für 4 h bei Raumtemperatur gerührt und anschließend unter reduziertem Druck eingeengt. Der Rückstand wurde in 2 mL

N,N-Dimethylformamid gelöst und mit 61 mg (0,33 mmol) Dibenzo[b,d]furan-2-ol sowie 285 mg (2,06 mmol) $K_2CO_3$ versetzt. Das Reaktionsgemisch wurde für 4 h bei 70 °C und für 3 d bei RT gerührt, an RP-Kieselgel adsorbiert und mittels MPLC an RP-Kieselgel chromatographisch aufgetrennt (Gradient: $H_2O$/Acetonitril). Es wurden 48 mg 2-({6-[(Dibenzo[b,d]furan-2-yloxy)methyl]-5-methoxypyridin-3-yl}oxy)-N-methoxy-N-methylethanamin erhalten.

$^1$H-NMR(400.0 MHz, CDCl3): $\delta$ = 7.9991(2.4);7.9934(2.7);7.9143(1.4);7.8951(1.5);7.6111(2.2); 7.6049(2.5);7.5409(1.3);7.5202(2.1);7.4563(2.1);7.4506(1.1);7.4471(1.0);7.4336(3.0);7.4295(2.1);7.4115 (0.8);7.4083(0.8);7.3311(1.0);7.3289(1.0);7.3104(1.6);7.2936(0.7);7.2916(0.7);7.2605(8.3);7.1897(1.4); 7.1832(1.5);7.1673(1.2);7.1609(1.3);6.8950(2.2);6.8894(2.5);5.2982(2.7);5.2508(8.2);4.2445(1.3);4.2311 (2.5);4.2178(1.4);3.8825(14.1);3.5365(16.0);3.0517(1.8);3.0383(3.5);3.0248(1.8);2.6834(13.9);1.5841 (6.1);-0.0002(7.8).

Herstellung von (5-{2-[Methoxy(methyl)amino]ethoxy}pyridin-2-yl)methanol

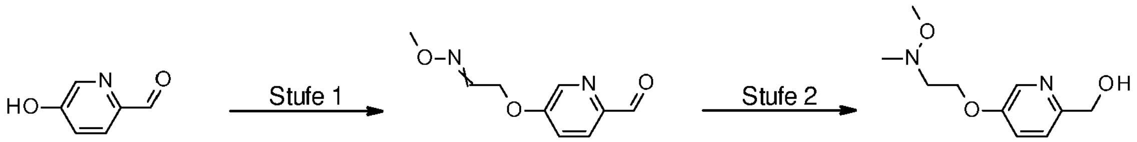


Stufe 1: Herstellung von 5-[2-(Methoxyimino)ethoxy]pyridin-2-carbaldehyd

Eine Lösung von 1,97 g (16,0 mmol) 5-Hydroxypyridin-2-carbaldehyd in 30 mL Acetonitril wurde bei Raumtemperatur mit 4,42 g (32,0 mmol) $K_2CO_3$ und einer Lösung von 2,41 g (22,4 mmol) 2-Chlor-N-methoxyethanimin in 30 mL Acetonitril versetzt. Das Reaktionsgemisch wurde für 40 h unter Rückfluss erhitzt und weitere 4 d bei Raumtemperatur gerührt. Es wurde vom Bodensatz abfiltriert, das Filtrat unter reduziertem Druck eingeengt und der Rückstand in 15 mL Acetonitril aufgenommen. Es wurde erneut filtriert und das Filtrat unter reduziertem Druck eingeengt. So konnten 1,91 g 5-[2-(Methoxyimino)ethoxy]pyridin-2-carbaldehyd als Rückstand erhalten werden.

$^1$H-NMR(400.0 MHz, CDCl3): $\delta$ = 10.0000(4.9);8.4785(2.5);8.4717(2.5);8.4568(0.8);8.4500(0.8); 7.9809(2.4);7.9592(2.5);7.5613(1.3);7.5469(2.6);7.5328(1.4);7.3812(1.3);7.3743(1.3);7.3594(1.2);7.3530 (1.2);7.3203(0.4);7.3136(0.4);7.2985(0.4);7.2918(0.4);7.2652(6.8);6.9597(0.4);6.9506(0.8);6.9413(0.4); 4.9494(1.7);4.9403(1.7);4.7838(5.2);4.7695(5.1);3.9757(5.0);3.9550(0.3);3.9161(16.0);3.9032(1.0); 3.8898(0.4);1.6375(3.0);-0.0001(3.9).

Stufe 2: Herstellung von (5-{2-[Methoxy(methyl)amino]ethoxy}pyridin-2-yl)methanol

Eine Lösung von 1,91 g (9,83 mmol) 5-[2-(Methoxyimino)ethoxy]pyridin-2-carbaldehyd in 15 mL Essigsäure wurde bei 10 °C mit 926 mg (14,7 mmol) Natriumcyanoborhydrid versetzt und 2 h bei Raumtemperatur gerührt. Unter Kühlung wurden anschließend bei 20 °C 12,1 g (147 mmol) einer 36,5%igen wässrigen Formaldehyd-Lösung hinzugefügt. Nach 10 min wurden weitere 926 mg (14,7 mmol) Natriumcyanoborhydrid zugesetzt. Das Reaktionsgemisch wurde über Nacht bei Raumtemperatur gerührt und dann das Lösungsmittel unter reduziertem Druck entfernt. Der Rückstand wurde mit 50 mL Wasser versetzt, mit einer 50%igen Natriumhydroxid-Lösung auf pH 10 eingestellt und mehrmals mit Essigsäureethylester extrahiert. Die vereinigten organischen Phasen wurden mit Natriumsulfat getrocknet, filtriert und das Lösungsmittel unter reduziertem Druck entfernt. Der Rückstand wurde mittels MPLC an Kieselgel chromatographisch aufgetrennt (Gradient: Essigsäureethylester /Cyclohexan). Es wurden 1,08 g 5-{2-[Methoxy(methyl)amino]ethoxy}pyridin-2-yl)methanol erhalten.

$^1$H-NMR(400.0 MHz, CDCl3): $\delta$ = 8.2963(2.0);8.2894(2.0);7.2869(1.1);7.2799(1.0);7.2697(2.7); 7.2657(2.0);7.2585(1.7);7.2065(2.4);7.1852(1.4);4.9625(0.5);4.7643(0.5);4.7038(7.6);4.2049(1.5);4.1912 (2.9);4.1775(1.6);3.5311(16.0);3.0514(2.2);3.0377(4.2);3.0239(2.1);2.6800(15.3);-0.0002(1.2).

Herstellung von 2-{[6-([(5,6-Dichlor-1-naphthyl)oxy]methyl}-2-(methylsulfanyl)pyridin-3-yl}oxy}-N-methoxy-N-methylethanamin

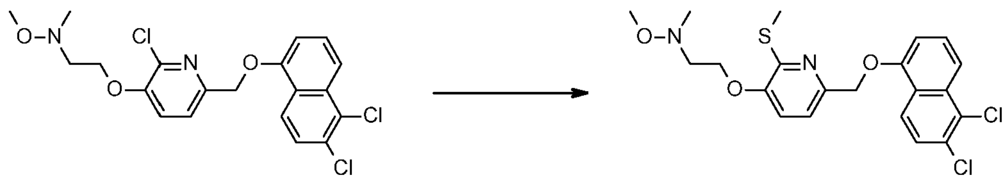

Eine Lösung von 46 mg (0,10 mmol) 2-[(2-Chlor-6-{[(5,6-dichlor-1-naphthyl)oxy]methyl}pyridin-3-yl)oxy]-N-methoxy-N-methylethanamin in Dimethylacetamid wurde mit 14,6 mg (0,21 mmol) Natriummethanthiolat versetzt und über Nacht bei Raumtemperatur gerührt. Anschließend wurden weitere 7,3 mg (0,10 mmol) Natriummethanthiolat hinzugefügt. Nach 2 h bei Raumtemperatur wurden erneut 7,3 mg (0,10 mmol) Natriummethanthiolat hinzugegeben und das Reaktionsgemisch über Nacht gerührt. Das Reaktionsgemisch wurde mit Wasser versetzt und mehrmals mit Essigsäureethylester extrahiert. Die vereinigten organischen Phasen wurden mit Natriumsulfat getrocknet, filtriert und das Lösungsmittel unter reduziertem Druck entfernt. Der Rückstand wurde und mittels HPLC chromatographisch aufgetrennt (Gradient: $H_2O$/Acetonitril). So wurden 3 mg 2-{[6-{[(5,6-Dichlor-1-naphthyl)oxy]methyl}-2-(methylsulfanyl)pyridin-3-yl]oxy}-N-methoxy-N-methylethanamin erhalten.

$^1$H-NMR(601.6 MHz, CDCl3): δ = 8.2606(1.7);8.2455(1.8);7.8618(1.6);7.8475(1.7);7.5069(1.2); 7.5011(2.6);7.4936(1.8);7.4861(2.4);7.4797(1.2);7.2589(26.6);7.1854(1.7);7.1718(2.0);7.0330(2.2);7.019 3(1.9);6.9921(1.7);6.9793(1.6);5.3152(6.8);4.2239(1.9);4.2140(4.0);4.2041(2.0);3.5268(16.0);3.1054(2.2); 3.0956(4.4);3.0857(2.1);2.6824(14.6);2.5371(15.1);1.5443(15.4);1.2554(1.0);0.0691(3.3);0.0051(0.6); -0.0002(16.0);-0.0056(0.7).

Herstellung von 2-[(5-([(4-Chlor-1-naphthyl)oxy]methyl]pyridin-2-yl)oxy]-N-methoxy-N-methylethanamin

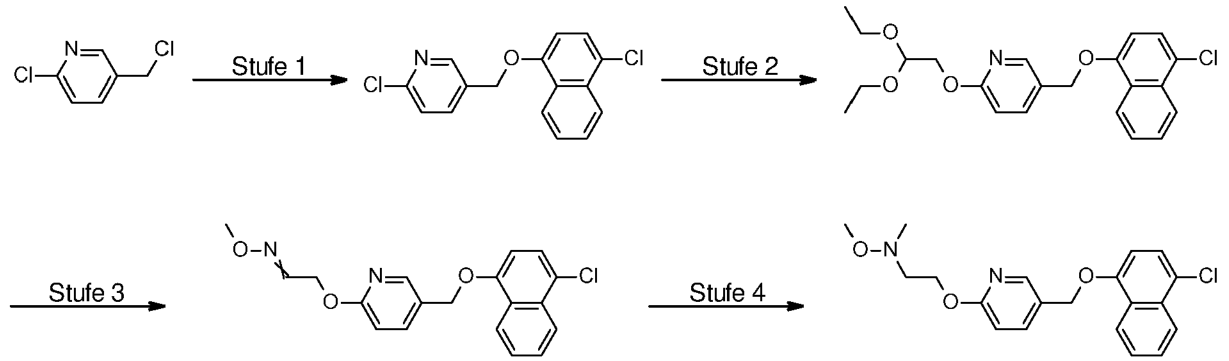


Stufe 1: Herstellung von 2-Chlor-5-1[(4-chlor-1-naphthyl)oxy]methyl}pyridin

Eine Lösung von 5,64 g (34,8 mmol) 2-Chlor-5-(chlormethyl)pyridin in 40 mL N,N-Dimethylformamid wurde mit 6,22 g (34,8 mmol) 4-Chlor-1-naphthol und 14,4 g (104 mmol) $K_2CO_3$ versetzt. Das Reaktionsgemisch wurde für 16 h bei 70 °C gerührt und anschließend unter reduziertem Druck eingeengt. Der Rückstand wurde in Essigsäureethylester gelöst und diese Lösung mit Wasser sowie einer gesättigten Ammoniumchlorid-Lösung gewaschen. Die organische Phase wurde daraufhin mit gesättigter Ammoniumchlorid-Lösung, die vereinigten wässrigen Phasen mehrfach mit Essigsäureethylester extrahiert. Die organischen Phasen wurden vereinigt, mit gesättigter wässriger Natriumchlorid-Lösung gewaschen, mit Natriumsulfat getrocknet, filtriert und das Lösungsmittel unter reduziertem Druck entfernt. Der Rückstand wurde an Kieselgel adsorbiert und mittels MPLC an Kieselgel chromatographisch aufgetrennt (Gradient: Cyclohexan/Essigsäureethylester). Es wurden 9,72 g 2-Chlor-5-{ [(4-chlor-1-naphthyl)oxy]methyl}pyridin erhalten.

$^1$H-NMR(400.0 MHz, $d_6$-DMSO): δ = 8.6347(4.5);8.6289(4.7);8.2855(3.6);8.2647(3.9);8.1440(3.6); 8.1229(4.0);8.0817(2.7);8.0759(2.7);8.0612(2.9);8.0553(2.9);7.7505(1.7);7.7325(3.3); 7.7140(2.2); 7.6592(2.5); 7.6462(6.3);7.6409(3.8);7.6256(6.5);7.6154(5.7);7.5948(5.0);7.1313(5.5);7.1104(5.1); 5.3830(16.0); 3.3373(32.0);2.5096(30.8);2.5056(39.6);2.5016(30.3);0.0074(0.9);-0.0002(21.8).

Stufe 2: Herstellung von 5-1[(4-Chlor-1-naphthyl)oxylmethyl}-2-(2,2-diethoxyethoxy)pyridin

Eine Lösung von 882 mg (6,58 mmol) 2,2-Diethoxyethanol in Tetrahydrofuran wurde mit 359 mg (8,22 mmol)

Natriumhydrid (55%ige Dispersion in Mineralöl) versetzt und für 30 min bei Raumtemperatur gerührt. Anschließend wurde 1,00 g (3,29 mmol) 2-Chlor-5-{ [(4-chlor-1-naphthyl)oxy]methyl}pyridin gelöst in 20 ml Tetrahydrofuran hinzugefügt und die Reaktionslösung für 4 h bei 60 °C und für 12 h bei Raumtemperatur gerührt. Daraufhin wurden weitere 359 mg (8,22 mmol) Natriumhydrid (55%ige Dispersion in Mineralöl) hinzugefügt und weitere 6 h bei 60 °C und über Nacht bei Raumtemperatur gerührt. Das Reaktionsgemisch wurde mit Wasser und Essigsäureethylester versetzt und die Phasen getrennt. Die organische Phase wurde mit Wasser gewaschen und die wässrige Phase mehrfach mit Essigsäureethylester extrahiert. Die organischen Phasen wurden vereinigt und mit gesättigter wässriger Natriumchlorid-Lösung gewaschen. Sie wurden mit Natriumsulfat getrocknet, filtriert und das Lösungsmittel unter reduziertem Druck entfernt. Der Rückstand wurde an Kieselgel adsorbiert und mittels MPLC an Kieselgel chromatographisch aufgetrennt (Gradient: Cyclohexan/Essigsäureethylester). Es wurden 128 mg 5-{[(4-Chlor-1-naphthyl)oxy]methyl}-2-(2,2-diethoxyethoxy)pyridin und 891 mg einer verunreinigten Fraktion erhalten. Letztere wurde mittels MPLC an RP-Kieselgel chromatographisch aufgetrennt (Gradient: Acetonitril/$H_2O$). Es wurden weitere 652 mg 5-{[(4-Chlor-1-naphthyl)oxy]methyl}-2-(2,2-diethoxyethoxy)pyridin erhalten.

$^1$H-NMR(400.0 MHz, $d_6$-DMSO): δ = 8.3586(1.9);8.3534(2.0);8.2309(1.4);8.2103(1.5);8.1313(1.5); 8.1105(1.6);7.9306(1.2);7.9246(1.2);7.9094(1.3);7.9034(1.3);7.7362(0.7);7.7333(0.7);7.7190(1.1);7.7158 (1.4);7.7124(0.8);7.6980(0.9);7.6950(0.9);7.6373(2.8);7.6166(3.5);7.5971(0.7);7.5944(0.6);7.1480(2.2); 7.1271(2.0);6.9259(2.1);6.9047(2.0);5.2679(5.9);4.8430(1.1);4.8297(2.5);4.8165(1.2);4.2634(4.4);4.2501 (4.2);3.7038(0.5);3.6861(1.6);3.6798(0.9);3.6684(1.8);3.6622(2.5);3.6507(0.7);3.6445(2.4);3.6269(0.7); 3.5846(0.7);3.5670(2.3);3.5608(0.7);3.5494(2.4);3.5430(1.8);3.5318(0.9);3.5254(1.6);3.5078(0.5);3.3325 (27.3);2.5258(0.6);2.5122(12.8);2.5081(24.9);2.5036(32.0);2.4991(23.6);2.4951(11.9);1.1454(7.9);1.1278 (16.0);1.1102(7.6);0.0079(0.8);-0.0002(20.6);-0.0083(0.9).

Stufe 3: Herstellung von 2-[(5-{[(4-Chlor-1-naphthyl)oxy]methyl}pyridin-2-yl)oxy]-N-methoxyethanimin

Eine Lösung von 200 mg (498 µmol) 5-{ [(4-Chlor-1-naphthyl)oxy]methyl}-2-(2,2-diethoxyethoxy)pyridin in 20 mL Ethanol wurde mit einer Lösung von 125 mg (1,49 mmol) O-Methylhydroxylaminhydrochlorid in 3 ml Wasser versetzt und über Nacht bei 65 °C gerührt. Die Lösungsmittel wurden anschließend unter reduziertem Druck entfernt. Das Reaktionsgemisch wurde mit Wasser und $CH_2Cl_2$ versetzt und die Phasen getrennt. Die organische Phase wurde mit Wasser gewaschen, mit Natriumsulfat getrocknet, filtriert und das Lösungsmittel unter reduziertem Druck entfernt. Der Rückstand wurde an Kieselgel adsorbiert und mittels MPLC an Kieselgel chromatographisch aufgetrennt (Gradient: Cyclohexan/Essigsäureethylester). Es wurden 160 mg 2-[(5-{[(4-Chlor-1-naphthyl)oxy]methyl}pyridin-2-yl)oxy]-N-methoxyethanimin erhalten.

$^1$H-NMR(400.0 MHz, $d_6$-DMSO): δ = 8.3813(2.0);8.3765(3.2);8.2340(2.3);8.2131(2.4);8.1323(2.4); 8.1114(2.7);7.9720(0.9);7.9659(1.0);7.9608(1.3);7.9546(1.3);7.9510(1.1);7.9446(1.1);7.9395(1.3);7.9335( 1.2);7.7372 (1.2);7.7344(1.2);7.7200(1.8);7.7169(2.3);7.6990(1.5);7.6960(1.4);7.6766(1.2);7.6628(2.4);7.6 490(1.3);7.6398(4.3);7.6193(5.6);7.5989(1.1);7.5963(1.0);7.1515(3.4);7.1306(3.2);7.0390(0.8);7.0298(1.8 );7.0208(0. 9);6.9829(1.5);6.9616(1.5);6.9545(2.0);6.9332(1.9);5.2769(9.9);5.0632(3.8);5.0541(3.8);4.904 3(4.8);4.8905(4.8);3.8585(11.2);3.7910(16.0);3.3331(34.8);2.5260(0.9);2.5083(35.1);2.5039(45.3);2.4994 (33.8);1.9903(1.2);1.1934(0.3);1.1756(0.6);0.0078(1.4);-0.0002(33.0).

Stufe 4: 2-[(5-{[(4-Chlor-1-naphthyl)oxy]methyl}pyridin-2-yl)oxy]-N-methoxy-N-methylethanamin

Eine Lösung von 140 mg (392 µmol) 2-[(5-{[(4-Chlor-1-naphthyl)oxy]methyl}pyridin-2-yl)oxy]-N-methoxyethanimin in 20 mL Essigsäure wurde mit 49 mg (0,79 mmol) Natriumcyanoborhydrid versetzt und 1 h bei Raumtemperatur gerührt. Anschließend wurden weitere 49 mg (0,79 mmol) Natriumcyanoborhydrid hinzugefügt. Nach 30 min bei Raumtemperatur wurden 2,98 mL (39,2 mmol) einer 36,5%igen wässrigen Formaldehyd-Lösung zugegeben und nach weiteren 20 min 74 mg (1,2 mmol) Natriumcyanoborhydrid zugesetzt. Das Reaktionsgemisch wurde für 2 h bei Raumtemperatur gerührt. Das Reaktionsgemisch wurde mit einer gesättigten wässrigen $NaHCO_3$-Lösung versetzt und mehrfach mit Essigsäureethylester extrahiert. Die vereinigten organischen Phasen wurden mit einer gesättigten wässrigen Natriumchlorid-Lösung gewaschen, mit Natriumsulfat getrocknet, filtriert und das Lösungsmittel unter reduziertem Druck entfernt. Der Rückstand wurde an Kieselgel adsorbiert und mittels MPLC an Kieselgel chromatographisch aufgetrennt (Gradient: Cyclohexan/Essigsäureethylester). Es wurden 78 mg 2-[(5-{[(4-Chlor-1-naphthyl)oxy] methyl}pyridin-2-yl)oxy] -N-methoxy-N-methylethanamin erhalten.

$^1$H-NMR(400.0 MHz, $d_6$-DMSO): δ = 8.3630(5.1);8.3579(5.5);8.2317(3.9);8.2108(4.1);8.1311(4.0); 8.1102(4.3);7.9169(3.2);7.9109(3.2);7.8956(3.3);7.8896(3.3);7.7361(1.8);7.7335(2.0);7.7160(3.8);7.6980 (2.3);7.6953(2.4);7.6387(6.2);7.6180(8.4);7.5966(1.7);7.5943(1.8);7.1509(5.6);7.1300(5.2);6.9034(5.4); 6.8821(5.2);5.2613(16.0);4.8123(1.3);4.7872(1.6);4.6588(1.7);4.4268(5.1);4.4124(10.6);4.3979(5.4); 3.4092(42.0);3.3340(86.0);3.2821(2.8);2.9629(4.6);2.9486(9.2);2.9341(4.6);2.6773(0.4);2.6731(0.6);

2.6687(0.4);2.5578(36.3);2.5082(62.5);2.5039(81.9);2.4997(64.1);2.3307(0.5);1.3969(1.8);1.2317(2.8); 0.8527(0.4);0.0077(0.8);-0.0002(16.2).

Herstellung von {3-[(5-{[(4-Chlor-1-naphthyl)oxy]methyl}pyridin-2-yl)oxy}pyrrolidin-1-yl}acetonitril

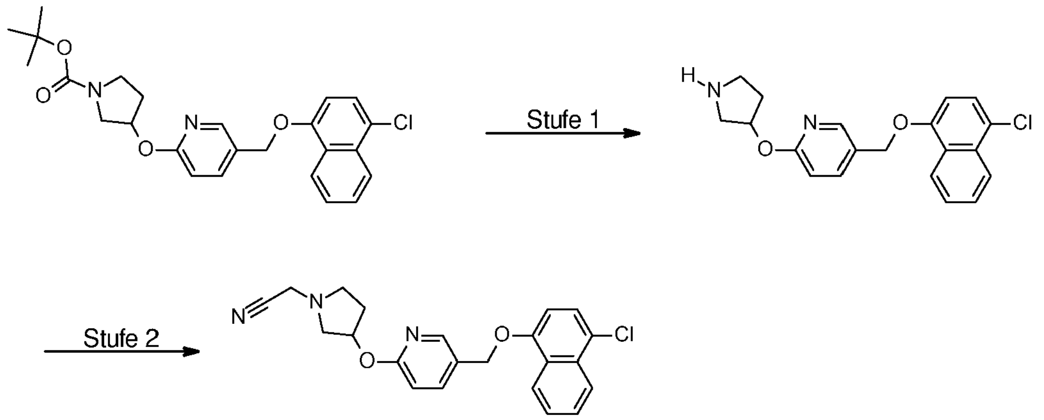


Stufe 1: Herstellung von 5-{[(4-Chlor-1-naphthyl)oxy]methyl}-2-(pyrrolidin-3-yloxy)pyridin

Eine Lösung von 280 mg (615 μmol) tert-Butyl-3-[(5-{[(4-chlor-1-naphthyl)oxy]methyl}pyridin-2-yl)oxy]pyrrolidin-1-carboxylat in 6 mL $CH_2Cl_2$ wurde bei 0 °C mit 1,54 ml (6,16 mmol) einer 4 M Lösung von Salzsäure in 1,4-Dioxan versetzt. Die Reaktionslösung wurde über Nacht bei Raumtemperatur gerührt, mit 5 mL Wasser versetzt und mit einer gesättigten wässrigen $NaHCO_3$-Lösung auf pH 8 eingestellt. Die Phasen wurden getrennt und die wässrige Phase zweimal mit 5 mL $CH_2Cl_2$ extrahiert. Die vereinigten organischen Phasen wurden mit einer gesättigten wässrigen Natriumchlorid-Lösung gewaschen, mit Natriumsulfat getrocknet, filtriert und das Lösungsmittel unter reduziertem Druck entfernt. Es wurden 249 mg 5-{ [(4-Chlor-1-naphthyl)oxy]methyl}-2-(pyrrolidin-3-yloxy)pyridin erhalten.

$^1$H-NMR(400.0 MHz, $d_6$-DMSO): δ = 8.3634(1.8);8.3580(1.7);8.2375(1.3);8.2173(1.4);8.1393(1.4); 8.1186(1.6);7.9133(1.1);7.9073(1.0);7.8920(1.1);7.8860(1.1);7.7434(0.7);7.7405(0.7);7.7262(1.0);7.7229 (1.3);7.7051(0.9);7.7021(0.8);7.6448(2.6);7.6241(3.3);7.6044(0.6);7.6018(0.6);7.1562(2.1);7.1353(1.9); 6.8639(1.8);6.8427(1.7);5.4316(0.5);5.4174(0.7);5.4022(0.4);5.2648(5.3);3.5757(16.0);3.5001(0.3); 3.4806(0.4);3.4702(0.4);3.3656(2.6);3.1757(0.6);3.1619(0.6);3.1449(0.8);3.1311(0.7);2.9862(0.6);2.9784( 0.4);2.9593 (0.8);2.9406(0.4);2.9270(0.8);2.9223(0.8);2.8958(1.0);2.8838(0.6);2.8750(0.5);2.8635(0.5); 2.8569(0.4);2.5318(0.8);2.5183(17.8);2.5140(35.3);2.5095(47.3);2.5050(34.4);2.5007(16.0);2.0926(0.4); 2.0759(0.5);2.0575(0.6);2.0415(0.5);1.8551(0.4);1.8380(0.4);1.8210(0.4).

Stufe 2: Herstellung von {3-[(5-{[(4-Chlor-1-naphthyl)oxy]methyl}pyridin-2-yl)oxy]pyrrolidin-1-yl}acetonitril

Eine Lösung von 90 mg (0,25 mmol) 5-{[(4-Chlor-1-naphthyl)oxy]methyl}-2-(pyrrolidin-3-yloxy)pyridin in 3 ml Tetrahydrofuran wurde mit 35 μL (0,25 mmol) Triethylamin und 18 μL (0,25 mmol) Bromacetonitril versetzt und für 30 min bei 70 °C gerührt. Das Lösungsmittel wurde anschließend unter reduziertem Druck entfernt. Der Rückstand wurde an Kieselgel adsorbiert und mittels MPLC an Kieselgel chromatographisch aufgetrennt (Gradient: Cyclohexan/Essigester). Es wurden 75 mg 3-[(5-{[(4-Chlor-1-naphthyl)oxy]methyl}pyridin-2-yl)oxy]pyrrolidin-1-yl}acetonitril erhalten.

$^1$H-NMR(400.0 MHz, $d_6$-DMSO): δ = 8.3569(3.4);8.3518(3.4);8.3142(4.9);8.2335(2.5);8.2124(2.7); 8.1313(2.6);8.1106(2.9);7.9180(2.2);7.9119(2.1);7.8967(2.2);7.8906(2.2);7.7358(1.2);7.7328(1.4);7.7187 (1.8);7.7154(2.5);7.7119(1.4);7.6976(1.6);7.6945(1.6);7.6371(5.9);7.6164(7.2);7.5985(1.2);7.5958(1.2); 7.1484(3.9);7.1275(3.6);6.8863(3.6);6.8650(3.5);5.7544(3.1);5.4337(0.4);5.4264(0.9);5.4188(1.0);5.4112 (1.2);5.4074(1.3);5.3999(1.1);5.3921(0.9);5.3850(0.5);5.2594(10.2);3.8567(16.0);3.3186(33.8);3.2947 (1.9);3.0038(1.5);2.9886(1.6);2.9775(2.0);2.9623(1.8);2.8469(0.6);2.8263(1.3);2.8108(1.4);2.7907(0.8); 2.7530(1.7);2.7463(1.7);2.7267(1.4);2.7201(1.4);2.6757(0.3);2.6712(0.5);2.6667(0.4);2.5830(0.8);2.5628 (1.6);2.5464(1.6);2.5420(1.2);2.5249(1.9);2.5111(25.9);2.5067(53.8);2.5022(74.6);2.4977(57.1);2.4934 (28.5);2.3698(0.4);2.3506(0.8);2.3356(1.5);2.3164(1.4);2.3016(0.9);2.2820(0.4);2.0742(1.4);1.9166(0.4); 1.9096(0.5);1.8961(0.7);1.8821(0.8);1.8750(0.8);1.8605(0.6);1.8472(0.4);1.8408(0.4);-0.0002(6.6).

Herstellung von 2-[(5-{[(4-Chlor-1-naphthyl)oxy]methyl}-6-fluorpyridin-2-yl)oxy]-N-methoxy-N-methylethanamin

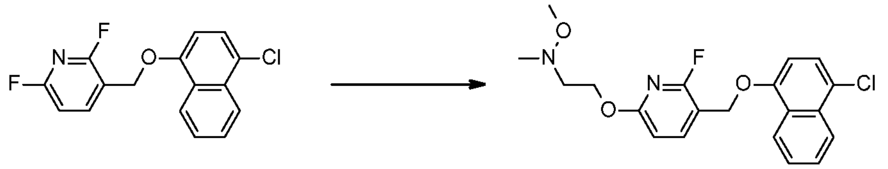

Eine Lösung von 52 mg (0,49 mmol) 2-[Methoxy(methyl)amino]ethanol in 3 mL Tetrahydrofuran und 0,5 mL N,N-Dimethylformamid wurde mit 43 mg (0,98 mmol) Natriumhydrid (55%ige Dispersion in Mineralöl) versetzt. Es wurde eine Lösung von 100 mg (327 mmol) 3-{[(4-Chlor-1-naphthyl)oxy]methyl}-2,6-difluorpyridin in 3 mL Tetrahydrofuran hinzugetropft. Das Reaktionsgemisch wurde für 2 d bei Raumtemperatur gerührt, mit Eis versetzt und anschließend mit Essigsäureethylester extrahiert. Die vereinigten organischen Phasen wurden mit einer gesättigten wässrigen Natriumchlorid-Lösung gewaschen, mit Natriumsulfat getrocknet, filtriert und das Lösungsmittel unter reduziertem Druck entfernt. Der Rückstand wurde an RP-Kieselgel adsorbiert und mittels MPLC an RP-Kieselgel chromatographisch aufgetrennt (Gradient: $H_2O$/Acetonitril). Es wurden 7 mg 2-[(5-{[(4-Chlor-1-naphthyl)oxy]methyl}-6-fluorpyridin-2-yl)oxy]-N-methoxy-N-methylethanamin erhalten.

$^1$H-NMR(400.0 MHz, $d_6$-DMSO): $\delta$ = 8.3185(0.4);8.2012(3.8);8.1806(4.1);8.1441(2.6);8.1346(4.3); 8.1235(3.5);8.1186(3.5);8.1138(5.0);8.0986(2.6);7.7379(2.0);7.7351(2.0);7.7207(3.0);7.7176(3.8);7.7141 (2.1);7.6997(2.6);7.6967(2.4);7.6441(6.7);7.6338(3.0);7.6312(3.0);7.6234(7.6);7.6165(2.7);7.6133(4.0); 7.6105(2.9);7.5956(1.9);7.5930(1.8);7.1689(6.1);7.1480(5.6);6.8636(4.3);6.8431(4.2);5.2692(16.0); 5.2479(0.4);4.3869(4.7);4.3729(9.7);4.3588(5.1);3.8703(3.1);3.4562(0.4);3.4080(45.7);3.3516(5.1);3.344 5(5.1);3.2886(0.4);3.2287(0.3);2.9594(4.3);2.9454(8.5);2.9314(4.4);2.6770(0.7);2.6724(0.9);2.6680(0.7); 2.5584(37.8);2.5257(2.1);2.5120(53.0);2.5079(106.6);2.5034(139.2);2.4989(102.1);2.4949(52.0);2.3874 (0.4);2.3346(0.7);2.3302(1.0);2.3256(0.7);1.2984(0.5);1.2584(0.8);1.2318(2.7);0.1460(0.3);0.0079(2.7); -0.0002(78.0);-0.0084(3.4);-0.1496(0.4).

Herstellung von (6-{2-[Methoxy(methyl)amino]ethoxy}pyridin-3-yl)methanol

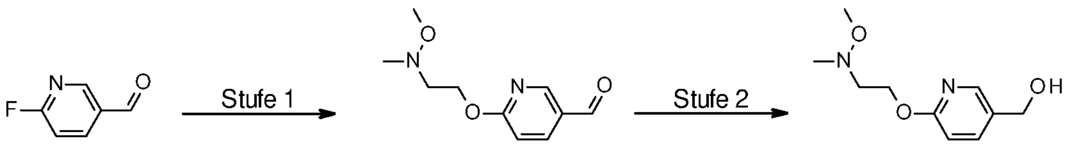


Stufe 1: Herstellung von 6-{2-[Methoxy(methyl)amino]ethoxy}nicotinaldehyd

Eine Lösung von 1,64 g (13,1 mmol) 6-Fluornicotinaldehyd in 30 mL Acetonitril wurde unter Rühren bei Raumtemperatur mit 686 mg (15,7 mmol) Natriumhydrid (55%ige Dispersion in Mineralöl) versetzt. Nach 10 min bei Raumtemperatur wurde eine Lösung von 1,38 g (13,1 mmol) 2-[Methoxy(methyl)amino]ethanol in 5 mL Acetonitril hinzugetropft. Das Reaktionsgemisch wurde über Nacht weitergerührt, anschließend mit Essigsäureethylester und Wasser versetzt und mehrmals mit Essigsäureethylester extrahiert. Die vereinigten organischen Phasen wurden mit Natriumsulfat getrocknet, filtriert und das Lösungsmittel unter reduziertem Druck entfernt. Der Rückstand wurde mittels MPLC an RP-Kieselgel chromatographisch aufgetrennt (Gradient: $H_2O$/Acetonitril). Es wurden 50 mg 6-{2-[Methoxy(methyl)amino]ethoxy}nicotinaldehyd erhalten.

$^1$H-NMR(400.0 MHz, $d_6$-DMSO): $\delta$ = 9.9600(16.0);8.7610(8.6);8.7555(8.3);8.3457(1.0);8.1297(5.1) ;8.1241(4.9);8.1081(5.5);8.1024(5.9);8.0814(0.6);8.075 2(0.5);8.0607(0.4);7.6942(0.5);7.6887(0.5);7.6727 (0.6);7.6671(0.5);7.2136(0.7);7.2067(0.6);7.1927(0.6);7.1859(0.5);7.0042(7.5);6.9826(7.2);6.8655(0.9); 6.8443(0.8);5.3751(2.9);5.1906(0.6);5.1765(1.0);5.1623(0.5);4.8916(0.4);4.5316(7.1);4.5175(14.4); 4.5033(7.6);4.4439(1.6);4.4299(1.5);3.4326(0.9);3.4004(56.2);3.3517(0.7);3.3210(24.3);2.9809(6.7); 2.9668(12.9);2.9526(6.7);2.9050(0.4);2.6761(0.5);2.6720(0.6);2.5761(1.0);2.5563(50.5);2.5068(89.3); 2.5025(113.6);2.4984(85.1);2.3338(0.5);2.3295(0.7);2.0957(0.8);2.0391(1.0);0.0038(9.9);-0.0004(50.2).

Stufe 2: Herstellung von (6-{2-[Methoxy(methyl)amino]ethoxy}pyridin-3-yl)methanol

Eine Lösung von 100 mg (476 μmol) 6-{2-[Methoxy(methyl)amino]ethoxy}nicotinaldehyd wurde in einer Mischung von 15 mL Methanol und 25 mL Tetrahydrofuran gelöst und bei 0 °C mit 27 mg (0,71 mmol) Natriumborhydrid versetzt. Die Reaktionslösung wurde für 1 h bei Raumtemperatur gerührt. Anschließend wurde Wasser hinzugefügt, die organischen Lösungsmittel unter reduziertem Druck entfernt und die wässrige Phase mehrmals mit Essigsäureethylester extrahiert. Die vereinigten organischen Phasen wurden mit Natriumsulfat getrocknet, filtriert und das Lösungsmittel unter reduziertem Druck entfernt. Es wurden 90 mg (6-{2-[Methoxy(methyl)amino]ethoxy}pyridin-3-yl)methanol erhalten.

$^1$H-NMR(400.0 MHz, $d_6$-DMSO): δ = 6.7875(0.4);6.7660(0.3);5.1460(0.5);4.4268(0.7);4.4128(0.6); 4.3792(0.4);4.3647(0.8);4.3501(0.4);3.3975(3.3);3.3805(1.3);3.3196(12.3);3.3030(4.8);3.2951(0.5); 3.2853(3.6);2.9374(0.4);2.9228(0.7);2.9083(0.3);2.6950(16.0);2.5447(2.8);2.5236(0.4);2.5103(8.4); 2.5059(17.3);2.5014(23.1);2.4968(16.9);2.4926(8.6);2.1965(1.8);2.1765(3.5);2.1560(2.4);1.9887(1.4); 1.9379(0.9);1.9203(2.2);1.9174(1.8);1.9117(0.5);1.9017(2.5);1.8914(0.4);1.8819(2.0);1.8621(0.6);1.3551 (0.5);1.2351(0.4);1.1924(0.4);1.1746(0.7);1.1568(0.4);-0.0001(2.2).

Herstellung von 6-Fluor-3'-(trifluormethoxy)biphenyl-3-ol

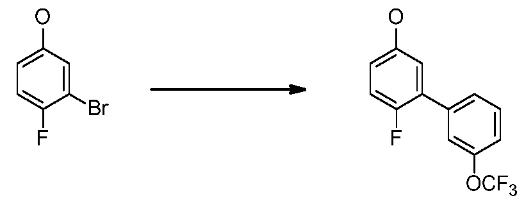

Eine Gemisch von 5,0 g (26 mmol) 3-Brom-4-fluorphenol, 7,00 g (34,0 mmol) [3-(Trifluormethoxy)phenyl]boronsäure, 7,60 g (55,0 mmol) Kaliumcarbonat, 35 mL Tetrahydrofuran und 7 mL Wasser wurde durch Einleiten eines Argonstroms von gelöstem Sauerstoff befreit und dann mit 0,50 g (0,43 mmol) Tetrakis-(triphenylphosphin)-palladium versetzt. Das Reaktionsgemisch wurde über Nacht bei 80 °C gerührt, dann unter reduziertem Druck eingeengt und mit 1 M Salzsäure auf pH 1 eingestellt. Es wurde mit Wasser und Essigsäureethylester versetzt, die Phasen getrennt und die wässrige Phase mehrmals mit Essigsäureethylester extrahiert. Die vereinigten organischen Phasen wurden mit einer gesättigten wässrigen Natriumchlorid-Lösung gewaschen, mit Natriumsulfat getrocknet, filtriert und das Lösungsmittel unter reduziertem Druck entfernt. Der Rückstand wurde mittels MPLC an RP-Kieselgel chromatographisch aufgetrennt (Gradient: $H_2O$/Acetonitril). Es wurden 4,85 g 6-Fluor-3'-(trifluormethoxy)biphenyl-3-ol erhalten.

$^1$H-NMR(400.0 MHz, $d_6$-DMSO): δ = 8.3185(1.1);7.6289(0.4);7.6203(1.7);7.6006(4.7);7.5807(3.6); 7.5675(0.4);7.5484(2.9);7.5453(3.0);7.5287(1.6);7.5257(1.5);7.4457(3.0);7.4083(1.8);7.4056(1.9);7.4028 (1.6);7.3882(1.5);7.3855(1.6);7.3826(1.4);7.1303(2.2);7.1081(2.7);7.1037(2.5);7.0815(2.6);6.8504(2.1); 6.8429(2.6);6.8339(2.1);6.8264(2.6);6.7931(1.5);6.7833(2.0);6.7755(1.3);6.7710(1.4);6.7614(1.6);6.7535 (1.0);2.5257(0.5);2.5123(15.4);2.5079(32.4);2.5034(43.5);2.4989(31.4);2.4945(15.1);2.0760(16.0);0.0080 (0.7);-0.0002(21.3);-0.0085(0.8).

Herstellung von 2-Chlor-5-{[(4-chlor-1-naphthyl)oxy]methyl}pyrimidin

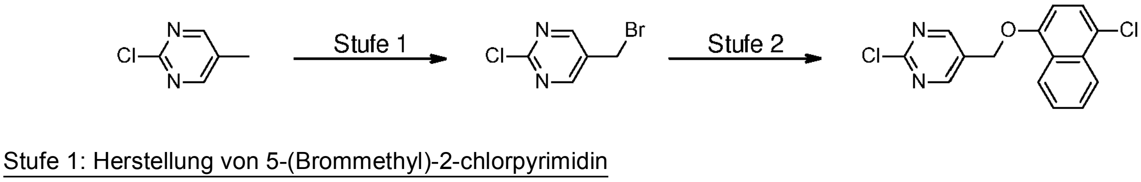


Stufe 1: Herstellung von 5-(Brommethyl)-2-chlorpyrimidin

Eine Lösung von 200 mg (1,56 mmol) 2-Chlor-5-methylpyrimidin, 277 mg (1,56 mmol) N-Bromsuccinimid und 26 mg (0,16 mmol) 2,2'-Azobis(2-methylpropionitril) in 17,6 mL Chlorbenzol für wurde für 16 h unter Rückfluss erhitzt. Anschließend wurde die Reaktionslösung mit einer wässrigen $Na_2SO_3$-Lösung und mit gesättigter wässriger $NaHCO_3$-Lösung gewaschen, mit Natriumsulfat getrocknet, filtriert und das Lösungsmittel unter reduziertem Druck entfernt. Der Rückstand wurde mittels MPLC an Kieselgel chromatographisch aufgetrennt (Gradient: Cyclohexan/Essigsäureethylester). Es wurden 76 mg 5-(Brommethyl)-2-chlorpyrimidin erhalten.

$^1$H-NMR(400.0 MHz, $d_6$-DMSO): δ = 8.9119(1.1);8.8913(15.0);8.8619(0.7);8.8222(1.3);8.6390(1.4); 5.5539(0.5);5.3766(0.9);4.8528(2.2);4.7481(16.0);4.7234(1.3);3.3281(67.0);2.6718(0.4);2.5248(0.8); 2.5114(23.6);2.5071(48.7);2.5027(64.9);2.4983(46.7);2.4941(22.6);2.3295(0.4);2.2725(2.8);2.2461(0.6); 1.4376(0.4);-0.0002(0.7).

Stufe 2: Herstellung von 2-Chlor-5-{[(4-chlor-1-naphthyl)oxy]methy}pyrimidin

Eine Suspension von 38 mg (0,21 mmol) 4-Chlor-1-naphthol und 60 mg (0,43 mmol) Kaliumcarbonat in 1 mL Acetonitril wurde für 1 h bei Raumtemperatur gerührt. Das Gemisch wurde auf 0 °C abgekühlt und tropfenweise mit einer Lösung von 76 mg (0,27 mmol) 5-(Brommethyl)-2-chlorpyrimidin in Acetonitril versetzt. Das Reaktionsgemisch wurde über Nacht bei Raumtemperatur gerührt und anschließend mit Wasser und einer gesättigten $NaHCO_3$-Lösung versetzt. Es wurde mehrmals mit Essigsäureethylester extrahiert. Die vereinigten organischen Phasen wurden mit Wasser und einer gesättigten wässrigen Natriumchlorid-Lösung gewaschen, mit Natriumsulfat getrocknet, filtriert und das Lösungsmittel unter reduziertem Druck entfernt. Der Rückstand wurde mittels MPLC an Kieselgel chromatographisch aufgetrennt (Gradient: Cyclohexan/Essigsäureethylester). Es wurden 70 mg 2-Chlor-5-{ [(4-chlor-1-naphthyl)oxy]methyl}pyrimidin erhalten.

$^1$H-NMR(400.0 MHz, $d_6$-DMSO): δ = 9.0173(16.0);8.9478(1.4);8.8875(0.4);8.3103(2.7);8.2893(2.8); 8.1476(2.8);8.1269(3.1);7.7565(1.4);7.7534(1.6);7.7393(2.2);7.7359(2.9);7.7323(1.5);7.7183(2.0);7.7150 (1.9);7.6608(6.9);7.6439(1.8);7.6402(8.3);7.6231(1.4);7.6202(1.3);7.1554(4.4);7.1485(0.6);7.1345(4.1); 7.1276(0.5);5.4057(11.9);5.3819(1.1);4.8531(0.5);3.3215(34.5);2.6719(0.4);2.5254(1.0);2.5206(1.5); 2.5120(25.1);2.5075(54.3);2.5029(73.9);2.4983(52.7);2.4938(24.5);2.3297(0.4);0.1459(0.4);0.0080(3.4); -0.0002(106.7);-0.0085(3.6);-0.1498(0.4).

Herstellung von 4-Chlor-3-methoxyphenol und 2-Chlor-5-methoxyphenol

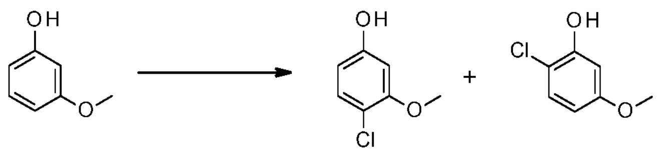

Eine Lösung von 300 mg (2,42 mmol) 3-Methoxyphenol in 2 mL $CH_2Cl_2$ wurde bei 0 °C mit 342 mg (2,54 mmol) Sulfurylchlorid versetzt. Die Reaktionslösung wurde für 1 h bei 0 °C gerührt und anschließend Wasser und $CH_2Cl_2$ hinzugefügt. Die Phasen wurden getrennt und die wässrige Phase mehrmals mit $CH_2Cl_2$ extrahiert. Die vereinigten organischen Phasen wurden mit einer gesättigten wässrigen Natriumchlorid-Lösung gewaschen, mit Natriumsulfat getrocknet, filtriert und das Lösungsmittel unter reduziertem Druck entfernt. Der Rückstand wurde an RP-Kieselgel adsorbiert und mittels MPLC an RP-Kieselgel chromatographisch aufgetrennt (Gradient: $H_2O$/Acetonitril). Es wurden 125 mg 4-Chlor-3-methoxyphenol und 194 mg 2-Chlor-5-methoxyphenol erhalten.

4-Chlor-3 -methoxyphenol:
$^1$H-NMR(400.0 MHz, $d_6$-DMSO): δ = 9.6746(2.2);7.1593(2.9);7.1379(3.1);7.0630(0.3);7.0428(0.5); 6.5162(2.7);6.5096(2.8);6.3642(1.8);6.3576(1.7);6.3484(0.4);6.3427(1.7);6.3362(1.7);6.3152(0.4);6.3094 (0.4);3.7876(0.8);3.7744(16.0);3.7390(0.5);3.6885(0.6);3.6800(2.9);3.3248(6.3);2.5240(0.4);2.5064(18.8); 2.5021(24.2);2.4978(17.3);2.0253(0.4);0.0078(0.5);-0.0002(11.6);-0.0073(0.4).

2-Chlor-5 -methoxyphenol:
$^1$H-NMR(400.0 MHz, $d_6$-DMSO): δ = 10.1114(4.9);7.2062(2.6);7.1843(2.8);6.5256(2.6);6.5185(2.9); 6.4149(1.6);6.4077(1.4);6.3929(1.5);6.3857(1.3);3.6893(16.0);3.3297(3.7);2.5112(4.3);2.5071(8.6);2.502 8(11.2);2.4984(7.9).

Herstellung von 5-[2-(Diethylamino)ethoxy]pyridin-2-carbaldehyd

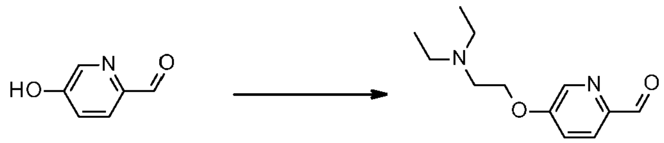

Eine Suspension von 1,0 g (8,1 mmol) 5-Hydroxypyridin-2-carbaldehyd und 3,4 g (25 mmol) $K_2CO_3$ in 25 mL N,N-Dimethylformamid wurde für 2 h bei 65 °C gerührt. Anschließend wurden bei 30 °C 13 mg (81μmol) Kaliumiodid und 2,43 g (9,14 mmol) 2-Brom-N,N-diethylethanaminhydrobromid (1:1) hinzugefügt und das Reaktionsgemisch für 16 h bei 65 °C gerührt. Es wurden dann weitere 1,27 g (4,87 mmol) 2-Brom-N,N-diethylethanaminhydrobromid (1:1), 1,7 g (12 mmol) Kaliumcarbonat und 13 mg (81 μmol) Kaliumiodid hinzugegeben und das Gemisch weitere 20 h bei 65 °C gerührt. Das Reaktionsgemisch wurde über Kieselgur filtriert und das Lösungsmittel unter reduziertem Druck entfernt. Der Rückstand wurde mittels MPLC an RP-Kieselgel chromatographisch aufgetrennt (Gradient: $H_2O$/Acetonitril). Nach dem Entfernen des Lösungsmittels unter reduziertem Druck wurde der Rückstand mit Wasser versetzt und mehrmals mit Ethylacetat extrahiert. Die vereinigten organischen Phasen wurden mit Natriumsulfat getrocknet, filtriert und das Lösungsmittel unter reduziertem Druck entfernt. Es wurden 803 mg 5-[2-(Diethylamino)ethoxy]pyridin-2-carbaldehyd erhalten.

$^1$H-NMR(400.0 MHz, $d_6$-DMSO): δ = 9.8858(4.6);8.4862(2.4);8.4793(2.5);7.9378(2.3);7.9161(2.6); 7.6092(1.2);7.6024(1.3);7.5874(1.1);7.5807(1.1);4.2358(2.2);4.2208(4.6);4.2059(2.3);3.3230(10.4); 2.8232(2.2);2.8083(4.5);2.7933(2.2);2.5739(2.3);2.5561(7.3);2.5383(7.5);2.5205(3.2);2.5073(16.8); 2.5030(22.2);2.4987(17.2);0.9826(8.0);0.9649(16.0);0.9471(7.7);-0.0002(20.2).

Herstellung von Ethyl-5-(4-ethylpiperazin-1-yl)-3-fluorpyridin-2-carboxylat

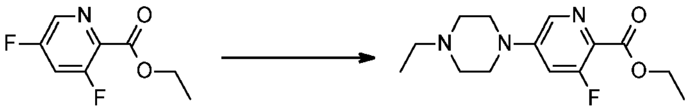

Eine Suspension von 1,98 g (17,4 mmol) 1-Ethylpiperazin, 2,50 g (13,4 mmol) Ethyl-3,5-difluorpyridin-2-carboxylat und 2,40 g (17,4 mmol) Kaliumcarbonat in 21 mL N,N-Dimethylacetamid wurde in einem Mikrowellenreaktor für 2 h bei 70 °C erhitzt. Das Reaktionsgemisch wurde anschließen mit Wasser versetzt und mehrmals mit Ethylacetat extrahiert. Die vereinigten organischen Phasen wurden mit Natriumsulfat getrocknet, filtriert und das Lösungsmittel unter reduziertem Druck entfernt. Der Rückstand wurde mittels MPLC an Kieselgel chromatographisch aufgetrennt (Gradient: Cyclohexan/Essigsäureethylester +1% v/v Triethylamin). Es wurden 1,46 g Ethyl-5-(4-ethylpiperazin-1-yl)-3-fluorpyridin-2-carboxylat erhalten.

$^1$H-NMR(400.0 MHz, $d_6$-DMSO): δ = 8.2421(2.7);8.2374(4.2);7.2262(2.2);7.2203(2.2);7.1891(2.2); 7.1832(2.2);4.2963(2.2);4.2786(7.0);4.2608(7.1);4.2431(2.3);3.4222(6.1);3.4097(7.9);3.3967(6.4);3.3230( 23.9);2.945 5(4.4);2.7859(3.6);2.5080(21.5);2.5036(28.6);2.4992(21.7);2.4833(6.8);2.4705(8.4);2.4579(6. 3);2.3926(1.9);2.3746(6.1);2.3566(6.2);2.3387(2.1);1.9587(3.8);1.3048(7.7);1.2870(16.0);1.2693(7.5); 1.0464(6.7);1.0284(13.9);1.0105(6.4);0.0079(0.8);-0.0002(18.1);-0.0082(0.8).

Herstellung von 2-[(6-{[(5,6-Dichlor-1-naphthyl)oxy]methy}-2-fluorpyridin-3-yl)oxy]-N-methoxy-N-methylethanamin

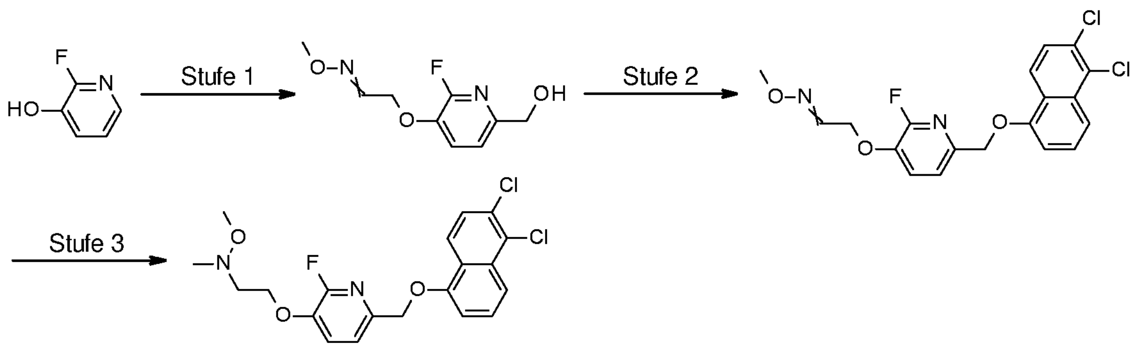


Stufe 1: Herstellung von {6-Fluor-5-[2-(methoxyimino)ethoxy]pyridin-2-yl}methanol

Eine Lösung von 300 mg (2,65 mmol) 2-Fluorpyridin-3-ol in 2,3 mL Wasser wurde mit 334 mg (3,97 mmol) Natriumhydrogencarbonat versetzt und 15 min bei Raumtemperatur gerührt. Nach Erwärmen auf 90 °C wurde das Gemisch portionsweise mit 696 $\mu$L (9,28 mmol) einer 37 %igen wäßrigen Formaldehyd-Lösung versetzt und 2,5 h bei 90 °C und weitere 16 h bei Raumtemperatur gerührt. Nach Zugabe von 2 g Eis wurde die Mischung mit 6 N wässriger Salzsäure auf pH 1 gestellt und mehrmals mit Essigsäureethylester extrahiert. Die vereinigten organischen Extrakte wurden über Natriumsulfat getrocknet, filtriert und das Lösungsmittel unter reduziertem Druck entfernt. Der Rückstand wurde in Dichlormethan aufgenommen, filtriert und das Lösungsmittel unter reduziertem Druck entfernt. Das so erhaltene Zwischenprodukt 2-Fluor-6-(hydroxymethyl)pyridin-3-ol wurde in 9 mL Acetonitril gelöst, mit 579 mg (4,19 mmol) Kaliumcarbonat und 413 mg (3,07 mmol, Reinheit 80 %) 2-Chlor-N-methoxyethanimin versetzt, anschließend 4 h unter Rückfluss erhitzt und weitere 16 h bei Raumtemperatur gerührt. Nach Abfiltrieren des Bodensatzes wurde das Filtrat an Kieselgel adsorbiert und mittels MPLC an Kieselgel chromatographisch aufgetrennt (Gradient: Cyclohexan/Essigsäureethylester). Es wurden 205 mg eines (E,Z)-Isomerengemisches von {6-Fluor-5-[2-(methoxyimino)ethoxy]pyridin-2-yl}methanol erhalten.

$^1$H-NMR(400.0 MHz, $d_6$-DMSO): $\delta$ = 7.7277(0.9);7.7073(1.0);7.7015(1.0);7.6810(1.0);7.6596(1.1); 7.6456(2.5);7.6316(1.2);7.6227(0.4);7.6167(0.4);7.5963(0.4);7.3251(1.6);7.3048(1.4);7.1226(0.4);7.1133 (0.9);7.1040(0.4);5.4528(0.7);5.4500(1.3);5.4382(1.6);5.4352(2.8);5.4235(0.8);5.4205(1.4);4.9464(1.8); 4.9371(1.8);4.7654(4.5);4.7514(4.4);4.4156(4.5);4.4009(4.4);3.8674(5.9);3.7989(16.0);3.3316(17.6);2.52 51(0.4);2.5112(9.1);2.5072(18.1);2.5028(23.6);2.4983(17.5);-0.0002(4.4).

Stufe 2: Herstellung von 2-[(6-{[(5,6-Dichlor-1-naphthyl)oxy]methyl}-2-fluorpyridin-3-yl)oxy]-N-methoxyethanimin

Eine Lösung von 190 mg (0,88 mmol) eines (E,Z)-Isomerengemisches von {6-Fluor-5-[2-(methoxy-imino)ethoxy]pyridin-2-yl}methanol in 6 mL Toluol wurden mit 194 $\mu$L (2,66 mmol) Thionylchlorid und einer katalytischen Menge N,N-Dimethylformamid versetzt. Das Reaktionsgemisch wurde für 3,5 h bei Raumtemperatur gerührt und anschließend unter reduziertem Druck eingeengt. Der Rückstand wurde in 6 mL N,N-Dimethylformamid gelöst und mit 246 mg (1,15 mmol) 5,6-Dichlor-1-naphthol sowie 368 mg (2,66 mmol) Kaliumcarbonat versetzt. Das Reaktionsgemisch wurde für 50 min bei 70 °C gerührt und anschließend unter reduziertem Druck eingeengt. Der Rückstand wurde in Dichlormethan aufgenommen, an Kieselgel adsorbiert und mittels MPLC an Kieselgel chromatographisch aufgetrennt (Gradient: Cyclohexan/Essigsäureethylester). Es wurden 332 mg eines (E,Z)-Isomerengemisches von 2-[(6-{[(5,6-Dichlor-1-naphthyl)oxy]methyl}-2-fluorpyridin-3-yl)oxy]-N-methoxyethanimin erhalten.

$^1$H-NMR(400.0 MHz, $d_6$-DMSO): $\delta$ = 8.2461(1.6);8.2385(0.8);8.2236(1.8);8.2158(0.9);8.1528(0.4); 8.1300(0.4);7.8100(1.9);7.8011(0.8);7.7885(2.7);7.7808(1.1);7.7752(0.8);7.7546(0.9);7.7077(3.1);7.6876 (3.4);7.6849(3.5);7.6688(4.6);7.6549(1.2);7.6479(1.4);7.6134(0.6);7.5859(1.7);7.5663(2.1);7.5548(0.4); 7.5472(0.6);7.5357(0.4);7.2592(2.2);7.2397(2.0);7.1517(0.5);7.1424(1.1);7.1331(0.5);7.0119(0.3);5.2828 (7.5);4.9992(2.0);4.9899(2.0);4.8222(4.0);4.8083(3.9);3.8747(6.9);3.8067(16.0);3.3316(25.0);2.6729(0.3); 2.5260(0.9);2.5125(20.4);2.5083(41.0);2.5038(53.8);2.4993(39.3);2.4950(19.3);2.3305(0.4);1.3971(0.4); 0.0079(0.6);-0.0002(16.4);-0.0084(0.6).

Stufe 3: 2-[(6-([(5,6-Dichlor-1-naphthyl)oxy]methyl}-2-fluorpvridin-3-yl)oxy]-N-methoxy-N-methylethanamin

Eine Lösung von 295 mg (0,72 mmol) eines (E,Z)-Isomerengemisches von 2-[(6-{[(5,6-Dichlor-1-naphthyl)oxy]methyl}-2-fluorpyridin-3-yl)oxy]-N-methoxyethanimin in 6,2 mL Essigsäure wurde mit 68 mg (1,08 mmol) Natriumcyanoborhydrid versetzt. Nach 30 min bei Raumtemperatur wurden 1,08 mL (14,4 mmol) einer 37 %igen wässrigen Formaldehyd-Lösung zugegeben und nach weiteren 5 min 68 mg (1,08 mmol) Natriumcyanoborhydrid zugesetzt. Das

Reaktionsgemisch wurde für 30 min bei Raumtemperatur gerührt und unter reduziertem Druck eingeengt. Der Rückstand wurde in Dichlormethan aufgenommen, an Kieselgel adsorbiert und mittels MPLC an Kieselgel chromatographisch aufgetrennt (Gradient: Cyclohexan/Essigsäureethylester). Es wurden 236 mg 2-[(6-{[(5,6-Dichlor-1-naphthyl)oxy]methyl}-2-fluorpyridin-3-yl)oxy]-N-methoxy-N-methyl-ethanamin erhalten.

$^1$H-NMR(400.0 MHz, $d_6$-DMSO): $\delta$ = 8.2363(2.0);8.2351(2.0);8.2136(2.2);8.2124(2.2);7.8066(1.6); 7.7851(3.2);7.7646(1.2);7.7589(1.0);7.7384(1.1);7.7052(3.3);7.6877(1.6);7.6825(3.2);7.6679(2.1);7.6468 (1.3);7.5581(2.0);7.5379(1.7);7.2605(1.9);7.2413(1.8);5.2740(6.6);4.2559(1.9);4.2421(4.0);4.2282(2.0); 3.4043(19.9);3.3348(27.4);2.9998(1.7);2.9861(3.5);2.9722(1.7);2.5742(16.0);2.5273(0.4);2.5139(10.8); 2.5095(22.1);2.5050(29.0);2.5005(21.1);2.4961(10.3);1.3968(0.4);0.0079(0.5);-0.0002(15.7);-0.0084(0.5).

Herstellung von 2-[(6-{[(5,6-Dichlor-1-naphthyl)oxy]methy}-5-fluorpyridin-3-yl)oxy]-N-methoxy-N-methylethanamin

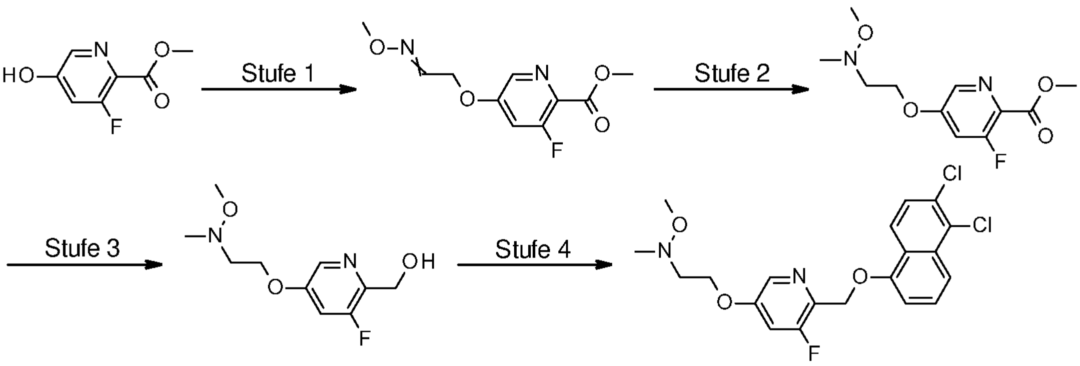


Stufe 1: Herstellung von Methyl-3-fluor-5-[2-(methoxyimino)ethoxy]pyridin-2-carboxylat

Eine Lösung von 7,59 g (44,4 mmol) Methyl-3-fluor-5-hydroxypyridin-2-carboxylat in 253 mL Acetonitril wurde bei Raumtemperatur mit 18,39 g (133 mmol) Kaliumcarbonat und 59,1 mL (44,4 mmol) einer 0,75 M Lösung von 2-Chlor-N-methoxyethanimin in Acetonitril versetzt. Das Reaktionsgemisch wurde für 27 h unter Rückfluss erhitzt und und weitere 3 d bei Raumtemperatur gerührt. Nach Abfiltrieren des Bodensatzes wurde das Filtrat unter reduziertem Druck eingeengt. Es wurden 5,17 g eines (E,Z)-Isomerengemisches von Methyl-3-fluor-5-[2-(methoxyimino)ethoxy]pyridin-2-carboxylat erhalten.

$^1$H-NMR(400.0 MHz, $d_6$-DMSO): $\delta$ = 8.3132(1.5);8.3107(1.6);8.3074(1.7);8.3004(0.7);8.2977(0.7); 8.2942(0.7);7.6847(1.0);7.6710(3.1);7.6648(1.2);7.6574(1.1);7.6391(1.0);7.6331(1.1);7.6238(0.4);7.6178 (0.4);7.5925(0.4);7.5864(0.4);7.1439(0.4);7.1344(0.8);7.1251(0.4);5.0357(1.5);5.0263(1.5);4.8625(4.0); 4.8488(3.9);3.8787(5.0);3.8551(16.0);3.8083(14.4);3.3195(2.3);2.5068(17.8);2.5024(23.4);2.4981(17.5); 0.0079(0.6);-0.0002(18.6);-0.0085(0.9).

Stufe 2: Herstellung von Methyl-3-fluor-5-{2-[methoxy(methyl)amino]ethoxy}pyridin-2-carboxylat

Eine Lösung von 5,17 g eines (E,Z)-Isomerengemisches von Methyl-3-fluor-5-[2-(methoxyimino)ethoxy]-pyridin-2-carboxylat in 180 mL Essigsäure wurde mit 2,01 g (32,0 mmol) Natriumcyanoborhydrid versetzt. Nach 1 h bei Raumtemperatur wurden 32,0 mL (427 mmol) einer 37 %igen wässrigen Formaldehyd-Lösung zugegeben und nach weiteren 15 min 2,01 g (32,0 mmol) Natriumcyanoborhydrid zugesetzt. Das Reaktionsgemisch wurde für 1 h bei Raumtemperatur gerührt und anschließend unter reduziertem Druck eingeengt. Der Rückstand wurde in Dichlormethan aufgenommen, an Kieselgel adsorbiert und mittels MPLC an Kieselgel chromatographisch aufgetrennt (Gradient: Cyclohexan/Essigsäureethylester). Es wurden 4,61 g Methyl-3-fluor-5-{2-[methoxy(methyl)amino]ethoxy}pyridin-2-carboxylat erhalten.

$^1$H-NMR(400.0 MHz, $d_6$-DMSO): $\delta$ = 8.2901(1.6);8.2874(2.2);8.2844(2.2);8.2817(2.1);7.6473(1.3); 7.6414(1.4);7.6153(1.3);7.6094(1.4);4.2890(2.2);4.2754(4.2);4.2617(2.2);3.8498(16.0);3.4048(19.4); 3.3192(5.5);2.9915(1.8);2.9779(3.7);2.9643(1.9);2.5678(16.0);2.5202(0.5);2.5112(8.5);2.5070(18.4); 2.5025(25.4);2.4981(19.5);1.9893(0.4);1.9092(6.7);0.0079(0.6);-0.0002(18.8);-0.0083(0.9).

Stufe 3: Herstellung von (3-Fluor-5-{2-[methoxy(methyl)amino]ethoxy}pyridin-2-yl)methanol

Eine Lösung von 7,84 g (30,4 mmol) Methyl-3-fluor-5-{2-[methoxy(methyl)amino]ethoxy}pyridin-2-carboxylat in 78 mL Ethanol wurden bei 10 °C mit 2,78 g (73,6 mmol) Natriumborhydrid versetzt und die Reaktionsmischung 1 h bei 10 °C und dann weitere 16 h bei Raumtemperatur gerührt. Nach Zugabe von 100 mL Wasser unter Eiskühlung wird 20 min bei Raumtemperatur gerührt. Anschließend wurde unter reduziertem Druck Ethanol weitgehend entfernt und der Rückstand mehrmals mit Essigsäureethylester extrahiert. Die vereinigten organischen Phasen wurden mit einer gesättigten wäßrigen Natriumchlorid-Lösung gewaschen, mit Natriumsulfat getrocknet, filtriert und das Filtrat unter reduziertem Druck eingeengt. Es wurden 5,31 g (3-Fluor-5-{2-[methoxy(methyl)amino]ethoxy}pyridin-2-yl)methanol erhalten.
$^1$H-NMR(400.0 MHz, $d_6$-DMSO): $\delta$ = 8.1383(2.4);8.1339(2.4);7.4605(1.4);7.4548(1.4);7.4312(1.4); 7.4254(1.4);5.1829(1.2);5.1683(2.5);5.1537(1.2);4.5150(2.9);4.5095(3.0);4.5004(2.9);4.4950(2.8);4.2006 (2.3);4.1868(4.6);4.1730(2.4);3.4028(18.6);3.3203(16.8);2.9662(2.1);2.9525(4.1);2.9386(2.1);2.5626 (16.0);2.5058(38.8);2.5016(49.8);2.4974(37.0);-0.0002(8.2).

Stufe 4: Herstellung von 2-[(6-{[(5,6-Dichlor-1-naphthyl)oxy]methyl}-5-fluorpyridin-3-yl)oxyl-N-methoxy-N-methylethanamin

1,0 g (4,34 mmol) (3-Fluor-5-{2-[methoxy(methyl)amino]ethoxy}pyridin-2-yl)methanol wurden mit 20 mL (273 mmol) Thionylchlorid und einer katalytischen Menge N,N-Dimethylformamid versetzt. Das Reaktionsgemisch wurde für 80 min bei Raumtemperatur gerührt und anschließend unter reduziertem Druck eingeengt. Der Rückstand wurde in 26 mL N,N-Dimethylformamid gelöst und mit 787 mg (3,69 mmol) 5,6-Dichlor-l-naphthol sowie 2,55 g (18,5 mmol) Kaliumcarbonat versetzt. Das Reaktionsgemisch wurde für 4 h 20 min bei 70 °C und weitere 15 h bei Raumtemperatur gerührt und anschließend unter reduziertem Druck eingeengt. Der Rückstand wurde in Dichlormethan aufgenommen, an Kieselgel adsorbiert und mittels MPLC an Kieselgel chromatographisch aufgetrennt (Gradient: Cyclohexan/Essigsäureethylester). Es wurden 1,16 g 2-[(6-{[(5,6-Dichlor-1-naphthyl)oxy]methyl}-5-fluorpyridin-3-yl)oxy]-N-methoxy-N-methylethanamin erhalten.
$^1$H-NMR(400.0 MHz, $d_6$-DMSO): $\delta$ = 8.2575(2.3);8.2531(2.4);8.0842(2.2);8.0615(2.5);7.8056(1.7); 7.7842(2.6);7.7031(1.6);7.6833(2.3);7.6593(3.7);7.6365(3.1);7.6107(1.4);7.6047(1.3);7.5811(1.4);7.5752 (1.4);7.3719(2.2);7.3525(1.9);5.3891(4.6);5.3853(4.7);4.2479(2.0);4.2342(4.2);4.2204(2.1);3.4116(19.6); 3.3190(60.2);2.9871(1.9);2.9735(3.7);2.9597(1.9);2.6759(0.5);2.6712(0.7);2.6670(0.5);2.5718(16.0); 2.5244(2.2);2.5108(43.8);2.5066(86.7);2.5022(117.2);2.4978(89.0);2.3336(0.5);2.3291(0.7);2.3247(0.5); 1.9000(0.4);0.0079(1.3);-0.0001(31.9);-0.0081(1.4).

Herstellung von {[2-({6-[(Dibenzo[b,d]furan-2-yloxy)methyl]-5-fluorpyridin-3-yl}oxy)ethyl](methyl)-amino}acetonitril

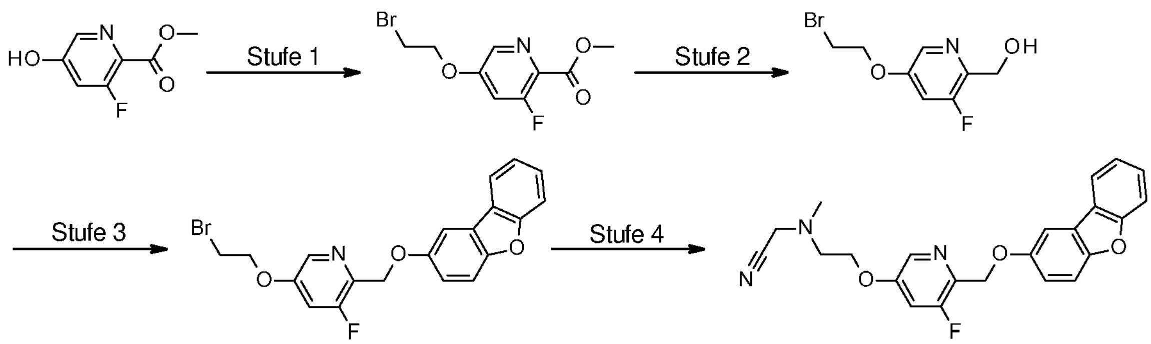


Stufe 1: Herstellung von Methyl-5-(2-bromethoxy)-3-fluorpyridin-2-carboxylat

Eine Lösung von 3,50 g (20,5 mmol) Methyl-3-fluor-5-hydroxypyridin-2-carboxylat in 100 mL Acetonitril wurde bei Raumtemperatur mit 7,07 g (51,1 mmol) Kaliumcarbonat und 8,9 mL (103 mmol) 1,2-Dibromethan versetzt. Das Reaktionsgemisch wurde über Nacht bei 80 °C gerührt, anschließend an Kieselgel adsorbiert und mittels MPLC an Kieselgel chromatographisch aufgetrennt (Gradient: Cyclohexan/Essigsäureethylester). Es wurden 1,96 g Methyl-5-(2-bromethoxy)-3-fluorpyridin-2-carboxylat erhalten.
$^1$H-NMR(400.0 MHz, $d_6$-DMSO): $\delta$ = 8.3079(1.6);8.3054(1.8);8.3021(1.8);7.6591(1.2);7.6532(1.2); 7.6275(1.2);7.6215(1.2);4.5409(2.1);4.5277(2.7);4.5142(2.3);3.8715(2.4);3.8545(16.0);3.8449(2.6);

3.3192(14.1);2.5107(13.3);2.5064(27.3);2.5019(37.7);2.4975(28.6);2.4932(14.4);1.3979(0.8);0.0080(0.4); -0.0002(10.4);-0.0083(0.5).

Stufe 2: Herstellung von [5-(2-Bromethoxy)-3-fluorpyridin-2-yl]methanol

Eine Lösung von 9,39 g (33,8 mmol) Methyl-5-(2-bromethoxy)-3-fluorpyridin-2-carboxylat in 300 mL Ethanol wurden unter Eiskühlung mit 3,19 g (84,4 mmol) Natriumborhydrid versetzt und die Reaktionsmischung 1 h unter Eiskühlung und anschließend über Nacht bei Raumtemperatur gerührt. Nach Zugabe von 250 mL Wasser unter Eiskühlung wurde 40 min bei Raumtemperatur gerührt, unter reduziertem Druck Ethanol weitgehend entfernt und der Rückstand mehrmals mit Essigsäureethylester extrahiert. Die vereinigten organischen Phasen wurden mit einer gesättigten wäßrigen Natriumchlorid-Lösung gewaschen, mit Natriumsulfat getrocknet, filtriert und das Filtrat unter reduziertem Druck eingeengt. Es wurden 5,96 g [5-(2-Bromethoxy)-3-fluorpyridin-2-yl]methanol (Reinheit 88 %) erhalten.

$^1$H-NMR(400.0 MHz, $d_6$-DMSO): $\delta$ = 8.1532(11.0);8.1489(10.9);8.1037(0.9);8.0999(0.9);7.4823(6.6); 7.4763(6.8);7.4532(6.7);7.4473(6.8);7.3980(0.6);7.3921(0.6);7.3686(0.6);7.3625(0.6);6.5251(0.4);5.2083 (5.5);5.1936(11.9);5.1789(6.3);5.1624(1.1);5.1480(0.5);4.5215(12.7);4.5161(14.0);4.5069(13.8);4.5016 (13.5);4.4908(1.7);4.4486(11.6);4.4354(15.8);4.4218(12.8);4.3831(0.6);4.3710(0.8);4.3578(0.7);4.1452 (0.6);4.1279(1.7);4.1104(1.7);4.0930(0.6);3.9835(0.7);3.9703(0.8);3.9581(0.6);3.8396(13.2);3.8260(16.0); 3.8128(12.1);3.3216(53.4);2.6750(0.7);2.6709(0.9);2.5061(112.3);2.5017(151.1);2.4974(118.2);2.3326 (0.6);2.3286(0.8);1.3559(1.8);1.3385(3.6);1.3210(1.7);0.1459(0.6);0.0079(5.5);-0.0002(130.8); -0.1497(0.6).

Stufe 3: Herstellung von 5-(2-Bromethoxy)-2-[(dibenzo[b,d]furan-2-yloxy)methyl]-3-fluorpyridin

516 mg (2,06 mmol) [5-(2-Bromethoxy)-3-fluorpyridin-2-yl]methanol wurden mit 11,1 mL (152 mmol) Thionylchlorid und einer katalytischen Menge N,N-Dimethylformamid versetzt. Das Reaktionsgemisch wurde für 80 min bei Raumtemperatur gerührt und anschließend unter reduziertem Druck eingeengt. Der Rückstand wurde in 14 mL N,N-Dimethylformamid gelöst und mit 388 mg (2,10 mmol) Dibenzo[b,d]-furan-2-ol sowie 1,43 g (10,3 mmol) Kaliumcarbonat versetzt. Das Reaktionsgemisch wurde für 2 h 40 min bei 70 °C gerührt und anschließend unter reduziertem Druck eingeengt. Der Rückstand wurde in Dichlormethan aufgenommen, an Kieselgel adsorbiert und mittels MPLC an Kieselgel chromatographisch aufgetrennt (Gradient: Cyclohexan/Essigsäureethylester). Es wurden 120 mg 5-(2-Bromethoxy)-2-[(dibenzo[b,d]furan-2-yloxy)methyl]-3 -fluorpyridin erhalten.

$^1$H-NMR(400.0 MHz, $d_6$-DMSO): $\delta$ = 8.2654(7.9);8.2602(7.8);8.1324(5.7);8.1134(6.2);7.8764(9.5); 7.8698(9.6);7.6849(6.7);7.6643(8.7);7.6304(10.0);7.6131(5.3);7.6079(15.2);7.5841(4.8);7.5781(4.6); 7.5414(3.2);7.5384(3.2);7.5205(6.1);7.5025(3.4);7.4996(3.2);7.4159(4.5);7.3969(7.2);7.3785(3.2);7.1949 (5.8);7.1883(5.6);7.1726(5.2);7.1659(5.1);5.2553(15.8);5.2507(16.0);4.4973(7.8);4.4843(10.2);4.4706 (8.4);4.4321(0.5);4.4199(0.6);4.4066(0.5);4.0096(0.6);3.9968(0.7);3.9842(0.5);3.8650(8.7);3.8513(10.3); 3.8383(7.9);3.5441(0.3);3.5294(0.4);3.3607(1876.4);2.6834(1.1);2.6791(1.5);2.6747(1.1);2.5321(3.9); 2.5187(89.6);2.5143(185.4);2.5099(255.8);2.5055(190.1);2.5013(91.8);2.3411(1.0);2.3368(1.4);2.3327 (1.0);1.4029(0.4);1.2389(0.3).

Stufe 4: {[2-({6-[(Dibenzo[b,d]furan-2-yloxy)methyl]-5-fluorpyridin-3-yl}oxy)ethyl](methyl)amino}-acetonitril

Eine Lösung von 117 mg (0,28 mmol) 5-(2-Bromethoxy)-2-[(dibenzo[b,d]furan-2-yloxy)methyl]-3-fluorpyridin in 2 mL Dimethylformamid wurden mit 99 mg (1,41 mmol) (Methylamino)acetonitril versetzt und 4 h bei 80 °C gerührt. Das Reaktionsgemisch wurde anschließend an Kieselgel adsorbiert und mittels MPLC an Kieselgel chromatographisch aufgetrennt (Gradient: Cyclohexan/Essigsäureethylester). Es wurden 95 mg {[2-({6-[(Dibenzo[b,d]furan-2-yloxy)methyl]-5-fluorpyridin-3-yl}oxy)ethyl](methyl)-amino}acetonitril erhalten.

$^1$H-NMR(400.0 MHz, $d_6$-DMSO): $\delta$ = 8.2459(2.6);8.2408(2.7);8.0776(2.6);8.0549(2.9);7.8046(1.9); 7.7832(2.9);7.7018(1.8);7.6818(2.6);7.6614(5.2);7.6389(3.4);7.5845(1.5);7.5786(1.5);7.5550(1.5);7.5491 (1.5);7.3692(2.5);7.3496(2.2);5.3903(5.1);5.3868(5.2);4.2442(2.3);4.2307(4.4);4.2171(2.3);3.8084(10.6); 3.3201(14.6);2.8214(2.3);2.8079(4.2);2.7943(2.2);2.6761(0.4);2.6717(0.6);2.6678(0.4);2.5247(1.3); 2.5069(68.8);2.5026(94.4);2.4983(71.4);2.3426(16.0);2.3298(0.8);0.0078(1.2);-0.0002(33.8);-0.0077(1.4).

Herstellung von 2-[(6-{[(5,6-Dichlor-1-naphthyl)oxy]methyl}-5-fluorpyridin-3-yl)oxy]-N,N-diethylethan-amin

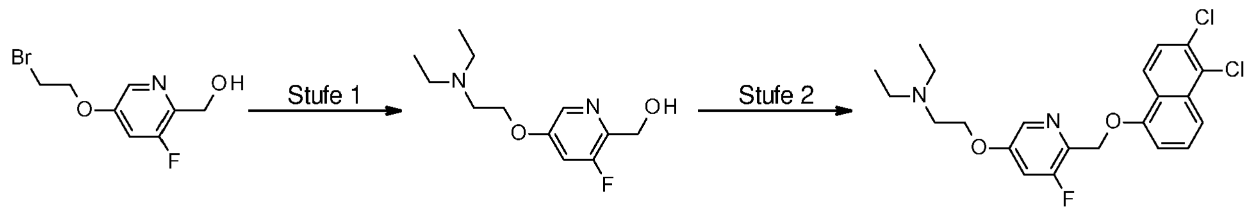


Stufe 1: Herstellung von {5-[2-(Diethylamino)ethoxy]-3-fluorpyridin-2-yl}methanol

Eine Lösung von 740 mg (2,96 mmol) [5-(2-Bromethoxy)-3-fluorpyridin-2-yl]methanol in 2 mL Dimethylformamid wurden mit 918 µL (8.88 mmol) Diethylamin versetzt und 5 h bei 75 °C gerührt. Das Reaktionsgemisch wurde mittels MPLC an RP-Kieselgel chromatographisch aufgetrennt (Gradient: Wasser/Acetonitril). Es wurden 613 mg {5-[2-(Diethylamino)ethoxy]-3-fluorpyridin-2-yl}methanol erhalten.

$^1$H-NMR(400.0 MHz, $d_6$-DMSO): δ = 8.1056(2.0);8.1019(2.0);7.4256(1.2);7.4196(1.2);7.3961(1.2); 7.3901(1.3);5.1802(0.5);5.1657(0.9);5.1512(0.5);4.5038(2.1);4.4959(2.1);4.1224(2.1);4.1074(4.6);4.0923 (2.2);3.3237(7.8);2.7819(2.2);2.7669(4.4);2.7518(2.0);2.5614(2.3);2.5436(7.3);2.5258(7.9);2.5064(26.0); 2.5019(33.3);2.4974(24.8);1.9039(1.0);0.9769(7.9);0.9592(16.0);0.9414(7.5);0.0079(1.1);-0.0002(28.0); -0.0083(1.3).

Stufe 2: Herstellung von 2-[(6-{[(5,6-Dichlor-1-naphthyl)oxy]methyl}-5-fluorpyridin-3-yl)oxy]-N,N-diethylethanamin

Eine Lösung von 634 mg (2,62 mmol) {5-[2-(Diethylamino)ethoxy]-3-fluorpyridin-2-yl}methanol in 30 mL Dichlormethan wurden mit 1,0 mL (13,7 mmol) Thionylchlorid und einer katalytischen Menge N,N-Dimethylformamid versetzt und 90 min bei Raumtemperatur gerührt. Nach Zugabe von 30 mL Toluol wurde das Reaktionsgemisch unter reduziertem Druck eingeengt. Der Rückstand wurde in 12 mL N,N-Dimethylformamid gelöst und ein Sechstel dieser Lösung zu 79 mg (0,37 mmol) 5,6-Dichlor-1-naphthol und 428 mg (1,31 mmol) Cäsiumcarbonat gegeben. Das Reaktionsgemisch wurde über Nacht bei 80 °C gerührt und anschließend mittels MPLC an RP-Kieselgel chromatographisch aufgetrennt (Gradient: Wasser/Acetonitril). Es wurden 144 mg 2-[(6-{[(5,6-Dichlor-1-naphthyl)oxy]methyl}-5-fluorpyridin-3-yl)oxy]-N,N-diethylethanamin erhalten.

$^1$H-NMR(400.0 MHz, $d_6$-DMSO): δ = 8.2239(2.2);8.2196(2.2);8.0771(2.1);8.0544(2.3);7.8029(1.6); 7.7815(2.3);7.7006(1.5);7.6807(2.1);7.6591(4.2);7.6363(2.8);7.5745(1.2);7.5687(1.3);7.5449(1.2);7.5391 (1.2);7.3669(2.0);7.3476(1.8);5.3842(4.2);5.3808(4.4);4.1683(1.9);4.1533(4.1);4.1383(2.0);3.3230(39.8); 2.8013(1.8);2.7865(3.8);2.7714(1.8);2.6720(0.4);2.6675(0.3);2.5675(2.1);2.5497(6.7);2.5319(7.2);2.5071 (57.4);2.5027(75.6);2.4983(56.7);2.3294(0.4);2.3249(0.3);0.9792(7.8);0.9615(16.0);0.9437(7.5);0.0078 (2.3);-0.0002(54.0);-0.0081(2.7).

Herstellung von N-{2-[(6-([(5,6-Dichlor-1-naphthyl)oxy]methyl}-5-fluorpyndin-3-yl)oxy]ethyl}-N,2-dimethylpropan-2-aminiumchlorid und N-{2-[(6-{[(5,6-Dichlor-1-naphthyl)oxy]methyl}-5-fluorpyridin-3- yl)oxy]ethyl}-N,2-dimethylpropan-2-amin

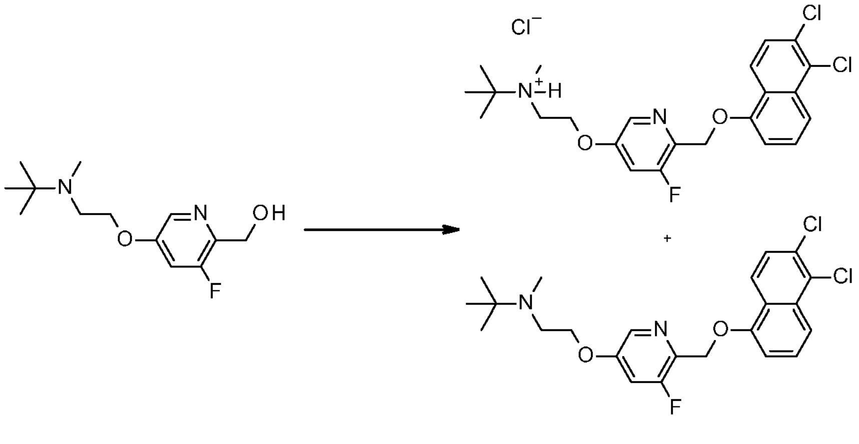

Eine Lösung von 596 mg (2,33 mmol) (5-{2-[tert-Butyl(methyl)amino]ethoxy}-3-fluorpyridin-2-yl)-methanol in

20 mL Dichlormethan wurden mit 1,0 mL (13,7 mmol) Thionylchlorid und einer katalytischen Menge N,N-Dimethylformamid versetzt und 90 min bei Raumtemperatur gerührt. Nach Zugabe von 30 mL Toluol wurde das Reaktionsgemisch unter reduziertem Druck eingeengt. Der Rückstand wurde in 12 mL N,N-Dimethylformamid gelöst und ein Sechstel dieser Lösung zu 70 mg (0,33 mmol) 5,6-Dichlor-1-naphthol und 378 mg (1,16 mmol) Cäsiumcarbonat gegeben. Das Reaktionsgemisch wurde über Nacht bei 80 °C gerührt und anschließend mittels MPLC an RP-Kieselgel chromatographisch aufgetrennt (Gradient: Wasser/Acetonitril). Der bei der Flüssiginjektion des Reaktionsgemisches in die MPLC nach Zusatz von Ethanol und Wasser in der Injektionsspritze ausgefallene Feststoff wurde durch Zugabe von Acetonitril und 1 N wässriger Salzsäure wieder gelöst. Es wurden 99 mg N-{2-[(6-{[(5,6-Dichlor-1-naphthyl)oxy]methyl}-5-fluorpyridin-3-yl)oxy]ethyl}-N,2-dimethylpropan-2-aminiumchlorid (Reinheit 78 %) und 29 mg N-{2-[(6-{[(5,6-Dichlor-1-naphthyl)oxy]methyl}-5-fluorpyridin-3-yl)oxy]ethyl}-N,2-dimethylpropan-2-amin erhalten.

N-{2-[(6-{[(5,6-Dichlor-1-naphthyl)oxy]methyl}-5-fluorpyridin-3-yl)oxy]ethyl}-N,2-dimethylpropan-2-aminiumchlorid (Reinheit 78 %):
$^1$H-NMR(400.0 MHz, $d_6$-DMSO): $\delta$ = 9.6357(0.4);8.3034(1.2);8.2990(1.2);8.0670(1.2);8.0443(1.3); 7.8101(1.0);7.7885(1.4);7.7061(0.8);7.6863(1.3);7.6764(1.9);7.6653(0.8);7.6537(2.2);7.6362(0.5);7.6235 (1.3);7.3742(1.2);7.3549(1.0);5.7570(0.8);5.4187(2.5);5.2320(0.6);4.5213(1.4);4.5096(1.0);3.8081(0.4); 3.7953(0.4);3.7817(0.4);3.7708(0.4);3.3260(52.0);2.8122(2.7);2.8002(2.9);2.6758(0.3);2.6716(0.4); 2.5068(60.7);2.5024(79.5);2.4980(60.3);2.3292(0.4);2.3249(0.4);1.3720(16.0);0.1460(0.3);0.0077(3.4); -0.0002(73.8);-0.0083(4.0);-0.1498(0.3).

N-{2-[(6-{[(5,6-Dichlor-1-naphthyl)oxy]methyl}-5-fluorpyridin-3-yl)oxylethyl}-N,2-dimethylpropan-2-amin:
$^1$H-NMR(400.0 MHz, $d_6$-DMSO): $\delta$ = 8.2188(1.1);8.2144(1.1);8.0759(1.0);8.0532(1.2);7.8031(0.8); 7.7816(1.2);7.7000(0.8);7.6800(1.1);7.6602(2.0);7.6378(1.4);7.5625(0.6);7.5566(0.6);7.5326(0.6);7.5268 (0.6);7.3651(1.0);7.3459(0.9);5.3811(2.2);4.1263(0.8);4.1109(1.6);4.0956(0.8);3.3230(51.0);2.7359(0.7); 2.7206(1.4);2.7050(0.7);2.6712(0.4);2.5064(54.4);2.5021(71.6);2.4977(54.5);2.3288(0.4);2.2272(4.9); 1.0083(16.0);0.0077(2.8);-0.0003(64.9).

Herstellung von 2-[(2-{[(4-Chlor-1-naphthyl)oxy]methyl}pyrimidin-5-yl)oxy]-N-methoxy-N-methylethanamin

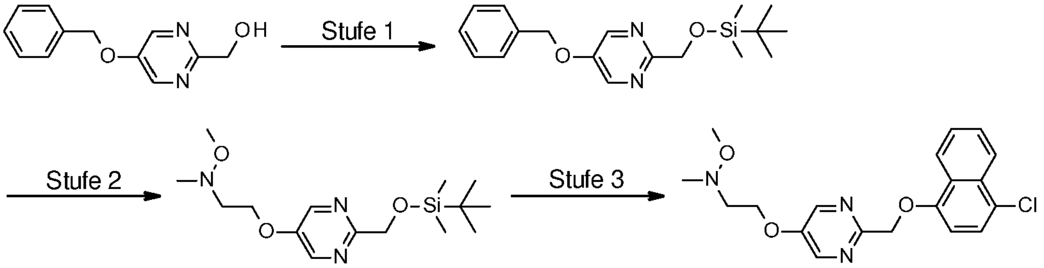


Stufe 1: Herstellung von 5-(Benzyloxy)-2-({[tert-butyl(dimethyl)silyl]oxy}methyl)pyrimidin

Eine Lösung von 744 mg (3,44 mmol) [5-(Benzyloxy)pyrimidin-2-yl]methanol (D. Bensen et al, WO2014/043272) und 341 mg (5,0 mmol) Imidazol in 3,5 mL N,N-Dimethylformamid wurde mit 616 mg (4,10 mmol) tert-Butyldimethylsilylchlorid versetzt und 30 min bei Raumtemperatur gerührt. Nach Zugabe von Essigsäureethylester wurde mit einer gesättigten wäßrigen Natriumchlorid-Lösung gewaschen, mit Magnesiumsulfat getrocknet, filtriert und das Filtrat unter reduziertem Druck eingeengt. Der Rückstand wurde in Dichlormethan aufgenommen, an Kieselgel adsorbiert und mittels MPLC an Kieselgel chromatographisch aufgetrennt (Gradient: Cyclohexan/Essigsäureethylester). Es wurden 0,83 g 5-(Benzyloxy)-2-({ [tert-butyl(dimethyl)silyl]oxy }methyl)pyrimidin erhalten.

$^1$H-NMR(400.0 MHz, CDCl3): $\delta$ = 8.3222(3.5);7.2949(1.8);7.2883(1.2);7.2807(1.3);7.1373(2.4); 5.0288(2.9);4.7408(3.4);1.4579(1.2);0.8133(0.7);0.8061(16.0);0.7990(0.9);0.0076(0.4);-0.0002(11.7); -0.0079(0.5);-0.1235(2.7).

Stufe 2: Herstellung von 2-{[2-({[tert-Butyl(dimethyl)silyl]oxy]methyl)pyrimidin-5-yl]oxy}-N-methoxy-N-methylethanamin

Eine Lösung von 470 mg (1.42 mmol) 5-(Benzyloxy)-2-({[tert-butyl(dimethyl)silyl]oxy}methyl)pyrimidin in 40 mL Methanol wurden am Hydrierreaktor H-Cube® an 10 % Pd/C bei 1 bar mit einem Fluss von 2 mL/min hydriert. Anschließend wurde die Reaktionslösung unter reduziertem Druck eingeengt. Es wurden 328 mg nicht aufgereinigtes 2-({[tert-Butyl(dimethyl)silyl]oxy}methyl)pyrimidin-5-ol als Zwischenprodukt erhalten. Eine Lösung von 150 mg des nicht aufgereinigten

Zwischenproduktes 2-({ [tert-Butyl(dimethyl)silyl]oxy}methyl)pyrimidin-5-ol in 1,5 mL N,N-Dimethylformamid wurden mit 224 mg (1,62 mmol) Kaliumcarbonat und 202 mg (0.81 mmol) 2-Brom-N-methoxy-N-methylethanaminhydrobromid (1:1) versetzt und 15 h bei 50 °C gerührt. Nach Zugabe von weiteren 62 mg (0.25 mmol) 2-Brom-N-methoxy-N-methylethanaminhydrobromid (1:1) wurde die Reaktionslösung nochmals 3 h bei 50 °C gerührt und anschließend unter reduziertem Druck eingeengt. Der Rückstand wurde mit Wasser versetzt und mehrmals mit Essigsäureethylester extrahiert. Die vereinigten organischen Phasen wurden mit einer gesättigten wäßrigen Natriumchlorid-Lösung gewaschen, mit Natriumsulfat getrocknet, filtriert und das Filtrat unter reduziertem Druck eingeengt. Das Rohprodukt wurde mittels MPLC an Kieselgel chromatographisch aufgetrennt (Gradient: Cyclohexan/Essigsäureethylester). Es wurden 43 mg 2-{[2-({ [tert-Butyl(dimethyl)silyl]oxy}methyl)pyrimidin-5-yl]oxy}-N-methoxy-N-methylethanamin erhalten.

$^1$H-NMR(400.0 MHz, $d_6$-DMSO): $\delta$ = 8.5493(3.9);4.7338(3.3);4.2697(0.7);4.2559(1.5);4.2421(0.8);

3.3996(6.2);3.3184(2.0);2.9778(0.7);2.9640(1.3);2.9502(0.7);2.5641(5.3);2.5066(8.9);2.5021(12.5);2.4978(9.6);0.8740(16.0);0.8639(1.7);0.0677(0.5);0.0602(11.7);0.0439(1.0);-0.0002(8.8);-0.0080(0.4).

Stufe 3: Herstellung von 2-[(2-{[(4-Chlor-1-naphthyl)oxy]methyl}pyrimidin-5-yl)oxy]-N-methoxy-N-methylethanamin

Eine Lösung von 40 mg (0,12 mmol) 2-{[2-({[tert-Butyl(dimethyl)silyl]oxy}methyl)pyrimidin-5-yl]oxy}-N-methoxy-N-methylethanamin in 0,7 mL Tetrahydrofuran wurden mit 250 µL (0,25 mmol) einer 1 M Lösung von Tetrabutylammoniumfluorid in Tetrahydrofuran versetzt und 30 min bei Raumtemperatur gerührt. Nach Zugabe von gesättigter wässriger Ammoniumchlorid-Lösung wurde mehrmals mit Essigsäureethylester extrahiert, die vereinigten organischen Phasen mit Magnesiumsulfat getrocknet, filtriert und das Filtrat unter reduziertem Druck eingeengt. Der Rückstand wurde in 1 mL Toluol gelöst und mit 25 µL (0,34 mmol) Thionylchlorid und einer katalytischen Menge N,N-Dimethylformamid versetzt. Das Reaktionsgemisch wurde für 2 h bei Raumtemperatur gerührt und anschließend unter reduziertem Druck eingeengt. Der Rückstand wurde in 1 mL N,N-Dimethylformamid gelöst und mit 27 mg (0,15 mmol) 4-Chlor-1-naphthol sowie 47 mg (0,34 mmol) Kaliumcarbonat versetzt. Das Reaktionsgemisch wurde für 2 h bei 70 °C gerührt und anschließend unter reduziertem Druck eingeengt. Der Rückstand wurde in Dichlormethan aufgenommen, an Kieselgel adsorbiert und mittels MPLC an Kieselgel chromatographisch aufgetrennt (Gradient: Cyclohexan/Essigsäureethylester). Es wurden 28 mg 2-[(2-{[(4-Chlor-1-naphthyl)oxy]-methyl}pyrimidin-5-yl)oxy]-N-methoxy-N-methylethanamin erhalten.

$^1$H-NMR(400.0 MHz, $d_6$-DMSO): $\delta$ = 8.6301(12.6);8.3049(1.5);8.2842(1.6);8.1276(1.6);8.1072(1.8); 7.7419(0.8);7.7388(0.8);7.7247(1.2);7.7214(1.6);7.7178(0.8);7.7037(1.1);7.7005(1.0);7.6548(1.1);7.6520 (1.1);7.6342(1.6);7.6311(1.1);7.6166(0.8);7.6138(0.7);7.5759(2.8);7.5551(3.0);7.0297(2.6);7.0087(2.4); 5.4358(8.1);4.2925(2.0);4.2788(4.2);4.2650(2.1);3.3999(20.0);3.3213(5.2);2.9859(1.8);2.9723(3.7); 2.9586(1.8);2.5655(16.0);2.5251(0.7);2.5202(1.1);2.5115(15.9);2.5071(33.7);2.5025(47.0);2.4980(34.8); 2.4937(16.6);0.0080(0.3);-0.0002(10.8);-0.0085(0.4).

Herstellung von 2-Brom-N-methoxy-N-methylethanaminhydrobromid (1:1)

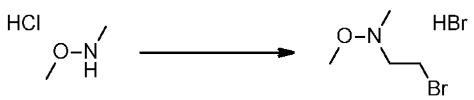

Eine Suspension von 100 g (1,02 mol) N-Methoxymethanaminhydrochlorid (1:1) und 435 g (3,15 mol) Kaliumcarbonat in 1 L Acetonitril wurde bei 0 °C langsam mit 463 g (2,46 mol) 1,2-Dibromethan versetzt. Anschließend wurde 20 min bei 0 °C, 70 h bei Raumtemperatur und 24 h bei 50 °C gerührt. Es wurde vom Bodensatz abfiltriert und das Filtrat unter reduziertem Druck eingeengt. Der Rückstand wurde mit einer Lösung von Chlorwasserstoff in Diethylether behandelt. Der ausgefallene Festtoff wurde abfiltriert und mit Diethylether gewaschen. Das so erhaltene Hydrochlorid wurde bei 0 °C in einer Lösung von Bromwasserstoff in Essigsäure gelöst und für 10 min gerührt. Anschließend wurde die Essigsäure unter reduziertem Druck entfernt. Es wurde der Rückstand in Dichlormethan gelöst, filtriert und das Filtrat eingeengt. So wurden 22 g 2-Brom-N-methoxy-N-methylethanaminhydrobromid (1:1) erhalten.

2-Brom-N-methoxy-N-methylethanaminhydrochlorid (1:1):
$^1$H-NMR (300.0 MHz, $d_6$-DMSO): $\delta$ = 3,61 (t, $J$ = 6,9 Hz, 2H), 3,55 (s, 3H), 3,14 (t, $J$ = 6,9 Hz, 2H), 2,67 (s, 3H) ppm.

**NMR-Daten ausgewählter Beispiele**

NMR -Peak-Listenverfahren

Die 1H-NMR-Daten ausgewählter Beispiele und Syntheseintermediate werden in Form von 1H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:

$$\delta_1(\text{Intensität}_1);\delta_2(\text{Intensität}_2);\ldots\ldots..;\delta_i(\text{Intensität}_i);\ldots\ldots;\delta_n(\text{Intensität}_n)$$

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von 1H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-$D_6$ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu 1H-NMR-Peaklisten können der Research Disclosure Database Number 564025 entnommen werden.

| **Beispiel** |
|---|
| 1: $^1$H-NMR(400.0 MHz, $d_6$-DMSO):<br>δ= 8.3682(5.1);8.3625(5.2);8.3181 (0.4);8.1644(5.2);8.1416(5.8);7.9202(3.4);7.9141(3.3);7.8989(3.5);7.8928 (3.5);7.8027(3.8);7 .7813(5.8);7.7092(4.0);7.6894(5.2);7.6673(10.2);7.6443(7.3);7.3154(4.8);7.2963(4.3);6.9035 (5.8);6.8823(5.6);6.0677(1.0);6.05 13(2.3);6.0349(1.0);5.2800(16.0);4.8119(2.1);4.7868(2.3);4.6582(2.5);4.4271 (5.5);4.4126(11.7);4.3981(5.8);4.3040(4.5);4.287 7(4.4);3.4089(48.4);3.3286(91.4);3.2818(4.0);2.9628(4.7); 2.9485(9.8);2.9340(4.8);2.6768(0.6);2.6723(0.9);2.6679(0.6);2.5578( 41.0);2.5256(2.1);2.5207(3.2);2.5120 (48.1);2.5077(98.0);2.5031(130.4);2.4986(95.8);2.4943(47.2);2.3344(0.6);2.3300(0.9);2.3 254(0.6);1.9897(0.4); 0.1460(0.8);0.0080(5.7);-0.0002(164.9);-0.0085(6.0);-0.1496(0.8) |
| 2: $^1$H-NMR(400.0 MHz, $d_6$-DMSO): |

(fortgesetzt)

| Beispiel |
|---|
| δ= 8.3630(5.1);8.3579(5.5);8.2317(3.9);8.21 08(4.1);8.1311 (4.0);8.11 02(4.3);7 .9169(3.2);7 .9109(3.2);7 .8956 (3.3);7 .8896(3.3);7 .7361(1.8);7.7335(2.0);7.7160(3.8);7.6980(2.3);7.6953(2.4);7.6387(6.2);7.6180(8.4);7.5966 (1.7);7.5943(1.8);7.1509(5.6);7.130 0(5.2);6.9034(5.4);6.8821(5.2);5.2613(16.0);4.8123(1.3);4.7872(1.6);4.6588 (1.7);4.4268(5.1);4.4124(10.6);4.3979(5.4);3.4092( 42.0);3.3340(86.0);3.2821(2.8);2.9629(4.6);2.9486(9.2); 2.9341(4.6);2.6773(0.4);2.6731(0.6);2.6687(0.4);2.5578(36.3);2.5082( 62.5);2.5039(81.9);2.4997(64.1);2.3307 (0.5);1.3969(1.8);1.2317(2.8);0.8527(0.4);0.0077(0.8);-0.0002(16.2) |
| 3: $^1$H-NMR(400.0 MHz, $d_6$-DMSO):<br>δ=8.3185(0.4):8.2012(3.8):8.1806(4.1):8.1441(2.6):8.1346(4.3):8.1235(3.5):8.1186(3.5):8.1138(5.0):8.0986 (2.6):7.7379(2.0):7 .7351(2.0);7.7207(3.0);7.7176(3.8);7.7141(2.1);7.6997(2.6);7.6967(2.4);7.6441(6.7);7.6338 (3.0);7.6312(3.0);7.6234(7.6);7.616 5(2.7);7.6133(4.0);7.6105(2.9);7.5956(1.9);7.5930(1.8);7.1689(6.1);7.1480 (5.6) ;6.8636(4.3);6.8431(4.2);5.2692(16.0);5.2479(0 .4);4.3869(4.7);4.3729(9.7);4.3588(5.1);3.8703(3.1); 3.4562(0.4);3.4080(45.7);3.3516(5.1);3.3445(5.1);3.2886(0.4);3.2287(0.3); 2.9594(4.3);2.9454(8.5);2.9314(4.4); 2.6770(0.7);2.6724(0.9);2.6680(0.7);2.5584(37.8);2.5257(2.1);2.5120(53.0);2.5079(106.6) ;2.5034(139.2); 2.4989(102.1);2.4949(52.0);2.3874(0.4);2.3346(0.7);2.3302(1.0);2.3256(0.7);1.2984(0.5);1.2584(0.8);1.2318(2. 7);0.1460(0.3);0.0079(2.7);-0.0002(78.0);-0.0084(3.4);-0.1496(0.4) |
| 4: $^1$H-NMR(400.0 MHz, $d_6$-DMSO):<br>δ=8.7828(0.3);8.3787(3.9);8.3135(0.4);8.2345(3.1);8.2135(3.3);8.1323(3.1);8.1216(0.5);8.1113(3.4);7.9396 (1.3);7.9336(1.4);7 .9289(1.6);7.9226(1.6);7.9186(1.5);7.9122(1.5);7.9076(1.6);7.9014(1.5);7.7353(1.6);7.7178 (2.5);7.6972(1.9);7.6385(5.2);7.618 2(6.4);7.5978(1.4);7.1552(2.5);7.1493(2.9);7.1343(2.3);7.1283(2.6);6.9164 (2.2);6.8994(2.8);6.8955(2.4);6.8783(2.5);5.7538(15 .6);5.6080(0.5);5.6025(0.9);5.5969(1.0);5.5910(0.9);5.5853 (0.5);5.5249(0.8);5.5199(1.0);5.5140(1.3);5.5090(1.0);5.5030(0.7);5.3798(1.2);5.2723(7.7);5.2687(8.4);3.8665 (0.8);3.8546(0.9);3.8370(1.1);3.8252(1.0);3.6564(0.3);3.6488(0.4);3.6312(1.0);3.624 4(1.0);3.6088(1.0);3.6019 (1.3);3.5904(1.6);3.5809(1.1);3.5719(2.5);3.5593(2.9);3.5391(2.5);3.5243(0.6);3.5158(0.6);3.5062(0. 6);3.3835 (0.5);3.3648(0.7);3.3596(0.8);3.3213(136.8);3.3179(114.0);2.6753(0.8);2.6709(1.1);2.6664(0.8);2.5238(3.4); 2.5106(7 0.5);2.5063(140.5);2.5018(185.4);2.4973(135.6);2.4929(67.1);2.3329(0.9);2.3285(1.2);2.3244(0.8); 2.3193(0.4);2.2901(0.3);2.2 795(0.7);2.2674(0.6);2.2554(0.8);2.2440(0.6);2.2328(0.4);2.2210(0.4);2.1919(0.7); 2.1808(0.8);2.1707(1.2);2.1585(0.8);2.1467( 1.0);2.1348(0.9);2.1242(0.4);2.1123(0.3);2.0909(0.6);2.0834(0.5); 2.0737(0.6);2.0675(0.4);1.9764(16.0);1.9303(13.8);1.2932(0. 4); 1.2338(0.5);0.0079(0.8);-0.0001(21.3);-0.0083 (0.8) |
| 5: $^1$H-NMR(400.0 MHz, $d_6$-DMSO):<br>δ= 8.3569(3.4);8.3518(3.4);8.3142(4.9);8.2335(2.5);8.2124(2.7);8.1313(2.6);8.1106(2.9);7.9180(2.2);7.9119 (2.1);7.8967(2.2);7 .8906(2.2);7.7358(1.2);7.7328(1.4);7.7187(1.8);7.7154(2.5);7.7119(1.4);7.6976(1.6);7.6945 (1.6);7.6371(5.9);7.6164(7.2);7.598 5(1.2);7.5958(1.2);7.1484(3.9);7.1275(3.6);6.8863(3.6);6.8650(3.5);5.7544 (3.1);5.4337(0.4);5.4264(0.9);5.4188(1.0);5.4112(1. 2);5.4074(1.3);5.3999(1.1);5.3921(0.9);5.3850(0.5);5.2594 (10.2);3.8567(16.0);3.3186(33.8);3.2947(1.9);3.0038(1.5);2.9886(1. 6);2.9775(2.0);2.9623(1.8);2.8469(0.6); 2.8263(1.3);2.8108(1.4);2.7907(0.8);2.7530(1.7);2.7463(1.7);2.7267(1.4);2.7201(1.4);2. 6757(0.3);2.6712(0.5); 2.6667(0.4);2.5830(0.8);2.5628(1.6);2.5464(1.6);2.5420(1.2);2.5249(1.9);2.5111(25.9);2.5067(53.8);2.5 022 (74.6);2.4977(57.1);2.4934(28.5);2.3698(0.4);2.3506(0.8);2.3356(1.5);2.3164(1.4);2.3016(0.9);2.2820(0.4); 2.0742(1.4);1.9 166(0.4);1.9096(0.5);1.8961(0.7);1.8821(0.8);1.8750(0.8);1.8605(0.6);1.8472(0.4);1.8408(0.4);- 0.0002(6.6) |
| 6: $^1$H-NMR(400.0 MHz, $d_6$-DMSO):<br>δ= 8.5420(2.1);8.2339(1.6);8.2138(1.8);8.1539(2.1);8.1369(1.8);8.1161(1.9);7.7406(0.7);7.7378(0.8);7.7235 (1.1);7.7204(1.6);7 .7026(1.0);7.6996(1.1);7.6473(3.4);7.6266(4.3);7.6079(0.7);7.6053(0.8);7.2144(1.0);7.1569 (2.5);7.1359(2.3);7.0776(2.2);6.940 7(1.1);5.3367(6.6);4.5456(2.2);4.5311(4.8);4.5166(2.4);3.4051(19.0);3.3241 (19.0);3.0037(2.1);2.9893(4.3);2.9747(2.0);2.5644( 16.0);2.5263(0.5);2.5128(11.1);2.5085(23.9);2.5040(34.0); 2.4997(26.8);1.3974(0.3);1.2314(0.6);0.0081(1.4);-0.0002(42.7) |
| 7: $^1$H-NMR(601.6 MHz, $d_6$-DMSO):<br>δ= 8.3415(2.4);8.3381(2.5);8.2473(1.5);8.2337(1.6);8.1303(1.6);8.1168(4.4);8.1136(3.1);7.7321(0.8);7.7301 (0.8);7.7206(1.1);7 .7184(1.5);7.7161(0.9);7.7066(1.0);7.7046(0.9);7.6356(3.7);7.6258(1.1);7.6218(4.0);7.6121 (0.8);7.6103(0.8);7.1365(2.4);7.122 6(2.3);5.2736(6.5);4.5246(2.2);4.5150(4.7);4.5054(2.3);3.4189(19.7);3.3156 (8.6);3.0036(1.8);2.9940(3.7);2.9844(1.8);2.5725(1 6.0);2.5113(4.3);2.5083(9.0);2.5053(12.3);2.5023(9.1); 2.4993(4.4);-0.0002(3.4) |

(fortgesetzt)

| Beispiel |
| --- |
| 8: $^1$H-NMR(400.0 MHz, $d_6$-DMSO): δ=8.3577(0.6);8.3447(0.6);8.3159(5.5);8.3106(5.1);8.1411(4.1);8.1219(4.4);8.0992(0.3);8.0927(0.4);7.9100 (0.4);7.9043(0.4);7 .8884(0.4);7.8833(0.5);7.8676(3.1);7.8616(3.3);7.8529(6.5);7.8465(9.1);7.8407(3.6);7.6752 (4.6);7.6546(5.9);7.6263(6.0);7.604 0(6.6);7.5300(2.2);7.5103(4.1);7.4920(2.3);7.4077(3.0);7.3891(4.9);7.3710 (2.2);7.2226(0.4);7.2155(0.4);7.2018(0.4);7.1843(3. 6);7.1778(3.4);7.1620(3.2);7.1555(3.1);6.9541(0.7);6.9328 (0.6);6.8792(5.3);6.8579(5.1);5.4036(2.0);5.1675(2.1);5.1493(16.0); 4.4156(4.8);4.4012(10.1);4.3867(5.1); 3.4009(39.3);3.3207(78.2);2.9537(4.6);2.9394(9.1);2.9249(4.5);2.6707(1.6);2.5506(34.4) ;2.5056(220.0);2.5017 (280.2);2.4975(205.6);2.3284(1.6);1.2338(0.5);1.0542(1.4);-0.0003(52.8) |
| 9: $^1$H-NMR(400.0 MHz, $d_6$-DMSO): δ= 8.3553(3.4);8.3498(3.5);8.1588(3.2);8.1362(3.6);7.9089(2.0);7.9029(2.0);7.8876(2.1);7.8816(2.1);7.8008 (2.4);7.7793(3.6);7 .7073(2.4);7.6875(3.2);7.6666(6.6);7.6440(4.4);7.4947(0.4);7.3123(3.0);7.2930(2.7);6.8694 (3.5);6.8482(3.3);5.7590(0.6);5.276 3(9.7);5.2439(0.6);4.3405(2.3);4.3248(4.7);4.3091(2.4);4.0567(0.4);4.0388 (1.0);4.0210(1.1);4.0032(0.4);3.3237(22.6);3.2048(0 .5);3.1873(0.5);2.8913(0.7);2.7764(1.5);2.7623(2.4);2.7324 (1.0);2.6764(0.4);2.6721(0.5);2.6676(0.4);2.5632(1.8);2.5463(4.1);2 .5262(5.1);2.5075(64.9);2.5031(83.5); 2.4986(62.1);2.3338(0.4);2.3299(0.5);2.3256(0.4);1.9900(4.5);1.2983(0.4);1.2578(0.5);1.2306(0.6);1.1932(1.2); 1.1754(2.2);1.1576(1.1);1.0387(0.5);1.0202(0.4);1.0027(0.4);0.9824(8.2);0.9646(16.0);0.9469(8.1);0.92 39(0.7); 0.0076(0.4);-0.0002(9.3) |
| 10: $^1$H-NMR(400.0 MHz, $d_6$-DMSO): δ= 8.3568(0.6);8.3167(0.4);8.2989(0.7);8.2934(0.7);8.2648(5.3);8.2595(5.4);8.0953(0.4);8.0890(0.4);7.8613 (0.4);7.8558(0.4);7 .8404(0.5);7.8345(0.5);7.8183(3.2);7.8125(3.1);7.7971(3.3);7.7911(3.2);7.6523(0.8);7.6326 (5.8);7.6261(4.6);7.6180(10.7);7.54 25(5.1);7.4331(2.6);7.4230(2.2);7.3075(2.7);7.2845(4.2);7.2589(3.3);7.2174 (3.1);7.2098(3.3);7.2014(3.4);7.1938(3.3);7.1063(1 .9);7.0975(3.0);7.0887(2.1);7.0841(1.8);7.0753(2.4);7.0666 (1.3);6.9446(0.7);6.9234(0.7);6.8681(5.5);6.8469(5.3);5.4001(2.2);5 .1213(2.2);5.1038(16.0);4.4109(5.0);4.3964 (10.6);4.3820(5.3);3.3978(43.2);3.3216(55.9);2.9501(4.8);2.9356(9.6);2.9212(4.7); 2.6759(0.8);2.6715(1.0); 2.6674(0.8);2.5487(37.1);2.5067(137.5);2.5025(176.7);2.4982(128.8);2.3290(1.0);1.2341(0.7);1.0540( 3.3); 0.0078(1.5);-0.0002(37.0) |
| 11: $^1$H-NMR(400.0 MHz, $d_6$-DMSO): δ= 8.2745(2.1);8.2689(2.2);7.8237(1.3);7.8177(1.3);7 .8024(1.4);7.7964(1.4);7.7459(1.2);7.7256(1.5);7.6538 (1.9);7.6157(1.4);7 .5956(2.5);7.5757(1.3);7.4299(1.0);7.4102(2.3);7.3900(1.9);7.3828(1.2);7.3620(1.0);7.3458 (2.2);7.3408(1.6);7.3068(1.6);7.287 4(1.2);7.0779(1.1);7.0728(1.1);7.0581(1.0);7.0525(1.0);6.8375(2.3);6.8163 (2.2);5.1410(6.4);4.3241(2.2);4.3083(4.6);4.2924(2. 2);3.3235(20.0);2.7629(2.0);2.7471(4.2);2.7313(2.1);2.5532 (2.2);2.5354(7.0);2.5174(9.1);2.5114(16.7);2.5072(32.3);2.5027(43 .3);2.4985(33.2);1.9924(0.3);0.9729(7.9); 0.9552(16.0);0.9374(7.7);0.0079(1.6);-0.0002(39.1) |
| 12: $^1$H-NMR(400.0 MHz, $d_6$-DMSO): δ= 8.2510(2.3);8.2456(2.5);7.8046(1.4);7.7988(1.4);7.7834(1.4);7.7774(1.5);7.6196(2.4);7.5468(0.5);7.5310 (2.8);7.5131(3.0);7 .4961(2.2);7.4928(2.7);7.4865(1.1);7.4794(0.5);7.4747(0.6);7.2915(1.2);7.2686(1.7);7.2431 (1.4);7.2003(1.2);7.1926(1.4);7.184 4(1.3);7.1766(1.3);7.0865(0.8);7.0777(1.3);7.0691(0.8);7.0643(0.7);7.0556 (1.0);7.0466(0.6);6.8330(2.5);6.8118(2.4);5 .0942(7. 1);4.3213(2.3);4.3054(4.7);4.2896(2.3);3.3235(21.3); 2.7603(2.2);2.7445(4.5);2.7287(2.1);2.5514(2.4);2.5336(7.5);2.5069(31.4) ;2.5026(41.4);2.4985(33.7);0.9714 (8.0);0.9536(16.0);0.9359(7.6);0.0078(1.6);-0.0002(35.0) |
| 13: $^1$H-NMR(400.0 MHz, $d_6$-DMSO): δ= 8.2824(5.7);8.2777(6.0);7.8343(3.1);7.8286(3.2);7.8131(3.2);7.8073(3.3);7.6765(6.7);7.6579(8.4);7.6192 (1.0);7.6029(0.9);7 .5972(0.8);7.4801(3.9);7.4612(8.1);7.4420(5.0);7.4285(0.9);7.4050(2.5);7.3858(7.8);7.3674 (5.9);7.3502(1.6);7.3064(0.4);7.282 5(5.7);7.2571(4.3);7.2380(3.2);7.1174(0.7);7.0960(0.6);7.0335(3.0);7.0280 (2.9);7.0131(2.7);7.0076(2.6);6.8712(5.7);6.8500(5. 5);5.1300(16.0);5.0934(1.3);5.0603(0.5);4.4136(5.1);4.3992 (10.4);4.3848(5.5);4.3687(0.5);3.4006(35.7);3.3209(33.5);2.9524(4 .8);2.9380(9.4);2.9237(4.8);2.6708(0.6); 2.5501(32.7);2.5017(91.7);2.3285(0.5);-0.0002(37.4) |
| 14: $^1$H-NMR(400.0 MHz, $d_6$-DMSO): |

(fortgesetzt)

| **Beispiel** |
|---|
| $\delta$= 8.2560(2.2);8.2507(2.2);7.8067(1.3);7 .8007(1.3);7 .7854(1.4);7.7794(1.4);7.6128(2.7);7.5904(3.0);7 .2262(1.7);7 .2230(1.8);7 .1475(1.6);7.1404(1.4);7.1250(1.4);7.1179(1.3);6.8295(2.3);6.8083(2.2);5.0954(7.0);4.3210(2.2);4.3052(4.7);4.2894(2.3);3.322 5(13.3);2.8913(0.8);2.7577(2.2);2.7418(4.4);2.7318(1.1);2.7260(2.1);2.5506(2.3);2.5328(7.3);2.5252(1.3);2.5070(26.5);2.5027( 34.8);2.4982(27.7);2.4731(0.4);0.9710(8.0);0.9532(16.0);0.9354(7.7);0.9238(0.4);0.0077(1.4);-0.0002(33.2);-0.0083(1.5) |
| 15: $^1$H-NMR(400.0 MHz, $d_6$-DMSO):<br>$\delta$= 10.1255(3.7);8.5176(0.7);8.5125(0.7);8.3133(1.0);8.2521(4.4);8.2469(4.6);7.9513(1.1);7.9366(0.6);7.9306(0.5);7.7996(3.0); 7.7936(2.9);7.7783(3.1);7.7723(3.1);7.5811(1.0);7.5637(1.3);7.5593(1.2);7.5449(6.8);7.5366(0.7);7.5226(7.3);7.4010(3.1);7.37 91(3.4);7.3724(1.6);7.3652(1.6);7.3476(6.6);7.3404(6.9);7.0780(0.7);7.0706(0.7);7.0575(4.1);7.0503(4.0);7.0352(3.5);7.0279(3 .4);6.9771(3.0);6.9701(3.3);6.8626(5.2);6.8414(5.0);6.7806(2.0);6.7736(1.8);6.7586(1.8);6.7516(1.7);6.5776(0.7);5.2005(3.0);5 .1944(1.4);5.0763(16.0);4.4088(4.9);4.3944(10.3);4.3799(5.2);3.8554(2.5);3.3989(43.8);3.3814(0.4);3.3145(135.2);2.9689(0.4) ;2.9485(4.5);2.9340(9.2);2.9196(4.5);2.8910(5.4);2.7320(4.4);2.6749(1.6);2.6704(2.3);2.6659(1.6);2.5584(2.0);2.5488(36.8);2.5237(5.2);2.5102(125.4);2.5058(255.3);2.5013(342.6);2.4968(257.0);2.4925(129.2);2.4464(0.7);2.4364(0.5);2.4308(0.5);2.404 1(0.4);2.3785(0.4);2.3326(1.8);2.3281(2.4);2.3236(1.8);2.1187(1,0);1.6615(0.4);1.6359(0.4);1.6221(0.4);1.6088(0.4);1.5962(0. 3);1.5755(0.5);1.5501(0.4);1.5224(0.4);1.4765(0.5);1.4705(0.5);1.3825(0.5);1.3699(1.2);1.3614(0.6);1.3365(1.3);1.2702(2.2);1. 2355(10.6);1.1693(5.5);1.1466(0.9);1.1371(0.9);1.1254(0.9);1.1054(0.7);1.0919(0.9);1.0482(0.8);1.0160(0.9);1.0000(0.8);0.99 07(0.4);0.9797(0.9);0.9656(3.9);0.9380(0.6);0.9301(0.6);0.9216(0.7);0.9086(0.8);0.9011(0.7);0.8539(4.1);0.8362(3.2);0.8102(1 .6);0.7549(0.4);0.1461(1.0);0.0080(7.8);-0.0002(245.8);-0.0085(9.5);-0.1496(1.1) |
| 16: $^1$H-NMR(400.0 MHz, $d_6$-DMSO):<br>$\delta$= 8.3453(2.5);8.3383(2.6);8.2325 (2.3);8.2098(2.6);7.7974(1.8);7.77 59(2.6);7.6988(3.1);7.6835(1.7);7 .67 61(3.0);7 .6634(2.4);7 .6426(1.3);7.6082(1.9);7.5867(2.9);7.5077(1.7);7.5005(1.7);7.4862(1.2);7.4789(1.2);7.2737(2.3);7.2542(2.1);5.3373(7.9);4.732 6(0.3);4.2089(2.2);4.1950(4.6);4.1811(2.5);3.4095(18.5);3.3270(30.8);2.9837(2.1);2.9698(4.2);2.9559(2.2);2.6720(0.4);2.5731( 16.0);2.5070(47.5);2.5029(61.3);2.4987(48.0);2.3296(0.4);-0.0002(41.4) |
| 17: $^1$H-NMR(400.0 MHz, $d_6$-DMSO):<br>$\delta$= 8.2363(2.0);8.2351(2.0);8.2136(2.2);8.2124(2.2);7 .8066(1.6);7 .7851(3.2);7 .7646(1.2);7 .7589(1.0);7 .7384(1.1);7 .7052(3.3);7 .6877(1.6);7.6825(3.2);7.6679(2.1);7.6468(1.3);7.5581(2.0);7.5379(1.7);7.2605(1.9);7.2413(1.8);5.2740(6.6);4.2559(1.9);4.242 1(4.0);4.2282(2.0);3.4043(19.9);3.3348(27,4);2.9998(1.7);2.9861(3.5);2.9722(1.7);2.5742(16.0);2.5273(0.4);2.5139(10.8);2.50 95(22.1);2.5050(29.0);2.5005(21.1);2.4961(10.3);1.3968(0.4);0.0079(0.5);-0.0002(15.7);-0.0084(0.5) |
| 18: $^1$H-NMR(400.0 MHz, $d_6$-DMSO):<br>$\delta$= 8.2408(2.2);8.2181(2.4);7.8110(1.7);7 .7894(2.4);7 .7145(1.8);7 .7070(3.4);7 .6932(4.6);7 .6844(3.3);7 .6725(2.3);7 .6511(4.5);7 .6302(1.7);7.2630(2.1);7.2436(1.9);5.3214(7.4);4.3047(0.4);4.2884(0.4);4.2748(2.0);4.2609(4.3);4.2468(2.2);3.4152(19.1);3.33 19(26.4);3.0247(2.0);3.0108(4.0);2.9968(2.0);2.6728(0.3);2.5893(16.0);2.5082(37.4);2.5039(49.2);2.4995(37.6);1.9901(1.0);1. 1757(0.5);-0.0002(7.8) |
| 19: $^1$H-NMR(400.0 MHz, $d_6$-DMSO):<br>$\delta$= 8.2574(1.9);8.2554(2.2);8.2515(2.2);8.1547(1.6);8.1345(1.8);8.1262(1.8);8.1051(1.8);7.7268(0.8);7.7237(0.9);7.7095(1.2);7 .7062(1.6);7.7026(0.9);7.6885(1.0);7.6854(1.0);7.6320(2.8);7.6114(4.0);7.6018(1.6);7.5989(1.3);7.5952(1.7);7.5921(1.2);7.578 0(2.1);7.5727(1.6);7.2083(2.6);7.1873(2.4);5.3679(4.5);5.3640(4.6);4.2478(2.0);4.2340(4.1);4.2203(2.1);3.4127(19.5);3.3195(8 .5);2.9877(1.8);2.9741(3.7);2.9602(1.9);2.5724(16.0);2.5254(0.6);2.5121(12.1);2.5078(24.4);2.5033(32.1);2.4988(23.8);2.4944 (11.9);2.0411(0.4);0.0080(0.4);-0.0002(12.7);-0.0084(0.5) |
| 20: $^1$H-NMR(400.0 MHz, $d_6$-DMSO): |

(fortgesetzt)

| Beispiel |
|---|
| δ= 8.2437(2.3);8.2381(2.1);8.1232(1.6);8.1042(1.6);7.8680(2.6);7.8614(2.6);7.6765(1.8);7.6559(2.4);7.6214 (2.7);7.5991(3.0);7 .5885(1.3);7.5825(1.3);7.5590(1.3);7.5531(1.3);7.5332(0.9);7.5299(1.0);7.5148(1.3);7.5120 (1.7);7.4942(1.0);7.4910(1.0);7.408 2(1.1);7.4063(1.2);7.3875(1.9);7.3708(0.8);7.3687(0.9);7.1882(1.7);7.1815 (1.6);7.1659(1.5);7.1592(1.5);5.2391(4.2);5.2344(4. 4);4.2428(1.9);4.2291(4.0);4.2153(2.1);3.4087(20.5);3.3180 (40.5);2.9839(1.8);2.9703(3.6);2.9566(1.8);2.6753(0.5);2.6708(0.6) ;2.6664(0.5);2.5693(16.0);2.5499(0.4); 2.5241(1.8);2.5106(36.0);2.5062(74.9);2.5017(104.0);2.4972(79.2);2.4928(39.7);2.3331( 0.4);2.3285(0.6); 2.3239(0.4);1.2343(0.3);0.0079(1.0);-0.0002(28.9);-0.0085(1.2) |
| 21: $^1$H-NMR(400.0 MHz, $d_6$-DMSO):<br>δ= 8.2562(2.0);8.2542(2.2);8.2503(2.2);8.1442(1.6);8.1241(1.7);8.0807(1.7);8.0597(1.9);7.8079(2.9);7.7872 (3.2);7.7219(0.8);7 .7189(0.9);7.7047(1.2);7.7014(1.6);7.6978(0.9);7.6836(1.0);7.6804(1.0);7.6087(1.5);7.6027 (2.4);7.5882(1.0);7.5848(1.7);7.579 6(1.7);7.5732(1.4);7.5673(0.9);7.5645(0.8);7.1682(2.6);7.1473(2.4);5.7551 (0.5);5.3679(4.3);5.3639(4.4);4.2470(1.9);4.2332(4. 0);4.2194(2.0);3.4113(20.4);3.3185(33.7);2.9867(1.8); 2.9731(3.5);2.9594(1.7);2.6759(0.4);2.6712(0.5);2.6667(0.4);2.5713(16.0);2.5245(1.5);2.5111(30.8);2.5067 (63.0);2.5021(86.4);2.4976(64.8);2.4932(31.8);2.3334(0.4);2.3290(0.5);2.3243(0.4);0.0080( 0.8);-0.0001(23.0);- 0.0083(0.9) |
| 22: $^1$H-NMR(400.0 MHz, $d_6$-DMSO):<br>δ= 8.2575(2.3);8.2531(2.4);8.0842(2.2);8.0615(2.5);7.8056(1.7);7.7842(2.6);7.7031(1.6);7.6833(2.3);7.6593 (3.7);7.6365(3.1 );7 .6107(1.4);7.6047(1.3);7.5811(1.4);7.5752(14);7.3719(2.2);7.3525(1.9);5.3891(4.6);5.3853 (4.7);4.2479(2.0);4.2342(4.2);4.220 4(2.1);3.4116(19.6);3.3190(60.2);2.9871(1.9);2.9735(3.7);2.9597(1.9); 2.6759(0.5);2.6712(0.7);2.6670(0.5);2.5718(16.0);2.524 4(2.2);2.5108(43.8);2.5066(86.7);2.5022(117.2);2.4978 (89.0);2.3336(0.5);2.3291(0.7);2.3247(0.5);1.9000(0.4);0.0079(1.3);-0.0001(31.9);-0.0081(1.4) |
| 23: $^1$H-NMR(400.0 MHz, $d_6$-DMSO):<br>δ= 8.3749(1.1);8.3699(0.8);8.3622(1.1);8.3522(1.1);8.2536(2.2);8.2495(2.2);8.0976(2.3);8.0914(2.4);8.0051 (1.2);7.9985(0.8);7 .9950(1.2);7.9876(0.9);7.9828(1.3);7.9751(0.3);7.9183(2.6);7.8964(2.8);7.5962(1.3);7.5902 (1.2);7.5668(1.3);7.5608(1.2);7.527 5(0.4);7.5148(2.3);7.5097(1.6);7.5046(1.8);7.5024(1.7);7.4972(1.5);7.4920 (2.4);7,4795(0.4);7.2324(1.4);7.2261(1.4);7.2105(1. 4);7.2042(1.4);5.7552(0.8);5.2892(3.8);5.2847(3.9);4.2445 (1.9);4.2308(4.0);4.2170(2.1);3.4087(20.1);3.3207(33.6);2.9842(1.8) ;2.9705(3.6);2.9567(1.8);2.8908(0.6); 2.7316(0.5);2.6711(0.4);2.5691(16.0);2.5246(1.0);2.5196(1.5);2.5110(23.6);2.5066(50.7); 2.5020(71.6);2.4975 (53.7);2.4932(25.9);2.3289(0.4);2.3244(0.3);-0.0002(6.3) |
| 24: $^1$H-NMR(400.0 MHz, $d_6$-DMSO):<br>δ= 8.2584(2.4);8.2542(2.4);7.8974(1.9);7.8764(2.1);7.6321(1.4);7.6105(3.6);7.6041(1.6);7.5802(1.4);7.5741 (1.6);7.5698(1.9);7 .5502(2.2);7.5295(1.1);7.4767(0.6);7.4622(0.8);7.4570(1.3);7.4426(1.2);7.4364(0.9);7.4221 (0.8);7.3719(1.2);7.3541(0.8);7.345 6(1.2);7.3267(0.8);7.3083(2.1);7.2892(1.8);5.7555(0.4);5.3716(4.8);5.3679 (4.9);4.2491(2.0);4.2353(4.2);4.2216(2.2);3.4128(19 .6);3.3197(21.3);2.9883(1.9);2.9747(3.8);2.9610(1.9); 2.6712(0.5);2.6667(0.4);2.5725(16.0);2.5065(61.6);2.5021(85.0);2.4978( 64.4);2.3334(0.4);2.3290(0.5);-0.0002 (6.4) |
| 25: $^1$H-NMR(400.0 MHz, $d_6$-DMSO):<br>δ= 8.3391(3.2);8.3327(3.4);8.1797(1.7);8.1590(1.8);8.1266(1.8);8.1057(2.0);7.7517(3.0);7.7453(3.0);7.7278 (0.8);7.7248(0.9);7 .7105(1.2);7.7073(1.6);7.6895(1.0);7.6865(1.0);7.6266(2.9);7.6184(1.2);7.6157(1.2);7.6059 (3.3);7.6015(1.2);7.5979(1.7);7.580 1(0.8);7.5777(0.8);7.1867(2.7);7.1657(2.5);5.3920(7.8);4.2614(2.0);4.2476 (4.1);4.2338(2.1);3.4131(20.3);3.3212(25.3);2.9832( 1.8);2.9695(3.7);2.9558(1.8);2.6719(0.4);2.5710(16.0); 2.5249(1.0);2.5114(23.1);2.5070(49.0);2.5024(68.8);2.4980(51.6);2.493 8(25.0);2.3293(0.4);0.0079(0.4);- 0.0002(10.7);-0.0085(0.4) |
| 26: $^1$H-NMR(400.0 MHz, $d_6$-DMSO):<br>δ= 8.3409(3.2);8.3345(3.4);8.3152(0.4);8.1070(2.3);8.0844(2.6);7.8031(1.8);7.7815(2.7);7.7538(3.0);7.7475 (3.1);7.6984(1.6);7 .6784(2.4);7.6648(3.5);7.6575(1.5);7.6421(3.1);7.3507(2.2);7.3316(2.0);5.4132(8.0);4.2614 (2.0);4.2477(4.2);4.2341(2.2);3.412 7(19.5);3.3192(21.4);2.9827(2.0);2.9690(3.9);2.9555(1.9);2.6712(0.9); 2.5708(16.0);2.5062(110.3);2.5019(150.1);2.4976(115.0 );2.3328(0.6);2.3291(0.9);2.3246(0.7);0.0077(0.8);- 0.0002(20.4) |
| 27: $^1$H-NMR(400.0 MHz, $d_6$-DMSO): |

(fortgesetzt)

| **Beispiel** |
|---|
| δ= 8.2420(2.7);8.2368(2.6);8.1482(1.9);8.1259(4.1);8.1045(2.3);8.0787(0.6);7.7272(0.9);7.7246(1.0);7.7069(1.8);7.6891(1.1);7 .6863(1.2);7.6318(3.1);7.6112(3.9);7.5965(1.8);7.5761(2.4);7.5524(1.4);7.5465(1.5);7.2055(3.0);7.1845(2.7);5.3686(5.1);5.365 1(5.4);4.5718(1.0);4.3877(1.5);4.2425(2.2);4.2289(4.4);4.2153(2.3);3.8070(10.2);3.3200(44.7);3.0008(2.4);2.8201(2.3);2.8152( 2.4);2.8066(4.3);2.7931(2.2);2.6710(0.7);2.6666(0.6);2.5241(1.7);2.5062(88.9);2.5017(126.0);2.4974(99.5);2.3413(16.0);2.328 9(1.0);2.2877(0.4);0.0079(1.8);-0.0003(55.6);-0.0082(2.5) |
| 28: $^{1}$H-NMR(400.0 MHz, $d_6$-DMSO):<br>δ= 8.2459(2.6);8.2408(2.7);8.0776(2.6);8.0549(2.9);7.8046(1.9);7.7832(2.9);7.7018(1.8);7.6818(2.6);7.6614(5.2);7.6389(3.4);7.5845(1.5);7.5786(1.5);7.5550(1.5);7.5491(1.5);7.3692(2.5);7.3496(2.2);5.3903(5.1);5.3868(5.2);4.2442(2.3);4.2307(4.4);4.217 1(2.3);3.8084(10.6);3.3201(14.6);2.8214(2.3);2.8079(4.2);2.7943(2.2);2.6761(0.4);2.6717(0.6);2.6678(0.4);2.5247(1.3);2.5069( 68.8);2.5026(94.4);2.4983(71.4);2.3426(16.0);2.3298(0.8);0.0078(1.2);-0.0002(33.8);-0.0077(1.4) |
| 29: $^{1}$H-NMR(400.0 MHz, $d_6$-DMSO):<br>δ= 8.2324(2.5);8.2271(2.7);8.1230(1.9);8.1039(2.1);7.8677(3.1);7.8611(3.2);7.6765(2.2);7.6559(2.9);7.6209(3.2);7.5985(3.6);7 .5629(1.5);7.5570(1.5);7.5333(2.5);7.5294(2.0);7.5124(2.0);7.4942(1.1);7.4914(1.1);7.4078(1.5);7.3888(2.4);7.3705(1.1);7.186 9(1.8);7.1803(1.8);7.1646(1.7);7.1580(1.7);5.7549(0.4);5.2407(5.0);5.2364(5.2);4.2388(2.2);4.2253(4.3);4.2117(2.3);3.8055(10 .4);3.3197(18.6);2.8180(2.2);2.8045(4.2);2.7909(2.2);2.6757(0.5);2.6714(0.6);2.6671(0.5);2.5245(1.5);2.5066(79.4);2.5022(10 9.8);2.4979(82.8);2.3404(16.0);2.3290(1.2);0.0079(1.3);-0.0002(39.8);-0.0079(1.6) |
| 30: $^{1}$H-NMR(400.0 MHz, $CDCl_3$):<br>δ= 8.2065(2.0);8.1997(2.0);7.9262(1.5);7.9069(1.6);7.5833(2.4);7.5768(2.5);7.5423(1.4);7.5217(2.2);7.4626(2.3);7.4559(1.0);7 .4536(0.9);7.4401 (3.2);7.4172(0.8);7.3375(1.2);7.3184(1.8);7.3000(0.8);7.2610(7.7);7.1522(1.4);7.1457(1.4);7.1299(1.3);7.123 3(1.4);7.1162(1.9);7.1096(1.8);5.2523(8.4);4.2062(1.4);4.1927(2.7);4.1790(1.5);3.8629(0.5);3.5287(16.0);3.0459(1.9);3.0323(3 .8);3.0186(1.9);2.6759(14.0);2.4599(11.8);2.0045(1.6);1.6104(5.7);-0.0002(5.0) |
| 31: $^{1}$H-NMR(400.0 MHz, $CDCl_3$):<br>δ= 8.3769(2.0);8.3697(2.1);7.9042(1.4);7 .8849(1.5);7.5447(1.4);7.5240(2.1);7.5073(2.3);7.5008(3.9);7.4777(3.7);7 .4551(3.2);7 .4396(1.4);7.4369(1.4);7.4192(0.8);7.4161(0.7);7.3344(1.1);7.3154(1.7);7.3006(1.5);7.2945(1.7);7.2793(1.1);7.2720(1.1);7.261 1(7.1);7.1498(1.4);7.1432(1.3);7.1275(1.2);7.1209(1.2);5.2499(7.4);4.2145(1.3);4.2009(2.7);4.1872(1.5);3.5300(16.0);3.5175(0 .4);3.4761(0.4);3.0549(2.0);3.0412(3.7);3.0274(1.9);2.9545(1.1);2.8839(1.1);2.6793(14.1);1.6102(4.4);1.2555(0.6);0.9208(0.6); -0.0002(3.6) |
| 32: $^{1}$H-NMR(400.0 MHz, $d_6$-DMSO):<br>δ= 8.2239(2.2);8.2196(2.2);8.0771(2.1);8.0544(2.3);7.8029(1.6);7.7815(2.3);7.7006(1.5);7.6807(2.1);7.6591(4.2);7.6363(2.8);7 .5745(1.2);7.5687(1.3);7.5449(1.2);7.5391(1.2);7.3669(2.0);7.3476(1.8);5.3842(4.2);5.3808(4.4);4.1683(1.9);4.1533(4.1);4.138 3(2.0);3.3230(39.8);2.8013(1.8);2.7865(3.8);2.7714(1.8);2.6720(0.4);2.6675(0.3);2.5675(2.1);2.5497(6.7);2.5319(7.2);2.5071(5 7.4);2.5027(75.6);2.4983(56.7);2.3294(0.4);2.3249(0.3);0.9792(7.8);0.9615(16.0);0.9437(7.5);0.0078(2.3);-0.0002(54.0);-0.0081(2.7) |
| 33: $^{1}$H-NMR(400.0 MHz, $d_6$-DMSO):<br>δ= 8.3143(3.0);8.3071(3.0);8.2285(2.6);8.2058(2.9);7.7954(2.1);7.7739(3.0);7.6976(3.9);7.6800(2.1);7.6750(3.9);7.6602(2.7);7 .6392(1.5);7.5932(2.2);7.5718(3.2);7.4760(1.9);7.4687(1.9);7.4546(1.4);7.4472(1.4);7.2663(2.5);7.2470(2.3);5.3328(8.9);4.126 5(2.1);4.1114(4.3);4.0963(2.2);3.3268(102.8);2.7997(1.6);2.7846(3.0);2.7697(1.5);2.6765(0.4);2.6718(0.5);2.6677(0.4);2.5726( 1.8);2.5549(5.0);2.5371(5.4);2.5074(65.8);2.5030(86.3);2.4986(64.2);2.3344(0.4);2.3296(0.5);2.3253(0.4);0.9853(8.0);0.9676( 16.0);0.9498(7.7);0.0076(2.2);-0.0001(50.7);-0.0082(2.6) |
| 34: $^{1}$H-NMR(400.0 MHz, $d_6$-DMSO): |

(fortgesetzt)

| Beispiel |
|---|
| δ= 8.2074(0.9);8.1210(0.7);8.1021 (0.8);8.0766(0.5);8.0541 (0.5);7.8635(1.2);7.8571(1.2);7.8031 (0.4);7.7817 (0.6);7.6999(0.4);7 .6769(1.0);7.6559(1.2);7.6377(0.6);7.6192(1.2);7.5968(1.3);7.5330(1.0);7.5115(1.2);7.4939 (0.5);7.4073(0.6);7.3882(0.9);7.367 2(0.6);7.3459(0.4);7.1854(0.7);7.1788(0.7);7.1631(0.6);7.1564(0.6);5.3846 (1.0);5.2348(1.8);5.2313(1.8);4.1224(0.8);4.1071(1. 5);4.0917(0.8);3.3215(25.5);2.7331(0.8);2.7181(1.6);2.7032 (0.8);2.6714(0.4);2.5066(49.3);2.5022(63.5);2.4978(46.9);2.3292(0 .4);2.2264(5.6);1.0064(16.0);0.0078(3.0);-0.0002(61.6);-0.0085(2.8) |
| 35: $^1$H-NMR(400.0 MHz, $d_6$-DMSO):<br>δ= 8.2924(1.1);8.2875(1.1);8.1219(0.8);8.1029(0.9);7.8707(1.2);7.8642(1.3);7.6815(0.9);7.6604(1.2);7.6352 (0.6);7.6269(1.6);7.6045(1.8);7.5363(0.4);7.5180(0.8);7.4992(0.4);7.4123(0.6);7.3934(1.0);7.3746(0.5);7.1862 (0.7);7.1798(0.7);7.1638(0.7);7.157 2(0.7);5.2718(2.1);5.2686(2.1);4.5248(0.8);4.5135(1.3);4.5012(0.7);3.7673 (0.3);3.3262(45.3);2.8103(2.7);2.7981(2.7);2.6719(0 .3);2.5066(46.0);2.5025(59.6);2.4984(45.0);2.3293(0.4); 1.3683(16.0);0.0074(2.5);-0.0002(48.8);-0.0071(2.7) |
| 36: $^1$H-NMR(400.0 MHz, $d_6$-DMSO):<br>δ= 8.2188(1.1);8.2144(1.1);8.0759(1.0);8.0532(1.2);7.8031(0.8);7.7816(1.2);7.7000(0.8);7.6800(1.1);7.6602 (2.0);7.6378(1.4);7 .5625(0.6);7.5566(0.6);7.5326(0.6);7.5268(0.6);7.3651(1.0);7.3459(0.9);5.3811(2.2);4.1263 (0.8);4.1109(1.6);4.0956(0.8);3.323 0(51.0);2.7359(0.7);2.7206(1.4);2.7050(0.7);2.6712(0.4);2.5064(54.4); 2.5021(71.6);2.4977(54.5);2.3288(0.4);2.2272(4.9);1.00 83(16.0);0.0077(2.8);-0.0003(64.9) |
| 37: $^1$H-NMR(400.0 MHz, $d_6$-DMSO):<br>δ= 9.6357(0.4);8.3034(1.2);8.2990(1.2);8.0670(1.2);8.0443(1.3);7.8101(1.0);7.7885(1.4);7.7061(0.8);7.6863 (1.3);7.6764(1.9);7 .6653(0.8);7.6537(2.2);7.6362(0.5);7.6235(1.3);7.3742(1.2);7.3549(1.0);5.7570(0.8);5.4187 (2.5);5.2320(0.6);4.5213(1.4);4.509 6(1.0);3.8081(0.4);3.7953(0.4);3.7817(0.4);3.7708(0.4);3.3260(52.0);2.8122 (2.7);2.8002(2.9);2.6758(0.3);2.6716(0.4);2.5068(6 0.7);2.5024(79.5);2.4980(60.3);2.3292(0.4);2.3249(0.4); 1.3720(16.0);0.1460(0.3);0.0077(3.4);-0.0002(73.8);-0.0083(4.0);-0.1498(0.3) |
| 38:$^1$H-NMR(400.0 MHz,$d_6$-DMSO):<br>δ=8.2372(0.9);8.2319(0.9);8.0784(0.8);8.0557(0.9);7.8028(0.6);7.7813(0.9);7.7006(0.6);7.6802(0.8);7.6587 (1.5);7.6358(1.0);7 .5627(0.5);7.5570(0.5);7.5331(0.5);7.5273(0.5);7.3661(0.8);7.3467(0.7);5.3820(1.8);4.1287 (0.7);4.1143(1.5);4.0997(0.8);3.324 1(13.0);2.8546(0.8);2.5075(17.2);2.5032(22.8);2.4989(17.5);1.0437(16.0); 0.9913(0.4);-0.0002(15.9) |
| 39: $^1$H-NMR(400.0 MHz, $d_6$-DMSO):<br>δ= 8.2072(1.3);8.2029(1.3);8.0748(1.2);8.0521(1.4);7.8022(0.9);7.7808(1.4);7.6993(0.9);7.6794(1.2);7.6582 (2.5);7.6355(1.6);7 .5510(0.7);7.5451(0.7);7.5214(0.7);7.5157(0.7);7.3653(1.2);7.3460(1.0);5.3819(2.6);5.3787 (2.6);4.0399(1.0);4.0231(2.1);4.006 4(1.1);3.3234(48.2);3.0440(0.4);3.0275(1.1);3.0111(1.5);2.9948(1.1);2.9781 (0.4);2.8059(1.1);2.7892(2.1);2.7724(1.0);2.6717(0 .3);2.5069(46.3);2.5026(59.2);2.4983(44.7);2.3292(0.3); 0.9833(16.0);0.9670(15.8);-0.0002(29.6) |
| 40: $^1$H-NMR(400.0 MHz, $d_6$-DMSO):<br>δ= 8.1954(1.2);8.1908(1.2);8.1205(0.9);8.1020(1.0);7.8628(1.4);7.8562(1.5);7.6764(1.0);7.6559(1.4);7.6190 (1.5);7.5966(1.7);7 .5295(1.2);7.5235(0.8);7.5118(1.0);7.4999(0.8);7.4939(1.2);7.4060(0.7);7.3875(1.1);7.3694 (0.5);7.1840(0.9);7.1774(0.9);7.161 7(0.8);7.1551(0.8);5.2327(2.4);5.2283(2.5);4.0363(1.0);4.0196(2.1);4.0027 (1.0);3.3223(30.4);3.0419(0.4);3.0257(1.1);3.0093(1 .5);2.9929(1.1);2.9766(0.4);2.8025(1.0);2.7858(2.1);2.7691 (1.0);2.5067(39.0);2.5023(51.5);2.4979(38.6);0.9816(16.0);0.9653( 15.7);0.0078(1.4);-0.0002(34.1);-0.0081 (1.5) |
| 41: $^1$H-NMR(400.0 MHz, $d_6$-DMSO):<br>δ= 8.3156(0.4);8.2202(8.0);8.2161(7.8);8.1213(5.7);8.1022(5.9);7.8651(9.3);7.8585(9.5);7.6760(6.6);7.6555 (8.5);7.6193(9.5);7 ,5969(10.7);7.5597(4.6);7.5538(4.6);7.5297(7,7);7.5243(5.1);7.5113(6.1);7.4936(3.3);7.4904 (3.4);7.4055(4.5);7.3869(7.0);7.36 80(3.2);7.1846(5.7);7.1780(5.7);7.1623(5.2);7.1556(5.4);5.2350(15.4);5.2305 (16.0);4.2097(7.0);4.1952(14.8);4.1806(7.3);3.32 41(469.6);2.6765(7.5);2.6709(4.5);2.6623(13.8);2.6478(6.3); 2.5240(6.8);2.5105(130.9);2.5062(266.3);2.5017(357.5);2.4972(26 7.6);2.4928(135.0);2.4181(10.1);2.3515 (0.4);2.3449(0.5);2.3328(1.6);2.3284(2.2);2.3240(1.6);1.5094(2.9);1.4951(8,0);1.4810( 12.8);1.4677(10.6); 1.4543(4.5);1.3924(2.4);1.3779(4.7);1.3648(5.1);0.1455(2.0);0.0075(17.1);-0.0006(444.8);-0.0088(19.9);-0.1503(2.0) |

(fortgesetzt)

| Beispiel |
|---|
| 42: $^{1}$H-NMR(601.6 MHz, $d_6$-DMSO): δ= 19.9783(1.6);8.2312(7.6);8.2281(7.5);8.0748(8.2);8.0596(9.0);7.7987(7 .0);7.7844(9.2);7.6920(6.5);7.6789 (8.5);7.6648(5.8); 7.6517(13.7);7.6367(13.3);7.5695(4.5);7.5655(4.4);7.5498(4.5);7.5459(4.4);7.3627(7.7); 7.3500(7.1);5.7506(1.5);5.3847(16.0);5.3821(15.9);4.2114(4.2);4.2024(8.3);4.1927(4.4);3.3067(438.5);2.6661 (6.0);2.6130(2.3);2.5223(3.9);2.5191(4.7);2.5161(4.4); 2.5073(132.5);2.5042(290.9);2.5012(411.2);2.4981 (292.1);2.4951(133.3);2.4207(6.4);2.3855(2.9);1.4915(6.6);1.4822(9.8);1.47 30(8.0);1.3697(4.0);0.0964(1.7); 0.0052(13.0);-0.0002(462.9);-0.0057(14.5);-0.1000(1.8) |
| 43: $^{1}$H-NMR(400.0 MHz, $d_6$-DMSO): δ= 8.2123(2.2);8.2083(2.3);8.1219(1.6);8.1037(1.7);7.8660(2.5);7.8595(2.6);7.6768(1.8);7.6563(2.3);7.6205 (2.6);7.5982(2.8);7 .5483(1.3);7.5425(1.3);7.5333(0.9);7.5301(1.0);7.5186(1.5);7.5126(2.9);7.4943(0.9);7.4912 (0.9);7.4063(1.2);7.3878(2.0);7.368 9(0.9);7.1866(1.6);7.1800(1.5);7.1643(1.4);7.1576(1.4);5.2348(4.3);5.2303 (4.6);4.1569(1.9);4.1419(4.2);4.1269(2.0);3.3246(61 .2);2.8417(0.4);2.8256(1.0);2.8093(1.3);2.7928(1.0);2.7764 (0.4);2.7314(1.8);2.7164(3.6);2.7015(1.7);2.6715(0.4);2.5070(47.6); 2.5025(63.5);2.4981(48.7);2.3290(0.4); 2.2011(13.7);0.9526(16.0);0.9362(15.8);0.1461(0.3);0.0077(3.3);-0.0003(70.5);-0.1496(0.3) |
| 44: $^{1}$H-NMR(601.6 MHz, $d_6$-DMSO): δ= 8.2221(2.1);8.2194(2.0);8.0750(2.0);8.0599(2.2);7.7991(1.7);7.7848(2.3);7.6921(1.6);7.6790(2.1);7.6649 (1.4);7.6527(3.6);7 .6377(3.4);7.5565(1.2);7.5525(1.2);7.5369(1.2);7.5329(1.2);7.3619(1.9);7.3493(1.8);5.3839 (4.1);5.3812(4.1);4.1572(1.9);4.147 2(4.1);4.1373(2.0);3.3061(60.1);2.8332(0.3);2.8224(0.8);2.8113(1.1);2.8006 (0.8);2.7900(0.3);2.7309(1.4);2.7209(2.9);2.7108(1 .4);2.6130(0.5);2.5224(0.9);2.5192(1.0);2.5162(1.0);2.5074 (29.5);2.5044(64.4);2.5013(90.7);2.4983(64.0);2.4952(29.0);2.3855 (0.5);2.2033(11.7);0.9525(16.0);0.9416 (15.9);0.0965(0.4);0.0053(3.2);-0.0002(108.5);-0.0057(3.3);-0.1003(0.4) |
| 45: $^{1}$H-NMR(400.0 MHz, $d_6$-DMSO): δ= 9.8139(0.6);8.3462(0.3);8.2000(5.2);8.0747(5.3);8.0520(5.9);7.9530(0.4);7.7919(3.9);7.7705(5.8);7.6953 (3.7);7.6754(5.1 );7 .6525(8.6);7.6295(6.6);7.5328(0.4);7.3693(5.0);7.3501(4,4);7.3249(2.5);7.3195(2.4);7.2912 (2.6);7.2859(2.4);5.3230(10.9);5.31 99(11.0);3.5125(0.6);3.5000(0.7);3.4877(0.6);3.4221(0.3);3.4081(0.4); 3.3219(219.8);3.3084(8.2);3.2958(9.4);3.2832(7.1);3.24 16(0.5);3.2279(0.4);2.8908(2.3);2.7316(2.0);2.6755 (1.3);2.6709(1.8);2.6665(1.3);2.5241(6.9);2.5108(110.7);2.5064(218.3);2.5 019(286.9);2.4974(212.0);2.4931 (107.5);2.4745(8.6);2.3956(1.9);2.3775(5.4);2.3595(5.6);2.3415(2.2);2.3334(1.9);2.3287(2.0); 2.3242(1.4); 2.3090(0.5);2.3019(0.5);2.0226(0.4);1.2980(0.9);1.2855(0.4);1.2580(1.3);1.2487(0.4);1.2331(0.5);1.0503(7.8); 1.03 24(16.0);1.0145(7.6);0.1457(0.6);0.0076(5.0);-0.0004(125.4);-0.0086(5.1);-0.1498(0.6) |
| 46: $^{1}$H-NMR(400.0 MHz, $d_6$-DMSO): δ= 9.9870(1.3);8.3450(0.6);8.3393(0.6);8.1868(3.1);8.1656(0.5);8.1451(0.5);8.1203(2.3);8.1024(2.4);7.9529 (2.4);7.8917(0.7);7 .8852(0.7);7.8599(3.7);7.8534(3.9);7.7514(0.7);7.7292(0.8);7.7220(0.5);7.7016(0.7);7.6748 (2.7);7.6542(3.4);7.6133(4.0);7.590 9(4.2);7.5432(0.6);7.5310(1.3);7.5277(1.5);7.5099(2.4);7.4921(1.4);7.4889 (1.3);7.4589(0.6);7.4051(1.9);7.3865(3.0);7.3675(1. 4);7.3347(0.5);7.3101(1.6);7.3046(1.7);7.2772(1.8);7.2713 (1.9);7.2561(0.5);7.2496(0.4);7.1770(2.3);7.1704(2.2);7.1617(0.6);7. 1547(2.0);7.1480(2.0);7.1295(0.4);6.6915 (0.5);6.6860(0.5);5.1773(6.2);5.1735(6.4);4.0380(0.4);4.0202(0.4);3.3232(111.6);3.2 989(4.4);3.2869(5.5); 3.2742(4.4);3.1834(0.4);3.1717(0.4);3.1590(0.3);2.8906(16.0);2.7312(14.2);2.6756(0.6);2.6712(0.9);2.66 66 (0.7);2.5107(57.8);2.5065(115.6);2.5021(153.8);2.4976(113.4);2.4933(58.0);2.4166(0.9);2.3950(1.7);2.3773 (3.3);2.3593(3.4 );2.3407(1.5);2.3335(1.2);2.3289(1.3);2.3239(1.4);2.3085(0.8);2.3047(0.9);2.0229(0.4);1.9890 (1.6);1.9093(0.3);1.7677(0.6);1.2 983(0.4);1.2582(0.6);1.1927(0.4);1.1749(0.8);1.1572(0.4);1.0487(5.3);1.0308 (10.9);1.0128(5.1);0.9946(1.0);0.9908(0.8);0.984 9(0.5);0.9769(1.7);0.9589(0.8);-0.0002(9.0) |
| 47: $^{1}$H-NMR(400.0 MHz, $d_6$-DMSO): δ= 8.1917(2.5);8.1869(2.5);7.6312(3.3);7.6181(4.5);7.5419(3.0);7.5357(1.7);7.5118(1.4);7.5058(1.4);7.4287 (1.1);7.2980(1.3);7 .2751(1.9);7.2492(1.8);7.2452(1.6);7.2373(1.6);7.2291(1.4);7.2212(1.4);7.1122(0.9);7.1031 (1.4);7.0944(0.9);7.0899(0.8);7.080 7(1.1);7.0720(0.6);5.2010(4.8);5.1969(5.0);4.1566(2.0);4.1417(4.2);4.1267 (2.0);3.3220(64.4);2.7917(1.8);2.7770(3.4);2.7621(1 .7);2.6713(0.7);2.6666(0.6);2.5609(2.0);2.5431(6.2);2.5251 (8.3);2.5065(97,0);2.5021(125.6);2.4978(93.4);2.3334(0.5);2.3290(0.7);2.3246(0.5);1.2327(0.4);0.9732(7.9); 0.9555(16.0);0.9377(7.6);0.1461(0.4);0.0076(3.8);-0.0003(86.7);-0.0078(4.0);-0.1496(0.4) |
| 48: $^{1}$H-NMR(400.0 MHz, $d_6$-DMSO): |

(fortgesetzt)

| Beispiel |
|---|
| δ= 8.2215(2.6);8.2198(2.7);8.2161(2.6);7.6364(0.8);7.6306(0.4);7.6167(1.8);7.5947(1.6);7.5789(1.5);7.5733 (1.3);7.5494(1.5);7 .5438(1.3);7.5030(4.0);7.4813(3.3);7.4408(4.4);7.4243(1.3);7.1504(2.2);7.1433(3.3);7.1292 (2.1);7.1216(1.0);7.1074(1.6);7.099 8(1.1);5.2043(5.2);4.2338(2.1);4.2203(4.3);4.2065(2.2);3.4021(19.5);3.3195 (13.8);2.9769(2.0);2.9633(4.0);2.9497(2.0);2.8912( 0.6);2.7320(0.6);2.6710(0.6);2.5643(16.0);2.5062(90.2); 2.5020(103.9);2.4977(70.8);2.3288(0.6);0.0000(7.0) |
| 49: $^1$H-NMR(400.0 MHz, $d_6$-DMSO):<br>δ= 8.2284(2.4);8.2243(2.4);7.6277(2.5);7.5787(1.4);7.5729(1.4);7.5495(1.9);7.5430(2.0);7.5322(3.0);7.5133 (2.9);7.4960(2.8);7 .4911(2.6);7.4858(1.2);7.4777(0.5);7.4727(0.6);7.4675(0.4);7.2864(1.2);7.2636(1.8);7.2378 (1.6);7.2317(1.4);7.2239(1.5);7.215 7(1.3);7.2079(1.4);7.0972(0.8);7.0882(1.3);7.0795(0.9);7.0748(0.8);7.0659 (1.0);7.0573(0.6);5.1994(4.8);5.1955(4.8);4.2356(2. 1);4.2219(4.2);4.2081(2.2);4.0379(0.3);3.4030(19.0);3.3178 (17.8);2.9779(2.0);2.9643(3.9);2.9506(1.9);2.6748(0.4);2.6707(0.5) ;2.6662(0.4);2.5649(16.0);2.5059(70.7); 2.5016(90.3);2.4974(67.2);2.3328(0.4);2.3284(0.6);1.9887(1.3);1.3975(2.0);1.1925(0.4 );1.1748(0.7);1.1569 (0.3);-0.0002(55.6) |
| 50: $^1$H-NMR(400.0 MHz, $d_6$-DMSO):<br>δ= 8.3105(0.6);8.2279(2.2);8.2237(2.2);7.6330(3.0);7.6202(3.8);7.5792(1.3);7.5733(1.3);7.5499(3.1);7.5446 (2.1);7.4409(0.6);7 .4305(1.0);7.4150(0.5);7.3015(1.2);7.2788(1.7);7.2758(1.5);7.2526(1.9);7.2424(1.4);7.2342 (1.2);7.2264(1.3);7.1183(0.8);7.109 0(1.2);7.1006(0.8);7.0958(0.7);7.0868(0.9);7.0782(0.5);5.2072(4.1);5.2032 (4.2);4.2370(1.9);4.2233(4.0);4.2095(2.0);4.0399(0. 6);4.0221(0.6);3.4049(19.8);3.3230(5.3);2.9796(1.8);2.9660 (3.7);2.9522(1.8);2.5667(16.0);2.5267(0.4);2.5132(8.7);2.5089(17. 9);2.5044(23.6);2.4999(17.2);2.4956(8.5); 1.9903(2.5);1.3968(6.3);1.1940(0.7);1.1762(1.3);1.1584(0.7);0.0079(0.8);-0.0002(22.0);-0.0084(0.8) |
| 51: $^1$H-NMR(400.0 MHz, $d_6$-DMSO):<br>δ= 8.1869(1.0);8.1828(1.0);7.6309(1.4);7.6178(1.9);7.5447(0.9);7.5284(0.6);7.5231(0.6);7.4993(0.6);7.4932 (0.6);7 .4287(0.5);7 .2967(0.5);7.2739(0.8);7.2709(0.7);7.2479(0.8);7.2450(0.7);7.2363(0.7);7.2280(0.6);7.2203 (0.6);7.1112(0.4);7.1020(0.6);7.093 5(0.4);7.0888(0.3);7.0798(0.5);5.2007(2.0);5.1968(2.0);4.1153(0.7);4.0995 (1.5);4.0841(0.8);3.3214(27.8);2.7259(0.7);2.7103(1 .4);2.6949(0.7);2.6710(0.4);2.5064(44.2);2.5020(58.1); 2.4976(43.3);2.3287(0.3);2.2201(5.0);1.0006(16.0);0.0078(1.4);-0.0002(33.0);-0.0079(1.6) |
| 52: $^1$H-NMR(601.6 MHz, $CDCl_3$):<br>δ= 8.2606(1.7);8.2455(1.8);7.8618(1.6);7.8475(1.7);7.5069(1.2);7.5011(2.6);7.4936(1.8);7.4861(2.4);7.4797 (1.2);7.2589(26.6); 7.1854(1.7);7.1718(2.0);7.0330(2.2);7.0193(1.9);6.9921(1.7);6.9793(1.6);5.3152(6.8);4.2239 (1.9);4.2140(4.0);4.2041(2.0);3.52 68(16.0);3.1054(2.2);3.0956(4.4);3.0857(2.1);2.6824(14.6);2.5371(15.1); 1.5443(15.4);1.2554(1.0);0.0691(3.3);0.0051(0.6);-0.0002(16.0);-0.0056(0.7) |
| 53: $^1$H-NMR(400.0 MHz, $d_6$-DMSO):<br>δ= 8.2352(2.6):8.2294(2.8):7.7460(1.4):7.7261(1.9):7.6561(2.4):7.6161(1.6):7.5960(2.8):7.5812(1.7):7.5759 (2.8):7.5519(1.4):7 .5462(1.4);7.4251(1.1);7.4055(2.7);7.3844(4.0);7.3784(3.9);7.3629(1.2);7.3073(2.0);7.2885 (1.4);7.0919(1.4);7.0860(1.4);7.071 6(1.3);7.0654(1.3);5.2406(4.9);5.2372(5.0);4.2383(2.2);4.2246(4.4);4.2109 (2.3);3.4048(18.8);3.3206(40.4);2.9804(2.0);2.9668( 4.1);2.9530(2.0);2.6715(0.5);2.5666(16.0);2.5064(63.4); 2.5023(84.2);2.3289(0.5);-0.0002(5.2) |
| 54: $^1$H-NMR(400.0 MHz, $d_6$-DMSO):<br>δ= 8.2232(2.2);8.2191(2.2);7.5725(1.3);7.5665(1.3);7.5430(1.3);7.5371(1.2);7.3059(3.0);7.2840(3.3);6.7956 (2.4);6.7888(2.6);6 .6528(1.6);6.6460(1.4);6.6310(1.4);6.6241(1.4);5.1373(4.3);5.1328(4.3);4.2323(1.9);4.2185 (4.0);4.2047(2.0);4.1110(1.1);4.0936(3.6);4.0761(3.6);4.0587(1.1);3.4042(20.4);3.3208(28.5);2.9767(1.8); 2.9630(3.6);2.9494(1.8);2.6710(0.3);2.5655(16.0);2.524 2(1.2);2.5106(23.4);2.5063(47.5);2.5018(62.6);2.4973 (44.7);2.4930(21.5);2.3286(0.4);1.9888(1.0);1.3517(3.8);1.3343(8.0);1.3 169(3.7);1.1749(0.5);0.0079(2.6);- 0.0003(66.7);-0.0085(2.8) |
| 55: $^1$H-NMR(400.0 MHz, $d_6$-DMSO): |

(fortgesetzt)

| Beispiel |
|---|
| δ= 8.2290(2.0);8.2248(2.1);8.2116(0.6);7.5788(1.2);7.5729(1.2);7.5538(0.6);7.5495(1.3);7.5433(1.4);7.5354 (1.1);7 .5189(1.1);7 .5144(0.7);7.4996(0.8);7.4299(2.5);7.4270(2.1);7.4078(1.8);7.2865(1.2);7.2821(0.7);7.2773 (0.6);7.2730(0.6);7.2637(1.8);7.260 3(2.3);7.2530(1.0);7.2379(1.9);7.2296(1.6);7.2215(1.3);7.2133(1.2);7.2055 (1.3);7.0988(0.8);7.0895(1.2);7.0810(0.8);7.0762(0. 7);7.0675(0.9);7.0587(0.6);5.1970(4.0);5.1924(4.2);4.2353 (1.9);4.2215(3.9);4.2077(2.0);3.4034(20.3);3.3187(11.7);2.9779(1.8) ;2.9642(3.5);2.9504(1.7);2.5651(16.0); 2.5243(0.8);2.5194(1.3);2.5109(19.8);2.5065(41.0);2.5019(54.3);2.4974(39.0);2.4930(18 .9);2.3286(0.3);1.3974 (0.8);0.0079(1.4);-0.0002(43.1);-0.0085(1.6) |
| 56: $^1$H-NMR(400.0 MHz, $d_6$-DMSO):<br>δ= 8.2224(2.2);8.2182(2.2);7.5789(1.2);7.5730(1.3);7.5494(1.3);7.5435(1.3);7.5050(2.8);7.4990(4.8);7.4917 (5.7);7.4884(5.6);7 ,4797(0.6);7.4745(0.5);7.4661(3.0);7.4282(1.2);7.4236(1.2);7.4208(1.2);7.4139(1.0);7.4085 (0.9);7.4054(0.7);7.4023(0.7);7.131 3(1.9);7.1240(3.3);7.1122(2.2);7.1046(1.0);7.0905(1.7);7.0829(1.2);5.1973 (4.0);5.1930(4.3);4.2343(1.9);4.2205(3.9);4.2067(2. 0);3.4025(20.5);3.3206(13.3);2.9767(1.7);2.9630(3.5); 2.9493(1.8);2.5643(16.0);2.5245(0.6);2.5109(18.2);2.5065(39.1);2.5021( 53.2);2.4976(39.1);2.4932(19.4);-0.0002(1.3) |
| 57: $^1$H-NMR(400.0 MHz, $d_6$-DMSO):<br>δ= 8.1747(1.3);8.1698(1.3);7.6300(1.7);7.6169(2.3);7.5415(1.2);7.5163(0.7);7.5106(0.7);7.4867(0.7);7.4808 (0.7);7.4273(0.6);7 .2956(0.6);7.2726(0.9);7.2702(0.9);7.2468(0.9);7.2424(0.8);7.2343(0.8);7.2262(0.7);7.2183 (0.8);7.1093(0.4);7.1002(0.7);7.091 7(0.5);7.0872(0.4);7.0780(0.6);7.0694(0.3);5.1998(2.4);5.1960(2.6);4.0291 (1.0);4.0125(2.1);3.9959(1.1);3.3220(19.5);3.0369(0 .4);3.0203(1.1);3.0041(1.5);2.9877(1.1);2.9714(0.4);2.7956 (1.1);2.7790(2.1);2.7622(1.0);2.5069(30.6);2.5025(41.0);2.4981(31. 9);0.9755(16.0);0.9591(15.8);0.0078(1.2);-0.0002(26.4) |
| 58: $^1$H-NMR(400.0 MHz, $d_6$-DMSO):<br>δ= 8.2274(2.3);8.2230(2.4);8.1437(0.7);8.1392(0.7);7.5757(1.3);7.5701(1.3);7.5463(1.3);7.5407(1.3);7.4547 (0.4);7.4485(0.4);7 .4253(0.4);7.4196(0.4);7.3143(2.5);7.2925(2.7);6.8153(2.3);6.8087(2.6);6.6680(1.4);6.6614 (1.4);6.6462(1.4);6.6396(1.3);5.147 9(4.5);5.1441(4.8);4.2344(2.0);4.2208(4.0);4.2071(2.3);4.1970(1.0);4.1827 (1.4);4.1688(0.9);4.1557(0.4);4.1164(0.4);3.8423(0. 4);3.8252(13.9);3.7615(0.9);3.4918(1.5);3.4866(1.6);3.4050 (19.5);3.3879(1.9);3.3753(1.6);3.3666(0.6);3.3525(0.9);3.3222(8.1) ;2.9781(2.0);2.9647(4.4);2.9516(3.3);2.9383 (1.2);2.9135(0.4);2.6707(0.5);2.5663(16.0);2.5415(1.6);2.5056(69.1);2.5017(89.8); 2.4978(71.8);2.3284(0.5); 2.1399(8.2);1.2346(0.8);-0.0002(6.8) |
| 59: $^1$H-NMR(400.0 MHz, $CDCl_3$):<br>δ= 8.1852(2.0);8.1785(2.0);7.4807(0.4);7.4663(3.6);7.4488(2.1);7.4295(0.6);7.3884(1.8);7.2613(9.6);7.2274 (0.9);7 .2100(0.8);7 .1062(1.9);7.0987(2.1);7.0887(1.7);7.0800(1.3);7.0685(2.0);7.0439(1.6);7.0089(0.9);7.0000 (1.3);6.9915(0.8);6.9867(0.6);6.978 1(0.7);6.9691(0.4);5.1702(8.1);4.2028(1.4);4.1891(2.7);4.1755(1.5);3.8616 (0.5);3.5267(16.0);3.0449(2.0);3.0312(3.8);3.0175(1 .9);2.6755(14.1);2.4267(11.7);1.5856(7.7);1.1956(0.4); 0.0703(0.5);-0.0002(6.2) |
| 60: $^1$H-NMR(400.0 MHz, $d_6$-DMSO):<br>δ= 8.1926(2.2);8.1888(2.2);7.6313(2.8);7.6184(3.8);7.5469(2.0);7.5366(1.5);7.5306(1.3);7.5069(1.2);7.5010 (1.3);7.4399(0.6);7 .4301(1.0);7.2980(1.1);7.2753(1.6);7.2723(1.5);7.2493(1.6);7.2454(1.4);7.2372(1.4);7.2291 (1.2);7.2213(1.2);7.1126(0.8);7.103 3(1.2);7.0949(0.8);7.0902(0.7);7.0812(0.9);7.0724(0.6);5.2002(4.2);5.1960 (4.3);4.1495(1.9);4.1345(4.0);4.1195(2.0);3.3235(86 .6);2.8369(0.4);2.8204(1.0);2.8040(1.3);2.7876(1.0);2.7709 (0.4);2.7247(1.7);2.7098(3.5);2.6948(1.7);2.6758(0.4);2.6713(0.6);2.6668(0.4);2.5066(68.5);2.5022(90.5); 2.4977(68.7);2.3333(0.4);2.3288(0.5);2.3244(0.4);2.1958(13.6);0.9480(16.0);0.9316(15.7 );0.1460(0.5);0.0078 (4.4);-0.0002(102.1);-0.0084(5.0);-0.1497(0.4) |
| 61: $^1$H-NMR(400.0 MHz, $d_6$-DMSO):<br>δ= 8.2221(2.1);8.2186(2.1);8.2165(2.1);7.6128(1.1);7.6054(1.1);7.5787(1.1);7.5729(1.1);7.5492(1.1);7.5429 (1.4 );7.5264(0.5);7 .5219(0.8);7.5063(0.9);7.5021(0.6);7.4879(1.7);7.4755(1.2);7.4668(1.8);7.4534(1.3);7.3101 (0.7);7.3048(0.8);7.2977(1.2);7.289 4(0.6);7.2848(1.3);7.2789(2.0);7.2624(0.8);7.2560(0.6);7.2419(0.4);7.1239 (1.0);7.1171(2.4);7.1129(2.0);7.1080(0.9);7.1006(0. 7);7.0914(1.2);7.0852(1.0);7.0784(0.6);5.1949(2.7);5.1906 (2.8);5.1518(1.6);5.1474(1.6);4.2331(1.8);4.2194(3.8);4.2056(1.9);3. 4028(19.2);3.3207(25.8);2.9762(1.8); 2.9625(3.6);2.9488(1.8);2.6711(0.4);2.5644(16.0);2.5243(0.9);2.5107(27.1);2.5064(56.0); 2.5020(74.1);2.4975 (52.8);2.4933(25.4);2.3288(0.4);-0.0001(1.6) |

(fortgesetzt)

| Beispiel |
|---|
| 62: $^1$H-NMR(400.0 MHz, $d_6$-DMSO): δ= 8.2058(0.9);8.2014(0.9);7.6317(1.2);7.6187(1.6);7.5487(0.8);7.5295(0.5);7.5239(0.5);7.5001(0.5);7.4944 (0.5);7.4285(0.4);7 .2984(0.4);7.2750(0.6);7.2491(0.7);7.2387(0.5);7.2307(0.5);7.2230(0.5);7.1050(0.5);7.0823 (0.4);5.2020(1.7);5.1987(1.7);4.117 3(0.7);4.1027(1.5);4.0882(0.8);3.3233(6.8);2.8589(0.5);2.8458(0.8);2.8330 (0.5);2.5073(14.7);2.5031(18.8);2.4989(14.3);1.132 2(0.3);1.0378(16.0);0.9945(0.5);-0.0002(13.3) |
| 63: $^1$H-NMR(400.0 MHz, $d_6$-DMSO): δ= 8.2228(2.5);8.2190(2.6);7.5774(1.4);7.5715(1.3);7.5478(1.4);7.5424(1.3);7.4880(0.8);7.4722(3.6);7.4486 (5.8);7.4429(5.9);7 .4264(2.6);7.4090(1.4);7.4006(0.4);7.3922(0.4);7.0805(3.7);7.0723(2.6);7.0651(2.1);7.0576 (0.7);5.1881(4.6);5.1849(4.6);5.151 6(0.5);4.2326(2.1);4.2189(4.4);4.2052(2.2);3.9033(5.3);3.4019(18.5);3.3203 (53.9);3.1749(0.4);3.1617(0.3);2.9756(2.0);2.9620( 4.0);2.9484(2.0);2.6706(1.1);2.5637(16.0);2.5056(155.7); 2.5017(193.2);2.3284(1.1);1.2362(0.4);0.0001(4.3) |
| 64: $^1$H-NMR(400.0 MHz, $CDCl_3$): δ= 8.3620(2.1);8.3550(2.1);7.5060(0.9);7.4863(1.9);7.4732(1.8);7.4613(1.6);7.4516(2.2);7.4415(2.2);7.4219 (1.0);7 .4043(2.0);7 .3825(1.0);7.3628(2.0);7.3429(1.3);7.3005(1.4);7.2934(1.4);7.2790(1.2);7.2718(1.2);7.2609 (6.5);7.2064(3.0);7.2018(2.5);7.180 1(2.1);7.1600(1.2);7.0158(1.1);7.0107(1.0);6.9957(1.0);6.9900(0.9);5.2100 (7.1);5.1371(0.4);4.2130(1.4);4.1995(2.8);4.1858(1. 6);3.5295(16.0);3.0543(2.1);3.0406(3.9);3.0268(2.0);2.6795 (14.0);1.5948(4.6);1.2550(0.5);-0.0002(3.2) |
| 65: $^1$H-NMR(400.0 MHz, $CDCl_3$): δ= 8.3545(2.0);8.3476(2.0);7.5140(1.9);7.4450(1.6);7.4232(2.5);7.4053(1.2);7.4004(1.3);7.3832(0.8);7.3637 (2.2);7.3520(1.6);7 .3471(3.3);7.3422(1.4);7.3324(0.4);7.3276(0.4);7.2997(1.4);7.2924(1.3);7.2782(1.1);7.2707 (1.3);7.2627(9.4);7.0882(0.9);7.065 6(1.7);7.0406(2.0);7.0316(1.3);7.0237(1.1);7.0159(1.2);6.9478(0.8);6.9391 (1.1);6.9305(0.8);6.9254(0.6);6.9171(0.8);6.9082(0. 5);5.1486(7.2);4.2143(1.3);4.2008(2.6);4.1871(1.4);3.5291 (16.0);3.0549(1.9);3.0411(3.7);3.0273(1.9);2.9569(0.5);2.8850(0.4); 2.6797(14.1);2.0454(0.3);1.6117(10.1); 1.2558(0.6);0.9214(0.4);-0.0002(4.5) |
| 66: $^1$H-NMR(400.0 MHz, $d_6$-DMSO): δ= 8.1958(0.9);8.1915(0.9);7.7452(0.5);7.7246(0.6);7.6530(0.8);7.6150(0.6);7.5949(1.0);7.5750(0.5);7.5327 (0.5);7.5268(0.5);7 .5031(0.5);7.4973(0.5);7.4214(0.4);7.4018(1.0);7.3812(1.5);7.3754(1.2);7.3701(0.8);7.3636 (0.4);7.3056(0.7);7.2855(0.5);7.086 1(0.5);7.0815(0.5);7.0657(0.4);7.0611(0.4);5.2362(1.6);5.2321(1.7);4.1174 (0.7);4.1018(1.4);4.0862(0.7);3.3230(25.8);2.7278(0 .6);2.7128(1.3);2.6976(0.6);2.5066(27.6);2.5021(36.4); 2.4976(27.0);2.2220(4.8);1.0024(16.0);0.0080(0.8);-0.0002(22.8);-0.0083(1.1) |
| 67: $^1$H-NMR(400.0 MHz, $d_6$-DMSO): δ= 8.1841(1.3);8.1797(1.3);7.7438(0.7);7.7232(1.0);7.6496(1.2);7.6145(0.8);7.5944(1.5);7.5745(0.8);7.5205 (0.7);7.5147(0.7);7 .4911(0.8);7.4852(0.8);7.4205(0.6);7.4007(1,4);7.3803(2.1);7.3745(1.7);7.3687(1.2);7.3044 (1.0);7.2841(0.7);7.0846(0.7);7.079 0(0.7);7.0641(0.6);7.0593(0.6);5.2355(2.5);5.2316(2.6);4.0318(1.0);4.0153 (2.1);3.9985(1.0);3.3221(19.9);3.0394(0.4);3.0227(1.0);3.0065(1.3);2.9901(1.0);2.9737(0.4);2.7986(0.9);2.7821 (1.8);2.7655(0.9);2.5069(28.2);2.5025(37.4);2.4981(28.2);0.9781(1 6.0);0.9618(15.8);0.0079(1.0);-0.0002 (25.8);-0.0082(1.2) |
| 68: $^1$H-NMR(400.0 MHz, $d_6$-DMSO): δ= 8.2305(0.8);8.2177(2.5);8.2134(2.6);7.5983(0.4);7.5832(1.6);7.5774(1.7);7.5689(3.0);7.5537(1.9);7.5472 (3.9);7.4180(0.5);7 .3984(0.5);7.3898(0.5);7.3858(0.5);7.1369(3.0);7.1289(2.5);7.1219(1.2);7.1068(2.0);7.0998 (1.4);7.0890(0.4);6.9972(0.7);6.868 4(1.4);6.8614(0.8);6.7394(0.7);6.7325(0.4);5.2855(1.0);5.2029(4.9);5.1994 (5.0);4.2306(2.9);4.2174(4.5);4.2038(2.3);3.4069(5. 7);3.4002(17.8);3.3571(0.5);3.3197(26.8);2.9737(2.5); 2.9604(4.2);2.9469(2.2);2.6752(0.4);2.6716(0.5);2.5630(16.0);2.5063(71 .1);2.5022(86.3);2.4986(66.7);2.3290 (0.6);2.1406(0.7);1.2333(0.3);1.0551(1.0);0.1461(0.4);-0.0003(76.8);-0.1494(0.4) |
| 69: $^1$H-NMR(400.0 MHz, $d_6$-DMSO): |

(fortgesetzt)

| Beispiel |
|---|
| δ= 8.2012(2.1);8.1969(2.1);7.7444(1.2);7.7248(1.5);7.6537(1.9);7.6151(1.4);7.5949(2.5);7.5750(1.3);7.5402 (1.2);7 .5342(1.2);7 .5106(1.2);7.5046(1.2);7.4224(1.0);7.4025(2.3);7.3818(3.5);7.3760(3.0);7.3631(1.0);7.3054 (1.6);7.2859(1.1);7.0873(1.1);7.082 4(1.1);7.0671(1.0);7.0623(1.0);5.2356(4.0);5.2316(4.1);4.1519(1.9);4.1369 (4.0);4.1218(2.0);3.3227(66.7);2.8382(0.4);2.8219(1 .0);2.8055(1.4);2.7891(1.0);2.7728(0.4);2.7267(1.7);2.7118 (3.5);2.6968(1.7);2.6754(0.4);2.6710(0.5);2.6665(0.3);2.5105(26.3); 2.5064(51.5);2.5019(68.5);2.4975(52.1); 2.3289(0.4);2.1972(13.8);0.9493(16.0);0.9329(15.7);0.1456(0.4);0.0078(3.5);-0.0003(77.2);-0.0084(4.0);-0.1499(0.4) |
| 70: $^1$H-NMR(400.0 MHz, $d_6$-DMSO):<br>δ= 8.2399(2.5);8.2358(2.6);7.6755(3.4);7.6563(3.9);7.5811(1.4);7.5752(1.5);7.5515(1.4);7.5458(1.5);7.4789 (1.8);7.4606(3.9);7 .4412(2.4);7.3985(1.2);7.3859(1.6);7.3787(2.9);7.3675(2.1);7.3590(2.0);7.3494(0.8);7.3108 (2.6);7.3066(2.1);7.2567(2.0);7.237 4(1.5);7.0452(1.4);7.0398(1.4);7.0247(1.2);7.0201(1.2);5.2181(5.4);4.2368 (2.2);4.2232(4.4);4.2094(2.3);3.4042(18.3);3.3182(1 4.2);2.9787(2.0);2.9652(4.0);2.9513(2.0);2.6701(0.4); 2.5653(16.0);2.5054(55.6);2.5012(74.4);2.4972(59.0);2.3277(0.5);0.0074 (1.9);-0.0002(43.0) |
| 71: $^1$H-NMR(400.0 MHz, $d_6$-DMSO):<br>δ= 8.2304(2.2);8.2263(2.2);7.5763(1.4);7.5681(2.6);7.5640(1.8);7.5471(4.3);7.4984(1.9);7.4804(3.7);7.4611 (2.0);7.4291(0.8);7 .4259(1.4);7.4227(0.9);7.4135(0.5);7.4076(1.7);7.3895(0.6);7.2630(1.2);7.2404(1.7);7.2374 (1.6);7.2147(1.5);7.1731(1.2);7.165 3(1.4);7.1570(1.3);7.1492(1.4);7.0680(0.8);7.0587(1.3);7.0503(0.8);7.0456 (0.8);7.0367(1.0);7.0280(0.6);5.1873(4.3);5.1829(4. 6);4.2340(1.9);4.2203(4.0);4.2066(2.1);3.4025(19.6);3.3189 (19.4);2.9769(1.8);2.9633(3.6);2.9495(1.8);2.6708(0.4);2.5643(16. 0);2.5409(36.2);2.5239(0.9);2.5104(22.3); 2.5061(46.1);2.5016(61.5);2.4971(45.7);2.4929(23.3);2.3283(0.4);0.0079(1.5);-0.0002(41.0);-0.0084(1.9) |
| 72: $^1$H-NMR(400.0 MHz, $d_6$-DMSO):<br>δ= 8.2219(2.1);8.2178(2.1);8.2162(2.1);7.6083(2.9);7.5858(4.5);7.5798(1.4);7.5561(1.3);7.5501 (1.3);7.2664 (1.2);7.2634(1.6);7 .2598(1.8);7.2568(1.6);7.1697(1.7);7.1625(1.5);7.1472(1.5);7.1400(1.4);5.2136(4.3);5.2091 (4.5);4.2319(1.9);4.2182(4.0);4.204 4(2.0);3.4006(19.7);3.3191(17.2);2.9742(1.8);2.9606(3.6);2.9469(1.8); 2.5633(16.0);2.5246(0.6);2.5111(16.6);2.5067(34.8);2.5 022(46.6);2.4977(34.1);2.4933(16.9);0.0079(1.2);-0.0002(34.3);-0.0085(1.4) |
| 73: $^1$H-NMR(400.0 MHz, $CDCl_3$):<br>δ= 8.3509(2.2);8.3439(2.2);7.4043(1.7);7.3830(2.4);7.3484(2.4);7.3261(2.7);7.2941(1.5);7.2869(1.5);7.2725 (1.2);7.2619(8.0);6 .9842(1.7);6.9811(1.8);6.8920(1.5);6.8850(1.3);6.8697(1.4);6.8626(1.2);5.1194(8.6);4.2128 (1.5);4.1992(3.0);4.1857(1.6);3.528 7(16.0);3.0542(2.1);3.0405(4.1);3.0268(2.1);2.6801(14.5);1.5757(4.9);-0.0002(4.0) |
| 74: $^1$H-NMR(400.0 MHz, $d_6$-DMSO):<br>δ= 8.2142(0.9);8.2102(0.9);7.7448(0.5);7.7254(0.7);7.6562(0.8);7.6155(0.6);7.5954(1.0);7.5755(0.5);7.5340 (0.5);7 .5282(0.5);7 .5044(0.5);7.4988(0.5);7.4233(0.4);7.4035(0.9);7.3830(1.6);7.3777(1.4);7.3608(0.4);7.3065 (0.7);7.2875(0.5);7.0889(0.5);7.0833(0.5);7.0684(0.4);7.0630(0.4);5.2369(1.7);5.2339(1.8);4.1196(0.7);4.1051 (1.5);4.0906(0.8);3.3231(6.2);2.8607(0.5);2.8477(0. 8);2.5069(16.4);2.5026(21.0);2.4985(16.2);1.0388(16.0);-0.0002(14.4) |
| 75: $^1$H-NMR(400.0 MHz, $d_6$-DMSO):<br>δ= 8.3163(0.4);8.2018(8.1);8.1979(8.2);7.6515(0.8);7.6313(10.5);7.6184(14.4);7.6005(0.5);7.5485(11.6);7.5432 (10.0);7.5190(4 .7);7.5131(4.8);7.4400(2.4);7.4287(3.6);7.2975(4.2);7.2747(6.1);7.2717(5.7);7.2487(6.4);7.2458 (5.6);7.2376(5.1);7.2295(4.6);7 .2216(4.8);7.1115(2.9);7.1022(4.5);7.0938(3.0);7.0890(2.7);7.0804(3.5);7.0714 (2.1);5.2944(0.4);5.2013(15.2);5.1970(16.0);4.2 036(6.0);4.1892(12.3);4.1747(6.3);3.3233(298.1);2.6715(6.4); 2.6576(8.9);2.6433(4.6);2.5665(0.3);2.5246(5.7);2.5111(113.4);2 .5068(233.9);2.5023(316.0);2.4978(238.1); 2.4935(122.0);2.4151(9.7);2.3334(1.6);2.3290(2.1);2.3246(1.6);1.5067(2.8);1.4927( 7.9);1.4788(12.4);1.4653 (10.2);1.4520(4.4);1.3777(4.9);1.3648(5.2);0.1460(1.6);0.0080(14.3);-0.0002(382.2);-0.0084(18.7);-0.1497(1.7) |
| 76: $^1$H-NMR(400.0 MHz, $d_6$-DMSO): |

(fortgesetzt)

| Beispiel |
|---|
| δ= 8.2183(2.2);8.2141(2.2);7.6110(3.0);7.6036(3.1);7.5733(1.3);7.5674(1.2);7.5439(1.3);7.5379(1.3);7.4737 (3.1);7.4515(3.5);7 .1070(1.7);7.0996(1.7);7.0848(1.5);7.0774(1.5);5.1509(4.4);5.1463(4.5);4.2318(2.0);4.2181 (4.1);4.2043(2.1);3.4036(20.2);3.31 79(37.6);2.9756(1.8);2.9619(3.6);2.9482(1.8);2.6754(0.4);2.6707(0.5); 2.6662(0.4);2.5647(16.0);2.5239(1.5);2.5107(27.8);2.50 62(56.4);2.5017(77.1);2.4972(58.0);2.4929(28.6); 2.3328(0.3);2.3286(0.4);2.3239(0.3);0.0078(0.8);-0.0002(19.3);-0.0083(0.7) |
| 77: $^1$H-NMR(400.0 MHz, $d_6$-DMSO):<br>δ= 8.2715(1.2);8.2678(1.3);7.6359(1.6);7.6225(3.1);7.5935(0.6);7.5886(0.6);7.5553(1.3);7.4356(0.7);7.3054 (0.7);7.2827(1.0);7 .2567(1.0);7.2453(0.8);7.2371(0.8);7.2294(0.8);7.1110(0.5);7.1017(0.9);7.0934(0.5);7.0885 (0.5);7.0796(0.6);7.0710(0.4);5.233 3(2.4);4.5080(0.8);4.4966(1.5);4.4853(0.9);3.8146(0.4);3.8012(0.6);3.7666 (0.4);3.3245(89.8);2.8089(2.8);2.7966(2.8);2.6710(0 .7);2.5061(92.1);2.5018(120.1);2.4975(90.8);2.3287(0.7); 1.3635(16.0);1.3346(0.5);1.2859(0.3);1.2759(0.4);1.2607(0.4);0.1458 (0.5);0.0077(5.3);-0.0002(106.9);-0.1498 (0.5) |
| 78: $^1$H-NMR(400.0 MHz, $d_6$-DMSO):<br>δ= 8.2004(2.9);8.1956(3.0);7.7443(1.6);7.7249(2.2);7.6529(2.7);7.6153(1.9);7.5950(3.4);7.5751(1.8);7.5453 (1.6);7.5397(1.7);7 .5160(1.6);7.5101(1.6);7.4222(1.4);7.4024(3.1);7.3817(5.0);7.3760(4.2);7.3637(1.4);7.3052 (2.3);7.2858(1.6);7.0869(1.6);7.081 7(1.6);7.0669(1.4);7.0612(1.4);5.2364(5.6);5.2325(5.8);4.1597(2.0);4.1448 (4.2);4.1297(2.1);3.3225(106.7);2.7944(1.6);2.7797( 3.0);2.7653(1.6);2.6753(0.6);2.6711(0.8);2.6670(0.7); 2.5627(1.9);2.5452(5.4);2.5266(6.7);2.5062(112.4);2.5019(147.5);2.4976 (111.2);2.3286(0.8);2.3242(0.6); 1.2325(0.4);0.9755(8.0);0.9577(16.0);0.9399(7.7);0.1458(0.5);0.0072(4.4);-0.0003(99.7);-0.1499(0.5) |
| 79: $^1$H-NMR(400.0 MHz, $d_6$-DMSO):<br>δ= 8.1711(1.4);8.1657(1.4);7.6022(1.6);7.5798(1.8);7.5242(0.7);7.5183(0.8);7.4946(0.7);7.4888(0.8);7.2579 (1.1);7.2546(1.2);7 .1613(0.9);7.1541(0.9);7.1388(0.9);7.1316(0.8);5.2069(2.7);5.2029(2.9);4.0289(1.0);4.0124 (2.1);3.9958(1.1);3.3205(22.6);3.03 76(0.4);3.0214(0.9);3.0050(1.2);2.9888(0.9);2.9724(0.4);2.7935(0.9);2.7770 (1.7);2.7606(0.8);2.6711(0.3);2.5063(41.9);2.5019( 56.4);2.4975(44.0);2.3289(0.3);0.9756(16.0);0.9593(15.8); 0.0078(1.5);-0.0003(37.1) |
| 80: $^1$H-NMR(400.0 MHz, $d_6$-DMSO):<br>δ= 8.2373(2.4);8.2321(2.4);7.7484(1.6);7.7439(3.0);7.7398(2.0);7.6616(1.4);7.6424(1.8);7.5830(1.4);7.5771 (1.4);7.5535(1.4);7 .5476(1.4);7.5073(1.1);7.4876(2.6);7.4683(1.8);7.4396(1.9);7.4366(1.6);7.4193(1.0);7.4093 (1.2);7.3894(2.5);7.3689(2.9);7.363 0(2.6);7.3584(2.0);7.2887(1.8);7.2693(1.3);7.0715(1.3);7.0661(1.3);7.0512 (1.2);7.0457(1.1);5.2353(4.6);5.2313(4.9);4.2381(2. 1);4.2245(4.2);4.2107(2.2);3.4045(19.0);3.3176(23.4); 2.9795(2.0);2.9659(3.9);2.9522(2.0);2.6745(0.5);2.6700(0.6);2.6658(0.5) ;2.5658(16.0);2.5055(78.8);2.5011 (104.0);2.4968(79.7);2.3324(0.4);2.3279(0.6);2.3233(0.5);0.1457(0.3);0.0078(3.5);-0.0003(67.5) |
| 81: $^1$H-NMR(400.0 MHz, $d_6$-DMSO):<br>δ= 8.2834(2.7);8.2766(2.7);7.7355(1.4);7.7159(1.9);7.6390(2.4);7.6132(1.6);7.5931(2.8);7.5732(1.4);7.5016 (1.5);7.4803(3.4);7 .4499(2.1);7.4429(2.0);7.4284(1.0);7.4203(1.9);7.3998(2.6);7.3800(3.1);7.3526(2.2);7.3475 (2.7);7.3433(2.0);7.2960(2.0);7.276 4(1.4);7.0730(14);7.0680(1.4);7.0526(1.2);7.0476(1.2);5.1979(8.2);4.1121 (2.1);4.0970(4.4);4.0819(2.2);3.3227(32.6);2.8907(1 .5);2.7889(1.8);2.7739(3.5);2.7588(1.7);2.7314(1.5);2.6713 (0.4);2.5639(2.1);2.5462(6.0);2.5284(6.7);2.5064(52.7);2.5022(68.7 );2.4980(53.2);2.3292(0.4);0.9772(8.0); 0.9595(16.0);0.9417(7.6);-0.0002(39.7) |
| 82: $^1$H-NMR(400.0 MHz, $d_6$-DMSO):<br>δ= 8.2766(2.8);8.2700(2.7);7.6122(2.8);7.5412(0.6);7.5271(3.3);7.5096(3.5);7.4895(4.1);7.4827(1.5);7.4684 (4.0);7.4477(2.4);7 .4407(2.2);7.4262(0.9);7.4191(0.9);7.2820(1.3);7.2590(2.0);7.2565(1.8);7.2334(1.6);7.2005 (1.4);7.1928(1.6);7.1845(1.5);7.176 8(1.5);7.0853(0.9);7.0761(1.5);7.0676(1.0);7.0629(0.9);7.0538(1.2);7.0452 (0.7);5.1540(8.8);4.1121(2.1);4.0970(4.4);4.0819(2. 2);3.3226(30.0);2.8906(1.0);2.7875(1.8);2.7726(3.4);2.7575 (1.7);2.7313(1.0);2.6713(0.4);2.5635(2.0);2.5459(5.8);2.5280(6.6); 2.5065(54.7);2.5022(70.0);2.4978(53.0); 2.3287(0.4);0.9770(8.0);0.9593(16.0);0.9415(7.7);-0.0002(40.4) |
| 83: $^1$H-NMR(400.0 MHz, $d_6$-DMSO): |

(fortgesetzt)

| Beispiel |
|---|
| δ= 8.2217(2.1);8.2175(2.1);7.5758(1.2);7.5699(1.2);7.5464(1.2);7.5404(1.3);7.4155(1.4);7.4079(1.5);7.4014 (1.4);7.3937(1.4);7 .3305(1.3);7.3082(2.5);7.2864(1.6);7.0916(0.8);7.0821(1.2);7.0742(0.9);7.0688(0.8);7.0595 (0.9);7.0513(0.6);5.1463(4.4);5.141 6(4.6);4.2327(2.0);4.2190(4.1);4.2052(2.1);3.4037(19.8);3.3178(11.4); 2.9760(1.8);2.9623(3.6);2.9486(1.8);2.6706(0.3);2.5648( 16.0);2.5240(0.9);2.5103(20.0);2.5061(41.0);2.5016 (54.4);2.4971(39.9);2.4928(19.9);0.0078(1.5);-0.0002(42.3);-0.0084(1.8) |
| 84: $^1$H-NMR(400.0 MHz, $d_6$-DMSO):<br>δ= 8.1977(7.8);8.1938(7.7);7.6042(10.6);7.5817(11.8);7.5549(4.6);7.5490(4.5);7.5253(4.6);7.5193(4.6);7.2610 (6.1);7.2574(6.5 );7.2544(5.5);7.1643(6.2);7.1571(5.4);7.1418(5.5);7.1346(5.0);5.2091(15.5);5.2046(16.0); 4.2008(7.0);4.1863(14.8);4.1718(7.2) ;3.3223(177.0);2.6673(7.6);2.6530(13.7);2.6385(6.5);2.5246(4.1);2.5111 (79.8);2.5068(161.4);2.5022(214.8);2.4977(159.4);2.4 934(79.6);2.4113(9.9);2.3337(1.1);2.3291(1.4);2.3245 (1.1);1.5042(2.7);1.4897(7.6);1.4760(12.3);1.4626(10.2);1.4493(4.4);1.4 272(0.4);1.3916(2.4);1.3763(4.6); 1.3636(4.8);1.3499(2.7);0.1459(1.2);0.0079(10.9);-0.0002(277.7);-0.0085(12.2);-0.1498(1.2) |
| 85: $^1$H-NMR(400.0 MHz, $d_6$-DMSO):<br>δ= 8.3166(1.8);8.2379(2.1);8.2338(2.1);7.5832(1.2);7.5773(1.2);7.5525(2.1);7.5473(3.0);7.5282(3.0);7.5249 (2.7);7.5038(1.1);7 .4878(1.0);7.4686(0.4);7.4097(1.1);7.3899(2.3);7.3696(2.7);7.3638(2.2);7.3587(1.7);7.3001 (1.6);7.2799(1.1);7.2239(0.4);7.218 8(0.7);7.2136(0.5);7.1990(1.0);7.1949(0.8);7.1817(0.4);7.1784(0.5);7.1728 (0.4);7.0731(1.1);7.0670(1.2);7.0528(1.0);7.0482(1. 0);5.2311(4.0);5.2267(4.2);4.2379(1.9);4.2242(3.9);4.2104 (2.0);3.4049(20.4);3.3190(13.0);3.2952(0.8);2.9795(1.8);2.9659(3.6) ;2.9522(1.8);2.6747(0.4);2.6704(0.5); 2.6660(0.4);2.5660(16.0);2.5235(1.3);2.5101(31.1);2.5058(63.5);2.5013(84.4);2.4968(62. 1);2.4925(31.1); 2.3325(0.4);2.3280(0.5);2.3237(0.4);1.4862(0.6);0.0080(2.1);-0.0002(56.0);-0.0084(2.5) |
| 86: $^1$H-NMR(400.0 MHz, $d_6$-DMSO):<br>δ= 8.2255(2.6);8.2199(2.6);7.5717(1.2);7.5659(1.3);7.5421(1.2);7.5366(1.3);7.4518(2.9);7.4301(3.2);6.7893 (2.3);6.7828(2.6);6 .6318(1.6);6.6253(1.5);6.6099(1.5);6.6035(1.5);5.1457(5.1);5.1187(0.4);4.2330(2.1);4.2194 (4.2);4.2058(2.2);3.8184(15.2);3.80 64(1.0);3.7621(0.8);3.4049(19.3);3.3750(143.9);3.3649(121.2);3.3631 (127.5);3.3602(136.8);3.3574(126.4);2.9777(2.0);2.9642( 4.2);2.9503(2.3);2.6734(0.4);2.5661(16.0);2.5395 (0.4);2.5260(1.6);2.5083(53.7);2.5040(71.4);2.4996(52.8);2.3304(0.4);1.0552 (0.9);-0.0002(40.5) |
| 87: $^1$H-NMR(400.0 MHz, $d_6$-DMSO):<br>δ= 8.2104(8.0);8.2063(8.1);7.7440(4.4);7.7246(6.0);7.6544(7.4);7.6153(5.3);7.5951(9.6);7.5752(5.0);7.5527 (4.4);7.5470(4.5);7 .5231(4.4);7.5173(4.5);7.4217(3.9);7.4019(8.7);7.3815(14.2);7.3760(11.7);7.3635(3.8); 7.3606(3.8);7.3053(6.2);7.2851(4.3);7.0 861(4.4);7.0817(4.2);7.0659(3.9);7.0612(3.8);5.2367(15.1);5.2327 (16.0);4.2063(5.2);4.1919(10.4);4.1776(5.4);3.3214(118.9);2.6713(5.4);2.6596(7.8);2.6452(4.2);2.5106(94.3); 2.5064(191.6);2.5020(257.6);2.4975(197.0);2.4161(9.2);2.3331(1.3);2.3287(1. 7);2.3242(1.3);1.5081(2.7); 1.4941(7.6);1.4806(11.7);1.4674(9.7);1.4542(4.2);1.3783(4.7);1.3656(5.0);0.1457(1.3);0.0078(12.2) ;-0.0003 (308.0);-0.0085(16.0);-0.1498(1.4) |
| 88: $^1$H-NMR(400.0 MHz, $d_6$-DMSO):<br>δ= 8.2751(2.4);8.2685(2.4);7.5974(3.0);7.5749(3.4);7.4882(1.2);7.4669(3.4);7.4469(2.2);7.4399(2.1);7.4254 (0.8);7.4183(0.8);7 .2310(1.9);7.2276(2.0);7.1410(1.8);7.1338(1.5);7.1185(1.6);7.1113(1.4);5.1533(8.5);4.1123 (2.1);4.0971(44);4.0820(2.2);3.324 6(26.5);2.7864(1.7);2.7714(3.3);2.7562(1.6);2.5636(1.9);2.5460(5.6);2.5282 (6.1);2.5076(29.1);2.5031(37.3);2.4987(27.5);0.97 72(8.0);0.9595(16.0);0.9417(7.6);0.0079(1.0);-0.0002(22.5);- 0.0084(1.0) |
| 89: $^1$H-NMR(400.0 MHz, $d_6$-DMSO):<br>δ= 8.2208(2.9);8.2174(2.8);7.5806(1.5);7.5750(1.4);7.5509(1.5);7.5456(1.4);7.4655(1.2);7.4412(2.2);7.4170 (1.4 );7.2678(1.3);7 .2568(1.3);7.1466(1.0);7.1380(1.5);7.1297(1.0);7.1239(0.9);7.1151(1.2);7.1067(0.6);5.1735 (5.8);5.1702(5.8);4.2314(2.4);4.217 9(4.6);4.2042(2.4);3.4015(18.5);3.3203(19.6);2.9745(2.4);2.9612(4.3); 2.9476(2.2);2.6719(0.4);2.5639(16.0);2.5022(79.0);2.32 89(0.5);2.1396(0.5);-0.0003(5.8) |
| 90: $^1$H-NMR(400.0 MHz, $CDCl_3$): |

(fortgesetzt)

| Beispiel |
|---|
| δ= 7.9991(2.4);7.9934(2.7);7.9143(1.4);7.8951(1.5);7.6111(2.2);7.6049(2.5);7.5409(1.3);7.5202(2.1);7.4563 (2.1);7.4506(1.1);7 .4471(1.0);7.4336(3.0);7.4295(2.1);7.4115(0.8);7.4083(0.8);7.3311(1.0);7.3289(1.0);7.3104 (1.6);7.2936(0.7);7.2916(0.7);7.260 5(8.3);7.1897(1.4);7.1832(1.5);7.1673(1.2);7.1609(1.3);6.8950(2.2);6.8894 (2.5);5.2982(2.7);5.2508(8.2);4.2445(1.3);4.2311(2. 5);4.2178(1.4);3.8825(14.1);3.5365(16.0);3.0517(1.8); 3.0383(3.5);3.0248(1.8);2.6834(13.9);1.5841(6.1);-0.0002(7.8) |
| 91: $^{1}$H-NMR(400.0 MHz, $CDCl_3$):<br>δ= 8.3169(1.0);8.3156(1.1);8.2959(1.1);8.2945(1.1);8.1787(1.1);8.1576(1.2);7.9816(2.2);7.9758(2.3);7.5960 (0.6);7.5928(0.6);7 .5789(0.8);7.5754(1.2);7.5717(0.6);7.5578(0.8);7.5545(0.8);7.4901(0.8);7.4872(0.8);7.4730 (0.6);7.4695(1.1);7.4661(0.9);7.456 1(2.2);7.4523(0.8);7.4491(0.6);7.4354(2.3);7.2608(8.4);6.9779(2.0);6.9572 (1.8);6.8945(1.9);6.8887(1.9);5.3119(6.8);4.2513(1. 0);4.2379(1.9);4.2244(1.1);3.8448(12.9);3.5443(16.0); 3.0581(1.5);3.0446(3.0);3.0310(1.6);2.6982(0.4);2.6885(13.3);1.5994(3. 0);-0.0002(7.9) |
| 92: $^{1}$H-NMR(400.0 MHz, $d_6$-DMSO):<br>δ= 8.1830(0.9);8.1788(0.8);7.6034(1.1);7.5809(1.2);7.5353(0.5);7.5295(0.5);7.5056(0.5);7.5001(0.5);7.2616 (0.7);7.2581(0.7);7 .1631(0.6);7.1560(0.6);7.1406(0.6);7.1335(0.5);5.2085(1.7);5.2043(1.8);4.1146(0.7);4.0992 (1.5);4.0837(0.7);3.3229(34.3);2.72 33(0.6);2.7082(1.1);2.6925(0.5);2.5064(31.1);2.5020(41.1);2.4975(30.7); 2.2194(4.2);1.0004(16.0);0.0079(1.0);-0.0002(25.2);-0.0083(1.2) |
| 93: $^{1}$H-NMR(400.0 MHz, $CDCl_3$):<br>δ= 8.1801(1.7);8.1575(1.8);7.9828(2.4);7.9770(2.5);7.8378(1.5);7.8162(1.7);7.5315(1.2);7.5115(1.7);7.4905 (1.2);7.4211(2.6);7 ,3984(2.4);7.2607(7,7);7.1157(1.7);7.0963(1.6);6.8980(2.2);6.8923(2.2);5.3209(7.6);5.2984 (0.5);4.2530(1.2);4.2397(2.4);4.226 3(1.4);3.8462(13.9);3.5441(16.0);3.5306(0.4);3.0593(1.8);3.0458(3.4); 3.0323(1.8);2.6891(14.0);1.6428(0.5);1.2543(0.4);-0.0002(7.4) |
| 94: $^{1}$H-NMR(400.0 MHz, $CDCl_3$):<br>δ= 7.9898(2.3);7.9840(2.4);7.5949(1.7);7.5915(2.3);7.5738(2.5);7.5722(2.4);7.5712(2.4);7.4384(1.4);7.4341 (0.6);7.4203(2.8);7 .4172(1.6);7.4008(1.6);7.3563(1.0);7.3514(0.7);7.3482(1.2);7.3451(0.7);7.3365(2.1);7.3298 (1.5);7.3247(0.5);7.3165(1.5);7.311 7(0.6);7.3084(0,4);7.3032(1.2);7.2978(1.7);7.2931(1.4);7.2593(7.8);7.1876 (0.9);7.1854(1.2);7.1840(1.2);7.1815(1.0);7.1686(0. 8);7.1663(1.0);7.1647(1.0);7.1625(0.8);7.0589(0.8);7.0569 (0.9);7.0526(0.9);7.0506(0.9);7.0384(0.8);7.0364(0.8);7.0321(0.8);7.0300(0.8);6.8786(2.1);6.8728(2.1);5.2129 (7.5);4.2383(1.0);4.2250(2.1);4.2115(1.2);3.8548(13.3);3.5347(16.0);3.0481(1.6);3.0 346(3.1);3.0212(1.6); 2.6817(13.6);2.0037(2.6);1.5799(4.1);-0.0002(7.1) |
| 95: $^{1}$H-NMR(400.0 MHz, $d_6$-DMSO):<br>δ= 8.2213(2.3);8.2162(2.4);7.5729(1.3);7.5670(1.4);7.5434(1.3);7.5376(1.4);7.4138(2.7);7.3248(1.5);7.3115 (1.0);7.3074(1.8);7 .1053(0.8);7.0844(2.4);7.0670(3.4);7.0630(2.8);7.0463(0.4);7.0411(0.6);5.1368(4.8);5.1327 (5.2);4.2328(2.1);4.2191(4.3);4.205 4(2.2);3.4046(18.8);3.3177(23.1);2.9764(2.0);2.9629(3.9);2.9492(2.0); 2.6746(0.4);2.6703(0.5);2.5652(16.0);2.5057(60.7);2.50 14(80.9);2.4972(62.3);2.3319(0.3);2.3281(0.5);0.0077 (2.4);-0.0002(54.6) |
| 96: $^{1}$H-NMR(400.0 MHz, $d_6$-DMSO):<br>δ= 8.2540(1.6);8.2315(1.8);7.7973(1.2);7.7758(1.8);7.7004(2.3);7.6854(1.2);7.6777(2.2);7.6654(1.7);7.6445 (0.9);7.3819(1.6);7 .3620(1.9);7.2945(1.6);7.2753(1.4);7.1674(1.8);7.1476(1.6);5.7569(16.0);5.2505(5.2);4.1336 (1.4);4.1193(3.0);4.1050(1.5);3.87 85(11.7);3.4033(13.2);3.3195(26.2);2.9761(1.4);2.9619(2.8);2.9477(1.4); 2.6709(0.6);2.5695(11.2);2.5061(74.3);2.5018(96.5);2 .4976(71.2);2.3285(0.6);1.2335(0.6);0.1462(0.6);0.0073 (6.2);-0.0001(126.0);-0.1496(0.6) |
| 97: $^{1}$H-NMR(400.0 MHz, $d_6$-DMSO):<br>δ= 9.9594(0.4);8.2457(2.4);8.2230(2.6);7.7922(1.9);7.7708(2.7);7.6965(3.2);7.6791(1.9);7.6739(3.3);7.6593 (2.4);7.6384(1.3);7 .3698(2.4);7.3498(2.8);7.2878(2.3);7.2684(2.1);7.1449(2.7);7.1251(2.3);5.2364(7.8);4.5171 (0.3);4.3665(1.3);4.3490(4.1);4.331 5(4.2);4.3139(1.4);4.1371(2.1);4.1226(4.5);4.1080(2.3);3.4096(18.6);3.4001 (2.0);3.3204(31.9);2.9799(2.3);2.9655(4.6);2.9511( 2.3);2.6709(0.6);2.5723(16.0);2.5558(1.6);2.5058(75.0); 2.5019(95.4);2.4980(72.3);2.3286(0.5);1.3184(4.2);1.3009(8.7);1.2834 (4.2);1.2308(0.6);0.1457(0.5);-0.0004 (99.6);-0.1499(0.5) |
| 98: $^{1}$H-NMR(400.0 MHz, $d_6$-DMSO): |

(fortgesetzt)

| Beispiel |
|---|
| δ= 8.8265(10.2);8.2428(1.7);8.2224(1.9);8.1369(1.8);8.1159(2.0);7.7393(0.9);7.7217(16);7.7036(1.1);7.6554 (2.7);7.6350(3.9); 7.6179(1.7);7.6001(0.9);7.1752(2.6);7.1544(2.4);5.7563(0.6);5.2934(7.3);4.4866(2.3);4.4722 (4.7);4.4579(2.4);3.9492(1.2);3.41 05(18.4);3.3537(0.5);3.3216(84.3);2.9878(2.0);2.9736(4.1);2.9592(2.0); 2.6713(0.4);2.5655(16.0);2.5067(54.7);2.5023(69.9);2. 4980(50.8);2.3293(0.4);0.0080(0.9);0.0000(19.5) |
| 99: $^1$H-NMR(400.0 MHz, $d_6$-DMSO):<br>δ= 8.6301(12.6);8.3049(1.5);8.2842(1.6);8.1276(1.6);8.1072(1.8);7.7419(0.8);7.7388(0.8);7.7247(1.2);7.7214 (1.6);7.7178(0.8); 7.7037(1.1);7.7005(1.0);7.6548(1.1);7.6520(1.1);7.6342(1.6);7.6311(1.1);7.6166(0.8);7.6138 (0.7);7.5759(2.8);7.5551(3.0);7.02 97(2.6);7.0087(2.4);5.4358(8.1);4.2925(2.0);4.2788(4.2);4.2650(2.1);3.3999 (20.0);3.3213(5.2);2.9859(1.8);2.9723(3.7);2.9586( 1.8);2.5655(16.0);2.5251(0.7);2.5202(1.1);2.5115(15.9); 2.5071(33.7);2.5025(47.0);2.4980(34.8);2.4937(16.6);0.0080(0.3);-0.0002(10.8);-0.0085(0.4) |

**Biologische** Beispiele

Ctenocephalides felis - in-vitro Kontakttests mit adulten Katzenflöhen

Für die Beschichtung der Teströhrchen werden zunächst 9 mg Wirkstoff in 1 ml Aceton p.a. gelöst und anschließend mit Aceton p.a. auf die gewünschte Konzentration verdünnt. 250 µl der Lösung werden durch Drehen und Kippen auf einem Rotationsschüttler (2 h Schaukelrotation bei 30 rpm) homogen auf den Innenwänden und dem Boden eines 25ml Glasröhrchens verteilt. Bei 900 ppm Wirkstofflösung und 44,7 $cm^2$ Innenoberfläche wird bei homogener Verteilung eine Flächendosis von 5 $\mu g/cm^2$ erreicht.

Nach Abdampfen des Lösungsmittels werden die Gläschen mit 5-10 adulten Katzenflöhen (*Ctenocephalides felis*) besetzt, mit einem gelochten Kunststoffdeckel verschlossen und liegend bei Raumtemperatur und Umgebungsfeuchte inkubiert. Nach 48 h wird die Wirksamkeit bestimmt. Hierzu werden die Gläschen aufrecht gestellt und die Flöhe auf den Boden des Gläschens geklopft. Flöhe, die unbeweglich auf dem Boden verbleiben oder sich unkoordiniert bewegen, gelten als tot bzw. angeschlagen.

Eine Substanz zeigt gute Wirkung gegen *Ctenocephalides felis*, wenn in diesem Test bei einer Aufwandmenge von 5 $\mu g/cm^2$ mindestens 80% Wirkung erzielt wurde. Dabei bedeutet 100% Wirkung, dass alle Flöhe angeschlagen oder tot waren. 0% Wirkung bedeutet, dass keine Flöhe geschädigt wurden.

Bei diesem Test zeigen z.B. die folgenden Verbindungen der Herstellungsbeispiele eine Wirkung von 100% bei einer Aufwandmenge von 5 $\mu g/cm^2$ (=500g/ha): 1, 2, 9,13, 16, 19, 20, 21, 22, 23, 24, 32, 37, 39, 44, 48, 49, 53, 54, 55, 56, 57, 59, 60, 61, 62, 63, 64, 65, 68, 69, 70, 71, 72, 75, 76, 78, 86, 89, 99

**Rhipicephalus sanguineus - in-vitro Kontakttests mit Adulten der braunen Hundezecke**

Für die Beschichtung der Teströhrchen werden zunächst 9 mg Wirkstoff in 1 ml Aceton p.a. gelöst und anschließend mit Aceton p.a. auf die gewünschte Konzentration verdünnt. 250 µl der Lösung werden durch Drehen und Kippen auf einem Rotationsschüttler (2 h Schaukelrotation bei 30 rpm) homogen auf den Innenwänden und dem Boden eines 25ml Glasröhrchens verteilt. Bei 900 ppm Wirkstofflösung und 44,7 $cm^2$ Innenoberfläche wird bei homogener Verteilung eine Flächendosis von 5 $\mu g/cm^2$ erreicht.

Nach Abdampfen des Lösungsmittels werden die Gläschen mit 5-10 adulten Hundezecken *(Rhipicephalus sanguineus)* besetzt, mit einem gelochten Kunststoffdeckel verschlossen und liegend im Dunkeln bei Raumtemperatur und Umgebungsfeuchte inkubiert. Nach 48 h wird die Wirksamkeit bestimmt. Hierzu werden die Zecken auf den Boden des Gläschens geklopft und auf einer Wärmeplatte bei 45-50°C maximal 5 min. inkubiert. Zecken, die unbeweglich auf dem Boden verbleiben oder sich so unkoordiniert bewegen, dass sie nicht gezielt der Wärme durch nach oben klettern ausweichen können, gelten als tot bzw. angeschlagen.

Eine Substanz zeigt gute Wirkung gegen *Rhipicephalus sanguineus*, wenn in diesem Test bei einer Aufwandmenge von 5 $\mu g/cm^2$ mindestens 80% Wirkung erzielt wurde. Dabei bedeutet 100% Wirkung, dass alle Zecken angeschlagen oder tot waren. 0% Wirkung bedeutet, dass keine Zecken geschädigt wurden.

Bei diesem Test zeigen z.B. die folgenden Verbindungen der Herstellungsbeispiele eine Wirkung von 100% bei einer Aufwandmenge von 5 $\mu g/cm^2$ (=500g/ha): 1, 2, 9, 13, 16, 19, 20, 21, 22, 23, 24, 27, 28, 31, 48, 49, 53, 54, 55, 56, 57, 59, 60, 61, 62, 63, 64, 65, 68, 69, 70, 71, 72, 75, 76, 78, 80, 86, 89, 99

**Boophilus microplus - Diptest**

| | |
|---|---|
| Testtiere: | Rinderzecken (*Boophilus microplus)* Stamm Parkhurst,SP-resistent |
| Lösungsmittel: | Dimethylsulfoxid |

10 mg Wirkstoff werden in 0,5 ml Dimethylsulfoxid gelöst. Zwecks Herstellung einer geeigneten Formulierung verdünnt man die Wirkstofflösung mit Wasser auf die jeweils gewünschte Konzentration.

Diese Wirkstoffzubereitung wird in Röhrchen pipettiert. 8-10 gesogene, adulte, weibliche Rinderzecken (*Boophilus microplus*) werden in ein weiteres Röhrchen mit Löchern überführt. Das Röhrchen wird in die Wirkstoffzubereitung getaucht wobei alle Zecken vollständig benetzt werden. Nach Ablaufen der Flüssigkeit werden die Zecken auf Filterscheiben in Kunststoffschalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigen z.B. die folgenden Verbindungen der Herstellungsbeispiele eine Wirkung von 100% bei einer Aufwandmenge von 100ppm: 19, 54, 86, 99

Bei diesem Test zeigen z.B. die folgenden Verbindungen der Herstellungsbeispiele eine Wirkung von 95% bei einer Aufwandmenge von 100ppm: 16, 58

Bei diesem Test zeigen z.B. die folgenden Verbindungen der Herstellungsbeispiele eine Wirkung von 90% bei einer Aufwandmenge von 100ppm: 55

Bei diesem Test zeigen z.B. die folgenden Verbindungen der Herstellungsbeispiele eine Wirkung von 80% bei einer Aufwandmenge von 100ppm: 23

**Boophilus microplus -Injektionstest**

Lösungsmittel: Dimethylsulfoxid

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration.

1μl der Wirkstofflösung wird in das Abdomen von 5 vollgesogenen, adulten, weiblichen Rinderzecken *(Boophilus microplus)* injiziert. Die Tiere werden in Schalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigen z.B. die folgenden Verbindungen der Herstellungsbeispiele eine Wirkung von 100% bei einer Aufwandmenge von 20μg/Tier: 1, 2, 4, 5, 13, 16, 17, 18, 19, 20, 21, 22, 23, 24, 25, 27, 28, 29, 30, 31, 32, 36, 38, 44, 47, 48, 49, 53, 54, 55, 56, 58, 59, 60, 61, 63, 64, 65, 68, 70, 71, 72, 76, 80, 86, 89, 90, 91, 93, 99

Bei diesem Test zeigen z.B. die folgenden Verbindungen der Herstellungsbeispiele eine Wirkung von 95% bei einer Aufwandmenge von 20μg/Tier: 57

Bei diesem Test zeigen z.B. die folgenden Verbindungen der Herstellungsbeispiele eine Wirkung von 90% bei einer Aufwandmenge von 20μg/Tier: 9, 39

Bei diesem Test zeigen z.B. die folgenden Verbindungen der Herstellungsbeispiele eine Wirkung von 85% bei einer Aufwandmenge von 20μg/Tier: 69

Bei diesem Test zeigen z.B. die folgenden Verbindungen der Herstellungsbeispiele eine Wirkung von 80% bei einer

Aufwandmenge von 20µg/Tier: 33, 34, 37, 73, 81, 83, 94

Ctenocephalides felis - Oraltest

Lösungsmittel: Dimethylsulfoxid

Zwecks Herstellung einer zweckmäßigen Wirkstoffzubereitung vermischt man 10 mg Wirkstoff mit 0,5 ml Dimethylsulfoxid. Durch Verdünnen mit citriertem Rinderblut erhält man die gewünschte Konzentration.

Ca. 20 nüchterne adulte Katzenflöhe (*Ctenocephalides felis*) werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann.

Nach 2 Tagen wird die Abtötung in % bestimmt. Dabei bedeutet 100 %, dass alle Flöhe abgetötet wurden; 0 % bedeutet, dass keiner der Flöhe abgetötet wurde.

Bei diesem Test zeigen z.B. die folgenden Verbindungen der Herstellungsbeispiele eine Wirkung von 100% bei einer Aufwandmenge von 100ppm: 4, 29

Bei diesem Test zeigen z.B. die folgenden Verbindungen der Herstellungsbeispiele eine Wirkung von 95% bei einer Aufwandmenge von 100ppm: 20, 63, 68, 72

Bei diesem Test zeigen z.B. die folgenden Verbindungen der Herstellungsbeispiele eine Wirkung von 90% bei einer Aufwandmenge von 100ppm: 22, 55, 61

Bei diesem Test zeigen z.B. die folgenden Verbindungen der Herstellungsbeispiele eine Wirkung von 80% bei einer Aufwandmenge von 100ppm: 2, 19, 21, 81

**Lucilia cuprina - Test**

Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Ca. 20 L1-Larven der Australischen Schafgoldfliege (*Lucilia cuprina*) werden in ein Testgefäß überführt, welches gehacktes Pferdefleisch und die Wirkstoffzubereitung der gewünschten Konzentration enthält.

Nach 2 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 100% bei einer Aufwandmenge von 100ppm: 1, 2, 4, 5, 16, 20, 21, 22, 23, 28, 29, 31, 48, 49, 53, 54, 55, 56, 59, 61, 63, 68, 70, 71, 72, 80, 93, 99

Bei diesem Test zeigen z.B. die folgenden Verbindungen der Herstellungsbeispiele eine Wirkung von 98% bei einer Aufwandmenge von 100ppm: 19

Bei diesem Test zeigen z.B. die folgenden Verbindungen der Herstellungsbeispiele eine Wirkung von 90% bei einer Aufwandmenge von 100ppm: 65, 77

Bei diesem Test zeigen z.B. die folgenden Verbindungen der Herstellungsbeispiele eine Wirkung von 80% bei einer Aufwandmenge von 100ppm: 38, 90, 91

Musca domestica-Test

Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.
Gefäße, die einen Schwamm enthalten, der mit Zuckerlösung und der Wirkstoffzubereitung der gewünschten Konzentration behandelt wurde, werden mit 10 adulten Stubenfliegen *(Musca domestica)* besetzt.
Nach 2 Tagen wird die Abtötung in % bestimmt. Dabei bedeutet 100 %, dass alle Fliegen abgetötet wurden; 0 % bedeutet, dass keine der Fliegen abgetötet wurde.
Bei diesem Test zeigen z.B. die folgenden Verbindungen der Herstellungsbeispiele eine Wirkung von 80% bei einer Aufwandmenge von 100ppm: 4
**Meloidogyne incognita- Test**

Lösungsmittel: 125,0 Gewichtsteile Aceton

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.
Gefäße werden mit Sand, Wirkstofflösung, einer Ei-Larven-Suspension des südlichen Wurzelgallenälchens *(Meloidogyne incognita)* und Salatsamen gefüllt. Die Salatsamen keimen und die Pflänzchen entwickeln sich. An den Wurzeln entwickeln sich die Gallen.
Nach 14 Tagen wird die nematizide Wirkung anhand der Gallenbildung in % bestimmt. Dabei bedeutet 100 %, dass keine Gallen gefunden wurden; 0 % bedeutet, dass die Zahl der Gallen an den behandelten Pflanzen der unbehandelten Kontrolle entspricht.
Bei diesem Test zeigen z. B. die folgenden Verbindungen der Herstellungsbeispiele Wirkung von 90% bei einer Aufwandmenge von 20ppm: 4

Myzus persicae - Sprühtest

| | |
|---|---|
| Lösungsmittel: | 78 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.
Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.
Nach 5 Tagen wird die Wirkung in % bestimmt. Dabei bedeutet 100 %, dass alle Blattläuse abgetötet wurden; 0 % bedeutet, dass keine Blattläuse abgetötet wurden.
Bei diesem Test zeigen z. B. die folgenden Verbindungen der Herstellungsbeispiele Wirkung von 90% bei einer Aufwandmenge von 500g/ha: 10, 48, 50, 53, 59
Bei diesem Test zeigen z. B. die folgenden Verbindungen der Herstellungsbeispiele Wirkung von 90% bei einer Aufwandmenge von 100g/ha: 39

**Nezara viridula -Sprühtest**

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.
Gerstenpflanzen (*Hordeum vulgare*), die mit Larven der Grünen Reiswanze (*Nezara viridula*) infiziert sind, werden mit einer Wirkstoffzubereitung der gewünschten Konzentration gespritzt.
Nach 4 Tagen wird die Wirkung in % bestimmt. Dabei bedeutet 100 %, dass alle Reiswanzen abgetötet wurden; 0 % bedeutet, dass keine Reiswanzen abgetötet wurden.
Bei diesem Test zeigen z. B. die folgenden Verbindungen der Herstellungsbeispiele Wirkung von 100% bei einer Aufwandmenge von 500g/ha: 50

**Nilaparvata lugens -Test**

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.
Reispflanzen *(Oryza sativa)* werden mit der Wirkstoffzubereitung der gewünschten Konzentration gespritzt und anschließend mit der Braunrückigen Reiszikade (*Nilaparvata lugens*) infiziert.
Nach 4 Tagen wird die Wirkung in % bestimmt. Dabei bedeutet 100 %, dass alle Reiszikaden abgetötet wurden; 0 % bedeutet, dass keine Reiszikaden abgetötet wurden.
Bei diesem Test zeigen z. B. die folgenden Verbindungen der Herstellungsbeispiele Wirkung von 100% bei einer Aufwandmenge von 500g/ha: 20, 50, 53
Bei diesem Test zeigen z. B. die folgenden Verbindungen der Herstellungsbeispiele Wirkung von 90% bei einer Aufwandmenge von 500g/ha: 22

**Phaedon cochleariae - Sprühtest**

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.
Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.
Nach 7 Tagen wird die Wirkung in % bestimmt. Dabei bedeutet 100 %, dass alle Käferlarven abgetötet wurden; 0 % bedeutet, dass keine Käferlarven abgetötet wurden.
Bei diesem Test zeigen z. B. die folgenden Verbindungen der Herstellungsbeispiele Wirkung von 100% bei einer Aufwandmenge von 500g/ha: 1, 2, 4, 5, 8, 10, 11, 13, 19, 20, 21, 22, 23, 27, 29, 31, 33, 34, 36, 37, 38, 39, 40, 43, 44, 45, 47, 48, 49, 50, 52, 53, 54, 56, 57, 58, 60, 62, 64, 66, 69, 70, 76, 79, 80, 98, 99
Bei diesem Test zeigen z. B. die folgenden Verbindungen der Herstellungsbeispiele Wirkung von 83% bei einer Aufwandmenge von 500g/ha: 9, 16, 30, 35, 51, 55, 59, 67, 71, 77, 82, 91
Bei diesem Test zeigen z. B. die folgenden Verbindungen der Herstellungsbeispiele Wirkung von 100% bei einer Aufwandmenge von 100g/ha: 63

**Spodoptera frugiperda - Sprühtest**

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von 100% bei einer Aufwandmenge von 500g/ha: 1, 2, 4, 8, 9, 10, 11, 12, 18, 19, 20, 21, 22, 23, 25, 27, 29, 31, 32, 33, 34, 36, 37, 38, 39, 42, 44, 45, 47, 48, 49, 50, 51, 52, 53, 54, 55, 56, 57, 58, 59, 60, 61, 62, 63, 65, 66, 67, 68, 71, 74, 77, 78, 81, 99

Bei diesem Test zeigen z. B. die folgenden Verbindungen der Herstellungsbeispiele Wirkung von 83% bei einer Aufwandmenge von 500g/ha: 5, 16, 24, 46, 64, 69, 72, 75, 80, 85, 86, 90

**Tetranychus urticae - Sprühtest, OP-resistent**

| | |
|---|---|
| Lösungsmittel: | 78,0 GewichtsteileAceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 100% bei einer Aufwandmenge von 500g/ha: 1, 7, 10, 11, 12, 30, 48, 49, 50, 51, 53, 54, 55, 56, 58, 59, 63, 65, 72, 73, 83, 84, 89

Bei diesem Test zeigen z. B. die folgenden Verbindungen der Herstellungsbeispiele eine Wirkung von 90% bei einer Aufwandmenge von 500g/ha: 2, 13, 21, 23, 27, 32, 34, 41, 47, 61, 62, 64, 68, 70, 75, 87

Bei diesem Test zeigen z. B. die folgenden Verbindungen der Herstellungsbeispiele eine Wirkung von 90% bei einer Aufwandmenge von 100g/ha: 44, 76

**Anopheles Test (ANPHGB Oberflächenbehandlung)**

Lösungsmittel: Aceton + 2000 ppm Rapsölmethylester (RME)

Zur Herstellung einer zweckmäßigen Wirkstoffzubereitung löst man den Wirkstoff im Lösungsmittel (2mg/ml). Die Wirkstoffzubereitung wird auf eine glasierte Kachel pipettiert und nach Abtrocknen werden adulte Mücken der Spezies Anopheles gambiae Stamm RSPH (homozygot kdr) auf die behandelte Kachel gesetzt. Die Expositionszeit beträgt 30 Minuten.

24 Stunden nach Kontakt zur behandelten Oberfläche wird die Mortalität in % bestimmt. Dabei bedeutet 100%, dass alle Mücken abgetötet wurden; 0% bedeutet, dass keine Mücken abgetötet wurden.

Bei diesem Test zeigen z.B. die folgenden Verbindungen der Herstellungsbeispiele Wirkung von 80-100% bei

einer Aufwandmenge von 100 mg/m$^2$: 1, 2, 3, 5, 6, 11, 16, 17, 18, 19, 20, 21, 22, 23, 24, 34, 53, 70, 76, 90, 95, 98,

Bei diesem Test zeigen z.B. die folgenden Verbindungen der Herstellungsbeispiele Wirkung von 80-100% bei einer Aufwandmenge von 20 mg/m$^2$: 1, 2, 3, 5, 8, 10, 12, 13, 16, 17, 18, 19, 20, 21, 22, 23, 24, 25, 26, 28, 29, 30, 31, 48, 49, 50, 53, 54, 55, 56, 58, 59, 64, 65, 68, 70, 71, 72, 73, 76, 80, 85, 89, 93, 95, 96, 98, 99

**Anopheles Test (ANPHFU Oberflächenbehandlung)**

Lösungsmittel: Aceton + 2000 ppm Rapsölmethylester (RME)

Zur Herstellung einer zweckmäßigen Wirkstoffzubereitung löst man den Wirkstoff im Lösungsmittel (2mg/ml). Die Wirkstoffzubereitung wird auf eine glasierte Kachel pipettiert und nach Abtrocknen werden adulte Mücken der Spezies Anopheles funestus Stamm FUMOZ-R (Hunt et al., Med Vet Entomol. 2005 Sep; 19(3):271-5) auf die behandelte Kachel gesetzt. Die Expositionszeit beträgt 30 Minuten.

24 Stunden nach Kontakt zur behandelten Oberfläche wird die Mortalität in % bestimmt. Dabei bedeutet 100%, dass alle Mücken abgetötet wurden; 0% bedeutet, dass keine Mücken abgetötet wurden.

Bei diesem Test zeigen z.B. die folgenden Verbindungen der Herstellungsbeispiele Wirkung von 80-100% bei einer Aufwandmenge von 100 mg/m$^2$:1, 2, 3, 6, 15, 18, 19, 20, 21, 22, 23, 24, 53, 70, 76, 90,

Bei diesem Test zeigen z.B. die folgenden Verbindungen der Herstellungsbeispiele Wirkung von 80-100% bei einer Aufwandmenge von 20 mg/m$^2$:1, 2, 3, 5, 10, 18, 19, 20, 21, 22, 23, 25, 28, 30, 31, 48, 49, 50, 53, 54, 55, 56, 58, 59, 64, 65, 68, 70, 71, 72, 73, 76, 80, 85, 86, 89, 96, 99

**Aedes Test (AEDSAE Oberflächenbehandlung)**

Lösungsmittel: Aceton + 2000 ppm Rapsölmethylester (RME)

Zur Herstellung einer zweckmäßigen Wirkstoffzubereitung löst man den Wirkstoff im Lösungsmittel (2mg/ml). Die Wirkstoffzubereitung wird auf eine glasierte Kachel pipettiert und nach Abtrocknen werden adulte Mücken der Spezies Aedes aegypti Stamm MONHEIM auf die behandelte Kachel gesetzt. Die Expositionszeit beträgt 30 Minuten.

24 Stunden nach Kontakt zur behandelten Oberfläche wird die Mortalität in % bestimmt. Dabei bedeutet 100%, dass alle Mücken abgetötet wurden; 0% bedeutet, dass keine Mücken abgetötet wurden.

Bei diesem Test zeigen z.B. die folgenden Verbindungen der Herstellungsbeispiele Wirkung von 80-100% bei einer Aufwandmenge von 100 mg/m$^2$:1, 2, 3, 11, 14, 15, 16, 18, 19, 20, 21, 22, 23, 24, 53, 70, 76, 83, 90, 94, 95,

Bei diesem Test zeigen z.B. die folgenden Verbindungen der Herstellungsbeispiele Wirkung von 80-100% bei einer Aufwandmenge von 20 mg/m$^2$:1, 2, 8, 10, 11, 13, 16, 18, 19, 20, 21, 22, 23, 24, 28, 30, 31, 48, 49, 50, 53, 54, 55, 56, 58, 59, 61, 63, 64, 65, 68, 70, 71, 72, 73, 76, 80, 83, 85, 86, 89, 90, 91, 94, 96, 97, 99

**Patentansprüche**

1. Verbindungen der Formel (I)

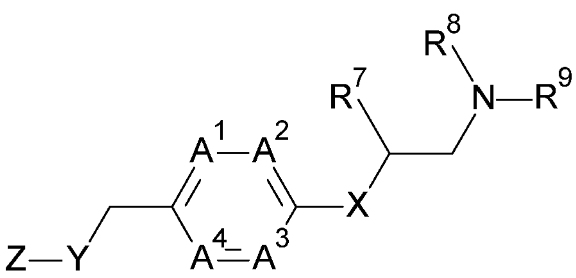

(I)

in welcher

Z für gegebenenfalls ein- oder mehrfach gleich oder verschieden mit Halogen, Cyano, $(C_1-C_4)$-Alkyl, $(C_1-C_4)$-Halogenalkyl, $(C_1-C_4)$-Alkoxy, $(C_1-C_4)$-Halogenalkoxy, $(C_1-C_4)$-Alkylsulfanyl, $(C_1-C_4)$-Halogenalkylsulfanyl, $(C_3-C_6)$-Cycloalkyl substituiertes Naphthyl, Dibenzo[b,d]furanyl, Dibenzo[b,d]thiophenyl oder für substituiertes Phenyl der Substrukturformel (II) steht,

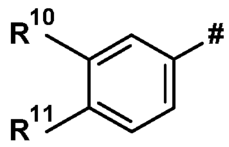

(II)

und das substituierte Phenyl der Substrukturformel (II) gegebenenfalls bis zu zwei weitere Substituenten ausgewählt aus der Gruppe von Halogen, Cyano, ($C_1$-$C_4$)-Alkyl, ($C_1$-$C_4$)-Halogenalkyl, ($C_1$-$C_4$)-Alkoxy, ($C_1$-$C_4$)-Halogenalkoxy, ($C_1$-$C_4$)-Alkylsulfanyl, ($C_3$-$C_6$)-Cycloalkyl oder ($C_3$-$C_6$)-Halogencycloalkyl tragen kann,
Y für O steht,
$A^1$ für N oder -$CR^1$ steht,
$A^2$ für N oder -$CR^2$ steht,
$A^3$ für N oder -$CR^3$ steht,
$A^4$ für N oder -$CR^4$ steht,
dabei mindestens eines und maximal zwei der Atome $A_1$, $A_2$, $A_3$ oder $A_4$ im aromatischen Ring für N steht,
X für Sauerstoff, oder -$NR^6$ steht,
$R^1$, $R^2$, $R^3$ und $R^4$ jeweils unabhängig voneinander für Wasserstoff, Halogen, Nitro, Cyano, Aminocarbonyl, Aminosulfonyl, jeweils gegebenenfalls substituiertes ($C_1$-$C_4$)-Alkyl, ($C_2$-$C_4$)-Alkenyl, ($C_2$-$C_4$)-Alkinyl, ($C_3$-$C_6$)-Cycloalkyl, ($C_1$-$C_4$)-Alkoxy, ($C_2$-$C_4$)-Alkenyloxy, ($C_2$-$C_4$)-Alkinyloxy, ($C_3$-$C_6$)-Cycloalkoxy, *N*-Mono-($C_1$-$C_4$)-alkylamino, *N*-Mono-($C_3$-$C_6$)-Cycloalkylamino, *N,N*-Di-($C_1$-$C_4$)-alkylamino, *N,N*-Di-($C_3$-$C_6$)-Cycloalkylamino, *N,N*-($C_3$-$C_6$)-Cycloalkyl-($C_1$-$C_4$)-alkylamino, *N*-($C_1$-$C_4$)-Alkanoylamino, *N*-($C_3$-$C_6$)-Cycloalkanoylamino, *N*-($C_1$-$C_4$)-Alkarioyl-*N*-($C_1$-$C_4$)-Alkylamino, *N*-($C_3$-$C_6$)-Cycloalkanoyl-*N*-($C_1$-$C_4$)-Alkylamino, *N*-($C_3$-$C_6$)-Cycloalkanoyl-*N*-($C_3$-$C_6$)-Cycloalkylamino, *N*-($C_1$-$C_4$)-Alkanoyl-*N*-($C_3$-$C_6$)-Cycloalkylamino, ($C_1$-$C_4$)-Alkoxycarbonyl, ($C_3$-$C_6$)-Cycloalkoxycarbonyl, ($C_1$-$C_4$)-Alkanoyl, ($C_3$-$C_6$)-Cycloalkanoyl, ($C_1$-$C_4$)-Alkylsulfanyl, ($C_1$-$C_4$)-Alkylsulfinyl, ($C_1$-$C_4$)-Alkylsulfonyl, ($C_3$-$C_6$)-Cycloalkylsulfanyl, ($C_3$-$C_6$)-Cycloalkylsulfinyl, ($C_3$-$C_6$)-Cycloalkylsulfonyl, *N*-($C_1$-$C_4$)-Alkylaminocarbonyl, *N*-($C_3$-$C_6$)-Cycloalkylaminocarbonyl, *N,N*-Di-($C_1$-$C_4$)-alkylaminocarbonyl, *N,N*-Di-($C_3$-$C_6$)-Cycloalkylaminocarbonyl, *N,N*-($C_3$-$C_6$)-Cycloalkyl-($C_1$-$C_4$)-Alkylaminocarbonyl, -CH=N-O-[($C_1$-$C_4$)-Alkyl], -CH=N-O-[($C_3$-$C_6$)-Cycloalkyl], -C[($C_1$-$C_4$)-Alkyl]=N-O-[($C_1$-$C_4$)-Alkyl], -C[($C_3$-$C_6$)-Cycloalkyl]=N-O-[($C_1$-$C_4$)-Alkyl], -C[($C_1$-$C_4$)-Alkyl]=N-O-[($C_3$-$C_6$)-Cycloalkyl], -C[($C_3$-$C_6$)-Cycloalkyl]=N-O-[($C_3$-$C_6$)-Cycloalkyl] stehen,
$R^5$ für Wasserstoff oder gegebenenfalls substituiertes ($C_1$-$C_4$)-Alkyl, ($C_3$-$C_6$)-Cycloalkyl, ($C_1$-$C_3$)-Alkanoyl steht,
$R^6$ für Wasserstoff oder gegebenenfalls substituiertes ($C_1$-$C_4$)-Alkyl, ($C_3$-$C_6$)-Cycloalkyl, ($C_1$-$C_4$)-Alkanoyl oder einen Ringschluss mit $R^8$ der aus 1 bis 3 $CH_2$-Gruppen gebildet wird, steht,
$R^7$ für Wasserstoff oder gegebenenfalls substituiertes ($C_1$-$C_4$)-Alkyl, ($C_3$-$C_6$)-Cycloalkyl oder einen Ringschluss mit $R^8$ der aus 1 bis 3 $CH_2$-Gruppen gebildet wird, steht,
$R^8$ für Wasserstoff oder für gegebenenfalls substituiertes ($C_1$-$C_4$)-Alkyl, ($C_2$-$C_4$)-Alkenyl, ($C_2$-$C_4$)-Alkinyl, ($C_3$-$C_6$)-Cycloalkyl steht oder ist zusammen mit $R^6$ oder $R^7$ zu einem Ring geschlossen der aus 1 bis 3 $CH_2$-Gruppen gebildet wird oder ist zusammen mit $R^9$ zu einem 4 bis 6 gliedrigen heterozyklischen Ring geschlossen, welcher im Falle eines 5 bis 6 gliedrigen heterozyklischen Ringes weitere Heteroatome enthalten kann und gegebenenfalls ein- oder mehrfach gleich oder verschieden mit Halogen, Cyano oder jeweils gegebenenfalls ein oder mehrfach gleich oder verschieden mit Halogen substituiertes ($C_1$-$C_4$)-Alkyl, ($C_3$-$C_6$)-Cycloalkyl, ($C_1$-$C_4$)-Alkoxy substituiert ist,
$R^9$ für jeweils gegebenenfalls substituiertes ($C_1$-$C_4$)-Alkyl, ($C_3$-$C_6$)-Cycloalkyl, ($C_2$-$C_4$)-Alkenyl, ($C_2$-$C_4$)-Alkinyl, *N*-Mono-($C_1$-$C_4$)-alkylamino, *N,N*-Di-($C_1$-$C_4$)-alkylamino, ($C_1$-$C_4$)-Alkoxy, ($C_2$-$C_4$)-Alkenyloxy, ($C_2$-$C_4$)-Alkinyloxy, ($C_3$-$C_6$)-Cycloalkoxy, ($C_1$-$C_4$)-Alkylcarbonyl, ($C_1$-$C_4$)-Alkylsulfinyl, ($C_1$-$C_4$)-Alkylsulfonyl, ($C_1$-$C_4$)-Alkoxycarbonyl steht, für den Fall das $R^7$ für Wasserstoff oder gegebenenfalls substituiertes ($C_1$-$C_4$)-Alkyl, ($C_3$-$C_6$)-Cycloalkyl steht oder für den Fall das $R^7$ mit $R^8$ einen 5- oder 6- gliedrigen Ring bildet, oder
$R^9$ für jeweils gegebenenfalls substituiertes ($C_1$-$C_4$)-Alkyl, ($C_3$-$C_6$)-Cycloalkyl, ($C_2$-$C_4$)-Alkenyl, ($C_2$-$C_4$)-Alkinyl, *N*-Mono-($C_1$-$C_4$)-alkylamino, *N,N*-Di-($C_1$-$C_4$)-alkylamino, ($C_1$-$C_4$)-Alkoxy, ($C_2$-$C_4$)-Alkenyloxy, ($C_2$-$C_4$)-Alkinyloxy, ($C_3$-$C_4$)-Cycloalkoxy, ($C_1$-$C_4$)-Alkylsulfinyl, ($C_1$-$C_4$)-Alkylsulfonyl steht, für den Fall das $R^7$ einen 4-gliedrigen Ring zusammen mit $R^8$ bildet, oder $R^8$ und $R^9$ für einen 4 bis 6 gliedrigen heterozyklischen Ringschluss stehen, welcher im Falle eines 5 bis 6 gliedrigen heterozyklischen Ringes weitere Heteroatome enthalten kann und gegebenenfalls ein oder mehrfach gleich oder verschieden mit Halogen, Cyano oder jeweils gegebenenfalls ein oder mehrfach gleich oder verschieden mit Halogen substituiertes ($C_1$-$C_4$)-Alkyl, ($C_3$-$C_6$)-Cycloalkyl, ($C_1$-$C_4$)-Alkoxy substituiert ist,
$R^{10}$ für Halogen, Nitro, Cyano, -$SF_5$ oder für gegebenenfalls ein- oder mehrfach gleich oder verschieden mit

Halogen, Cyano, Nitro, $-SF_5$, $(C_1-C_4)$-Alkyl, $(C_2-C_4)$-Alkenyl, $(C_2-C_4)$-Alkinyl, $(C_1-C_4)$-Halogenalkyl, $(C_2-C_4)$-Halogenalkenyl, $(C_1-C_4)$-Alkoxy, $(C_1-C_4)$-Halogenalkoxy, $(C_2-C_4)$-Alkenyloxy, $(C_2-C_4)$-Halogenalkenyloxy, $(C_2-C_4)$-Alkinyloxy, $(C_3-C_6)$-Cycloalkoxy, $(C_3-C_6)$-Halogencycloalkoxy, $(C_3-C_6)$-Cycloalkyl, $(C_3-C_6)$-Halogencycloalkyl, $(C_1-C_4)$-Alkylsulfanyl, $(C_1-C_4)$-Alkylsulfinyl, $(C_1-C_4)$-Alkylsulfonyl, $(C_1-C_4)$-Halogenalkylsulfonyl, $(C_1-C_4)$-Halogenalkylsulfanyl, $(C_1-C_4)$-Halogenalkylsulfinyl, $(C_3-C_6)$-Cycloalkylsulfanyl, $(C_3-C_6)$-Cycloalkylsulfinyl, $(C_3-C_6)$-Cycloalkylsulfonyl, $C_6$-,$C_{10}$-,$C_{14}$-Aryl, $C_6$-,$C_{10}$-,$C_{14}$-Aryloxy, Benzyl, Benzyloxy, Benzylthio, $C_6$-,$C_{10}$-,$C_{14}$-Arylthio, $C_6$-,$C_{10}$-,$C_{14}$-Arylamino, Benzylamino, Heterocyclyl, Trialkylsilyl substituiertes Phenyl, Pyrid-2-yl, Pyrid-3-yl, Pyrid-4-yl, Thiophen-2-yl, Thiophen-3-yl, $(C_1-C_4)$-Alkyl, $(C_1-C_4)$-Alkenyl, $(C_1-C_4)$-Alkinyl, $(C_3-C_6)$-Cycloalkyl, $(C_1-C_4)$-Alkoxy, $(C_2-C_4)$-Alkenyloxy, $(C_2-C_4)$-Alkinyloxy, $(C_3-C_6)$-Cycloalkoxy, $(C_1-C_4)$-Alkylsulfanyl, $(C_1-C_4)$-Alkylsulfinyl, $(C_1-C_4)$-Alkylsulfonyl, $(C_3-C_6)$-Cycloalkylsulfanyl, $(C_3-C_6)$-Cycloalkylsulfinyl, $(C_3-C_6)$-Cycloalkylsulfonyl, -CH=N-O-[$(C_1-C_4)$-Alkyl], -C[$(C_1-C_4)$-Alkyl]=N-O-[$(C_1-C_4)$-Alkyl] steht,
$R^{11}$ für Wasserstoff, Halogen, Cyano oder Nitro oder für jeweils gegebenenfalls substituiertes $(C_1-C_4)$-Alkyl, $(C_3-C_5)$-Cycloalkyl, $(C_1-C_4)$-Alkoxy, $(C_1-C_4)$-Alkylsulfanyl, $(C_1-C_4)$-Alkylsulfinyl, $(C_1-C_4)$-Alkylsulfonyl, -CH=N-O-[$(C_1-C_4)$-Alkyl], -C[$(C_1-C_4)$-Alkyl]=N-O-[$(C_1-C_4)$-Alkyl] steht, sowie
Salze, Metallkomplexe, N-Oxide und tautomere Formen der Verbindungen der Formel (I),
unter der Maßgabe, dass die Verbindung 1-[4-(6-{2-[3-(Chlormethyl)phenyl]ethyl}pyridin-3-yl)piperazin-1-yl]ethanon ausgenommen ist.

**2.** Verbindungen der Formel (I) gemäß Anspruch 1, in welcher

$R^1$, $R^2$, $R^3$ und $R^4$ jeweils unabhängig voneinander für einen Substituenten ausgewählt aus der Gruppe von Wasserstoff, Halogen, Nitro, Cyano, oder ein jeweils gegebenenfalls ein- oder mehrfach gleich oder verschieden mit Halogen, Cyano, Nitro, $(C_1-C_4)$-Alkyl, $(C_1-C_4)$-Halogenalkyl, $(C_1-C_4)$-Alkoxy, $(C_1-C_4)$-Halogenalkoxy, $(C_3-C_6)$-Cycloalkyl, $(C_3-C_6)$-Halogencycloalkyl, substituiertes $(C_1-C_4)$-Alkyl, $(C_2-C_4)$-Alkenyl, $(C_2-C_4)$-Alkinyl, $(C_3-C_6)$-Cycloalkyl, $(C_1-C_4)$-Alkoxy, $(C_2-C_4)$-Alkenyloxy, $(C_2-C_4)$-Alkinyloxy, $(C_3-C_6)$-Cycloalkoxy, $(C_1-C_4)$-Alkoxycarbonyl, $(C_3-C_6)$-Cycloalkoxycarbonyl, *N*-Mono-$(C_1-C_4)$-alkylamino, *N*,*N*-Di-$(C_1-C_4)$-alkylamino, $(C_1-C_4)$-Alkylsulfanyl, $(C_1-C_4)$-Alkylsulfinyl, $(C_1-C_4)$-Alkylsulfonyl, $(C_3-C_6)$-Cycloalkylsulfanyl, $(C_3-C_6)$-Cycloalkylsulfinyl, $(C_3-C_6)$-Cycloalkylsulfonyl stehen,
$R^5$ für Wasserstoff oder gegebenenfalls ein oder mehrfach gleich oder verschieden mit Halogen substituiertes $(C_1-C_4)$-Alkyl, $(C_3-C_4)$-Cycloalkyl, $(C_1-C_3)$-Alkanoyl steht,
$R^6$ für Wasserstoff oder gegebenenfalls ein oder mehrfach gleich oder verschieden mit Halogen substituiertes $(C_1-C_4)$-Alkyl, $(C_3-C_4)$-Cycloalkyl, $(C_1-C_4)$-Alkanoyl oder einen Ringschluss mit $R^8$ der aus 1 bis 3 $CH_2$-Gruppen gebildet wird, steht,
$R^7$ für Wasserstoff oder gegebenenfalls ein oder mehrfach gleich oder verschieden mit Halogen substituiertes $(C_1-C_4)$-Alkyl, $(C_3-C_4)$-Cycloalkyl oder einen Ringschluss mit $R^8$ der aus 1 bis 3 $CH_2$-Gruppen gebildet wird, steht,
$R^8$ für Wasserstoff oder für gegebenenfalls ein oder mehrfach gleich oder verschieden mit Halogen, Nitro oder Cyano substituiertes $(C_1-C_4)$-Alkyl, $(C_2-C_4)$-Alkenyl, $(C_2-C_4)$-Alkinyl, $(C_3-C_4)$-Cycloalkyl oder einen Ringschluss zusammen mit $R^6$ oder $R^7$ der aus 1 bis 3 $CH_2$-Gruppen gebildet wird oder einen 4 bis 6 gliedrigen heterozyklischen Ringschluss zusammen mit $R^9$, welcher im Falle eines 5 bis 6 gliedrigen heterozyklischen Ringes weitere Heteroatome ausgewählt aus der Gruppe von N, O enthalten kann und gegebenenfalls ein- oder mehrfach gleich oder verschieden mit Halogen, $(C_1-C_4)$-Alkyl oder $(C_1-C_4)$-Halogenalkyl substituiert ist, steht,
$R^9$ für jeweils gegebenenfalls ein- oder mehrfach gleich oder verschieden mit Halogen, Cyano, Nitro oder $(C_3-C_6)$-Cycloalkyl substituiertes $(C_1-C_4)$-Alkyl, $(C_2-C_4)$-Alkenyl, $(C_3-C_4)$-Cycloalkyl, $(C_1-C_4)$-Alkoxy, $(C_2-C_4)$-Alkenyloxy, $(C_3-C_6)$-Cycloalkoxy, $(C_1-C_4)$-Alkylcarbonyl, $(C_1-C_4)$-Alkylsulfinyl, $(C_1-C_4)$-Alkylsulfonyl, $(C_1-C_4)$-Alkoxycarbonyl steht, für den Fall das $R^7$ für Wasserstoff steht oder gegebenenfalls ein- oder mehrfach gleich oder verschieden mit Halogen substituiertes $(C_1-C_4)$-Alkyl, $(C_3-C_4)$-Cycloalkyl oder für den Fall das $R^7$ mit $R^8$ einen 5- oder 6-gliedrigen Ring bildet, oder
$R^9$ für jeweils gegebenenfalls ein- oder mehrfach gleich oder verschieden mit Halogen, Cyano, Nitro oder $(C_3-C_6)$-Cycloalkyl substituiertes $(C_1-C_4)$-Alkyl, $(C_2-C_4)$-Alkenyl, $(C_3-C_4)$-Cycloalkyl, $(C_1-C_4)$-Alkoxy, $(C_2-C_4)$-Alkenyloxy, $(C_3-C_4)$-Cycloalkoxy, $(C_1-C_4)$-Alkylsulfinyl, $(C_1-C_4)$-Alkylsulfonyl steht, für den Fall das $R^7$ einen 4-gliedrigen Ring zusammen mit $R^8$ bildet, oder $R^8$ und $R^9$ für einen 4 bis 6 gliedrigen heterozyklischen Ringschluss stehen, welcher im Falle eines 5 bis 6 gliedrigen heterozyklischen Ringes weitere Heteroatome ausgewählt aus der Gruppe von N, O enthalten kann und gegebenenfalls ein oder mehrfach gleich oder verschieden mit Halogen, $(C_1-C_4)$-Alkyl oder $(C_1-C_4)$-Halogenalkyl substituiert ist,
$R^{10}$ für Halogen oder für gegebenenfalls ein- oder mehrfach gleich oder verschieden mit Halogen, Cyano, Nitro, $(C_1-C_4)$-Alkyl, $(C_1-C_4)$-Halogenalkyl, $(C_1-C_4)$-Alkoxy, $(C_1-C_4)$-Halogenalkoxy, $(C_3-C_6)$-Cycloalkyl, $(C_3-C_6)$-Halogencycloalkyl, substituiertes Phenyl, Pyrid-2-yl, Pyrid-3-yl, Pyrid-4-yl, Thiophen-2-yl, Thiophen-3-yl, $(C_1-C_4)$-Alkyl, $(C_1-C_4)$-Alkenyl, $(C_1-C_4)$-Alkinyl, $(C_3-C_6)$-Cycloalkyl, $(C_1-C_4)$-Alkoxy, $(C_2-C_4)$-Alkenyloxy,

$(C_2-C_4)$-Alkinyloxy, $(C_3-C_6)$-Cycloalkoxy, $(C_1-C_4)$-Alkylsulfanyl, $(C_1-C_4)$-Alkylsulfinyl, $(C_1-C_4)$-Alkylsulfonyl, $(C_3-C_6)$-Cycloalkylsulfanyl, $(C_3-C_6)$-Cycloalkylsulfinyl, $(C_3-C_6)$-Cycloalkylsulfonyl, -CH=N-O-[$(C_1-C_4)$-Alkyl], -C[$(C_1-C_4)$-Alkyl]=N-O-[$(C_1-C_4)$-Alkyl steht,

$R^{11}$ für Wasserstoff, Halogen, Cyano oder Nitro oder für gegebenenfalls ein- oder mehrfach gleich oder verschieden mit Halogen, Cyano, Nitro, $(C_1-C_4)$-Alkoxy oder $(C_3-C_6)$-Cycloalkyl substituiertes $(C_1-C_4)$-Alkyl, $(C_1-C_4)$-Alkoxy, $(C_1-C_4)$-Alkylsulfanyl, $(C_1-C_4)$-Alkylsulfinyl, $(C_1-C_4)$-Alkylsulfonyl, $(C_3-C_5)$-Cycloalkyl, -CH=N-O-[$(C_1-C_4)$-Alkyl], -C[$(C_1-C_4)$-Alkyl]=N-O-[$(C_1-C_4)$-Alkyl] steht.

**3.** Verbindungen der Formel (I) nach einem der Ansprüche 1 bis 2, in welcher

zumindest eines der Atome $A_3$ oder $A_4$ im aromatischen Ring für Stickstoff steht,

$R^1$, $R^2$, $R^3$ und $R^4$ jeweils unabhängig voneinander für einen Substituenten ausgewählt aus der Gruppe von Wasserstoff, Halogen, $(C_1-C_4)$-Alkyl, $(C_1-C_4)$-Halogenalkyl, $(C_2-C_4)$-Alkenyl, $(C_2-C_4)$-Halogenalkenyl, $(C_1-C_4)$-Alkoxy, $(C_1-C_4)$-Halogenalkoxy, $(C_3-C_6)$-Cycloalkyl, $(C_3-C_6)$-Halogencycloalkyl, $(C_1-C_4)$-Alkylsulfanyl, $(C_1-C_4)$-Alkylsulfinyl oder $(C_1-C_4)$-Alkylsulfonyl stehen,

$R^6$ für Wasserstoff, $(C_1-C_4)$-Alkyl oder einen Ringschluss mit $R^8$ der aus 1 bis 3 $CH_2$-Gruppen gebildet wird, steht,

$R^7$ für Wasserstoff, $(C_1-C_4)$-Alkyl, $(C_3-C_4)$-Cycloalkyl oder einen Ringschluss mit $R^8$ der aus 1 bis 3 $CH_2$-Gruppen gebildet wird, steht,

$R^8$ für Wasserstoff oder für gegebenenfalls ein- oder mehrfach gleich oder verschieden mit Halogen substituiertes $(C_1-C_4)$-Alkyl, $(C_2-C_4)$-Alkenyl, $(C_2-C_4)$-Alkinyl, $(C_3-C_4)$-Cycloalkyl oder einen Ringschluss zusammen mit $R^6$ oder $R^7$ der aus 1 bis 3 $CH_2$-Gruppen gebildet wird oder einen 4 bis 6 gliedrigen heterozyklischen Ringschluss zusammen mit $R^9$, der aus 3 bis 5 $CH_2$-Gruppen gebildet ist und der gegebenenfalls ein oder mehrfach gleich oder verschieden mit Halogen, $(C_1-C_4)$-Alkyl oder $(C_1-C_4)$-Halogenalkyl substituiert ist, steht,

$R^9$ für jeweils gegebenenfalls ein- oder mehrfach gleich oder verschieden mit Halogen, Cyano, Nitro oder $(C_3-C_6)$-Cycloalkyl substituiertes $(C_1-C_4)$-Alkyl, $(C_3-C_6)$-Cycloalkyl, $(C_1-C_6)$-Alkoxy, $(C_1-C_6)$-Alkylcarbonyl steht, für den Fall das $R^7$ für Wasserstoff steht oder für $(C_1-C_4)$-Alkyl, $(C_3-C_4)$-Cycloalkyl oder für den Fall das $R^7$ mit $R^8$ einen 5- oder 6-gliedrigen Ring bildet, oder $R^9$ für jeweils gegebenenfalls ein- oder mehrfach gleich oder verschieden mit Halogen, Cyano, Nitro oder $(C_3-C_6)$-Cycloalkyl substituiertes $(C_1-C_4)$-Alkyl, $(C_3-C_4)$-Cycloalkyl, $(C_1-C_4)$-Alkoxy steht, für den Fall das $R^7$ einen 4-gliedrigen Ring zusammen mit $R^8$ bildet, oder $R^8$ und $R^9$ für einen 4 bis 6 gliedrigen heterozyklischen Ringschluss stehen, der aus 3 bis 5 $CH_2$-Gruppen gebildet ist und der gegebenenfalls ein- oder mehrfach gleich oder verschieden mit Halogen, $(C_1-C_4)$-Alkyl oder $(C_1-C_4)$-Halogenalkyl substituiert ist,

$R^{10}$ für Halogen oder für gegebenenfalls ein- oder mehrfach gleich oder verschieden mit Halogen, Cyano, Nitro, $(C_1-C_4)$-Alkyl, $(C_1-C_4)$-Halogenalkyl, $(C_1-C_4)$-Alkoxy, $(C_1-C_4)$-Halogenalkoxy, $(C_3-C_6)$-Cycloalkyl, $(C_3-C_6)$-Halogencycloalkyl substituiertes Phenyl, Pyrid-2-yl, Pyrid-3-yl, Pyrid-4-yl, Thiophen-2-yl, Thiophen-3-yl oder für gegebenenfalls ein- oder mehrfach gleich oder verschieden mit Halogen, Cyano, Nitro, substituiertes $(C_1-C_4)$-Alkyl, $(C_1-C_4)$-Alkenyl, $(C_1-C_4)$-Alkinyl, $(C_3-C_6)$-Cycloalkyl, $(C_1-C_4)$-Alkoxy, $(C_2-C_4)$-Alkenyloxy, $(C_2-C_4)$-Alkinyloxy, $(C_3-C_6)$-Cycloalkoxy, $(C_1-C_4)$-Alkylsulfanyl, $(C_1-C_4)$-Alkylsulfinyl, $(C_1-C_4)$-Alkylsulfonyl, $(C_3-C_6)$-Cycloalkylsulfanyl, $(C_3-C_6)$-Cycloalkylsulfinyl, $(C_3-C_6)$-Cycloalkylsulfonyl, -CH=N-O-[$(C_1-C_4)$-Alkyl], -C[$(C_1-C_4)$-Alkyl]=N-O-[$(C_1-C_4)$-Alkyl steht,

$R^{11}$ für Wasserstoff, Halogen, Cyano oder Nitro oder für gegebenenfalls ein- oder mehrfach gleich oder verschieden mit Halogen, Cyano oder $(C_3-C_6)$-Cycloalkyl substituiertes $(C_1-C_4)$-Alkyl, $(C_1-C_4)$-Alkoxy, $(C_1-C_4)$-Alkylsulfanyl, $(C_1-C_4)$-Alkylsulfinyl, $(C_1-C_4)$-Alkylsulfonyl, $(C_3-C_5)$-Cycloalkyl, steht.

**4.** Verbindungen der Formel (I) nach einem der Ansprüche 1 bis 3, in welcher

Z für gegebenenfalls ein- oder mehrfach gleich oder verschieden mit Halogen, Cyano, $(C_1-C_4)$-Alkyl, $(C_1-C_4)$-Halogenalkyl substituiertes Naphthyl, oder für unsubstituiertes Dibenzo[b,d]furanyl oder Dibenzo[b,d]thiophenyl oder für substituiertes Phenyl der Substrukturformel (II) steht,

$R^1$, $R^2$, $R^3$ und $R^4$ für einen Substituenten ausgewählt aus der Gruppe von Wasserstoff, Halogen, $(C_1-C_4)$-Alkyl, $(C_1-C_4)$-Halogenalkyl, $(C_1-C_4)$-Alkoxy oder $(C_1-C_4)$-Alkylsulfanyl stehen,

$R^6$ für Wasserstoff, $(C_1-C_3)$-Alkyl oder einen Ringschluss mit $R^8$ der aus 1 bis 2 $CH_2$-Gruppen gebildet wird, steht,

$R^7$ für Wasserstoff oder einen Ringschluss mit $R^8$ der aus 1 bis 3 $CH_2$-Gruppen gebildet wird, steht,

$R^8$ für Wasserstoff oder für gegebenenfalls ein oder mehrfach gleich oder verschieden mit Halogen substituiertes $(C_1-C_4)$-Alkyl oder einen Ringschluss zusammen mit $R^6$ der aus 1 bis 2 $CH_2$-Gruppen gebildet wird oder einen Ringschluss zusammen mit $R^7$ der aus 1 bis 3 $CH_2$-Gruppen gebildet wird oder einen 4 bis 6 gliedrigen heterozyklischen Ringschluss zusammen mit $R^9$ der aus 3 bis 5 $CH_2$-Gruppen gebildet ist und der gegebenenfalls ein- oder mehrfach gleich oder verschieden mit Halogen, $(C_1-C_4)$-Alkyl oder $(C_1-C_4)$-Halogenalkyl substituiert

ist, steht,
$R^9$ für jeweils gegebenenfalls ein- oder mehrfach gleich oder verschieden mit Halogen, Cyano oder ($C_3$-$C_6$)-Cycloalkyl substituiertes ($C_1$-$C_6$)-Alkyl, ($C_3$-$C_6$)-Cycloalkyl oder ($C_1$-$C_6$)-Alkoxy steht, oder $R^8$ und
$R^9$ für einen 4 bis 6 gliedrigen heterozyklischen Ringschluss stehen der aus 3 bis 5 $CH_2$-Gruppen gebildet ist und der gegebenenfalls ein- oder mehrfach gleich oder verschieden mit Halogen, ($C_1$-$C_4$)-Alkyl oder ($C_1$-$C_4$)-Halogenalkyl substituiert ist,
$R^{10}$ für Halogen, ($C_1$-$C_4$)-Alkyl, ($C_1$-$C_4$)-Alkoxy, ($C_1$-$C_4$)-Halogenalkoxy oder für gegebenenfalls ein- oder mehrfach gleich oder verschieden mit Halogen, Cyano, ($C_1$-$C_4$)-Alkyl, ($C_1$-$C_4$)-Halogenalkyl, ($C_1$-$C_4$)-Alkoxy oder ($C_1$-$C_4$)-Halogenalkoxy substituiertes Phenyl steht,
$R^{11}$ für Wasserstoff, Halogen, Nitro oder Cyano oder für gegebenenfalls ein- oder mehrfach gleich oder verschieden mit Halogen substituiertes ($C_1$-$C_4$)-Alkyl, ($C_3$-$C_5$)-Cycloalkyl oder ($C_1$-$C_4$)-Alkoxy steht.

**5.** Verbindungen der Formel (I) nach einem der Ansprüche 1 bis 4, in welcher

Z für ein Naphthyl der Substrukturformel (III):

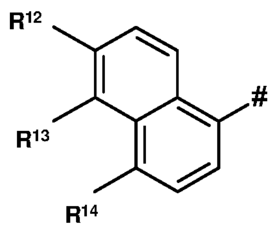

(III)

oder für unsubstituiertes Dibenzo[b,d]furanyl oder Dibenzo[b,d]thiophenyl oder für substituiertes Phenyl der Substrukturformel (II) steht.
$R^1$ für Wasserstoff, Halogen, ($C_1$-$C_4$)-Alkyl oder ($C_1$-$C_4$)-Alkoxy steht,
$R^2$ für Wasserstoff, Halogen oder ($C_1$-$C_4$)-Halogenalkyl steht,
$R^3$ für Wasserstoff, Halogen, ($C_1$-$C_4$)-Alkoxy oder ($C_1$-$C_4$)-Alkylsulfanyl steht,
$R^4$ für Wasserstoff oder Halogen steht,
$R^6$ für Wasserstoff, oder für einen Piperazin Ringschluss mit $R^8$, steht,
$R^7$ für Wasserstoff oder für einen Pyrrolidin Ringschluss mit $R^8$, steht,
$R^8$ für Wasserstoff, ($C_1$-$C_4$)-Alkyl oder für einen Piperazin Ringschluss zusammen mit $R^6$ oder für einen Pyrrolidin Ringschluss zusammen mit $R^7$ oder für einen Piperidin Ringschluss zusammen mit $R^9$, steht,
$R^9$ für jeweils gegebenenfalls ein- oder mehrfach gleich oder verschieden mit Halogen oder Cyano substituiertes ($C_1$-$C_4$)-Alkyl oder ($C_1$-$C_4$)-Alkoxy steht, oder
$R^8$ und $R^9$ für einen Piperidin Ringschluss stehen,
$R^{10}$ für Halogen, ($C_1$-$C_4$)-Alkyl, ($C_1$-$C_4$)-Alkoxy, ($C_1$-$C_4$)-Halogenalkoxy oder für ein Phenyl der Substrukturformel (IV):

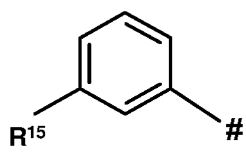

(IV)

$R^{11}$ für Wasserstoff oder Halogen oder für gegebenenfalls ein- oder mehrfach gleich oder verschieden mit Halogen substituiertes ($C_1$-$C_4$)-Alkyl oder ($C_1$-$C_4$)-Alkoxy steht,
$R^{12}$, $R^{13}$ und $R^{14}$ jeweils unabhängig voneinander für Wasserstoff oder Halogen stehen,
$R^{15}$ für Wasserstoff, Halogen, Cyano, ($C_1$-$C_4$)-Alkyl, ($C_1$-$C_4$)-Halogenalkyl, ($C_1$-$C_4$)-Alkoxy oder ($C_1$ $C_4$)-Halogenalkoxy steht.

**6.** Formulierung enthaltend Verbindungen der Formel (I) gemäß einem der Ansprüche 1 bis 5 sowie zumindest einen Hilfsstoff.

**7.** Formulierung gemäß Anspruch 6 zusätzlich enthaltend einen weiteren agrochemischen Wirkstoff.

**8.** Verbindungen der Formel (Va) oder (Vb-1):

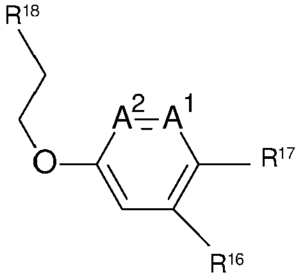

(Va) ,

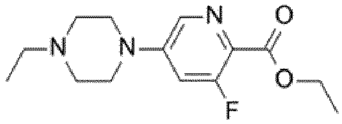

(Vb-1)

in welcher

$A^1$ für N oder CH steht,
$A^2$ für N oder CH steht, wobei zumindest $A^1$ oder $A^2$ für N stehen und vorzugsweise $A^1$ für N und $A^2$ für CH steht,
$R^{16}$ für Wasserstoff, Methyl, Fluor oder Methoxy steht,
$R^{17}$ für Cyano, Hydroxymethyl, Methylcarboxy, Ethylcarboxy oder 3-Bromphenoxymethyl steht, $R^{18}$ bei den Verbindungen der Formel (Va) für einen Rest:

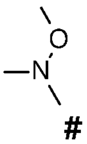 , 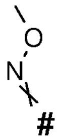 , 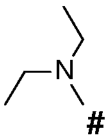

oder Brom steht, mit der Maßgabe, dass folgende Verbindungen nicht umfasst sind:

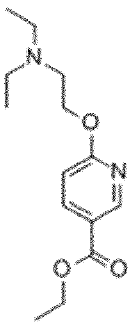 , 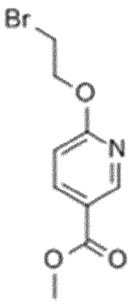 , 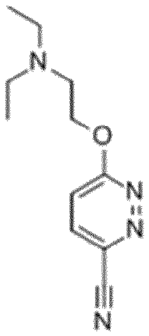 , 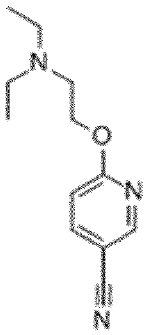 , 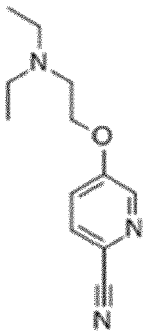 , 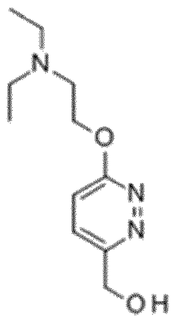 ,

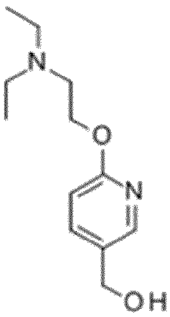 , 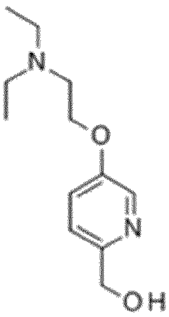 .

**9.** Verfahren zur Bekämpfung von tierischen Schädlingen **dadurch gekennzeichnet, dass** man eine Verbindung der Formel (I) gemäß einem der Ansprüche 1 bis 5 oder eine Formulierung gemäß einem der Ansprüche 6 oder 7 auf die tierischen Schädlinge und/oder ihren Lebensraum einwirken lässt, wobei die chirurgische, therapeutische und diagnostische Behandlung des menschlichen oder tierischen Körpers ausgeschlossen ist.

**10.** Verwendung von Verbindungen der Formel (I) gemäß einem der Ansprüche 1 bis 5 oder von Formulierungen gemäß einem der Ansprüche 6 oder 7 zur Bekämpfung von tierischen Schädlingen, wobei die chirurgische, therapeutische und diagnostische Behandlung des menschlichen oder tierischen Körpers ausgeschlossen ist.

**11.** Verwendung gemäß Anspruch 10, wobei die tierischen Schädlinge Mücken sind.

**12.** Verwendung von Verbindungen der Formel (I) gemäß einem der Ansprüche 1 bis 5 oder von Formulierungen gemäß einem der Ansprüche 6 oder 7 zur Herstellung von Arzneimitteln zur Bekämpfung von Ektoparasiten bei Tieren.

**13.** Verfahren zur Bekämpfung von tierischen Schädlingen **dadurch gekennzeichnet, dass** man eine Verbindung der Formel (I) gemäß einem der Ansprüche 1 bis 5 oder eine Formulierung gemäß Anspruch 6 oder 7 auf die tierischen Schädlinge und/oder ihren Lebensraum einwirken lässt, wobei die chirurgische, therapeutische und diagnostische Behandlung des menschlichen oder tierischen Körpers ausgeschlossen ist.

## Claims

**1.** Compounds of the formula (I)

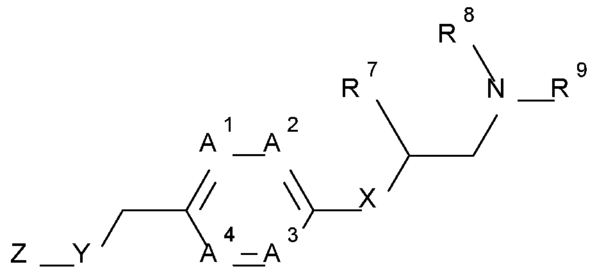

(I),

in which
Z represents naphthyl, dibenzo[b,d]furanyl, dibenzo[b,d]thiophenyl, optionally singly or multiply substituted by identical or different substituents selected from the group consisting of halogen, cyano, $(C_1-C_4)$ -alkyl, $(C_1-C_4)$ -haloalkyl, $(C_1-C_4)$ -alkoxy, $(C_1-C_4)$-haloalkoxy, $(C_1-C_4)$ -alkylsulfanyl, $(C_1-C_4)$-haloalkylsulfanyl, $(C_3-C_6)$ -cycloalkyl, or represents substituted phenyl of the substructure formula (II),

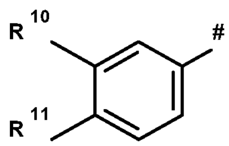

(II)

and the substituted phenyl of the substructure formula (II) may optionally bear up to two further substituents selected from the group consisting of halogen, cyano, $(C_1-C_4)$ -alkyl, $(C_1-C_4)$ -haloalkyl, $(C_1-C_4)$ -alkoxy, $(C_1-C_4)$-haloalkoxy, $(C_1-C_4)$-alkylsulfanyl, $(C_3-C_6)$-cycloalkyl and $(C_3-C_6)$-halocycloalkyl,
Y represents O,
$A^1$ represents N or $-CR^1$,
$A^2$ represents N or $-CR^2$,
$A^3$ represents N or $-CR^3$,

$A^4$ represents N or $-CR^4$,
where at least one and at most two of the atoms $A_1$, $A_2$, $A_3$ and $A_4$ in the aromatic ring represents N,
X represents oxygen or $-NR^6$,
$R^1$, $R^2$, $R^3$ and $R^4$ each independently represent hydrogen, halogen, nitro, cyano, aminocarbonyl, aminosulfonyl or in each case optionally substituted $(C_1-C_4)$-alkyl, $(C_2-C_4)$-alkenyl, $(C_2-C_4)$-alkynyl, $(C_3-C_6)$-cycloalkyl, $(C_1-C_4)$-alkoxy, $(C_2-C_4)$-alkenyloxy, $(C_2-C_4)$-alkynyloxy, $(C_3-C_6)$-cycloalkoxy, *N*-mono-$(C_1-C_4)$-alkylamino, *N*-mono-$(C_3-C_6)$-cycloalkylamino, *N,N*-di-$(C_1-C_4)$-alkylamino, *N,N*-di-$(C_3-C_6)$-cycloalkylamino, *N,N*-$(C_3-C_6)$-cycloalkyl-$(C_1-C_4)$ - alkylamino, *N*-$(C_1-C_4)$-alkanoylamino, *N*-$(C_3-C_6)$-cycloalkanoylamino, *N*-$(C_1-C_4)$-alkanoyl-*N*-$(C_1-C_4)$ - alkylamino, *N*-$(C_3-C_6)$-cycloalkanoyl-*N*-$(C_1-C_4)$ -alkylamino, *N*-$(C_3-C_6)$-cycloalkanoyl-*N*-$(C_3-C_6)$ -cycloalkylamino, *N*-$(C_1-C_4)$-alkanoyl-*N*-$(C_3-C_6)$-cycloalkylamino, $(C_1-C_4)$-alkoxycarbonyl, $(C_3-C_6)$-cycloalkoxycarbonyl, $(C_1-C_4)$-alkanoyl, $(C_3-C_6)$-cycloalkanoyl, $(C_1-C_4)$-alkylsulfanyl, $(C_1-C_4)$-alkylsulfinyl, $(C_1-C_4)$-alkylsulfonyl, $(C_3-C_6)$-cycloalkylsulfanyl, $(C_3-C_6)$-cycloalkylsulfinyl, $(C_3-C_6)$-cycloalkylsulfonyl, *N*-$(C_1-C_4)$-alkylaminocarbonyl, *N*-$(C_3-C_6)$-cycloalkylaminocarbonyl, *N,N*-di-$(C_1-C_4)$-alkylaminocarbonyl, *N,N*-di-$(C_3-C_6)$-cycloalkylaminocarbonyl, *N,N*-$(C_3-C_6)$-cycloalkyl- $(C_1-C_4)$-alkylaminocarbonyl, -CH=N-O-[$(C_1-C_4)$-alkyl], -CH=N-O-[$(C_3-C_6)$ -cycloalkyl], -C[$(C_1-C_4)$-alkyl]=N-O-[$(C_1-C_4)$ - alkyl], -C[$(C_3-C_6)$-cycloalkyl]=N-O-[$(C_1-C_4)$-alkyl], -C[$(C_1-C_4)$-alkyl]=N-O-[$(C_3-C_6)$-cycloalkyl], -C[$(C_3-C_6)$- cycloalkyl]=N-O-[$(C_3-C_6)$-cycloalkyl],
$R^5$ represents hydrogen or optionally substituted $(C_1-C_4)$-alkyl, $(C_3-C_6)$-cycloalkyl, $(C_1-C_3)$-alkanoyl,
$R^6$ represents hydrogen or optionally substituted $(C_1-C_4)$-alkyl, $(C_3-C_6)$-cycloalkyl, $(C_1-C_4)$-alkanoyl or a ring closure with $R^8$ formed by 1 to 3 $CH_2$ groups,
$R^7$ represents hydrogen or optionally substituted $(C_1-C_4)$-alkyl, $(C_3-C_6)$-cycloalkyl or a ring closure with $R^8$ formed by 1 to 3 $CH_2$ groups,
$R^8$ represents hydrogen or represents optionally substituted $(C_1-C_4)$-alkyl, $(C_2-C_4)$-alkenyl, $(C_2-C_4)$-alkynyl, $(C_3-C_6)$-cycloalkyl or together with $R^6$ or $R^7$ is closed in a ring formed by 1 to 3 $CH_2$ groups or together with $R^9$ is closed in a 4- to 6-membered heterocyclic ring which, in the case of a 5- to 6-membered heterocyclic ring, may contain further heteroatoms, and optionally singly or multiply substituted by identical or different halogen substituents, cyano or by $(C_1-C_4)$-alkyl, $(C_3-C_6)$-cycloalkyl, $(C_1-C_4)$-alkoxy, each of which is optionally singly or multiply substituted by identical or different halogen substituents,
$R^9$ represents in each case optionally substituted $(C_1-C_4)$-alkyl, $(C_3-C_6)$-cycloalkyl, $(C_2-C_4)$-alkenyl, $(C_2-C_4)$-alkynyl, *N*-mono-$(C_1-C_4)$ -alkylamino, *N,N*-di-$(C_1-C_4)$ - alkylamino, $(C_1-C_4)$-alkoxy, $(C_2-C_4)$-alkenyloxy, $(C_2-C_4)$-alkynyloxy, $(C_3-C_6)$-cycloalkoxy, $(C_1-C_4)$-alkylcarbonyl, $(C_1-C_4)$-alkylsulfinyl, $(C_1-C_4)$-alkylsulfonyl, $(C_1-C_4)$-alkoxycarbonyl, in the case that $R^7$ represents hydrogen or optionally substituted $(C_1-C_4)$-alkyl, $(C_3-C_6)$-cycloalkyl or in the case that $R^7$ together with $R^8$ forms a 5- or 6-membered ring, or
$R^9$ represents in each case optionally substituted $(C_1-C_4)$-alkyl, $(C_3-C_6)$-cycloalkyl, $(C_2-C_4)$-alkenyl, $(C_2-C_4)$-alkynyl, *N*-mono-$(C_1-C_4)$ -alkylamino, *N,N*-di-$(C_1-C_4)$ - alkylamino, $(C_1-C_4)$-alkoxy, $(C_2-C_4)$-alkenyloxy, $(C_2-C_4)$-alkynyloxy, $(C_3-C_4)$-cycloalkoxy, $(C_1-C_4)$-alkylsulfinyl, $(C_1-C_4)$-alkylsulfonyl, in the case that $R^7$ forms a 4-membered ring together with $R^8$, or
$R^8$ and $R^9$ represent a 4- to 6-membered heterocyclic ring closure which, in the case of a 5- to 6-membered heterocyclic ring, may contain further heteroatoms and is optionally singly or multiply substituted by identical or different halogen substituents, cyano or by $(C_1-C_4)$-alkyl, $(C_3-C_6)$-cycloalkyl, $(C_1-C_4)$-alkoxy, each of which is optionally singly or multiply substituted by identical or different halogen substituents,
$R^{10}$ represents halogen, nitro, cyano, $-SF_5$ or represents phenyl, pyrid-2-yl, pyrid-3-yl, pyrid-4-yl, thiophen-2-yl, thiophen-3-yl, $(C_1-C_4)$-alkyl, $(C_1-C_4)$-alkenyl, $(C_1-C_4)$-alkynyl, $(C_3-C_6)$-cycloalkyl, $(C_1-C_4)$-alkoxy, $(C_2-C_4)$-alkenyloxy, $(C_2-C_4)$-alkynyloxy, $(C_3-C_6)$-cycloalkoxy, $(C_1-C_4)$-alkylsulfanyl, $(C_1-C_4)$-alkylsulfinyl, $(C_1-C_4)$-alkylsulfonyl, $(C_3-C_6)$-cycloalkylsulfanyl, $(C_3-C_6)$-cycloalkylsulfinyl,
$(C_3-C_6)$-cycloalkylsulfonyl, -CH=N-O-[$(C_1-C_4)$-alkyl], -C[$(C_1-C_4)$-alkyl]=N-O-[$(C_1-C_4)$-alkyl], optionally singly or multiply substituted by identical or different substituents from the group consisting of halogen, cyano, nitro, $-SF_5$, $(C_1-C_4)$-alkyl, $(C_2-C_4)$-alkenyl, $(C_2-C_4)$-alkynyl, $(C_1-C_4)$-haloalkyl, $(C_2-C_4)$-haloalkenyl, $(C_1-C_4)$-alkoxy, $(C_1-C_4)$-haloalkoxy, $(C_2-C_4)$-alkenyloxy, $(C_2-C_4)$-haloalkenyloxy, $(C_2-C_4)$-alkynyloxy, $(C_3-C_6)$-cycloalkoxy, $(C_3-C_6)$-halocycloalkoxy, $(C_3-C_6)$-cycloalkyl, $(C_3-C_6)$-halocycloalkyl, $(C_1-C_4)$-alkylsulfanyl, $(C_1-C_4)$-alkylsulfinyl, $(C_1-C_4)$-alkylsulfonyl, $(C_1-C_4)$-haloalkylsulfonyl, $(C_1-C_4)$-haloalkylsulfanyl, $(C_1-C_4)$-haloalkylsulfinyl, $(C_3-C_6)$-cycloalkylsulfanyl, $(C_3-C_6)$-cycloalkylsulfinyl, $(C_3-C_6)$-cycloalkylsulfonyl, $C_6$-, $C_{10}$-, $C_{14}$-aryl, $C_6$-, $C_{10}$-, $C_{14}$-aryloxy, benzyl, benzyloxy, benzylthio, $C_6$-, $C_{10}$-, $C_{14}$-arylthio, $C_6$-, $C_{10}$-, $C_{14}$-arylamino, benzylamino, heterocyclyl and trialkylsilyl,
$R^{11}$ represents hydrogen, halogen, cyano or nitro or represents in each case optionally substituted $(C_1-C_4)$-alkyl, $(C_3-C_5)$-cycloalkyl, $(C_1-C_4)$-alkoxy, $(C_1-C_4)$-alkylsulfanyl, $(C_1-C_4)$-alkylsulfinyl, $(C_1-C_4)$-alkylsulfonyl, -CH=N-O-[$(C_1-C_4)$ -alkyl], -C[$(C_1-C_4)$-alkyl]=N-O-[$(C_1-C_4)$-alkyl], and
salts, metal complexes, N-oxides and tautomeric forms of the compounds of the formula (I),
with the proviso that the compound 1-[4-(6-{2-[3-(chloromethyl)phenyl]ethyl}pyridin-3-yl)piperazin-1-yl]ethanone is excluded.

**2.** Compounds of the formula (I) according to Claim 1, in which

$R^1$, $R^2$, $R^3$ and $R^4$ each independently represent a substituent selected from the group consisting of hydrogen, halogen, nitro, cyano, or represent $(C_1-C_4)$-alkyl, $(C_2-C_4)$-alkenyl, $(C_2-C_4)$-alkynyl, $(C_3-C_6)$-cycloalkyl, $(C_1-C_4)$-alkoxy, $(C_2-C_4)$-alkenyloxy, $(C_2-C_4)$ - alkynyloxy, $(C_3-C_6)$-cycloalkoxy, $(C_1-C_4)$-alkoxycarbonyl, $(C_3-C_6)$-cycloalkoxycarbonyl, *N*-mono-$(C_1-C_4)$-alkylamino, *N*,*N*-di-$(C_1-C_4)$ -alkylamino, $(C_1-C_4)$ -alkylsulfanyl, $(C_1-C_4)$-alkylsulfinyl, $(C_1-C_4)$-alkylsulfonyl, $(C_3-C_6)$-cycloalkylsulfanyl, $(C_3-C_6)$-cycloalkylsulfinyl, $(C_3-C_6)$-cycloalkylsulfonyl, each of which is optionally singly or multiply substituted by identical or different substituents from the group consisting of halogen, cyano, nitro, $(C_1-C_4)$-alkyl, $(C_1-C_4)$-haloalkyl, $(C_1-C_4)$-alkoxy, $(C_1-C_4)$-haloalkoxy, $(C_3-C_6)$-cycloalkyl, $(C_3-C_6)$-halocycloalkyl,
$R^5$ represents hydrogen or represents $(C_1-C_4)$-alkyl, $(C_3-C_4)$-cycloalkyl, $(C_1-C_3)$-alkanoyl, optionally singly or multiply substituted by identical or different halogen substituents,
$R^6$ represents hydrogen or represents $(C_1-C_4)$-alkyl, $(C_3-C_4)$-cycloalkyl, $(C_1-C_4)$-alkanoyl, optionally singly or multiply substituted by identical or different halogen substituents, or a ring closure with $R^8$ formed by 1 to 3 $CH_2$ groups,
$R^7$ represents hydrogen or represents $(C_1-C_4)$-alkyl, $(C_3-C_4)$-cycloalkyl, optionally singly or multiply substituted by identical or different halogen substituents, or a ring closure with $R^8$ formed by 1 to 3 $CH_2$ groups,
$R^8$ represents hydrogen or represents $(C_1-C_4)$-alkyl, $(C_2-C_4)$-alkenyl, $(C_2-C_4)$-alkynyl, $(C_3-C_4)$-cycloalkyl, optionally singly or multiply substituted by identical or different substituents from the group consisting of halogen, nitro and cyano, or a ring closure together with $R^6$ or $R^7$ formed by 1 to 3 $CH_2$ groups, or a 4- to 6-membered heterocyclic ring closure together with $R^9$ which, in the case of a 5- to 6-membered heterocyclic ring, may contain further heteroatoms selected from the group consisting of N and O and optionally singly or multiply substituted by identical or different substituents from the group consisting of halogen, $(C_1-C_4)$ -alkyl and $(C_1-C_4)$-haloalkyl,
$R^9$ represents $(C_1-C_4)$ -alkyl, $(C_2-C_4)$-alkenyl, $(C_3-C_4)$-cycloalkyl, $(C_1-C_4)$ -alkoxy, $(C_2-C_4)$-alkenyloxy, $(C_3-C_6)$-cycloalkoxy, $(C_1-C_4)$ -alkylcarbonyl, $(C_1-C_4)$-alkylsulfinyl, $(C_1-C_4)$ -alkylsulfonyl, $(C_1-C_4)$-alkoxycarbonyl, each of which is optionally singly or multiply substituted by identical or different substituents from the group consisting of halogen, cyano, nitro or $(C_3-C_6)$ -cycloalkyl, in the case that $R^7$ represents hydrogen or $(C_1-C_4)$ -alkyl, $(C_3-C_4)$-cycloalkyl, optionally singly or multiply substituted by identical or different halogen substituents or in the case that $R^7$ with $R^8$ forms a 5- or 6-membered ring, or
$R^9$ represents $(C_1-C_4)$ -alkyl, $(C_2-C_4)$-alkenyl, $(C_3-C_4)$-cycloalkyl, $(C_1-C_4)$ -alkoxy, $(C_2-C_4)$-alkenyloxy, $(C_3-C_4)$-cycloalkoxy, $(C_1-C_4)$ -alkylsulfinyl, $(C_1-C_4)$ - alkylsulfonyl, in each case optionally singly or multiply substituted by identical or different substituents from the group consisting of halogen, cyano, nitro or $(C_3-C_6)$-cycloalkyl, in the case that $R^7$ forms a 4-membered ring together with $R^8$, or
$R^8$ and $R^9$ represent a 4- to 6-membered heterocyclic ring closure which, in the case of a 5- to 6-membered heterocyclic ring, may contain further heteroatoms selected from the group consisting of N, O and optionally singly or multiply substituted by identical or different substituents from the group consisting of halogen, $(C_1-C_4)$-alkyl and $(C_1-C_4)$ -haloalkyl,
$R^{10}$ represents halogen or represents phenyl, pyrid-2-yl, pyrid-3-yl, pyrid-4-yl, thiophen-2-yl, thiophen-3-yl, $(C_1-C_4)$ -alkyl, $(C_1-C_4)$ -alkenyl, $(C_1-C_4)$ -alkynyl, $(C_3-C_6)$ - cycloalkyl, $(C_1-C_4)$-alkoxy, $(C_2-C_4)$-alkenyloxy, $(C_2-C_4)$ - alkynyloxy, $(C_3-C_6)$-cycloalkoxy, $(C_1-C_4)$-alkylsulfanyl, $(C_1-C_4)$-alkylsulfinyl, $(C_1-C_4)$-alkylsulfonyl, $(C_3-C_6)$ - cycloalkylsulfanyl, $(C_3-C_6)$-cycloalkylsulfinyl, $(C_3-C_6)$ - cycloalkylsulfonyl, -CH=N-O-[$(C_1-C_4)$-alkyl], -C[$(C_1-C_4)$-alkyl]=N-O-[$(C_1-C_4)$-alkyl, optionally singly or multiply substituted by identical or different substituents from the group consisting of halogen, cyano, nitro, $(C_1-C_4)$-alkyl, $(C_1-C_4)$-haloalkyl, $(C_1-C_4)$-alkoxy, $(C_1-C_4)$-haloalkoxy, $(C_3-C_6)$-cycloalkyl, $(C_3-C_6)$-halocycloalkyl, $R^{11}$ represents hydrogen, halogen, cyano or nitro or represents $(C_1-C_4)$-alkyl, $(C_1-C_4)$-alkoxy, $(C_1-C_4)$-alkylsulfanyl, $(C_1-C_4)$-alkylsulfinyl, $(C_1-C_4)$-alkylsulfonyl, $(C_3-C_5)$ -cycloalkyl, -CH=N-O-[$(C_1-C_4)$-alkyl], -C[$(C_1-C_4)$-alkyl]=N-O-[$(C_1-C_4)$-alkyl], optionally singly or multiply substituted by identical or different substituents from the group consisting of halogen, cyano, nitro, $(C_1-C_4)$-alkoxy and $(C_3-C_6)$-cycloalkyl.

**3.** Compounds of the formula (I) according to Claim 1 or 2 in which

at least one of the atoms $A_3$ or $A_4$ in the aromatic ring represents nitrogen,
$R^1$, $R^2$, $R^3$ and $R^4$ each independently represent a substituent selected from the group consisting of hydrogen, halogen, $(C_1-C_4)$-alkyl, $(C_1-C_4)$-haloalkyl, $(C_2-C_4)$-alkenyl, $(C_2-C_4)$-haloalkenyl, $(C_1-C_4)$-alkoxy, $(C_1-C_4)$-haloalkoxy, $(C_3-C_6)$-cycloalkyl, $(C_3-C_6)$-halocycloalkyl, $(C_1-C_4)$-alkylsulfanyl, $(C_1-C_4)$-alkylsulfinyl and $(C_1-C_4)$-alkylsulfonyl,

$R^6$ represents hydrogen, ($C_1$-$C_4$)-alkyl or a ring closure with $R^8$ formed by 1 to 3 $CH_2$ groups,
$R^7$ represents hydrogen, ($C_1$-$C_4$)-alkyl, ($C_3$-$C_4$)-cycloalkyl or a ring closure with $R^8$ formed by 1 to 3 $CH_2$ groups,
$R^8$ represents hydrogen or represents ($C_1$-$C_4$)-alkyl, ($C_2$-$C_4$)-alkenyl, ($C_2$-$C_4$)-alkynyl, ($C_3$-$C_4$)-cycloalkyl, optionally singly or multiply substituted by identical or different halogen substituents, or a ring closure together with $R^6$ or $R^7$ formed by 1 to 3 $CH_2$ groups, or a 4- to 6-membered heterocyclic ring closure together with $R^9$ which is formed by 3 to 5 $CH_2$ groups and is optionally singly or multiply substituted by identical or different substituents from the group consisting of halogen, ($C_1$-$C_4$)-alkyl and ($C_1$-$C_4$)-haloalkyl,
$R^9$ represents ($C_1$-$C_4$) -alkyl, ($C_3$-$C_6$) -cycloalkyl, ($C_1$-$C_6$)-alkoxy, ($C_1$-$C_6$)-alkylcarbonyl, each of which is optionally singly or multiply substituted by identical or different substituents from the group consisting of halogen, cyano, nitro or ($C_3$-$C_6$)-cycloalkyl, in the case that $R^7$ represents hydrogen or represents ($C_1$-$C_4$)-alkyl, ($C_3$-$C_4$)-cycloalkyl or in the case that $R^7$ together with $R^8$ forms a 5- or 6-membered ring, or
$R^9$ represents ($C_1$-$C_4$)-alkyl, ($C_3$-$C_4$)-cycloalkyl, ($C_1$-$C_4$)-alkoxy, each of which is optionally singly or multiply substituted by identical or different substituents from the group consisting of halogen, cyano, nitro or ($C_3$-$C_6$)-cycloalkyl, in the case that $R^7$ together with $R^8$ forms a 4-membered ring, or
$R^8$ and $R^9$ represent a 4- to 6-membered heterocyclic ring closure which is formed by 3 to 5 $CH_2$ groups and is optionally singly or multiply substituted by identical or different substituents from the group consisting of halogen, ($C_1$-$C_4$) -alkyl and ($C_1$-$C_4$) -haloalkyl,
$R^{10}$ represents halogen or represents phenyl, pyrid-2-yl, pyrid-3-yl, pyrid-4-yl, thiophen-2-yl, thiophen-3-yl, optionally singly or multiply substituted by identical or different substituents from the group consisting of halogen, cyano, nitro, ($C_1$-$C_4$)-alkyl, ($C_1$-$C_4$)-haloalkyl, ($C_1$-$C_4$)-alkoxy, ($C_1$-$C_4$)-haloalkoxy, ($C_3$-$C_6$)-cycloalkyl, ($C_3$-$C_6$)-halocycloalkyl, or represents ($C_1$-$C_4$)-alkyl, ($C_1$-$C_4$)-alkenyl, ($C_1$-$C_4$)-alkynyl, ($C_3$-$C_6$)-cycloalkyl, ($C_1$-$C_4$)-alkoxy, ($C_2$-$C_4$)-alkenyloxy, ($C_2$-$C_4$)-alkynyloxy, ($C_3$-$C_6$)-cycloalkoxy, ($C_1$-$C_4$)-alkylsulfanyl, ($C_1$-$C_4$)-alkylsulfinyl, ($C_1$-$C_4$)-alkylsulfonyl, ($C_3$-$C_6$)-cycloalkylsulfanyl, ($C_3$-$C_6$)-cycloalkylsulfinyl, ($C_3$-$C_6$)-cycloalkylsulfonyl, -CH=N-O-[($C_1$-$C_4$)-alkyl], -C[($C_1$-$C_4$)-alkyl]=N-O-[($C_1$-$C_4$)-alkyl, optionally singly or multiply substituted by identical or different substituents from the group consisting of halogen, cyano, nitro,
$R^{11}$ represents hydrogen, halogen, cyano or nitro or represents ($C_1$-$C_4$)-alkyl, ($C_1$-$C_4$)-alkoxy, ($C_1$-$C_4$)-alkylsulfanyl, ($C_1$-$C_4$)-alkylsulfinyl, ($C_1$-$C_4$)-alkylsulfonyl, ($C_3$-$C_5$)-cycloalkyl, optionally singly or multiply substituted by identical or different substituents from the group consisting of halogen, cyano and ($C_3$-$C_6$) -cycloalkyl.

**4.** Compounds of the formula (I) according to any of Claims 1 to 3 in which

Z represents naphthyl, optionally singly or multiply substituted by identical or different substituents from the group consisting of halogen, cyano, ($C_1$-$C_4$)-alkyl, ($C_1$-$C_4$)-haloalkyl, or represents unsubstituted dibenzo[b,d]furanyl or dibenzo[b,d]thiophenyl, or represents substituted phenyl of the substructure formula (II),
$R^1$, $R^2$, $R^3$ and $R^4$ represent a substituent selected from the group consisting of hydrogen, halogen, ($C_1$-$C_4$)-alkyl, ($C_1$-$C_4$)-haloalkyl, ($C_1$-$C_4$)-alkoxy and ($C_1$-$C_4$)-alkylsulfanyl,
$R^6$ represents hydrogen, ($C_1$-$C_3$)-alkyl or a ring closure with $R^8$ formed by 1 to 2 $CH_2$ groups,
$R^7$ represents hydrogen or a ring closure with $R^8$ formed by 1 to 3 $CH_2$ groups,
$R^8$ represents hydrogen or represents ($C_1$-$C_4$)-alkyl, optionally singly or multiply substituted by identical or different halogen substituents, or a ring closure together with $R^6$ formed by 1 to 2 $CH_2$ groups or a ring closure together with $R^7$ formed by 1 to 3 $CH_2$ groups or a 4- to 6-membered heterocyclic ring closure together with $R^9$ which is formed by 3 to 5 $CH_2$ groups and is optionally singly or multiply substituted by identical or different substituents from the group consisting of halogen, ($C_1$-$C_4$)-alkyl and ($C_1$-$C_4$)-haloalkyl,
$R^9$ represents ($C_1$-$C_6$)-alkyl, ($C_3$-$C_6$) -cycloalkyl or ($C_1$-$C_6$)-alkoxy, each of which is optionally singly or multiply substituted by identical or different substituents from the group consisting of halogen, cyano and ($C_3$-$C_6$) -cycloalkyl, or
$R^8$ and $R^9$ represent a 4- to 6-membered heterocyclic ring closure which is formed by 3 to 5 $CH_2$ groups and is optionally singly or multiply substituted by identical or different substituents from the group consisting of halogen, ($C_1$-$C_4$) -alkyl and ($C_1$-$C_4$) -haloalkyl,
$R^{10}$ represents halogen, ($C_1$-$C_4$) -alkyl, ($C_1$-$C_4$) -alkoxy, ($C_1$-$C_4$)-haloalkoxy or represents phenyl which is optionally singly or multiply substituted by identical or different substituents from the group consisting of halogen, cyano, ($C_1$-$C_4$) -alkyl, ($C_1$-$C_4$) -haloalkyl, ($C_1$-$C_4$) -alkoxy and ($C_1$-$C_4$)-haloalkoxy,
$R^{11}$ represents hydrogen, halogen, nitro or cyano or represents ($C_1$-$C_4$) -alkyl, ($C_3$-$C_5$)-cycloalkyl or ($C_1$-$C_4$)-alkoxy, optionally singly or multiply substituted by identical or different halogen substituents.

**5.** Compounds of the formula (I) according to any of Claims 1 to 4 in which

Z represents a naphthyl of the substructure formula (III):

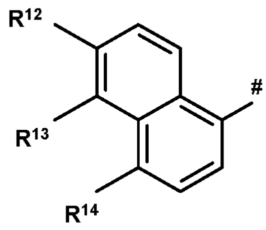

(III)

or represents unsubstituted dibenzo[b,d]furanyl or dibenzo[b,d]thiophenyl or represents substituted phenyl of the substructure formula (II),
$R^1$ represents hydrogen, halogen, $(C_1-C_4)$ -alkyl or $(C_1-C_4)$-alkoxy,
$R^2$ represents hydrogen, halogen or $(C_1-C_4)$-haloalkyl,
$R^3$ represents hydrogen, halogen, $(C_1-C_4)$ -alkoxy or $(C_1-C_4)$-alkylsulfanyl,
$R^4$ represents hydrogen or halogen,
$R^6$ represents hydrogen, or represents a piperazine ring closure with $R^8$,
$R^7$ represents hydrogen, or represents a pyrrolidine ring closure with $R^8$,
$R^8$ represents hydrogen, $(C_1-C_4)$-alkyl or represents a piperazine ring closure together with $R^6$ or represents a pyrrolidine ring closure together with $R^7$ or represents a piperidine ring closure together with $R^9$,
$R^9$ represents $(C_1-C_4)$ -alkyl or $(C_1-C_4)$ -alkoxy, each of which is optionally singly or multiply substituted by identical or different substituents from the group consisting of halogen and cyano, or
$R^8$ and $R^9$ represent a piperidine ring closure,
$R^{10}$ represents halogen, $(C_1-C_4)$ -alkyl, $(C_1-C_4)$ -alkoxy, $(C_1-C_4)$-haloalkoxy or represents a phenyl of the substructure formula (IV):

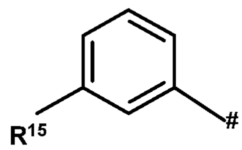

(IV)

$R^{11}$ represents hydrogen or halogen or represents $(C_1-C_4)$-alkyl or $(C_1-C_4)$-alkoxy, optionally singly or multiply substituted by identical or different halogen substituents,
$R^{12}$, $R^{13}$ and $R^{14}$ each independently represent hydrogen or halogen,
$R^{15}$ represents hydrogen, halogen, cyano, $(C_1-C_4)$-alkyl, $(C_1-C_4)$ -haloalkyl, $(C_1-C_4)$ -alkoxy or $(C_1-C_4)$ -haloalkoxy.

6. Formulation comprising compounds of the formula (I) according to any of Claims 1 to 5 and at least one auxiliary.

7. Formulation according to Claim 6, additionally comprising a further agrochemical active substance.

8. Compounds of the formula (Va) or (Vb-1):

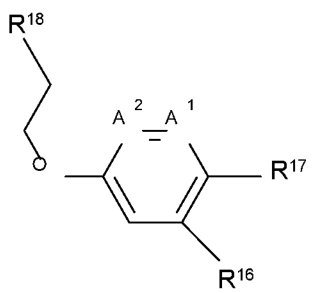

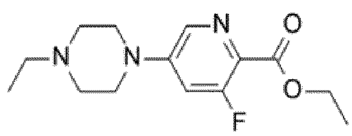

(Va) , (Vb-1)

in which
$A^1$ represents N or CH,
$A^2$ represents N or CH, where at least $A^1$ or $A^2$ represents N and preferably $A^1$ represents N and $A^2$ represents CH, $R^{16}$ represents hydrogen, methyl, fluorine or methoxy,
$R^{17}$ represents cyano, hydroxymethyl, methylcarboxy, ethylcarboxy or 3-bromophenoxymethyl,
$R^{18}$ in the compounds of the formula (Va) represents a radical:

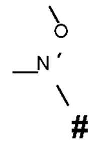 , 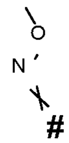 , 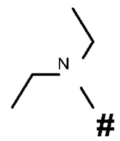

or bromine, with the proviso, that the following compounds are excluded:

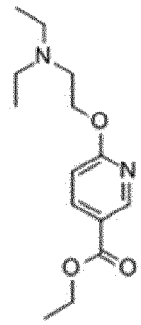 , 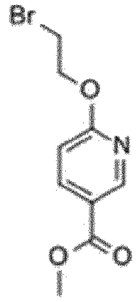 , 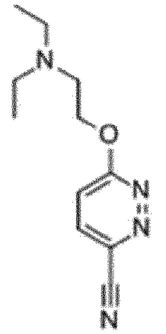 , 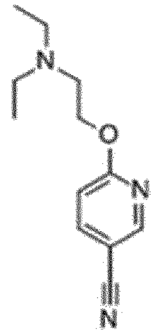 , 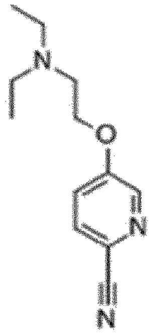 , 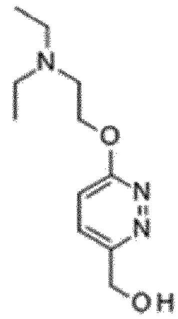 ,

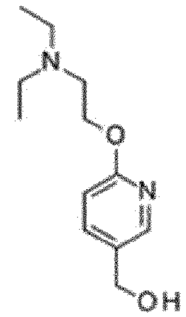 , 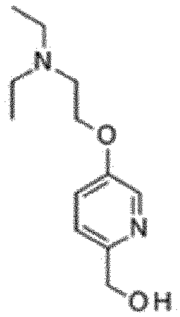 .

**9.** Method for controlling animal pests, **characterized in that** a compound of the formula (I) according to any of Claims 1 to 5 or a formulation according to Claim 6 or 7 is allowed to act on the animal pests and/or their habitat, excluded from this being the surgical, therapeutic and diagnostic treatment of the human or animal body.

**10.** Use of compounds of the formula (I) according to any of Claims 1 to 5 or of formulations according to Claim 6 or 7 for controlling animal pests, excluded from this being the surgical, therapeutic and diagnostic treatment of the human or animal body.

**11.** Use according to Claim 10, where the animal pests are mosquitoes.

**12.** Use of compounds of the formula (I) according to any of Claims 1 to 5 or of formulations according to Claim 6 or 7 for preparing medicaments for controlling ectoparasites on animals.

**13.** Method for controlling animal pests, **characterized in that** a compound of the formula (I) according to any of Claims 1 to 5 or a formulation according to Claim 6 or 7 is allowed to act on the animal pests and/or their habitat, excluded from this being the surgical, therapeutic and diagnostic treatment of the human or animal body.

## Revendications

**1.** Composés de formule (I)

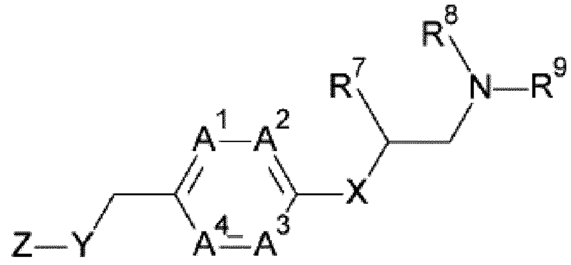

(I)

dans laquelle

Z représente un naphtyle, un dibenzo[b,d]furanyle, un dibenzo[b,d]thiophényle éventuellement une ou plusieurs fois substitué par des substituants identiques ou différents halogéno, cyano, alkyle en $C_1$-$C_4$, halogénalkyle en $C_1$-$C_4$, alcoxy en $C_1$-$C_4$, halogénalcoxy en $C_1$-$C_4$, alkylsulfanyle en $C_1$-$C_4$, halogénalkylsulfanyle en $C_1$-$C_4$, cycloalkyle en $C_3$-$C_6$, ou un phényle substitué de formule développée (II),

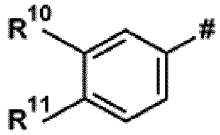

(II)

et le phényle substitué de formule développée (II) peut éventuellement porter jusqu'à deux substituants supplémentaires choisis dans le groupe des substituants halogéno, cyano, alkyle en $C_1$-$C_4$, halogénalkyle en $C_1$-$C_4$, alcoxy en $C_1$-$C_4$, halogénalcoxy en $C_1$-$C_4$, alkylsulfanyle en $C_1$-$C_4$, cycloalkyle en $C_3$-$C_6$ ou halogénocycloalkyle en $C_3$-$C_6$,

Y représente O,

$A^1$ représente N ou -$CR^1$,

$A^2$ représente N ou -$CR^2$,

$A^3$ représente N ou -$CR^3$,

$A^4$ représente N ou -$CR^4$,

au moins un et au maximum deux des atomes $A_1$, $A_2$, $A_3$ ou $A_4$ du noyau aromatique représentant N,

X représente un oxygène ou -$NR^6$,

$R^1$, $R^2$, $R^3$ et $R^4$ représentent chacun indépendamment des autres un hydrogène, un halogène, un nitro, un cyano, un aminocarbonyle, un aminosulfonyle, ou un radical alkyle en $C_1$-$C_4$, alcényle en $C_2$-$C_4$, alcynyle en $C_2$-$C_4$, cycloalkyle en $C_3$-$C_6$, alcoxy en $C_1$-$C_4$, alcényloxy en $C_2$-$C_4$, alcynyloxy en $C_2$-$C_4$, cycloalcoxy en $C_3$-$C_6$, *N*-mono-alkyle en $C_1$-$C_4$-amino, N-mono-cycloalkyle en $C_3$-$C_6$-amino, *N,N*-di-alkyle en $C_1$-$C_4$-amino, *N,N*-di-cycloalkyle en $C_3$-$C_6$-amino, *N,N*-cycloalkyle en $C_3$-$C_6$-alkyle en $C_1$-$C_4$-amino, N-alcanoyle en $C_1$-$C_4$-amino, N-cycloalcanoyle en $C_3$-$C_6$-amino, *N*-alcanoyle en $C_1$-$C_4$-*N*-alkyle en $C_1$-$C_4$-amino, N-cycloalcanoyle en $C_3$-$C_6$-*N*-alkyle en $C_1$-$C_4$-amino, N-cycloalcanoyle en $C_3$-$C_6$-*N*-cycloalkyle en $C_3$-$C_6$-amino, N-alcanoyle en $C_1$-$C_4$-*N*-cycloalkyle en $C_3$-$C_6$-amino, alcoxy en $C_1$-$C_4$-carbonyle, cycloalcoxy en $C_3$-$C_6$-carbonyle, alcanoyle en $C_1$-$C_4$, cycloalcanoyle en $C_3$-$C_6$, alkylsulfanyle en $C_1$-$C_4$, alkylsulfinyle en $C_1$-$C_4$, alkylsulfonyle en $C_1$-$C_4$, cycloalkylsulfanyle en $C_3$-$C_6$, cycloalkylsulfinyle en $C_3$-$C_6$, cycloalkylsulfonyle en $C_3$-$C_6$, N-alkyle en $C_1$-$C_4$-aminocarbonyle, N-cycloalkyle en $C_3$-$C_6$-aminocarbonyle, *N,N*-di-alkyle en $C_1$-$C_4$-aminocarbonyle, *N,N*-di-cycloalkyle en $C_3$-$C_6$-aminocarbonyle, *N,N*-cycloalkyle en $C_3$-$C_6$-alkyle en $C_1$-$C_4$-aminocarbonyle, -CH=N-O-[alkyle en $C_1$-$C_4$], -CH=N-O-[cycloalkyle en $C_3$-$C_6$], -C[alkyle en $C_1$-$C_4$]=N-O-[alkyle en $C_1$-$C_4$], -C[cycloalkyle en $C_3$-$C_6$]=N-O-[alkyle en $C_1$-$C_4$], -C[alkyle en $C_1$-$C_4$]=N-O-[cycloalkyle en $C_3$-$C_6$], - C[cycloalkyle en $C_3$-$C_6$]=N-O-[cycloalkyle en $C_3$-$C_6$], chacun éventuellement substitué,

$R^5$ représente un hydrogène ou un alkyle en $C_1$-$C_4$, un cycloalkyle en $C_3$-$C_6$, un alcanoyle en $C_1$-$C_3$ éventuellement substitué,

$R^6$ représente un hydrogène ou un alkyle en $C_1$-$C_4$, un cycloalkyle en $C_3$-$C_6$, un alcanoyle en $C_1$-$C_4$ éventuellement substitué ou est cyclisé en un cycle avec $R^8$, formé à partir de 1 à 3 groupes $CH_2$,

$R^7$ représente un hydrogène ou un alkyle en $C_1$-$C_4$, cycloalkyle en $C_3$-$C_6$ éventuellement substitué ou est cyclisé en un cycle avec $R^8$, formé à partir de 1 à 3 groupes $CH_2$,

$R^8$ représente un hydrogène ou un alkyle en $C_1$-$C_4$, un alcényle en $C_2$-$C_4$, un alcynyle en $C_2$-$C_4$, un cycloalkyle en $C_3$-$C_6$ éventuellement substitué ou, ensemble avec $R^6$ ou $R^7$, est cyclisé en un cycle formé à partir de 1 à 3 groupes $CH_2$ ou, ensemble avec $R^9$, est cyclisé en un cycle hétérocyclique à 4 à 6 chaînons, qui dans le cas d'un cycle hétérocyclique à 5 à 6 chaînons peut contenir des hétéroatomes supplémentaires et est éventuellement une ou plusieurs fois substitué par des substituants identiques ou différents halogéno, cyano, ou par un alkyle en $C_1$-$C_4$, cycloalkyle en $C_3$-$C_6$, alcoxy en $C_1$-$C_4$, chacun éventuellement substitué une ou plusieurs fois par des substituants identiques ou différents halogéno,

$R^9$ représente un radical alkyle en $C_1$-$C_4$, cycloalkyle en $C_3$-$C_6$, alcényle en $C_2$-$C_4$, alcynyle en $C_2$-$C_4$, *N*-mono-alkyle en $C_1$-$C_4$-amino, *N*,*N*-di-alkyle en $C_1$-$C_4$-amino, alcoxy en $C_1$-$C_4$, alcényloxy en $C_2$-$C_4$, alcynyloxy en $C_2$-$C_4$, cycloalcoxy en $C_3$-$C_6$, alkyle en $C_1$-$C_4$-carbonyle, alkylsulfinyle en $C_1$-$C_4$, alkylsulfonyle en $C_1$-$C_4$, alcoxy en $C_1$-$C_4$-carbonyle, chacun étant éventuellement substitué, dans le cas dans lequel $R^7$ représente un hydrogène ou un radical alkyle en $C_1$-$C_4$, cycloalkyle en $C_3$-$C_6$ éventuellement substitué, ou dans le cas dans lequel $R^7$ forme avec $R^8$ un cycle à 5 ou 6 chaînons, ou $R^9$ représente un radical alkyle en $C_1$-$C_4$, cycloalkyle en $C_3$-$C_6$, alcényle en $C_2$-$C_4$, alcynyle en $C_2$-$C_4$, *N*-mono-alkyle en $C_1$-$C_4$-amino, *N*,*N*-di-alkyle en $C_1$-$C_4$-amino, alcoxy en $C_1$-$C_4$, alcényloxy en $C_2$-$C_4$, alcynyloxy en $C_2$-$C_4$, cycloalcoxy en $C_3$-$C_4$, alkylsulfinyle en $C_1$-$C_4$, alkylsulfonyle en $C_1$-$C_4$, dans le cas dans lequel $R^7$ forme avec $R^8$ un cycle à 4 chaînons, ou

$R^8$ et $R^9$ sont cyclisés en un cycle hétérocyclique à 4 à 6 chaînons, qui dans le cas d'un cycle hétérocyclique à 5 à 6 chaînons peut contenir des hétéroatomes supplémentaires et est éventuellement substitué une ou plusieurs fois par des substituants identiques ou différents halogéno, cyano, ou par un un radical alkyle en $C_1$-$C_4$, cycloalkyle en $C_3$-$C_6$, alcoxy en $C_1$-$C_4$, chacun éventuellement une ou plusieurs fois substitué par des substituants identiques ou différents halogéno,

$R^{10}$ représente un halogène, un nitro, un cyano, -$SF_5$ ou un radical phényle, pyrid-2-yle, pyrid-3-yle, pyrid-4-yle, thiophén-2-yle, thiophén-3-yle, alkyle en $C_1$-$C_4$, alcényle en $C_1$-$C_4$, alcynyle en $C_1$-$C_4$, cycloalkyle en $C_3$-$C_6$, alcoxy en $C_1$-$C_4$, alcényloxy en $C_2$-$C_4$, alcynyloxy en $C_2$-$C_4$, cycloalcoxy en $C_3$-$C_6$, alkylsulfanyle en $C_1$-$C_4$, alkylsulfinyle en $C_1$-$C_4$, alkylsulfonyle en $C_1$-$C_4$, cycloalkylsulfanyle en $C_3$-$C_6$, cycloalkylsulfinyle en $C_3$-$C_6$, cycloalkylsulfonyle en $C_3$-$C_6$, -CH=N-O-[alkyle en $C_1$-$C_4$], -C[alkyle en $C_1$-$C_4$]=N-O-[alkyle en $C_1$-$C_4$], éventuellement une ou plusieurs fois substitué par des substituants identiques ou différents halogéno, cyano, nitro, -$SF_5$, alkyle en $C_1$-$C_4$, alcényle en $C_2$-$C_4$, alcynyle en $C_2$-$C_4$, halogénalkyle en $C_1$-$C_4$, halogénalcényle en $C_2$-$C_4$, alcoxy en $C_1$-$C_4$, halogénalcoxy en $C_1$-$C_4$, alcényloxy en $C_2$-$C_4$, halogénalcényloxy en $C_2$-$C_4$, alcynyloxy en $C_2$-$C_4$, cycloalcoxy en $C_3$-$C_6$, halogénocycloalcoxy en $C_3$-$C_6$, cycloalkyle en $C_3$-$C_6$, halogénocycloalkyle en $C_3$-$C_6$, alkylsulfanyle en $C_1$-$C_4$, alkylsulfinyle en $C_1$-$C_4$, alkylsulfonyle en $C_1$-$C_4$, halogénalkylsulfonyle en $C_1$-$C_4$, halogénalkylsulfanyle en $C_1$-$C_4$, halogénalkylsulfinyle en $C_1$-$C_4$, cycloalkylsulfanyle en $C_3$-$C_6$, cycloalkylsulfinyle en $C_3$-$C_6$, cycloalkylsulfonyle en $C_3$-$C_6$, aryle en $C_6$, en $C_{10}$, en C14, aryloxy en $C_6$, en $C_{10}$, en C14, benzyle, benzyloxy, benzylthio, arylthio en $C_6$, en $C_{10}$, en $C_{14}$, arylamino en $C_6$, en $C_{10}$, en $C_{14}$, benzylamino, hétérocyclyle, trialkylsilyle,

$R^{11}$ représente un hydrogène, un halogène, un cyano ou un nitro, ou un radical alkyle en $C_1$-$C_4$, cycloalkyle en $C_3$-$C_5$, alcoxy en $C_1$-$C_4$, alkylsulfanyle en $C_1$-$C_4$, alkylsulfinyle en $C_1$-$C_4$, alkylsulfonyle en $C_1$-$C_4$, -CH=N-O-[alkyle en $C_1$-$C_4$], -C[alkyle en $C_1$-$C_4$]=N-O-[alkyle en $C_1$-$C_4$],

ainsi que les sels, complexes métalliques, N-oxydes et formes tautomères des composés de formule (I),

à la condition d'exclure le composé 1-[4-(6-{2-[3-(chlorométhyl)phényl]éthyl}pyridin-3-yl)pipérazin-1-yl]éthanone.

**2.** Composés de formule (I) selon la revendication 1, dans laquelle

$R^1$, $R^2$, $R^3$ et $R^4$ représentent chacun indépendamment des autres un substituant choisi dans le groupe consistant en l'hydrogène, un halogène, un nitro, un cyano, ou un radical alkyle en $C_1$-$C_4$, alcényle en $C_2$-$C_4$, alcynyle en $C_2$-$C_4$, cycloalkyle en $C_3$-$C_6$, alcoxy en $C_1$-$C_4$, alcényloxy en $C_2$-$C_4$, alcynyloxy en $C_2$-$C_4$, cycloalcoxy en $C_3$-$C_6$, alcoxy en $C_1$-$C_4$-carbonyle, cycloalcoxy en $C_3$-$C_6$-carbonyle, N-mono-alkyle en $C_1$-$C_4$-amino, *N*,*N*-di-alkyle en $C_1$-$C_4$-amino, alkylsulfanyle en $C_1$-$C_4$, alkylsulfinyle en $C_1$-$C_4$, alkylsulfonyle en $C_1$-$C_4$, cycloalkylsulfanyle en $C_3$-$C_6$, cycloalkylsulfinyle en $C_3$-$C_6$, cycloalkylsulfonyle en $C_3$-$C_6$, chacun étant éventuellement une ou plusieurs fois substitué par des substituants identiques ou différents halogéno, cyano, nitro, alkyle en $C_1$-$C_4$, halogénalkyle en $C_1$-$C_4$, alcoxy en $C_1$-$C_4$, halogénalcoxy en $C_1$-$C_4$, cycloalkyle en $C_3$-$C_6$, halogénocycloalkyle en $C_3$-$C_6$,

$R^5$ représente un hydrogène ou un radical alkyle en $C_1$-$C_4$, cycloalkyle en $C_3$-$C_4$, alcanoyle en $C_1$-$C_3$ éventuellement une ou plusieurs fois substitué par des substituants identiques ou différents halogéno,

$R^6$ représente un hydrogène ou un radical alkyle en $C_1$-$C_4$, cycloalkyle en $C_3$-$C_4$, alcanoyle en $C_1$-$C_4$ éventuellement une fois ou plusieurs fois substitué par des substituants identiques ou différents halogéno, ou est cyclisé en un cycle avec $R^8$, formé à partir de 1 à 3 groupes $CH_2$,
$R^7$ représente un hydrogène ou un radical alkyle en $C_1$-$C_4$, cycloalkyle en $C_3$-$C_4$, éventuellement une ou plusieurs fois substitué par des substituants identiques ou différents halogéno, ou est cyclisé en un cycle avec $R^8$, qui est formé à partir de 1 à 3 groupes $CH_2$,
$R^8$ représente un hydrogène ou un radical alkyle en $C_1$-$C_4$, alcényle en $C_2$-$C_4$, alcynyle en $C_2$-$C_4$, cycloalkyle en $C_3$-$C_4$, éventuellement une ou plusieurs fois substitué par des substituants identiques ou différents halogéno, nitro ou cyano, ou est cyclisé en un cycle ensemble avec $R^6$ ou $R^7$, qui est formé à partir de 1 à 3 groupes $CH_2$, ou un cycle hétérocyclique à 4 à 6 chaînons ensemble avec $R^9$, qui dans le cas d'un cycle hétérocyclique à 5 à 6 chaînons peut contenir des hétéroatomes supplémentaires choisis dans le groupe consistant en N, O et éventuellement est substitué une ou plusieurs fois par des substituants identiques ou différents halogéno, alkyle en $C_1$-$C_4$ ou halogénalkyle en $C_1$-$C_4$ ,
$R^9$ représente un radical alkyle en $C_1$-$C_4$, alcényle en $C_2$-$C_4$, cycloalkyle en $C_3$-$C_4$, alcoxy en $C_1$-$C_4$, alcényloxy en $C_2$-$C_4$, cycloalcoxy en $C_3$-$C_6$, alkyle en $C_1$-$C_4$-carbonyle, alkylsulfinyle en $C_1$-$C_4$, alkylsulfonyle en $C_1$-$C_4$, alcoxy en $C_1$-$C_4$-carbonyle, chacun éventuellement substitué une ou plusieurs fois par des substituants identiques ou différents halogéno, cyano, nitro ou cycloalkyle en $C_3$-$C_6$, dans le cas dans lequel $R^7$ représente un hydrogène ou un radical alkyle en $C_1$-$C_4$, cycloalkyle en $C_3$-$C_4$ éventuellement une ou plusieurs fois substitué par des substituants identiques ou différents halogéno, ou dans le cas dans lequel, $R^7$, avec $R^8$, est cyclisé en un cycle à 5 ou 6 chaînons, ou
$R^9$ représente un radical alkyle en $C_1$-$C_4$, alcényle en $C_2$-$C_4$, cycloalkyle en $C_3$-$C_4$, alcoxy en $C_1$-$C_4$, alcényloxy en $C_2$-$C_4$, cycloalcoxy en $C_3$-$C_4$, alkylsulfinyle en $C_1$-$C_4$, alkylsulfonyle en $C_1$-$C_4$, chacun étant éventuellement substitué une ou plusieurs fois par des substituants identiques ou différents halogéno, cyano, nitro ou cycloalkyle en $C_3$-$C_6$, dans le cas dans lequel $R^7$ est cyclisé en un cycle à 4 chaînons ensemble avec $R^8$, ou $R^8$ et $R^9$ forment un cycle hétérocyclique à 4 à 6 chaînons, qui dans le cas d'un cycle hétérocyclique à 5 à 6 chaînons peut contenir des hétéroatomes supplémentaires choisis dans le groupe consistant en N, O, ou est éventuellement une ou plusieurs fois substitué par des substituants identiques ou différents halogéno, alkyle en $C_1$-$C_4$ ou halogénalkyle en $C_1$-$C_4$,
$R^{10}$ représente un halogène ou un radical phényle, pyrid-2-yle, pyrid-3-yle, pyrid-4-yle, thiophén-2-yle, thiophén-3-yle, alkyle en $C_1$-$C_4$, alcényle en $C_1$-$C_4$, alcynyle en $C_1$-$C_4$, cycloalkyle en $C_3$-$C_6$, alcoxy en $C_1$-$C_4$, alcényloxy en $C_2$-$C_4$, alcynyloxy en $C_2$-$C_4$, cycloalcoxy en $C_3$-$C_6$, alkylsulfanyle en $C_1$-$C_4$, alkylsulfinyle en $C_1$-$C_4$, alkylsulfonyle en $C_1$-$C_4$, cycloalkylsulfanyle en $C_3$-$C_6$, cycloalkylsulfinyle en $C_3$-$C_6$, cycloalkylsulfonyle en $C_3$-$C_6$, -CH=N-O- [alkyle en $C_1$-$C_4$], -C [alkyle en $C_1$-$C_4$] =N-O-[alkyle en $C_1$-$C_4$],
$R^{11}$ représente un hydrogène, un halogène, un cyano ou un nitro, ou un radical alkyle en $C_1$-$C_4$, alcoxy en $C_1$-$C_4$, alkylsulfanyle en $C_1$-$C_4$, alkylsulfinyle en $C_1$-$C_4$, alkylsulfonyle en $C_1$-$C_4$, cycloalkyle en $C_3$-$C_5$, -CH=N-O-[alkyle en $C_1$-$C_4$], -C [alkyle en $C_1$-$C_4$] =N-O- [alkyle en $C_1$-$C_4$], éventuellement une ou plusieurs fois substitué par des substituants identiques ou différents halogéno, cyano, nitro, alcoxy en $C_1$-$C_4$ ou cycloalkyle en $C_3$-$C_6$.

**3.** Composés de formule (I) selon l'une des revendications 1 à 2, dans laquelle

au moins l'un des atomes $A_3$ ou $A_4$ du cycle aromatique représente un azote,
$R^1$, $R^2$, $R^3$ et $R^4$ représentent chacun indépendamment des autres un substituant choisi dans le groupe consistant en l'hydrogène, un halogène, un radical alkyle en $C_1$-$C_4$, halogénalkyle en $C_1$-$C_4$, alcényle en $C_2$-$C_4$, halogénalcényle en $C_2$-$C_4$, alcoxy en $C_1$-$C_4$, halogénalcoxy en $C_1$-$C_4$, cycloalkyle en $C_3$-$C_6$, halogénocycloalkyle en $C_3$-$C_6$, alkylsulfanyle en $C_1$-$C_4$, alkylsulfinyle en $C_1$-$C_4$ ou alkylsulfonyle en $C_1$-$C_4$,
$R^6$ représente un hydrogène, un radical alkyle en $C_1$-$C_4$, ou est cyclisé en un cycle avec $R^8$, qui est formé à partir de 1 à 3 groupes $CH_2$,
$R^7$ représente un hydrogène, un radical alkyle en $C_1$-$C_4$, cycloalkyle en $C_3$-$C_4$, ou est cyclisé en un cycle avec $R^8$, qui est formé à partir de 1 à 3 groupes $CH_2$,
$R^8$ représente un hydrogène ou un radical alkyle en $C_1$-$C_4$, alcényle en $C_2$-$C_4$, alcynyle en $C_2$-$C_4$, cycloalkyle en $C_3$-$C_4$ éventuellement une ou plusieurs fois substitué par des substituants identiques ou différents halogéno, ou est cyclisé en un cycle avec $R^6$ ou $R^7$, qui est formé à partir de 1 à 3 groupes $CH_2$, ou est cyclisé en un cycle hétérocyclique à 4 à 6 chaînons avec $R^9$, qui est formé à partir de 3 à 5 groupes $CH_2$ et qui est éventuellement substitué une ou plusieurs fois par des substituants identiques ou différents halogéno, alkyle en $C_1$-$C_4$ ou halogénalkyle en $C_1$-$C_4$,
$R^9$ représente un radical alkyle en $C_1$-$C_4$, cycloalkyle en $C_3$-$C_6$, alcoxy en $C_1$-$C_6$, alkyle en $C_1$-$C_6$-carbonyle, chacun étant éventuellement une ou plusieurs fois substitué par des substituants identiques ou différents halogéno, cyano, nitro ou cycloalkyle en $C_3$-$C_6$, dans le cas dans lequel $R^7$ représente un hydrogène ou un radical

alkyle en $C_1$-$C_4$, cycloalkyle en $C_3$-$C_4$ ou dans le cas dans lequel $R^7$ forme avec $R^8$ un cycle à 5 ou 6 chaînons, ou $R^9$ représente un radical alkyle en $C_1$-$C_4$, cycloalkyle en $C_3$-$C_4$, alcoxy en $C_1$-$C_4$ chacun étant éventuellement une ou plusieurs fois substitué par des substituants identiques ou différents halogéno, cyano, nitro ou cycloalkyle en $C_3$-$C_6$, dans le cas dans lequel $R^7$ forme avec $R^8$ un cycle à 4 chaînons, ou $R^8$ et $R^9$ forment un cycle hétérocyclique à 4 à 6 chaînons, qui est formé à partir de 3 à 5 groupes $CH_2$, et qui est éventuellement une ou plusieurs fois substitué par des substituants identiques ou différents halogéno, alkyle en $C_1$-$C_4$ ou halogénalkyle en $C_1$-$C_4$,

$R^{10}$ représente un halogène ou un radical phényle, pyrid-2-yle, pyrid-3-yle, pyrid-4-yle, thiophén-2-yle, thiophén-3-yle éventuellement une ou plusieurs fois substitué par des substituants identiques ou différents halogéno, cyano, nitro, alkyle en $C_1$-$C_4$, halogénalkyle en $C_1$-$C_4$, alcoxy, halogénalcoxy en $C_1$-$C_4$, cycloalkyle en $C_3$-$C_6$, halogénocycloalkyle en $C_3$-$C_6$, ou un radical alkyle en $C_1$-$C_4$, alcényle en $C_1$-$C_4$, alcynyle en $C_1$-$C_4$, cycloalkyle en $C_3$-$C_6$, alcoxy en $C_1$-$C_4$, alcényloxy en $C_2$-$C_4$, alcynyloxy en $C_2$-$C_4$, cycloalcoxy en $C_3$-$C_6$, alkylsulfanyle en $C_1$-$C_4$, alkylsulfinyle en $C_1$-$C_4$, alkylsulfonyle en $C_1$-$C_4$, cycloalkylsulfanyle en $C_3$-$C_6$, cycloalkylsulfinyle en $C_3$-$C_6$, cycloalkylsulfonyle en $C_3$-$C_6$, -CH=N-O- [alkyle en $C_1$-$C_4$], -C [alkyle en $C_1$-$C_4$] =N-O-[alkyle en $C_1$-$C_4$], éventuellement une ou plusieurs fois substitué par des substituants identiques ou différents halogéno, cyano, nitro,

$R^{11}$ représente un hydrogène, un halogène, un cyano ou un nitro, ou un radical alkyle en $C_1$-$C_4$, alcoxy en $C_1$-$C_4$, alkylsulfanyle en $C_1$-$C_4$, alkylsulfinyle en $C_1$-$C_4$, alkylsulfonyle en $C_1$-$C_4$, cycloalkyle en $C_3$-$C_5$ éventuellement une ou plusieurs fois substitué par des substituants identiques ou différents halogéno, cyano ou cycloalkyle en $C_3$-$C_6$.

**4.** Composés de formule (I) selon l'une des revendications 1 à 3, dans laquelle

Z représente un naphtyle éventuellement une ou plusieurs fois substitué par des substituants identiques ou différents halogéno, cyano, alkyle en $C_1$-$C_4$, halogénalkyle en $C_1$-$C_4$, ou un dibenzo [b, d] furanyle ou un dibenzo[b,d]thiophényle non substitué ou un phényle substitué de formule développée (II), $R^1$, $R^2$, $R^3$ et $R^4$ représentent un substituant choisi dans le groupe consistant en l'hydrogène, un halogène, les radicaux alkyle en $C_1$-$C_4$, halogénalkyle en $C_1$-$C_4$, alcoxy en $C_1$-$C_4$ ou alkylsulfanyle en $C_1$-$C_4$,

$R^6$ représente un hydrogène, un radical alkyle en $C_1$-$C_3$ ou est cyclisé en un cycle avec $R^8$, qui est formé à partir de 1 à 2 groupes $CH_2$,

$R^7$ représente un hydrogène ou est cyclisé en un cycle avec $R^8$, qui est formé à partir de 1 à 3 groupes $CH_2$,

$R^8$ représente un hydrogène ou un radical alkyle en $C_1$-$C_4$ éventuellement une ou plusieurs fois substitué par des substituants identiques ou différents halogéno ou est cyclisé en un cycle avec $R^6$, qui est formé à partir de 1 à 2 groupes $CH_2$, ou est cyclisé en un cycle avec $R^7$, qui est formé à partir de 1 à 3 groupes $CH_2$, ou est cyclisé en un cycle hétérocyclique à 4 à 6 chaînons avec $R^9$, qui est formé à partir de 3 à 5 groupes $CH_2$ et qui est éventuellement une ou plusieurs fois substitué par des substituants identiques ou différents halogéno, alkyle en $C_1$-$C_4$ ou halogénalkyle en $C_1$-$C_4$,

$R^9$ représente un radical alkyle en $C_1$-$C_6$, cycloalkyle en $C_3$-$C_6$ ou alcoxy en $C_1$-$C_6$ éventuellement une ou plusieurs fois substitué par des substituants identiques ou différents halogéno, cyano ou cycloalkyle en $C_3$-$C_6$, ou $R^8$ et $R^9$ forment un cycle hétérocyclique à 4 à 6 chaînons, qui est formé à partir de 3 à 5 groupes $CH_2$ et qui est éventuellement une ou plusieurs fois substitué par des substituants identiques ou différents halogéno, alkyle en $C_1$-$C_4$ ou halogénalkyle en $C_1$-$C_4$,

$R^{10}$ représente un halogène, un radical alkyle en $C_1$-$C_4$, alcoxy en $C_1$-$C_4$, halogénalcoxy en $C_1$-$C_4$ ou un phényle éventuellement une ou plusieurs fois substitué par des substituants identiques ou différents halogéno, cyano, alkyle en $C_1$-$C_4$, halogénalkyle en $C_1$-$C_4$, alcoxy en $C_1$-$C_4$ ou halogénalcoxy en $C_1$-$C_4$,

$R^{11}$ représente un hydrogène, un halogène, un nitro ou un cyano ou représente un radical alkyle en $C_1$-$C_4$, cycloalkyle en $C_3$-$C_5$ ou alcoxy en $C_1$-$C_4$ éventuellement une ou plusieurs fois substitué par des substituants identiques ou différents halogéno.

**5.** Composés de formule (I) selon l'une des revendications 1 à 4, dans laquelle

Z représente un naphtyle de formule développée (III) :

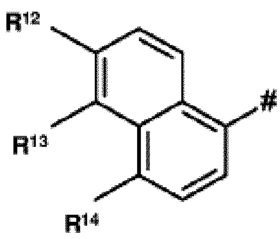

(III)

ou un dibenzo[b,d]furanyle ou dibenzo[b,d]thiophényle non substitué ou un phényle substitué de formule développée (II),
$R^1$ représente un hydrogène, un halogène, un radical alkyle en $C_1$-$C_4$ ou alcoxy en $C_1$-$C_4$,
$R^2$ représente un hydrogène, un halogène ou un radical halogénalkyle en $C_1$-$C_4$,
$R^3$ représente un hydrogène, un halogène, un radical alcoxy en $C_1$-$C_4$ ou alkylsulfanyle en $C_1$-$C_4$,
$R^4$ représente un hydrogène ou un halogène,
$R^6$ représente un hydrogène, ou une pipérazine formant un cycle avec $R^8$,
$R^7$ représente un hydrogène ou une pyrrolidine formant un cycle avec $R^8$,
$R^8$ représente un hydrogène, un radical alkyle en $C_1$-$C_4$ ou une pipérazine formant un cycle avec $R^6$ ou une pyrrolidine formant un cycle avec $R^7$ ou une pipéridine formant un cycle avec $R^9$,
$R^9$ représente un radical alkyle en $C_1$-$C_4$ ou alcoxy en $C_1$-$C_4$, chacun étant éventuellement une ou plusieurs fois substitué par des substituants identiques ou différents halogéno ou cyano, ou
$R^8$ et $R^9$ représentent une pipéridine formant un cycle, $R^{10}$ représente un halogène, un radical alkyle en $C_1$-$C_4$, alcoxy en $C_1$-$C_4$, halogénalcoxy en $C_1$-$C_4$, ou un phényle de formule développée (IV) :

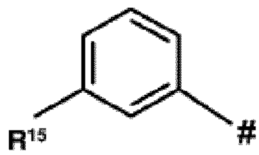

(IV)

$R^{11}$ représente un hydrogène ou un halogène, ou un radical alkyle en $C_1$-$C_4$ ou alcoxy en $C_1$-$C_4$ éventuellement une ou plusieurs fois substitué par des substituants identiques ou différents halogéno,
$R^{12,}$ $R^{13}$ et $R^{14}$ représentent chacun indépendamment des autres un hydrogène ou un halogène,
$R^{15}$ représente un hydrogène, un halogène, un cyano, un radical alkyle en $C_1$-$C_4$, halogénalkyle en $C_1$-$C_4$, alcoxy en $C_1$-$C_4$ ou halogénalcoxy en $C_1$-$C_4$.

**6.** Formulation contenant des composés de formule (I) selon l'une des revendications 1 à 5 ainsi qu'au moins un adjuvant.

**7.** Formulation selon la revendication 6, contenant en outre une matière active agrochimique supplémentaire.

**8.** Composés de formule (Va) ou (Vb-1) :

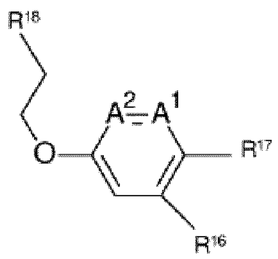

(Va) ,

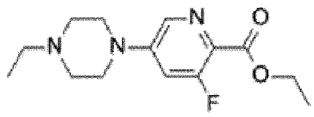

(Vb-1)

dans lesquelles

$A^1$ représente N ou CH,

$A^2$ représente N ou CH, au moins $A^1$ ou $A^2$ représentant N et de préférence $A^1$ représentant N et $A^2$ représentant CH,

$R^{16}$ représente un hydrogène, un méthyle, un fluoro ou un méthoxy,

$R^{17}$ représente un cyano, un hydroxyméthyle, un méthylcarboxy, un éthylcarboxy ou un 3-bromophénoxyméthyle,

$R^{18}$, dans les composés de formule (Va) représente un radical :

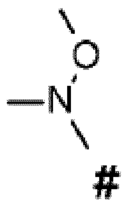 , 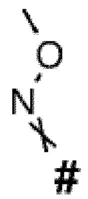 , 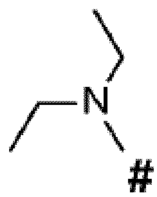

ou un bromo, à la condition de ne pas englober les composés suivants :

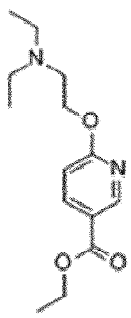 , 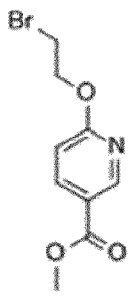 , 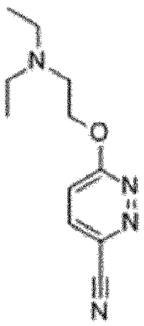 , 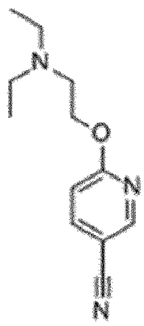 , 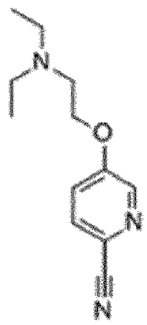 , 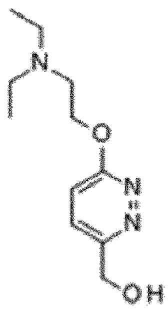 ,

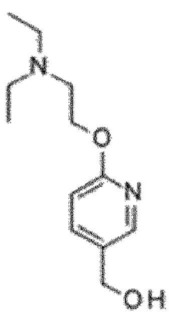 , 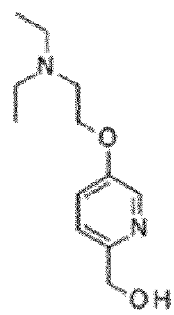 .

**9.** Procédé de lutte contre des ravageurs animaux, **caractérisé en ce qu'**on fait agir sur les ravageurs animaux et/ou leur habitat un composé de formule (I) selon l'une des revendications 1 à 5 ou une formulation selon l'une des

revendications 6 et 7, à l'exclusion du traitement chirurgical, thérapeutique et diagnostique du corps humain ou animal.

**10.** Utilisation de composés de formule (I) selon l'une des revendications 1 à 5 ou de formulations selon l'une des revendications 6 et 7 pour lutter contre des ravageurs animaux, à l'exclusion du traitement chirurgical, thérapeutique et diagnostique du corps humain ou animal.

**11.** Utilisation selon la revendication 10, les ravageurs animaux étant des moustiques.

**12.** Utilisation de composés de formule (I) selon l'une des revendications 1 à 5 ou de formulations selon l'une des revendications 6 et 7 pour fabriquer des médicaments destinés à lutter contre les ectoparasites des animaux.

**13.** Procédé de lutte contre des ravageurs animaux, **caractérisé en ce qu'**on laisse agir sur les ravageurs animaux et/ou leur habitat un composé de formule (I) selon l'une des revendications 1 à 5 ou une formulation selon la revendication 6 ou 7, à l'exclusion du traitement chirurgical, thérapeutique et diagnostique du corps humain ou animal.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2014179144 A
- WO 2005082089 A
- WO 2008073929 A
- WO 2009145360 A
- WO 2008073936 A
- WO 2008073461 A
- WO 2003064386 A
- WO 2006043635 A
- WO 2003106457 A
- WO 2006003494 A
- WO 2010052161 A
- EP 2647626 A
- WO 2004099160 A
- JP 2010018586 A
- WO 2012029672 A
- WO 2013144213 A
- WO 2010051926 A
- CN 103232431
- WO 2013050317 A1
- WO 2013162715 A2
- WO 2013162716 A2
- US 20140213448 A1
- CN 101337937 A
- CN 103109816 A
- WO 2012034403 A1
- WO 2011085575 A1
- CN 101337940 A
- CN 101715774 A
- CN 103524422 A
- CN 102391261 A
- US 20140275503 A1
- WO 2007040280 A1
- WO 2007040282 A1
- WO 2015058021 A1
- WO 2015058028 A1
- CN 103265527 A
- WO 2010132999 A
- EP 2050734 A
- US 20060052599 A
- WO 2013161312 A
- WO 2002017712 A
- WO 2015089139 A
- WO 2013019621 A
- WO 2005049572 A
- WO 2012082566 A
- WO 2016044789 A
- WO 2011044181 A
- WO 199848800 A
- EP 0678504 A
- WO 2006078619 A
- WO 2010071885 A
- WO 2006105304 A
- EP 0366085 A
- WO 2014028669 A
- US 20030092739 A
- EP 2236507 A
- EP 0135894 A
- WO 2012038851 A
- WO 2010054024 A
- WO 2014201206 A
- WO 2016059097 A
- WO 2010043377 A
- WO 2003084917 A
- WO 2016040185 A
- US 2015238641 A
- WO 2014207240 A
- WO 2014116684 A
- US 2015259317 F
- WO 2004041264 A
- WO 2004089925 A
- WO 2014043272 A, D. Bensen


### In der Beschreibung aufgeführte Nicht-Patentliteratur


- **BAUR et al.** *Pesticide Science,* 1997, vol. 51, 131-152
- The Pesticide Manua. British Crop Protection Council, 2012
- *CHEMICAL ABSTRACTS,* 885026-50-6
- *CHEMICAL ABSTRACTS,* 637360-23-7
- *CHEMICAL ABSTRACTS,* 872999-66-1
- *CHEMICAL ABSTRACTS,* 1225292-17-0
- *CHEMICAL ABSTRACTS,* 1440516-42-6
- *CHEMICAL ABSTRACTS,* 792914-58-0
- *CHEMICAL ABSTRACTS,* 1204776-60-2
- *CHEMICAL ABSTRACTS,* 1363400-41-2
- *CHEMICAL ABSTRACTS,* 1461743-15-6
- *CHEMICAL ABSTRACTS,* 1226889-14-0
- *CHEMICAL ABSTRACTS,* 1449220-44-3
- *CHEMICAL ABSTRACTS,* 1332628-83-7
- *CHEMICAL ABSTRACTS,* 1477923-37-7
- *CHEMICAL ABSTRACTS,* 1105672-77-2

- *CHEMICAL ABSTRACTS,* 1232543-85-9
- *CHEMICAL ABSTRACTS,* 1268277-22-0
- *CHEMICAL ABSTRACTS,* 1233882-22-8
- *CHEMICAL ABSTRACTS,* 1108184-52-6
- *CHEMICAL ABSTRACTS,* 1542271-46-4
- *CHEMICAL ABSTRACTS,* 1370358-69-2
- *CHEMICAL ABSTRACTS,* 1181213-14-8
- *CHEMICAL ABSTRACTS,* 934001-66-8
- *CHEMICAL ABSTRACTS,* 1477919-27-9
- *CHEMICAL ABSTRACTS,* 1452877-50-7
- **R. WEGLER.** Chemie der Pflanzenschutz- und Schädlingsbekämpfungsmittel. Springer Verlag, 1970, vol. 2, 401-412
- **HOUBEN-WEYL.** Methoden der Organischen Chemie. Georg Thieme Verlag, vol. 3, 760
- **HOUBEN-WEYL.** Methoden der Organischen Chemie. Georg Thieme Verlag, vol. 4, 361
- *Journal of Organic Chemistry,* 1957, vol. 22, 579
- **T. W. GREENE ; P. G. M. WUTS.** Protective Groups in Organic Synthesis. Wiley-Interscience, 1999
- **HUNT et al.** *Med Vet Entomol,* September 2005, vol. 19 (3), 271-5